# EUROPEAN PATENT APPLICATION

(11) **EP 4 834 881 A1**
(43) Date of publication of application: **04.11.2026**
(21) Application number: 24911451.3
(22) Date of filing: 27.12.2024
(51) Int. Cl.: C07D 215/56, C07D 401/04, C07D 471/04, C07D 519/00, A61K 31/4375, A61K 31/47, A61K 31/4709, A61K 31/444, A61P 37/00, A61P 29/00, A61P 17/04

(54) **QUINOLONE COMPOUNDS AND USE THEREOF**

(30) Priority: 29.12.2023 CN 202311857183
(71) Applicant: Alphamol Science Ltd (Shanghai), Shanghai 201210 (CN)
(72) Inventor: YUAN, Shuguang, Shenzhen, Guangdong 518000 (CN); LI, Yi, Shenzhen, Guangdong 518000 (CN); LI, Haijian, Shenzhen, Guangdong 518000 (CN); PAN, Qing, Shenzhen, Guangdong 518000 (CN)
(74) Representative: Vossius & Partner Patentanwälte Rechtsanwälte mbB
(86) International application number: PCT/CN2024/143121
(87) International publication number: WO 2025/140531

(57) **Abstract**

Provided herein are compounds of formula (IA) and other quinolone compounds, wherein the variables are as defined in the specification, and uses thereof in the treatment or prevention of diseases or disorders such as autoimmune diseases, allergic diseases, inflammatory diseases, and pain.

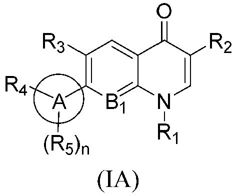

## Description

### TECHNICAL FIELD

The present disclosure relates to the field of medicine, and particularly to quinolone compounds or pharmaceutically acceptable salts thereof, and their use as immunomodulators in the treatment or prevention of diseases or disorders such as autoimmune diseases, allergic diseases, inflammatory diseases, and pain.

### BACKGROUND

Mast Cells (MC) are myeloid cells present in the connective tissues of our body, widely distributed around blood vessels and nerve endings in various tissues. They are a class of immune cell crucial for initiating inflammatory responses, and are primarily associated with the innate immune response, which is a non-specific branch of the immune system. Although playing a particularly important role in allergic reactions, they are also involved in many other immune functions. Mast cells have long been considered to be involved in the pathogenesis of allergic (hypersensitive) diseases, autoimmune diseases, and inflammatory diseases (especially inflammatory skin diseases and chronic inflammatory diseases). Mas-related G-protein-coupled receptors (MRGPRs) are involved in the signaling of pruritus, inflammation, and pain within the body. Among them, the MRGPRX2 target is widely distributed in barrier tissues and participates in regulating mast cell-related allergic reactions. When MRGPRX2 is activated, mast cell degranulation occurs; whereas the inhibition of MRGPRX2 significantly suppresses the degranulation of mast cell. MRGPRX2-mediated MC activation is independent of Immunoglobulin E (IgE) activation. Various basic secretagogues such as SP, vasoactive intestinal peptide, cationic antimicrobial peptides, and the classical activator compound 48/80 can act as ligands for MRGPRX2. In recent years, with in-depth research on MRGPRX2, its functions have been found to be increasingly important. MRGPRX2 is primarily involved in non-IgE-mediated reactions which are associated with mast cell activation, such as allergic and inflammatory reactions, neurogenic inflammation, pain, and pruritus, and can promote the innate immune response of the skin and intestines to harmful stimuli or pathogen invasion, such as atopic dermatitis, urticaria, alopecia areata, inflammatory bowel disease, asthma, etc. Therefore, MRGPRX2 antagonists can be used for the treatment or prevention of diseases or disorders such as autoimmune diseases, allergic diseases, inflammatory diseases, pruritus, and pain. Recent studies have also found that mast cell degranulation caused by MRGPRX2 activation promotes IgE-mediated allergic reactions. Researchers have observed that MRGPRX2 antagonists can significantly inhibit IgE-mediated anaphylactic shock *in vivo* (doi.org/10.1016/j.jaci.2024.11.021). This further demonstrates the application value of MRGPRX2 antagonists in diseases related to mast cell activation.

Autoimmune diseases are complex conditions, including psoriasis, atopic dermatitis, inflammatory bowel disease (IBD), urticaria, allergic rhinitis, asthma, and alopecia areata and the like. The pathogenesis usually involves, an abnormal immune system or hyperactive response triggers a disease, which attack the body's own tissues or organs, resulting in inflammation, damage, or dysfunction. The occurrence of these diseases may be related to many factors such as, genetics, environment, and infection. Autoimmune diseases affect about one-tenth of the population, and the global incidence of various autoimmune diseases has been increasing over time. A study published in *The Lancet* investigated the incidence of autoimmune diseases among over 22 million individuals from 2000 to 2019, during which over 970,000 individuals were diagnosed with at least one autoimmune disease (doi.org/10.1016/S0140-6736(23)00457-9). Autoimmune diseases cause a substantial burden of suffering on patients.

Psoriasis is an immune-mediated, chronic, systemic inflammatory skin disease with a widespread prevalence. The typical skin manifestation of psoriasis is erythema covered with silvery-white scales.

Atopic dermatitis is a disease caused by an immune system dysregulation. Its symptoms are relatively more severe in adolescents and adults, while its prevalence is higher in infant and children. Scaling and thickening of the affected skin area is a significant manifestation of atopic dermatitis.

Inflammatory bowel disease (IBD) is an idiopathic intestinal inflammatory disease involving the ileum, rectum, and colon. Clinical manifestations include diarrhea, abdominal pain, and even bloody stools. It is known that the inflammatory response caused by abnormal immune mucosal reactions in the intestine plays an important role in the pathogenesis of IBD.

Allergic rhinitis (AR) is a non-infectious chronic inflammatory disease primarily mediated by Immunoglobulin E (IgE) after atopic individuals are exposed to allergens. Clinical manifestations include nasal itching, sneezing, rhinorrhea, and nasal congestion. The global average prevalence of AR is as high as around 20%, which not only severely affects the quality of life of patients but is also an independent risk factor for inducing asthma. For establishing AR models, the most commonly used allergen is ovalbumin (OVA), which has the advantages of low toxicity and the ability of producing antibodies foe long period after sensitization. Mice primarily produce IgE during immune responses, a characteristic highly similar to humans, and they possess more comprehensive genetic data.

Bronchial asthma (referred to as asthma) is a chronic non-specific airway disease characterized by airway inflammation, airway mucus hypersecretion, airway hyperresponsiveness, and airway remodeling. With the deterioration of the climate and environment, its incidence has been increasing year by year, and it has now become a dangerous factor that severely endangers human life and health. Given the limitations of trials on human, asthma-related research is currently mainly conducted through animal models of asthma. An ideal animal model should reflect the pathophysiological mechanisms of human asthma as closely as possible, including allergic inflammation, eosinophilia, and the secretion of a large amount of mucus in the airways. As commonly used experimental animals for asthma models, BALB/c and C57BL/6 mice possess the following primary characteristics: a well-defined genetic background, numerous strains, low cost, and ease of inducing characteristic symptoms of asthma.

Rosacea is a chronic inflammatory skin disease involving the cheeks, nose, chin, and forehead. Its clinical manifestations are recurrent flushing, telangiectasia, persistent facial erythema, and papulopustules.

Urticaria is a common immune-related skin disease characterized mainly by wheals and pruritus. Mast cells are the key effector cells in the pathogenesis of urticaria. The main clinical symptom is localized edema, i.e., wheals, resulting from a temporary increase in vascular permeability in the skin and mucous membranes. Wheals may be accompanied by intense, severe pruritus and scratching.

### SUMMARY OF THE DISCLOSURE

This summary is provided to introduce some concepts in a simplified form, which will be further described in the detailed description below. This summary is not intended to identify key or essential features of the claimed subject matter, nor is it intended to limit the scope of the claimed subject matter.

The compounds of the present disclosure can be used through immunomodulatory mechanisms for the treatment or prevention of autoimmune diseases, allergic diseases, inflammatory diseases, pruritus, and pain, such as psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo. They have low toxicity and may be orally administered.

Specifically, the present disclosure provides a compound of formula (IA): 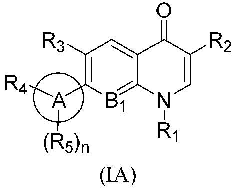 or a pharmaceutically acceptable salt, a stereoisomer, a tautomer, a hydrate, or a solvate thereof, wherein each variable is as defined herein, for use in antagonizing MRGPRX2.

The present disclosure also provides a compound of formula (IB): 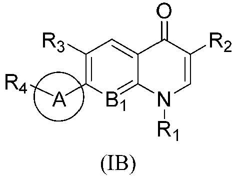 or a pharmaceutically acceptable salt, a stereoisomer, a tautomer, a hydrate, or a solvate thereof, wherein each variable is as defined herein.

The aforementioned compounds and the general and specific compounds disclosed in the context of the present disclosure, as well as pharmaceutically acceptable salts, stereoisomers, tautomers, hydrates, and solvates thereof, are referred to herein as "compounds of the present disclosure".

The compounds of the present disclosure are MRGPRX2 antagonists and can be used for the treatment or prevention of diseases or disorders mediated by MRGPRX2, particularly diseases or disorders caused by mast cell degranulation. More specifically, they can be used for the treatment or prevention of autoimmune diseases, allergic diseases, inflammatory diseases, pruritus, and pain, such as psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

Provided herein are the compounds of the present disclosure for use as MRGPRX2 antagonists.

Provided herein are the compounds of the present disclosure, for use in the treatment or prevention of a disease or disorder mediated by MRGPRX2.

Provided herein are the compounds of the present disclosure, for use in the treatment or prevention of a disease or disorder caused by mast cell degranulation.

Provided herein are the compounds of the present disclosure, for use in the treatment or prevention of autoimmune diseases, allergic diseases, inflammatory diseases, pruritus, and pain, such as psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

Provided herein is the use of the compounds of the present disclosure in the manufacture of a medicament for the treatment or prevention of autoimmune diseases, allergic diseases, inflammatory diseases, pruritus, and pain, such as psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

Provided herein are is a method for treating or preventing a disease or disorder mediated by MRGPRX2 comprises administering an effective amount of the compounds of the present disclosure or pharmaceutically acceptable salts thereof to a subject in need thereof.

Provided herein is a method for treating or preventing a disease or disorder in a subject, comprising to the subject in need thereof an effective amount of the compounds of the present disclosure or pharmaceutically acceptable salts thereof, the disease or disorder is autoimmune diseases, allergic diseases, inflammatory diseases, pruritus, and pain, such as psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

Provided herein is a method for antagonizing MRGPRX2 *in vitro* comprising utilizing a compound of the present disclosure or a pharmaceutically acceptable salt thereof in an *in vitro* assay for MRGPRX2.

Also provided herein is a pharmaceutical composition comprising the compounds of the present disclosure or pharmaceutically acceptable salts thereof, and one or more pharmaceutically acceptable excipients.

Additionally, provided herein is a pharmaceutical combination (combination product) or a kit, comprising a compound of the present disclosure or a pharmaceutically acceptable salt thereof (or a pharmaceutical composition thereof) as defined above, and one or more other therapeutic agents (or pharmaceutical compositions thereof). They are for simultaneous, separate, or sequential use in the treatment or prevention of a disease or disorder mediated by MRGPRX2.

### DETAILED DESCRIPTION OF THE DISCLOSURE

### Part A Embodiments of the Present Disclosure

**Embodiment 1.** A compound of formula (IA), or a pharmaceutically acceptable salt, stereoisomer, tautomer, hydrate, or solvate thereof, for use in the treatment or prevention of a disease or disorder mediated by MRGPRX2, 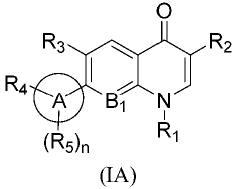 wherein:
R₁ is -C₃₋₆ cycloalkyl or -C₁₋₆ linear or branched alkyl;
R₂ is -COOH, -COOC₁₋₆ linear or branched alkyl, -C(O)NH₂, -C(O)-NH-C₁₋₆ linear or branched alkyl, -C(O)-N (C₁₋₆ linear or branched alkyl)₂ or -C(O)-NH-OH;
R₃ is halogen;
B₁ is -CR₆ or N, wherein R₆ is H, halogen or -O-C₁₋₆ linear or branched alkyl;
A is C₆₋₁₀ aryl or 5-6-membered heteroaryl;
R₄ is selected from the group consisting of -S(O)₂-NH₂, -S(O)₂-NHRₐ, -S(O)₂-NR_{b}R_{c}, -S(O)-NH₂, -S(O)-NH-Rₐ, -S(O)-NR_{b}R_{c}, -S-NH₂, -S-NH-Rₐ, -S-NR_{b}R_{c}, -S(O)₂-Rₐ, -S(O)-Rₐ, -NH-S(O)₂-Rₐ, -C(O)-NH₂, -C(O)-NH-Rₐ, -C(O)-NR_{b}R_{c}, -NH-C(O)-Rₐ, -C(O)-Rₐ, -NHR_{c}, -S-R_{d} and NO₂;
Rₐ and R_{d} are each independently selected from the group consisting of C₁₋₂₀ linear or branched alkyl, C₂₋₂₀ linear or branched alkenyl, C₂₋₂₀ linear or branched alkynyl, C₃₋₁₀-membered cycloalkyl and 4-10-membered heterocycloalkyl, wherein the heterocycloalkyl contains one or more heteroatoms selected from the group consisting of N, O, and S, and the alkyl, alkenyl, alkynyl, cycloalkyl or heterocycloalkyl is optionally substituted with 1, 2, or 3 substituents selected from the group consisting of: halogen, -OH, -NH₂, -CN, -NO₂, -SC₁₋₆ linear or branched alkyl, -OC₁₋₆ linear or branched alkyl, -NHC₁₋₆ linear or branched alkyl and -N (C₁₋₆ linear or branched alkyl)₂;
R_{b} and R_{c}, together with the N atom to which they are attached, form a 4-10-membered heterocycloalkyl group, wherein the heterocycloalkyl contains one or more heteroatoms selected from the group consisting of N, O, and S, and the heterocycloalkyl is optionally substituted with 1, 2, or 3 substituents selected from the group consisting of: halogen, -OH, -NH₂, -CN, -NO₂, -SC₁₋₆ linear or branched alkyl, -OC₁₋₆ linear or branched alkyl, -NHC₁₋₆ linear or branched alkyl and -N (C₁₋₆ linear or branched alkyl)₂;
R₅ is selected from the group consisting of halogen, -CN, -NH₂, -OH, -NH-C₁₋₆ linear or branched alkyl, -N (C₁₋₆ linear or branched alkyl)₂, C₁₋₆ linear or branched alkyl, C₁₋₆ linear or branched alkoxy, -C(O)-NH₂, -C(O)-NH-C₁₋₆ linear or branched alkyl, -C(O)-N (C₁₋₆ linear or branched alkyl)₂ and -NH-C(O)-C₁₋₆ linear or branched haloalkyl; and
n is 0, 1, 2 or 3.

**Embodiment** 2. The compound, or a pharmaceutically acceptable salt, stereoisomer, tautomer, hydrate or solvate thereof for use according to any one of the preceding embodiments, wherein R₁ is -C₃₋₄ cycloalkyl or -C₁₋₃ linear or branched alkyl.

**Embodiment** 3. The compound, or a pharmaceutically acceptable salt, stereoisomer, tautomer, hydrate or solvate thereof for use according to any one of the preceding embodiments, wherein R₁ is 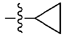 or -C₁₋₃ linear or branched alkyl.

**Embodiment** 4. The compound, or a pharmaceutically acceptable salt, stereoisomer, tautomer, hydrate or solvate thereof for use according to any one of the preceding embodiments, wherein R₁ is 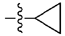 or

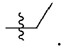

**Embodiment** 5. The compound, or a pharmaceutically acceptable salt, stereoisomer, tautomer, hydrate or solvate thereof for use according to any one of the preceding embodiments, wherein R₁ is

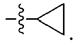

**Embodiment 6.** The compound, or a pharmaceutically acceptable salt, stereoisomer, tautomer, hydrate or solvate thereof for use according to any one of the preceding embodiments, wherein R₂ is -COOH, -COOC₁₋₃ linear or branched alkyl, -C(O)NH₂, -C(O)-NH-C₁₋₃ linear or branched alkyl, -C(O)-N (C₁₋₃ linear or branched alkyl)₂ or -C(O)-NH-OH.

**Embodiment** 7. The compound, or a pharmaceutically acceptable salt, stereoisomer, tautomer, hydrate or solvate thereof for use according to any one of the preceding embodiments, wherein R₂ is -COOH, -COOC₁₋₃ linear or branched alkyl or -C(O)NH₂.

**Embodiment 8.** The compound, or a pharmaceutically acceptable salt, stereoisomer, tautomer, hydrate or solvate thereof for use according to any one of the preceding embodiments, wherein R₂ is -COOH or -COOC₁₋₃ linear or branched alkyl.

**Embodiment 9.** The compound, or a pharmaceutically acceptable salt, stereoisomer, tautomer, hydrate or solvate thereof for use according to any one of the preceding embodiments, wherein R₂ is -COOH or -COOCH₃.

**Embodiment 10.** The compound, or a pharmaceutically acceptable salt, stereoisomer, tautomer, hydrate or solvate thereof for use according to any one of the preceding embodiments, wherein R₂ is -COOCH₃ or -COOCH₂CH₃.

**Embodiment 11.** The compound, or a pharmaceutically acceptable salt, stereoisomer, tautomer, hydrate or solvate thereof for use according to any one of the preceding embodiments, wherein R₂ is -COOH.

**Embodiment 12.** The compound, or a pharmaceutically acceptable salt, stereoisomer, tautomer, hydrate or solvate thereof for use according to any one of the preceding embodiments, wherein R₃ is F.

**Embodiment 13.** The compound, or a pharmaceutically acceptable salt, stereoisomer, tautomer, hydrate or solvate thereof for use according to any one of the preceding embodiments, wherein B₁ is -CR₆ or N, wherein R₆ is H, halogen or -O-C₁₋₃ linear or branched alkyl.

**Embodiment 14.** The compound, or a pharmaceutically acceptable salt, stereoisomer, tautomer, hydrate or solvate thereof for use according to any one of the preceding embodiments, wherein B₁ is -CR₆ or N, wherein R₆ is H, F, Cl or -OCH₃.

**Embodiment 15.** The compound, or a pharmaceutically acceptable salt, stereoisomer, tautomer, hydrate or solvate thereof for use according to any one of the preceding embodiments, wherein B₁ is -CR₆, wherein R₆ is H or -OCH₃.

**Embodiment 16.** The compound, or a pharmaceutically acceptable salt, stereoisomer, tautomer, hydrate or solvate thereof for use according to any one of the preceding embodiments, wherein B₁ is -CR₆, wherein R₆ is H.

**Embodiment 17.** The compound, or a pharmaceutically acceptable salt, stereoisomer, tautomer, hydrate or solvate thereof for use according to any one of the preceding embodiments, wherein B₁ is -CR₆, wherein R₆ is -OCH₃.

**Embodiment 18.** The compound, or a pharmaceutically acceptable salt, stereoisomer, tautomer, hydrate or solvate thereof for use according to any one of the preceding embodiments, wherein B₁ is N.

**Embodiment 19.** The compound, or a pharmaceutically acceptable salt, stereoisomer, tautomer, hydrate or solvate thereof for use according to any one of the preceding embodiments, wherein, A is phenyl, pyridyl, pyridazinyl, pyrimidinyl or pyrazinyl.

**Embodiment 20.** The compound, or a pharmaceutically acceptable salt, stereoisomer, tautomer, hydrate or solvate thereof for use according to any one of the preceding embodiments, wherein, A is phenyl or pyridyl.

**Embodiment 21.** The compound, or a pharmaceutically acceptable salt, stereoisomer, tautomer, hydrate or solvate thereof for use according to any one of the preceding embodiments, wherein, A is phenyl.

**Embodiment 22.** The compound, or a pharmaceutically acceptable salt, stereoisomer, tautomer, hydrate or solvate thereof for use according to any one of the preceding embodiments, wherein, A is pyridyl.

**Embodiment 23.** The compound, or a pharmaceutically acceptable salt, stereoisomer, tautomer, hydrate or solvate thereof for use according to any one of the preceding embodiments, wherein R₄ is selected from the group consisting of -S(O)₂-NH₂, -S(O)₂-NHRₐ, -S(O)₂-NR_{b}R_{c}, -S(O)-NH₂, -S(O)-NH-Rₐ, -S(O)-NR_{b}R_{c}, -S-NH₂, -S-NH-Rₐ, -S-NR_{b}R_{c}, -S(O)₂-Rₐ, -S(O)-Rₐ, -NH-S(O)₂-Rₐ, -C(O)-NH₂, -C(O)-NH-Rₐ, -C(O)-NR_{b}R_{c}, -NH-C(O)-Rₐ, -C(O)-Rₐ, -NHR_{d} and -S-R_{d}.

**Embodiment 24.** The compound, or a pharmaceutically acceptable salt, stereoisomer, tautomer, hydrate or solvate thereof for use according to any one of the preceding embodiments, wherein R₄ is selected from the group consisting of -S(O)₂-NH₂, -S(O)₂-NHRₐ, -S(O)₂-NR_{b}R_{c}, -S(O)-NH₂, -S(O)-NH-Rₐ, -S(O)-NR_{b}R_{c}, -S-NH-Rₐ, -S-NR_{b}R_{c}, -S(O)₂-Rₐ, -S(O)-Rₐ, -NH-S(O)₂-Rₐ, -C(O)-NH₂, -C(O)-NH-Rₐ, -C(O)-NR_{b}R_{c}, -NH-C(O)-Rₐ, -NHR_{d} and -S-R_{d}.

**Embodiment 25.** The compound, or a pharmaceutically acceptable salt, stereoisomer, tautomer, hydrate or solvate thereof for use according to any one of the preceding embodiments, wherein R₄ is selected from the group consisting of -S(O)₂-NH₂, -S(O)₂-NHRₐ, -S(O)₂-NR_{b}R_{c}, -S(O)-NH-Rₐ, -S-NH-Rₐ, -S(O)-Rₐ, -NH-S(O)₂-Rₐ, -C(O)-NH₂, -C(O)-NH-Rₐ, -C(O)-NR_{b}R_{c}, -NH-C(O)-Rₐ, -NHR_{d} and -S-R_{d}.

**Embodiment 26.** The compound, or a pharmaceutically acceptable salt, stereoisomer, tautomer, hydrate or solvate thereof for use according to any one of the preceding embodiments, wherein R₄ is selected from the group consisting of -S(O)₂-NH₂, -S(O)₂-NHRₐ, -S(O)₂-NR_{b}R_{c}, -S(O)-NH-Rₐ, -S-NH-Rₐ, -S(O)-Rₐ, -NH-S(O)₂-Rₐ, -C(O)-NH₂, -C(O)-NH-Rₐ, -NH-C(O)-Rₐ, -NHR_{d} and -S-R_{d}.

**Embodiment 27.** The compound, or a pharmaceutically acceptable salt, stereoisomer, tautomer, hydrate or solvate thereof for use according to any one of the preceding embodiments, wherein R₄ is selected from the group consisting of -S(O)₂-NH₂, -S(O)₂-NHRₐ, -S(O)₂-NR_{b}R_{c}, -NH-S(O)₂-Rₐ, -C(O)-NH₂, -C(O)-NH-Rₐ, -NH-C(O)-Rₐ, -NHR_{d} and -S-R_{d}.

**Embodiment 28.** The compound, or a pharmaceutically acceptable salt, stereoisomer, tautomer, hydrate or solvate thereof for use according to any one of the preceding embodiments, wherein R₄ is selected from the group consisting of -S(O)₂-NH₂, -S(O)₂-NHRₐ, -S(O)₂-NR_{b}R_{c}, -S(O)-NH-Rₐ, -S-NH-Rₐ, -NH-S(O)₂-Rₐ, -C(O)-NH₂, -C(O)-NH-Rₐ and -NHRₐ.

**Embodiment 29.** The compound, or a pharmaceutically acceptable salt, stereoisomer, tautomer, hydrate or solvate thereof for use according to any one of the preceding embodiments, wherein R₄ is selected from the group consisting of -S(O)₂-NH₂, -S(O)₂-NHRₐ, -S(O)₂-NR_{b}R_{c}, -NH-S(O)₂-Rₐ, -C(O)-NH₂, -C(O)-NH-Rₐ, -C(O)-NR_{b}R_{c} and -NH-C(O)-Rₐ.

**Embodiment 30.** The compound, or a pharmaceutically acceptable salt, stereoisomer, tautomer, hydrate or solvate thereof for use according to any one of the preceding embodiments, wherein R₄ is selected from the group consisting of -S(O)₂-NH₂, -S(O)₂-NHRₐ and -S(O)₂-NR_{b}R_{c}.

**Embodiment 31.** The compound, or a pharmaceutically acceptable salt, stereoisomer, tautomer, hydrate or solvate thereof for use according to any one of the preceding embodiments, wherein R₄ is -S(O)₂-NHRₐ.

**Embodiment 32.** The compound, or a pharmaceutically acceptable salt, stereoisomer, tautomer, hydrate or solvate thereof for use according to any one of the preceding embodiments, wherein Rₐ and R_{d} are each independently selected from the group consisting of C₁₋₁₅ linear or branched alkyl, C₂₋₁₅ linear or branched alkenyl, C₂₋₁₅ linear or branched alkynyl, C₃₋₁₀-membered cycloalkyl and 4-10-membered heterocycloalkyl, wherein the heterocycloalkyl contains one or more heteroatoms selected from the group consisting of N, O, and S, and the alkyl, alkenyl, alkynyl, cycloalkyl or heterocycloalkyl is optionally substituted with 1, 2, or 3 substituents selected from the group consisting of: halogen, -OH, -NH₂, -CN, -NO₂, -SC₁₋₆ linear or branched alkyl, -OC₁₋₆ linear or branched alkyl, -NHC₁₋₆ linear or branched alkyl and -N (C₁₋₆ linear or branched alkyl)₂.

**Embodiment 33.** The compound, or a pharmaceutically acceptable salt, stereoisomer, tautomer, hydrate or solvate thereof for use according to any one of the preceding embodiments, wherein Rₐ is selected from the group consisting of C₁₋₁₅ linear or branched alkyl, C₂₋₁₅ linear or branched alkenyl, C₂₋₁₅ linear or branched alkynyl, C₃₋₁₀-membered cycloalkyl and 4-10-membered heterocycloalkyl, wherein the heterocycloalkyl contains one or more heteroatoms selected from the group consisting of N, O, and S, and the alkyl is optionally substituted with 1, 2, or 3 substituents selected from the group consisting of: halogen, -OH, -NH₂, -CN, -SC₁₋₆ linear or branched alkyl, -OC₁₋₃ linear or branched alkyl and -NHC₁₋₃ linear or branched alkyl; for example, Rₐ is C₁₋₁₅ linear or branched alkyl, and the alkyl is optionally substituted with 1, 2, or 3 halogen.

**Embodiment 34.** The compound, or a pharmaceutically acceptable salt, stereoisomer, tautomer, hydrate or solvate thereof for use according to any one of the preceding embodiments, wherein Rₐ is selected from the group consisting of C₁₋₁₅ linear or branched alkyl, C₃₋₆-membered cycloalkyl and 4-6-membered heterocycloalkyl, wherein the heterocycloalkyl contains 1, 2 or 3 heteroatoms selected from the group consisting of N, O, and S, and the alkyl is optionally substituted with 1, 2, or 3 substituents selected from the group consisting of: halogen, -OH, -NH₂, -CN, -SC₁₋₃ linear or branched alkyl, -OC₁₋₃ linear or branched alkyl and -NHC₁₋₃ linear or branched alkyl; for example, Rₐ is C₁₋₁₅ linear alkyl, and the alkyl is optionally substituted with 1, 2, or 3 halogen.

**Embodiment 35.** The compound, or a pharmaceutically acceptable salt, stereoisomer, tautomer, hydrate or solvate thereof for use according to any one of the preceding embodiments, wherein Rₐ is selected from the group consisting of C₁₋₁₅ linear or branched alkyl, C₃₋₆-membered cycloalkyl and 4-6-membered heterocycloalkyl, wherein the heterocycloalkyl contains 1, 2 or 3 heteroatoms selected from the group consisting of N, O, and S, and the alkyl is optionally substituted with 1, 2, or 3 substituents selected from the group consisting of: halogen, -OH, -NH₂ and -CN; for example, Rₐ is C₁₋₁₅ linear alkyl, and the alkyl is optionally substituted with 1, 2, or 3 halogen (e.g., 3 F atoms).

**Embodiment 36.** The compound, or a pharmaceutically acceptable salt, stereoisomer, tautomer, hydrate or solvate thereof for use according to any one of the preceding embodiments, wherein Rₐ is selected from the group consisting of C₁₋₁₂ linear or branched alkyl, C₃₋₆-membered cycloalkyl and 4-6-membered heterocycloalkyl, wherein the heterocycloalkyl contains 1 or 2 heteroatoms selected from the group consisting of N, O, and S, and the alkyl is optionally substituted with 1, 2, or 3 substituents selected from the group consisting of: halogen, -OH and -NH₂; for example, Rₐ is C₁₋₁₂ linear or branched alkyl, and the alkyl is optionally substituted with 1, 2, or 3 halogen (e.g., 3 F atoms).

**Embodiment 37.** The compound, or a pharmaceutically acceptable salt, stereoisomer, tautomer, hydrate or solvate thereof for use according to any one of the preceding embodiments, wherein Rₐ is selected from the group consisting of C₁₋₉ linear or branched alkyl, C₅₋₆-membered cycloalkyl and 5-6-membered heterocycloalkyl, wherein the heterocycloalkyl contains 1 or 2 heteroatoms selected from the group consisting of N or O, and the alkyl is optionally substituted with 1, 2, or 3 substituents selected from the group consisting of: halogen, -OH and --NH₂; for example Rₐ is C₁₋₉ linear or branched alkyl, and the alkyl is optionally substituted with 1, 2, or 3 halogen (e.g., 3 F atoms).

**Embodiment 38.** The compound, or a pharmaceutically acceptable salt, stereoisomer, tautomer, hydrate or solvate thereof for use according to any one of the preceding embodiments, wherein Rₐ is selected from the group consisting of C₁₋₉ linear or branched alkyl, C₁₋₉ linear or branched alkyl-OH, C₁₋₉ linear or branched alkyl-NH₂, C₁₋₉ linear or branched haloalkyl (e.g., C₁₋₉ linear or branched chloroalkyl, C₁₋₉ linear or branched fluoroalkyl or C₁₋₈ linear or branched alkyl-CF₃), cyclohexyl and tetrahydropyranyl; for example C₁₋₉ linear alkyl or C₁₋₈ linear alkyl-CF₃.

**Embodiment 39.** The compound, or a pharmaceutically acceptable salt, stereoisomer, tautomer, hydrate or solvate thereof for use according to any one of the preceding embodiments, wherein Rₐ is selected from the group consisting of C₁₋₉ linear or branched alkyl, for example C₁₋₉ linear alkyl.

**Embodiment 40.** The compound, or a pharmaceutically acceptable salt, stereoisomer, tautomer, hydrate or solvate thereof for use according to any one of the preceding embodiments, wherein Rₐ is selected from the group consisting of C₁₋₉ linear or branched alkyl-OH.

**Embodiment 41.** The compound, or a pharmaceutically acceptable salt, stereoisomer, tautomer, hydrate or solvate thereof for use according to any one of the preceding embodiments, wherein Rₐ is selected from the group consisting of C₁₋₉ linear or branched alkyl-NH₂.

**Embodiment 42.** The compound, or a pharmaceutically acceptable salt, stereoisomer, tautomer, hydrate or solvate thereof for use according to any one of the preceding embodiments, wherein Rₐ is selected from the group consisting of C₁₋₉ linear or branched haloalkyl, for example C₁₋₉ linear or branched chloroalkyl, C₁₋₉ linear or branched fluoroalkyl or C₁₋₈ linear or branched alkyl-CF₃, for example C₁₋₈ linear alkyl-CF₃.

**Embodiment 43.** The compound, or a pharmaceutically acceptable salt, stereoisomer, tautomer, hydrate or solvate thereof for use according to any one of the preceding embodiments, wherein Rₐ is selected from the group consisting of cyclohexyl and tetrahydropyranyl.

**Embodiment 44.** The compound, or a pharmaceutically acceptable salt, stereoisomer, tautomer, hydrate or solvate thereof for use according to any one of the preceding embodiments, wherein R_{d} is selected from the group consisting of C₁₋₁₅ linear or branched alkyl, C₂₋₁₅ linear or branched alkenyl and C₂₋₁₅ linear or branched alkynyl, wherein the alkyl, alkenyl or alkynyl is optionally substituted with 1, 2, or 3 substituents selected from the group consisting of: halogen, -OH, -NH₂, -CN and -NO₂.

**Embodiment 45.** The compound, or a pharmaceutically acceptable salt, stereoisomer, tautomer, hydrate or solvate thereof for use according to any one of the preceding embodiments, wherein R_{d} is selected from the group consisting of C₁₋₁₂ linear or branched alkyl, C₂₋₁₂ linear or branched alkenyl and C₂₋₁₂ linear or branched alkynyl, wherein the alkyl, alkenyl or alkynyl is optionally substituted with 1, 2, or 3 substituents selected from the group consisting of: halogen, -OH and -CN.

**Embodiment 46.** The compound, or a pharmaceutically acceptable salt, stereoisomer, tautomer, hydrate or solvate thereof for use according to any one of the preceding embodiments, wherein R_{d} is selected from the group consisting of C₁₋₆ linear or branched alkyl, C₂₋₆ linear or branched alkenyl and C₂₋₆ linear or branched alkynyl, wherein the alkyl, alkenyl or alkynyl is optionally substituted with 1, 2, or 3 substituents selected from the group consisting of: halogen, -OH and -CN.

**Embodiment 47.** The compound, or a pharmaceutically acceptable salt, stereoisomer, tautomer, hydrate or solvate thereof for use according to any one of the preceding embodiments, wherein R_{d} is selected from the group consisting of C₁₋₆ linear or branched alkyl, C₂₋₆ linear or branched alkenyl and C₂₋₆ linear or branched alkynyl, wherein the alkyl is optionally substituted with 1, 2, or 3 substituents selected from the group consisting of: halogen, -OH and -CN.

**Embodiment 48.** The compound, or a pharmaceutically acceptable salt, stereoisomer, tautomer, hydrate or solvate thereof for use according to any one of the preceding embodiments, wherein R_{d} is selected from the group consisting of C₁₋₃ linear or branched alkyl, C₂₋₃ linear or branched alkenyl and C₂₋₃ linear or branched alkynyl, wherein the alkyl is optionally substituted with 1, 2, or 3 substituents selected from the group consisting of: halogen, -OH and -CN.

**Embodiment 49.** The compound, or a pharmaceutically acceptable salt, stereoisomer, tautomer, hydrate or solvate thereof for use according to any one of the preceding embodiments, wherein R_{d} is selected from the group consisting of C₁₋₃ linear or branched alkyl (e.g., n-propyl), C₂₋₃ linear or branched alkenyl (e.g., allyl) and C₂₋₃ linear or branched alkynyl (e.g., propargyl), wherein the alkyl is optionally substituted with 1 -OH (e.g., -C₁₋₃ linear alkyl-OH, such as hydroxyethyl).

**Embodiment 50.** The compound, or a pharmaceutically acceptable salt, stereoisomer, tautomer, hydrate or solvate thereof for use according to any one of the preceding embodiments, wherein R_{b} and R_{c}, together with the N atom to which they are attached, form a 4-10-membered heterocycloalkyl group, wherein the heterocycloalkyl contains one or more heteroatoms selected from the group consisting of N, O, and S, and the heterocycloalkyl is optionally substituted with 1, 2, or 3 substituents selected from the group consisting of: halogen, -OH, -NH₂, -CN, -NO₂, -SC₁₋₆ linear or branched alkyl, -OC₁₋₆ linear or branched alkyl, -NHC₁₋₆ linear or branched alkyl and -N (C₁₋₆ linear or branched alkyl)₂.

**Embodiment 51.** The compound, or a pharmaceutically acceptable salt, stereoisomer, tautomer, hydrate or solvate thereof for use according to any one of the preceding embodiments, wherein R_{b} and R_{c}, together with the N atom to which they are attached, form a 4-7-membered heterocycloalkyl group, wherein the heterocycloalkyl contains 1, 2 or 3 heteroatoms selected from the group consisting of N, O, and S, and the heterocycloalkyl is optionally substituted with 1, 2, or 3 substituents selected from the group consisting of: halogen, -OH, -NH₂, -CN, -SC₁₋₃ linear or branched alkyl, -OC₁₋₃ linear or branched alkyl and -NHC₁₋₃ linear or branched alkyl.

**Embodiment 52.** The compound, or a pharmaceutically acceptable salt, stereoisomer, tautomer, hydrate or solvate thereof for use according to any one of the preceding embodiments, wherein R_{b} and R_{c}, together with the N atom to which they are attached, form a 4-6-membered heterocycloalkyl group, wherein the heterocycloalkyl contains 1, 2 or 3 heteroatoms selected from the group consisting of N, O, and S, and the heterocycloalkyl is optionally substituted with 1, 2, or 3 substituents selected from the group consisting of: halogen, -OH, -NH₂, -CN, -SC₁₋₃ linear or branched alkyl, -OC₁₋₃ linear or branched alkyl and -NHC₁₋₃ linear or branched alkyl.

**Embodiment 53.** The compound, or a pharmaceutically acceptable salt, stereoisomer, tautomer, hydrate or solvate thereof for use according to any one of the preceding embodiments, wherein R_{b} and R_{c}, together with the N atom to which they are attached, form a 4-6-membered heterocycloalkyl group, wherein the heterocycloalkyl contains 1 or 2 heteroatoms selected from the group consisting of N, O, and S, and the heterocycloalkyl is optionally substituted with 1, 2, or 3 substituents selected from the group consisting of: halogen, -OH, -NH₂ and -CN.

**Embodiment 54.** The compound, or a pharmaceutically acceptable salt, stereoisomer, tautomer, hydrate or solvate thereof for use according to any one of the preceding embodiments, wherein R_{b} and R_{c}, together with the N atom to which they are attached, form a 4-6-membered heterocycloalkyl group, wherein the heterocycloalkyl contains 1 or 2 heteroatoms selected from the group consisting of N, O, and S, and the heterocycloalkyl is optionally substituted with 1, 2, or 3 halogen.

**Embodiment 55.** The compound, or a pharmaceutically acceptable salt, stereoisomer, tautomer, hydrate or solvate thereof for use according to any one of the preceding embodiments, wherein R_{b} and R_{c}, together with the N atom to which they are attached, form a 4-6-membered heterocycloalkyl group, wherein the heterocycloalkyl contains 1 or 2 heteroatoms selected from the group consisting of N or O, and the heterocycloalkyl is optionally substituted with two F atoms; for example, azetidinyl, pyrrolidinyl, piperidinyl, piperazinyl, morpholinyl, difluoroazetidinyl or 3,3-difluoroazetidinyl.

**Embodiment 56.** The compound, or a pharmaceutically acceptable salt, stereoisomer, tautomer, hydrate or solvate thereof for use according to any one of the preceding embodiments, wherein R_{b} and R_{c}, together with the N atom to which they are attached, form a 4-6-membered heterocycloalkyl group, wherein the heterocycloalkyl contains 1 heteroatom selected from the group consisting of N, and the heterocycloalkyl is optionally substituted with two F atoms; for example, azetidinyl, pyrrolidinyl, piperidinyl, difluoroazetidinyl or 3,3-difluoroazetidinyl.

**Embodiment 57.** The compound, or a pharmaceutically acceptable salt, stereoisomer, tautomer, hydrate or solvate thereof for use according to any one of the preceding embodiments, wherein R₅ is selected from the group consisting of halogen, -CN, -NH₂, -OH, -NH-C₁₋₆ linear or branched alkyl, C₁₋₆ linear or branched alkyl, C₁₋₆ linear or branched alkoxy, -C(O)-NH₂, -C(O)-NH-C₁₋₆ linear or branched alkyl and -NH-C(O)-C₁₋₆ linear or branched haloalkyl.

**Embodiment 58.** The compound, or a pharmaceutically acceptable salt, stereoisomer, tautomer, hydrate or solvate thereof for use according to any one of the preceding embodiments, wherein R₅ is selected from the group consisting of halogen, -CN, -NH₂, -OH, -NH-C₁₋₆ linear or branched alkyl, C₁₋₆ linear or branched alkyl, C₁₋₆ linear or branched alkoxy, -C(O)-NH₂, -C(O)-NH-C₁₋₆ linear or branched alkyl and -NH-C(O)-C₁₋₆ linear or branched haloalkyl.

**Embodiment 59.** The compound, or a pharmaceutically acceptable salt, stereoisomer, tautomer, hydrate or solvate thereof for use according to any one of the preceding embodiments, wherein R₅ is selected from the group consisting of halogen, -CN, -NH₂, -OH, -NH-C₁₋₆ linear or branched alkyl, C₁₋₆ linear or branched alkyl and C₁₋₆ linear or branched alkoxy.

**Embodiment 60.** The compound, or a pharmaceutically acceptable salt, stereoisomer, tautomer, hydrate or solvate thereof for use according to any one of the preceding embodiments, wherein R₅ is selected from the group consisting of halogen, -CN, -NH₂, -OH, -NH-C₁₋₃ linear or branched alkyl, C₁₋₃ linear alkyl and C₁₋₃ linear or branched alkoxy.

**Embodiment 61.** The compound, or a pharmaceutically acceptable salt, stereoisomer, tautomer, hydrate or solvate thereof for use according to any one of the preceding embodiments, wherein R₅ is selected from the group consisting of halogen (e.g., F), -CN, -NH₂ and C₁₋₃ linear alkoxy (e.g., methoxy).

**Embodiment 62.** The compound, or a pharmaceutically acceptable salt, stereoisomer, tautomer, hydrate or solvate thereof for use according to any one of the preceding embodiments, wherein n is 0, 1 or 2.

**Embodiment 63.** The compound, or a pharmaceutically acceptable salt, stereoisomer, tautomer, hydrate or solvate thereof for use according to any one of the preceding embodiments, wherein n is 0.

**Embodiment 64.** The compound, or a pharmaceutically acceptable salt, stereoisomer, tautomer, hydrate or solvate thereof for use according to any one of the preceding embodiments, wherein n is 1.

**Embodiment 65.** The compound, or a pharmaceutically acceptable salt, stereoisomer, tautomer, hydrate or solvate thereof for use according to any one of the preceding embodiments, wherein n is 1 or 2.

**Embodiment 66.** The compound, or a pharmaceutically acceptable salt, stereoisomer, tautomer, hydrate or solvate thereof for use according to embodiment 1, wherein:
A is phenyl or pyridyl;
R₄ is -S(O)₂-NHRₐ or -S(O)₂-NR_{b}R_{c};
Rₐ is selected from the group consisting of C₁₋₉ linear alkyl, cyclohexyl and tetrahydropyran, and the alkyl is optionally substituted with 1, 2, or 3 groups selected from the group consisting of halogen, -OH, -NH₂ and -SCH₃ (e.g., the alkyl is optionally substituted with 1 group selected from the group consisting of halogen, -OH, -NH₂ and -SCH₃, or optionally substituted with 3 halogen), for example -CF₃ or C₁₋₈ linear alkyl-CF₃;
R_{b} and R_{c}, together with the N atom to which they are attached, form a 4-6-membered heterocycloalkyl group, wherein the heterocycloalkyl contains 1 or 2 heteroatoms selected from the group consisting of N or O, and the heterocycloalkyl is optionally substituted with two F atoms (e.g., azetidinyl, pyrrolidinyl, piperidinyl, piperazinyl, morpholinyl, difluoroazetidinyl, 3,3-difluoroazetidinyl);
R₅ is selected from the group consisting of halogen, -CN, -NH₂, C₁₋₃ linear alkyl, C₁₋₃ linear alkoxy, -C(O)-NH₂ and -NH-C(O)-C₁₋₃ linear or branched haloalkyl; and
n is 0 or 1.

**Embodiment 67.** The compound, or a pharmaceutically acceptable salt, stereoisomer, tautomer, hydrate or solvate thereof for use according to embodiment 1, wherein:
A is phenyl or pyridyl;
R₄ is -S(O)-NH-Rₐ or -S-NH-Rₐ;
Rₐ is C₁₋₉ linear alkyl, optionally substituted with 1, 2, or 3 halogen (e.g., -CF₃ or C₁₋₈ linear alkyl-CF₃); and
n is 0.

**Embodiment 68.** The compound, or a pharmaceutically acceptable salt, stereoisomer, tautomer, hydrate or solvate thereof for use according to embodiment 1, wherein:
A is phenyl or pyridyl;
R₄ is -S(O)-Rₐ, Rₐ is C₁₋₆ linear or branched haloalkyl (e.g., R₄ is -S(O)-C₁₋₆ linear or branched chloroalkyl); and
n is 0.

**Embodiment 69.** The compound, or a pharmaceutically acceptable salt, stereoisomer, tautomer, hydrate or solvate thereof for use according to embodiment 1, wherein:
A is phenyl or pyridyl;
R₄ is -NH-S(O)₂-Rₐ;
Rₐ is linear or branched C₁₋₆ linear or branched alkyl; and
n is 0.

**Embodiment 70.** The compound, or a pharmaceutically acceptable salt, stereoisomer, tautomer, hydrate or solvate thereof for use according to embodiment 1, wherein:
A is phenyl or pyridyl;
R₄ is -C(O)-NH-Rₐ or -NH-C(O)-Rₐ;
Rₐ is linear or branched C₁₋₆ linear or branched alkyl, and the alkyl is optionally substituted with 1, 2, or 3 groups selected from the group consisting of halogen and -OH (e.g., the alkyl is optionally substituted with 1 group selected from the group consisting of halogen and OH, or optionally substituted with 2 or 3 halogen, for example -CF₂, -CF₃ or C₁₋₅ linear alkyl-CF₃);
R₅ is selected from the group consisting of halogen, -CN, -NH₂, C₁₋₃ linear alkyl, C₁₋₃ linear alkoxy, -C(O)-NH₂ and -NH-C(O)-C₁₋₃ linear or branched haloalkyl; and
n is 0 or 1.

**Embodiment 71.** The compound, or a pharmaceutically acceptable salt, stereoisomer, tautomer, hydrate, or solvate thereof for use according to any one of embodiment 1, wherein:
R₁ is 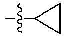 or 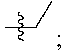 for example 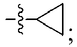
R₂ is -COOH or -COOC₁₋₃ linear or branched alkyl; for example -COOH or -COOCH₃;
R₃ is F;
B₁ is -CR₆ or N, wherein R₆ is H, halogen or -O-C₁₋₃ linear or branched alkyl, for example H, F or -O-CH₃;
A is phenyl or pyridyl;
R₄ is selected from the group consisting of -S(O)₂-NH₂, -S(O)₂-NHRₐ, -S(O)₂-NR_{b}R_{c}, -NH-S(O)₂-Rₐ, -C(O)-NH₂, -C(O)-NH-Rₐ, -C(O)-NR_{b}R_{c} and -NH-C(O)-Rₐ;
Rₐ is selected from the group consisting of C₁₋₁₂ linear or branched alkyl, C₃₋₆-membered cycloalkyl and 4-6-membered heterocycloalkyl, wherein the heterocycloalkyl contains 1 or 2 heteroatoms selected from the group consisting of N, O, and S, and the alkyl is optionally substituted with 1, 2, or 3 substituents selected from the group consisting of: halogen and -OH;
R_{b} and R_{c}, together with the N atom to which they are attached, form a 4-6-membered heterocycloalkyl group, wherein the heterocycloalkyl contains 1 or 2 heteroatoms selected from the group consisting of N, O, and S, and the heterocycloalkyl is optionally substituted with 1, 2, or 3 halogen; and
n is 0.

**Embodiment 72.** The compound, or a pharmaceutically acceptable salt, stereoisomer, tautomer, hydrate or solvate thereof for use according to any one of the preceding embodiments, wherein R₄ is -S(O)₂-NH₂.

**Embodiment 73.** The compound, or a pharmaceutically acceptable salt, stereoisomer, tautomer, hydrate or solvate thereof for use according to any one of the preceding embodiments, wherein R₄ is -S(O)₂-NHRₐ.

**Embodiment 74.** The compound, or a pharmaceutically acceptable salt, stereoisomer, tautomer, hydrate or solvate thereof for use according to any one of the preceding embodiments, wherein R₄ is -S(O)₂-NR_{b}R_{c}.

**Embodiment 75.** The compound, or a pharmaceutically acceptable salt, stereoisomer, tautomer, hydrate or solvate thereof for use according to any one of the preceding embodiments, wherein Rₐ is selected from the group consisting of C₁₋₁₀ linear or branched alkyl, C₃₋₆-membered cycloalkyl and 4-6-membered heterocycloalkyl, wherein the heterocycloalkyl contains 1 or 2 heteroatoms selected from the group consisting of N or O, and the alkyl is optionally substituted with 1, 2, or 3 substituents selected from the group consisting of: halogen and -OH;

**Embodiment 76.** The compound, or a pharmaceutically acceptable salt, stereoisomer, tautomer, hydrate or solvate thereof for use according to any one of the preceding embodiments, wherein Rₐ is C₁₋₉ linear or branched alkyl optionally substituted with 1, 2, or 3 hydroxyl or halogen groups (e.g., C₁₋₈ linear or branched alkyl-CF₃), or tetrahydropyranyl.

**Embodiment 77.** The compound, or a pharmaceutically acceptable salt, stereoisomer, tautomer, hydrate or solvate thereof for use according to any one of the preceding embodiments, wherein R_{b} and R_{c}, together with the N atom to which they are attached, form a 4-6-membered heterocycloalkyl group, wherein the heterocycloalkyl contains 1 or 2 heteroatoms selected from the group consisting of N and O, and the heterocycloalkyl is optionally substituted with 1 or 2 halogen (e.g., 2 F atoms); for example, azetidinyl, pyrrolidinyl, piperidinyl, piperazinyl, morpholinyl, difluoroazetidinyl or 3,3-difluoroazetidinyl.

**Embodiment 78.** The compound, or a pharmaceutically acceptable salt, stereoisomer, tautomer, hydrate or solvate thereof for use according to any one of the preceding embodiments, wherein the compound is selected from the group consisting of compounds of Example 1-173.

**Embodiment 79.** The compound, or a pharmaceutically acceptable salt, stereoisomer, tautomer, hydrate or solvate thereof for use according to any one of the preceding embodiments, wherein the disease or disorder is a disease or disorder caused by mast cell degranulation.

**Embodiment 80.** The compound, or a pharmaceutically acceptable salt, stereoisomer, tautomer, hydrate or solvate thereof for use according to any one of the preceding embodiments, wherein the disease or disorder is selected from the group consisting of autoimmune diseases, allergic diseases, inflammatory diseases, pruritus, and pain, such as inflammatory skin diseases.

**Embodiment 81.** The compound, or a pharmaceutically acceptable salt, stereoisomer, tautomer, hydrate or solvate thereof for use according to any one of the preceding embodiments, wherein the disease or disorder is selected from the group consisting of psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

**Embodiment 82.** The compound, or a pharmaceutically acceptable salt, stereoisomer, tautomer, hydrate or solvate thereof for use according to any one of the preceding embodiments, wherein the disease or disorder is selected from the group consisting of rosacea, psoriasis, urticaria, atopic dermatitis, inflammatory bowel disease, allergic rhinitis and asthma.

**Embodiment 83.** Use of the compound as defined in any one of the preceding embodiments 1-78, or a pharmaceutically acceptable salt, stereoisomer, tautomer, hydrate or solvate thereof in the manufacture of a medicament for use in the treatment or prevention of a disease or disorder mediated by MRGPRX2.

**Embodiment 84.** Use of the compound as defined in any one of the preceding embodiments 1-78, or a pharmaceutically acceptable salt, stereoisomer, tautomer, hydrate or solvate thereof in the manufacture of a medicament for use in the treatment or prevention of a disease or disorder caused by mast cell degranulation.

**Embodiment 85.** Use of the compound as defined in any one of the preceding embodiments 1-78, or a pharmaceutically acceptable salt, stereoisomer, tautomer, hydrate or solvate thereof in the manufacture of a medicament for use in the treatment or prevention of autoimmune diseases, allergic diseases, inflammatory diseases, pruritus, and pain, such as inflammatory skin diseases.

**Embodiment 86.** Use of the compound as defined in any one of the preceding embodiments 1-78, or a pharmaceutically acceptable salt, stereoisomer, tautomer, hydrate or solvate thereof in the manufacture of a medicament for use in the treatment or prevention of psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

**Embodiment 87.** Use of the compound as defined in any one of the preceding embodiments 1-78, or a pharmaceutically acceptable salt, stereoisomer, tautomer, hydrate or solvate thereof in the manufacture of a medicament for use in the treatment or prevention of rosacea, psoriasis, urticaria, atopic dermatitis, inflammatory bowel disease, allergic rhinitis and asthma.

**Embodiment 88.** A method for treating or preventing a disease or disorder in a subject, comprising administering to a subject in need thereof an effective amount of the compound as defined in any one of the preceding embodiments 1-78, or a pharmaceutically acceptable salt, stereoisomer, tautomer, hydrate, or solvate thereof, wherein the disease or disorder is a disease or disorder mediated by MRGPRX2.

**Embodiment 89.** A method for treating or preventing a disease or disorder in a subject, comprising administering to a subject in need thereof an effective amount of the compound as defined in any one of the preceding embodiments 1-78, or a pharmaceutically acceptable salt, stereoisomer, tautomer, hydrate, or solvate thereof, wherein the disease or disorder is a disease or disorder caused by mast cell degranulation.

**Embodiment 90.** A method for treating or preventing a disease or disorder in a subject, comprising administering to a subject in need thereof an effective amount of the compound as defined in any one of the preceding embodiments 1-78, or a pharmaceutically acceptable salt, stereoisomer, tautomer, hydrate, or solvate thereof, wherein the disease or disorder is selected from the group consisting of autoimmune diseases, allergic diseases, inflammatory diseases, pruritus, and pain, such as inflammatory skin diseases.

**Embodiment 91.** A method for treating or preventing a disease or disorder in a subject, comprising administering to a subject in need thereof an effective amount of the compound as defined in any one of the preceding embodiments 1-78, or a pharmaceutically acceptable salt, stereoisomer, tautomer, hydrate, or solvate thereof, wherein the disease or disorder is selected from the group consisting of psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

**Embodiment 92.** A method for treating or preventing a disease or disorder in a subject, comprising administering to a subject in need thereof an effective amount of the compound as defined in any one of the preceding embodiments 1-78, or a pharmaceutically acceptable salt, stereoisomer, tautomer, hydrate, or solvate thereof, wherein the disease or disorder is selected from the group consisting of rosacea, psoriasis, urticaria, atopic dermatitis, inflammatory bowel disease, allergic rhinitis and asthma.

**Embodiment 93.** Use of the compound as defined in any one of the preceding embodiments 1-78, or a pharmaceutically acceptable salt, stereoisomer, tautomer, hydrate or solvate thereof, or a pharmaceutically acceptable salt, stereoisomer, tautomer, hydrate, or solvate thereof for the treatment or prevention of a disease or disorder mediated by MRGPRX2.

**Embodiment 94.** Use of the compound as defined in any one of the preceding embodiments 1-78, or a pharmaceutically acceptable salt, stereoisomer, tautomer, hydrate or solvate thereof, or a pharmaceutically acceptable salt, stereoisomer, tautomer, hydrate, or solvate thereof for the treatment or prevention of a disease or disorder caused by mast cell degranulation.

**Embodiment 95.** Use of the compound as defined in any one of the preceding embodiments 1-78, or a pharmaceutically acceptable salt, stereoisomer, tautomer, hydrate or solvate thereof, or a pharmaceutically acceptable salt, stereoisomer, tautomer, hydrate, or solvate thereof for the treatment or prevention of autoimmune diseases, allergic diseases, inflammatory diseases, pruritus, and pain, such as inflammatory skin diseases.

**Embodiment 96.** Use of the compound as defined in any one of the preceding embodiments 1-78, or a pharmaceutically acceptable salt, stereoisomer, tautomer, hydrate or solvate thereof, or a pharmaceutically acceptable salt, stereoisomer, tautomer, hydrate, or solvate thereof for the treatment or prevention of psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

**Embodiment 97.** Use of the compound as defined in any one of the preceding embodiments 1-78, or a pharmaceutically acceptable salt, stereoisomer, tautomer, hydrate or solvate thereof, or a pharmaceutically acceptable salt, stereoisomer, tautomer, hydrate, or solvate thereof for the treatment or prevention of rosacea, psoriasis, urticaria, atopic dermatitis, inflammatory bowel disease, allergic rhinitis and asthma.

**Embodiment 98.** A pharmaceutical composition for the treatment or prevention of a disease or disorder mediated by MRGPRX2, comprising the compound as defined in any one of the preceding embodiments 1-78, or a pharmaceutically acceptable salt, stereoisomer, tautomer, hydrate, or solvate thereof.

**Embodiment 99.** A pharmaceutical composition for the treatment or prevention of a disease or disorder caused by mast cell degranulation, comprising the compound as defined in any one of the preceding embodiments 1-78, or a pharmaceutically acceptable salt, stereoisomer, tautomer, hydrate, or solvate thereof.

**Embodiment 100.** A method for antagonizing MRGPRX2 *in vitro,* comprising utilizing an effective amount of the compound as defined in any one of the preceding embodiments 1-78, or a pharmaceutically acceptable salt, stereoisomer, tautomer, hydrate, or solvate thereof in an *in vitro* assay for MRGPRX2.

### Embodiments of the Present Disclosure - Part B

**Embodiment 1.** A compound of formula (IB), or a pharmaceutically acceptable salt, stereoisomer, tautomer, hydrate, or solvate thereof, 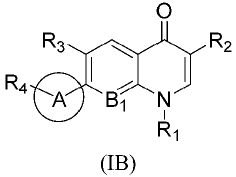 wherein:
R₁ is -C₃₋₆ cycloalkyl or -C₁₋₆ linear or branched alkyl;
R₂ is -COOH or -COOC₁₋₆ linear or branched alkyl;
R₃ is halogen;
B₁ is -CR₆ or N, wherein R₆ is H, halogen or -O-C₁₋₆ linear or branched alkyl; and
A is phenyl; and R₄ is selected from the group consisting of -S(O)₂-NH-C₈₋₁₂ linear or branched alkyl, -S(O)₂-NH-C₁₋₆ linear or branched alkyl-CF₃, -S(O)₂-NH-cyclohexyl, -S(O)₂-NR_{b}R_{c}, -S(O)-NH-C₁₋₆ linear or branched alkyl-CF₃, -S-NH-C₁₋₆ linear or branched alkyl-CF₃, -NH-S(O)₂-C₁₋₆ linear or branched alkyl, -C(O)-NH₂, -C(O)-NH-C₂₋₆ linear or branched alkyl, -C(O)-NR_{b}R_{c}, -NH-C(O)-CF₃, -NH-C(O)-CH₂Cl, -NH-C₁₋₆ linear or branched alkyl, -NH-C₁₋₆ linear or branched alkenyl and -NH-C₁₋₆ linear or branched alkynyl; wherein R_{b} and R_{c}, together with the N atom to which they are attached, form an azetidinyl or difluoroazetidinyl group;
or, A is pyridyl; and R₄ is selected from the group consisting of -S(O)₂-NH₂, -S(O)₂-NH-CH₂CH₂OH, -S(O)₂-NH-CH₂CH₂Cl, -S(O)₂-NH-C₄₋₆ linear or branched alkyl, -S(O)₂-NH-C₈₋₁₂ linear or branched alkyl, -S(O)₂-NH-cyclohexyl, -S(O)₂-NH-tetrahydropyranyl, -S(O)₂-NR_{b}R_{c}, -S(O)-NH-C₁₋₆ linear or branched alkyl, -S-NH-C₁₋₆ linear or branched alkyl-CF₃, -NH-S(O)₂-C₂₋₆ linear or branched alkyl, -C(O)-NH-C₃₋₆ branched alkyl, -C(O)-NR_{b}R_{c}, -NH-C(O)-CF₂, -NH-C(O)-CH₂CF₃, -NH-C₁₋₆ linear or branched alkyl and -S-C₁₋₆ linear or branched hydroxyalkyl; wherein R_{b} and R_{c}, together with the N atom to which they are attached, form an azetidinyl, difluoroazetidinyl, pyrrolidinyl, piperidinyl, piperazinyl or morpholinyl group.

**Embodiment 2.** The compound according to any one of the preceding embodiments, or a pharmaceutically acceptable salt, stereoisomer, tautomer, hydrate, or solvate thereof, wherein R₁ is -C₃₋₄ cycloalkyl or -C₁₋₃ linear or branched alkyl.

**Embodiment 3.** The compound according to any one of the preceding embodiments, or a pharmaceutically acceptable salt, stereoisomer, tautomer, hydrate, or solvate thereof, wherein R₁ is 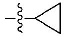 or -C₁₋₃ linear or branched alkyl.

**Embodiment 4.** The compound according to any one of the preceding embodiments, or a pharmaceutically acceptable salt, stereoisomer, tautomer, hydrate, or solvate thereof, wherein R₁ is 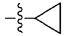 or

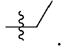

**Embodiment 5.** The compound according to any one of the preceding embodiments, or a pharmaceutically acceptable salt, stereoisomer, tautomer, hydrate, or solvate thereof, wherein R₁ is

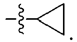

**Embodiment 6.** The compound according to any one of the preceding embodiments, or a pharmaceutically acceptable salt, stereoisomer, tautomer, hydrate, or solvate thereof, wherein R₂ is -COOH or -COOC₁₋₃ linear or branched alkyl.

**Embodiment 7.** The compound according to any one of the preceding embodiments 1-7, or a pharmaceutically acceptable salt, stereoisomer, tautomer, hydrate, or solvate thereof, wherein R₂ is -COOH or -COOCH₃.

**Embodiment 8.** The compound according to any one of the preceding embodiments 1-7, or a pharmaceutically acceptable salt, stereoisomer, tautomer, hydrate, or solvate thereof, wherein R₂ is -COOCH₃ or -COOCH₂CH₃.

**Embodiment 9.** The compound according to any one of the preceding embodiments 1-7, or a pharmaceutically acceptable salt, stereoisomer, tautomer, hydrate, or solvate thereof, wherein R₂ is -COOH.

**Embodiment 10.** The compound according to any one of the preceding embodiments 1-7, or a pharmaceutically acceptable salt, stereoisomer, tautomer, hydrate, or solvate thereof, wherein R₂ is -COOCH₃.

**Embodiment 11.** The compound according to any one of the preceding embodiments, or a pharmaceutically acceptable salt, stereoisomer, tautomer, hydrate, or solvate thereof, wherein R₃ is F.

**Embodiment 12.** The compound according to any one of the preceding embodiments, or a pharmaceutically acceptable salt, stereoisomer, tautomer, hydrate, or solvate thereof, wherein B₁ is -CR₆ or N, wherein R₆ is H, halogen or -O-C₁₋₃ linear or branched alkyl.

**Embodiment 13.** The compound according to any one of the preceding embodiments, or a pharmaceutically acceptable salt, stereoisomer, tautomer, hydrate, or solvate thereof, wherein B₁ is -CR₆ or N, wherein R₆ is H, F, Cl or -OCH₃.

**Embodiment 14.** The compound according to any one of the preceding embodiments, or a pharmaceutically acceptable salt, stereoisomer, tautomer, hydrate, or solvate thereof, wherein B₁ is -CR₆, wherein R₆ is H or -OCH₃.

**Embodiment 15.** The compound according to any one of the preceding embodiments, or a pharmaceutically acceptable salt, stereoisomer, tautomer, hydrate, or solvate thereof, wherein B₁ is -CR₆, wherein R₆ is H.

**Embodiment 16.** The compound according to any one of the preceding embodiments, or a pharmaceutically acceptable salt, stereoisomer, tautomer, hydrate, or solvate thereof, wherein B₁ is -CR₆, wherein R₆ is -OCH₃.

**Embodiment 17.** The compound according to any one of the preceding embodiments, or a pharmaceutically acceptable salt, stereoisomer, tautomer, hydrate, or solvate thereof, wherein B₁ is N.

**Embodiment 18.** The compound according to any one of the preceding embodiments, or a pharmaceutically acceptable salt, stereoisomer, tautomer, hydrate, or solvate thereof, wherein, A is phenyl; and R₄ is selected from the group consisting of -S(O)₂-NH-C₈₋₁₂ linear or branched alkyl, -S(O)₂-NH-C₁₋₆ linear or branched alkyl-CF₃, -S(O)₂-NH-cyclohexyl, -S(O)₂-NR_{b}R_{c}, -S(O)-NH-C₁₋₆ linear or branched alkyl-CF₃, -S-NH-C₁₋₆ linear or branched alkyl-CF₃, -NH-S(O)₂-C₁₋₆ linear or branched alkyl, -C(O)-NH₂, -C(O)-NH-C₂₋₆ linear or branched alkyl, -NH-C(O)-CF₃, -NH-C(O)-CH₂Cl, -NH-C₁₋₆ linear or branched alkenyl and -NH-C₁₋₆ linear or branched alkynyl; wherein R_{b} and R_{c}, together with the N atom to which they are attached, form an azetidinyl or difluoroazetidinyl group.

**Embodiment 19.** The compound according to any one of the preceding embodiments, or a pharmaceutically acceptable salt, stereoisomer, tautomer, hydrate, or solvate thereof, wherein, A is phenyl; and R₄ is selected from the group consisting of -S(O)₂-NH-C₈₋₁₂ linear or branched alkyl, -S(O)₂-NH-C₁₋₆ linear or branched alkyl-CF₃, -S(O)₂-NH-cyclohexyl, -S(O)₂-NR_{b}R_{c}, -NH-S(O)₂-C₁₋₆ linear or branched alkyl, -C(O)-NH₂, -C(O)-NH-C₂₋₆ linear or branched alkyl, -NH-C(O)-CF₃, -NH-C₁₋₆ linear or branched alkyl and -NH-C₁₋₆ linear or branched alkenyl; wherein R_{b} and R_{c}, together with the N atom to which they are attached, form an azetidinyl or difluoroazetidinyl group.

**Embodiment 20.** The compound according to any one of the preceding embodiments, or a pharmaceutically acceptable salt, stereoisomer, tautomer, hydrate, or solvate thereof, wherein, A is phenyl; and R₄ is selected from the group consisting of -S(O)₂-NH-C₈₋₁₀ linear alkyl, -S(O)₂-NH-C₂₋₄ linear alkyl-CF₃, -S(O)₂-NH-cyclohexyl, -S(O)₂-NR_{b}R_{c}, -NH-S(O)₂-C₂₋₄ linear alkyl, -C(O)-NH₂, -C(O)-NH-C₂₋₆ linear or branched alkyl, -NH-C(O)-CF₃, -NH-C₂₋₄ linear alkyl and -NH-C₂₋₄ linear alkenyl; wherein R_{b} and R_{c}, together with the N atom to which they are attached, form an azetidinyl or difluoroazetidinyl group.

**Embodiment 21.** The compound according to any one of the preceding embodiments 1-18, or a pharmaceutically acceptable salt, stereoisomer, tautomer, hydrate, or solvate thereof, wherein, A is phenyl; and R₄ is selected from the group consisting of -S(O)₂-NH-C₈₋₁₂ linear or branched alkyl, -S(O)₂-NH-C₁₋₆ linear or branched alkyl-CF₃, -S(O)₂-NH-cyclohexyl, -S(O)₂-NR_{b}R_{c}, -C(O)-NH₂, -C(O)-NH-C₂₋₆ linear or branched alkyl, -NH-C(O)-CF₃ and -NH-C₁₋₆ linear or branched alkenyl; wherein R_{b} and R_{c}, together with the N atom to which they are attached, form a difluoroazetidinyl group.

**Embodiment 22.** The compound according to any one of the preceding embodiments 1-18, or a pharmaceutically acceptable salt, stereoisomer, tautomer, hydrate, or solvate thereof, wherein, A is phenyl; and R₄ is selected from the group consisting of -S(O)₂-NH-C₈₋₁₂ linear alkyl, -S(O)₂-NH-C₁₋₆ linear alkyl-CF₃, -S(O)₂-NR_{b}R_{c}, -C(O)-NH-C₂₋₆ linear or branched alkyl, -NH-C(O)-CF₃ and -NH-C₁₋₆ linear alkenyl; wherein R_{b} and R_{c}, together with the N atom to which they are attached, form a difluoroazetidinyl group.

**Embodiment 23.** The compound according to any one of the preceding embodiments 1-18, or a pharmaceutically acceptable salt, stereoisomer, tautomer, hydrate, or solvate thereof, wherein, A is phenyl; and R₄ is selected from the group consisting of -S(O)₂-NH-C₈₋₁₀ linear alkyl, -S(O)₂-NH-C₂₋₄ linear alkyl-CF₃, -S(O)₂-NR_{b}R_{c}, -C(O)-NH-C₂₋₆ linear or branched alkyl, -NH-C(O)-CF₃ and -NH-C₂₋₄ linear alkenyl; wherein R_{b} and R_{c}, together with the N atom to which they are attached, form a difluoroazetidinyl group.

**Embodiment 24.** The compound according to any one of the preceding embodiments 1-18, or a pharmaceutically acceptable salt, stereoisomer, tautomer, hydrate, or solvate thereof, wherein, A is phenyl; and R₄ is selected from the group consisting of -S(O)₂-NH-n-nonyl, -S(O)₂-NH-n-propyl-CF₃, -S(O)₂-NR_{b}R_{c}, -C(O)-NH-n-propyl, -C(O)-NH-2,2-dimethylpropyl, -NH-C(O)-CF₃ and -NH-allyl; wherein R_{b} and R_{c}, together with the N atom to which they are attached, form a difluoroazetidinyl group.

**Embodiment 25.** The compound according to any one of the preceding embodiments 1-18, or a pharmaceutically acceptable salt, stereoisomer, tautomer, hydrate, or solvate thereof, wherein, A is phenyl; and R₄ is -S(O)₂-NH-C₈₋₁₂ linear or branched alkyl, or -S(O)₂-NH-C₁₋₆ linear or branched alkyl-CF₃.

**Embodiment 26.** The compound according to any one of the preceding embodiments, or a pharmaceutically acceptable salt, stereoisomer, tautomer, hydrate, or solvate thereof, wherein, A is pyridyl; and R₄ is selected from the group consisting of -S(O)₂-NH₂, -S(O)₂-NH-CH₂CH₂OH, -S(O)₂-NH-CH₂CH₂Cl, -S(O)₂-NH-C₄₋₆ linear or branched alkyl, -S(O)₂-NH-C₈₋₁₂ linear or branched alkyl, -S(O)₂-NH-cyclohexyl, -S(O)₂-NH-tetrahydropyranyl, -S(O)₂-NR_{b}R_{c}, -NH-S(O)₂-C₂₋₆ linear or branched alkyl, -C(O)-NH-C₃₋₆ branched alkyl, -NH-C(O)-CF₂, -NH-C(O)-CH₂CF₃, -NH-C₁₋₆ linear or branched alkyl and -S-C₁₋₆ linear or branched hydroxyalkyl; wherein R_{b} and R_{c}, together with the N atom to which they are attached, form an azetidinyl, difluoroazetidinyl, pyrrolidinyl, piperidinyl, piperazinyl or morpholinyl group.

**Embodiment 27.** The compound according to any one of the preceding embodiments, or a pharmaceutically acceptable salt, stereoisomer, tautomer, hydrate, or solvate thereof, wherein, A is pyridyl; and R₄ is selected from the group consisting of -S(O)₂-NH₂, -S(O)₂-NH-CH₂CH₂OH, -S(O)₂-NH-CH₂CH₂Cl, -S(O)₂-NH-C₄₋₆ linear or branched alkyl, -S(O)₂-NH-C₈₋₁₂ linear or branched alkyl, -S(O)₂-NH-cyclohexyl, -S(O)₂-NH-tetrahydropyranyl, -S(O)₂-NR_{b}R_{c}, -NH-S(O)₂-C₂₋₆ linear or branched alkyl, -C(O)-NH-C₃₋₆ branched alkyl, -NH-C(O)-CF₂, -NH-C(O)-CH₂CF₃ and -S-C₁₋₆ linear or branched hydroxyalkyl; wherein R_{b} and R_{c}, together with the N atom to which they are attached, form an azetidinyl, difluoroazetidinyl, pyrrolidinyl, piperidinyl, piperazinyl or morpholinyl group.

**Embodiment 28.** The compound according to any one of the preceding embodiments, or a pharmaceutically acceptable salt, stereoisomer, tautomer, hydrate, or solvate thereof, wherein, A is pyridyl; and R₄ is selected from the group consisting of -S(O)₂-NH₂, -S(O)₂-NH-CH₂CH₂OH, -S(O)₂-NH-CH₂CH₂Cl, -S(O)₂-NH-C₄₋₆ linear alkyl, -S(O)₂-NH-C₈₋₁₀ linear alkyl, -S(O)₂-NH-cyclohexyl, -S(O)₂-NH-tetrahydropyranyl, -S(O)₂-NR_{b}R_{c}, -NH-S(O)₂-C₂₋₄ linear alkyl, -C(O)-NH-C₃₋₆ branched alkyl, -NH-C(O)-CF₂, -NH-C(O)-CH₂CF₃ and -S-C₂₋₄ hydroxy linear alkyl; wherein R_{b} and R_{c}, together with the N atom to which they are attached, form an azetidinyl, difluoroazetidinyl, pyrrolidinyl, piperidinyl, piperazinyl or morpholinyl group.

**Embodiment 29.** The compound according to any one of the preceding embodiments 1-26, or a pharmaceutically acceptable salt, stereoisomer, tautomer, hydrate, or solvate thereof, wherein, A is pyridyl; and R₄ is selected from the group consisting of -S(O)₂-NH₂, -S(O)₂-NH-C₄₋₆ linear or branched alkyl, -S(O)₂-NH-C₈₋₁₂ linear or branched alkyl, -S(O)₂-NH-cyclohexyl, -S(O)₂-NH-tetrahydropyranyl, -S(O)₂-NR_{b}R_{c}, -C(O)-NH-C₃₋₆ branched alkyl, -NH-C(O)-CF₂ and -NH-C(O)-CH₂CF₃; wherein R_{b} and R_{c}, together with the N atom to which they are attached, form an azetidinyl, difluoroazetidinyl, pyrrolidinyl, piperidinyl, piperazinyl or morpholinyl group.

**Embodiment 30.** The compound according to any one of the preceding embodiments 1-26, or a pharmaceutically acceptable salt, stereoisomer, tautomer, hydrate, or solvate thereof, wherein, A is pyridyl; and R₄ is selected from the group consisting of -S(O)₂-NH₂, -S(O)₂-NH-cyclohexyl, -S(O)₂-NH-tetrahydropyranyl, -S(O)₂-NR_{b}R_{c}, -NH-C(O)-CF₂ and -NH-C(O)-CH₂CF₃; wherein R_{b} and R_{c}, together with the N atom to which they are attached, form an azetidinyl, difluoroazetidinyl, pyrrolidinyl, piperidinyl, piperazinyl or morpholinyl group.

**Embodiment 31.** The compound according to any one of the preceding embodiments 1-26, or a pharmaceutically acceptable salt, stereoisomer, tautomer, hydrate, or solvate thereof, wherein, A is pyridyl; and R₄ is selected from the group consisting of -S(O)₂-NH₂, -S(O)₂-NH-cyclohexyl, -S(O)₂-NH-tetrahydropyranyl, -S(O)₂-NR_{b}R_{c} and -NH-C(O)-CF₂; wherein R_{b} and R_{c}, together with the N atom to which they are attached, form an azetidinyl, difluoroazetidinyl, pyrrolidinyl or piperidinyl group.

**Embodiment 32.** The compound according to any one of the preceding embodiments, or a pharmaceutically acceptable salt, stereoisomer, tautomer, hydrate, or solvate thereof, wherein,
R₁ is 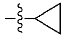 or 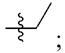 for example 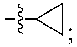
R₂ is -COOH or -COOC₁₋₃ linear or branched alkyl; for example -COOH or -COOCH₃;
R₃ is F;
B₁ is -CR₆ or N, wherein R₆ is H, halogen or -O-C₁₋₃ linear or branched alkyl, for example H, F, Cl or -O-CH₃; and
A is phenyl; and R₄ is selected from the group consisting of -S(O)₂-NH-C₈₋₁₀ linear alkyl, -S(O)₂-NH-C₂₋₄ linear alkyl-CF₃, -S(O)₂-NR_{b}R_{c}, -C(O)-NH-C₂₋₆ linear or branched alkyl, -NH-C(O)-CF₃ and -NH-C₂₋₄ linear alkenyl; wherein R_{b} and R_{c}, together with the N atom to which they are attached, form a difluoroazetidinyl group;
or, A is pyridyl; and R₄ is selected from the group consisting of -S(O)₂-NH₂, -S(O)₂-NH-cyclohexyl, -S(O)₂-NH-tetrahydropyranyl, -S(O)₂-NR_{b}R_{c}, -NH-C(O)-CF₂ and -NH-C(O)-CH₂CF₃; wherein R_{b} and R_{c}, together with the N atom to which they are attached, form an azetidinyl, difluoroazetidinyl, pyrrolidinyl, piperidinyl, piperazinyl or morpholinyl group.

**Embodiment 33.** The compound according to any one of the preceding embodiments, or a pharmaceutically acceptable salt, stereoisomer, tautomer, hydrate, or solvate thereof, wherein the compound is selected from the group consisting of Examples 9, 10, 13, 14, 38, 39, 40, 41, 46, 47, 51, 52, 60, 61, 62, 68, 69, 73, 95, 96, 98, 99, 100, 159 and 160.

**Embodiment 34.** The compound according to any one of the preceding embodiments, or a pharmaceutically acceptable salt, stereoisomer, tautomer, hydrate, or solvate thereof, wherein the compound is selected from the group consisting of Examples 12, 48, 49, 50, 79, 80, 81, 82, 87, 88, 89, 101, 102, 103, 104, 105, 106, 107, 108, 110, 113, 114, 119, 120, 121, 122, 123, 124, 126, 130, 167 and 168.

**Embodiment 35.** The compound according to any one of the preceding embodiments, or a pharmaceutically acceptable salt, stereoisomer, tautomer, hydrate, or solvate thereof, wherein the compound is selected from the group consisting of Examples 136, 137, 138, 139, 140, 141, 142, 143, 145, 146, 147, 148, 149, 150, 151, 152, 170, 171, 172 and 173.

**Embodiment 36.** A compound, which is selected from the group consisting of:

| **No.** | **Structure** |
|---|---|
| **7** | 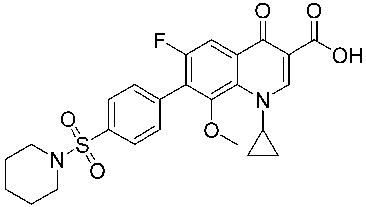 |
| **9** | 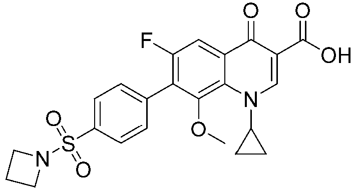 |
| **10** | 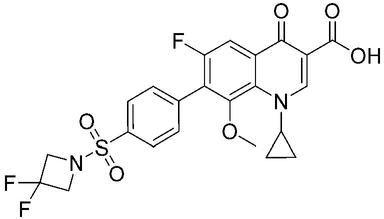 |
| **11** | 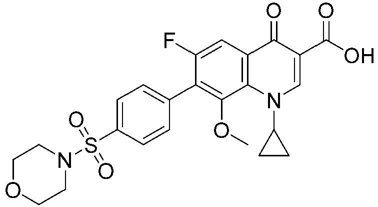 |
| **12** | 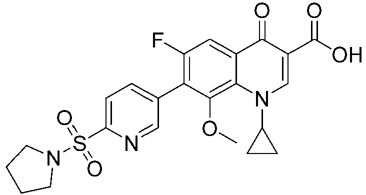 |
| **13** | 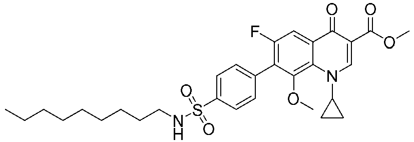 |
| **14** | 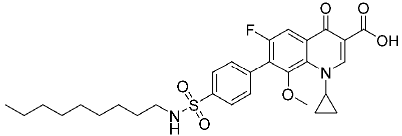 |
| **15** | 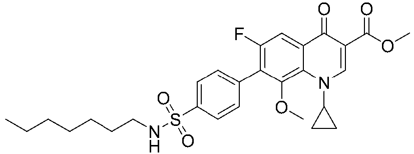 |
| **17** | 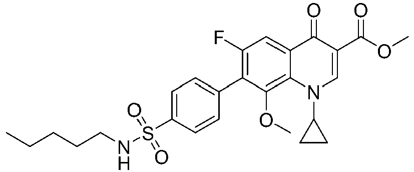 |
| **19** | 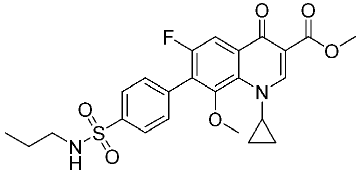 |
| **21** | 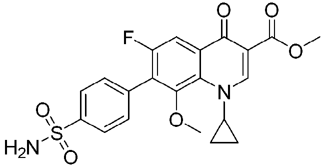 |
| **22** | 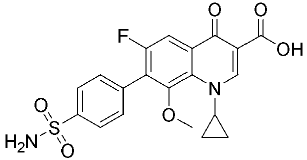 |
| **23** | 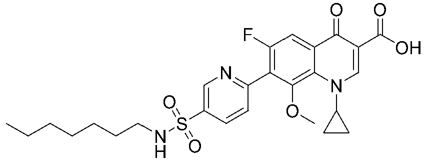 |
| **24** | 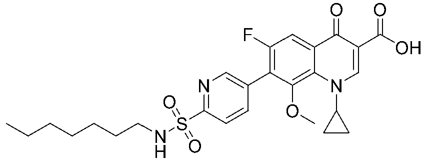 |
| **25** | 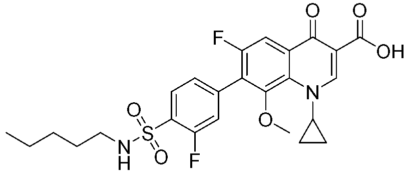 |
| **26** | 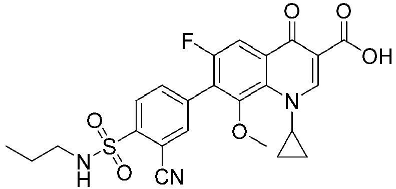 |
| **27** | 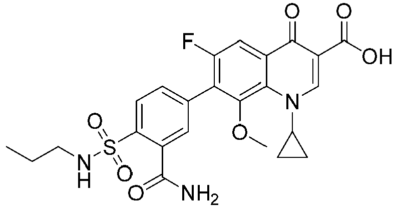 |
| **33** | 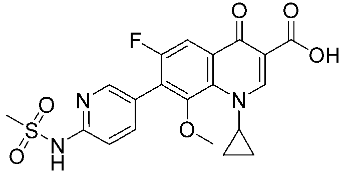 |
| **34** | 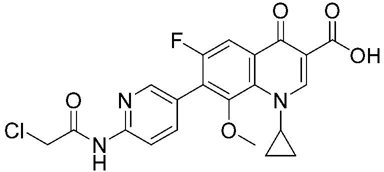 |
| **38** | 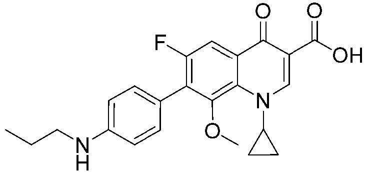 |
| **39** | 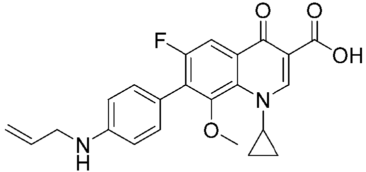 |
| **40** | 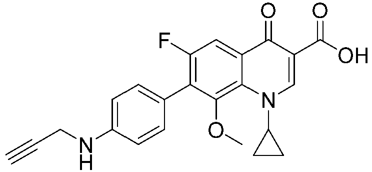 |
| **41** | 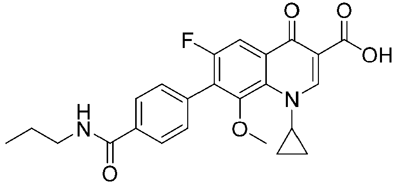 |
| **42** | 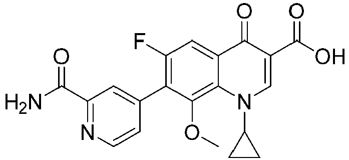 |
| **43** | 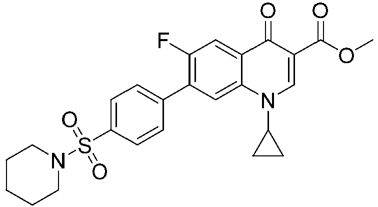 |
| **44** | 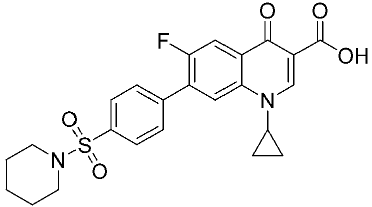 |
| **45** | 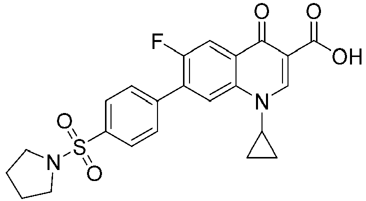 |
| **46** | 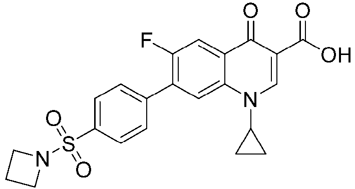 |
| **47** | 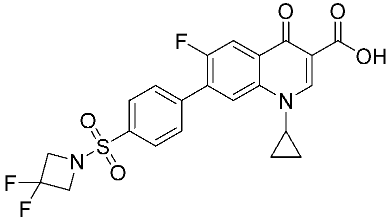 |
| **48** | 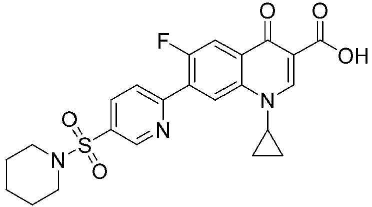 |
| **49** | 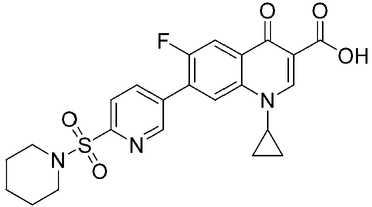 |
| **50** | 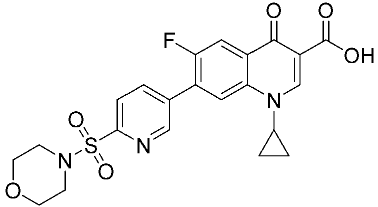 |
| **51** | 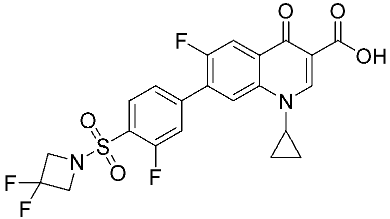 |
| **52** | 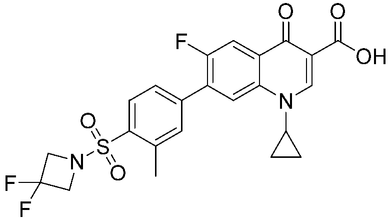 |
| **53** | 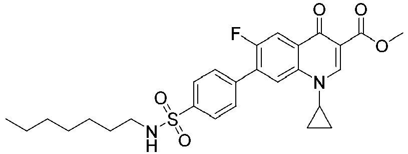 |
| **55** | 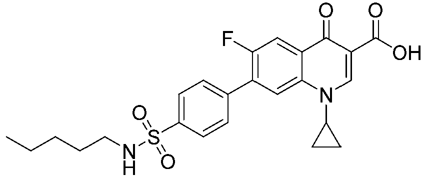 |
| **56** | 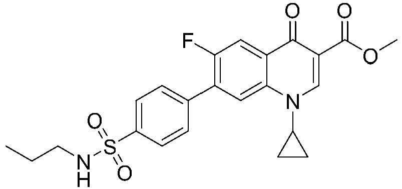 |
| **57** | 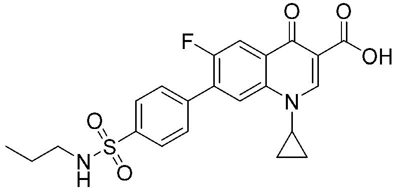 |
| **58** | 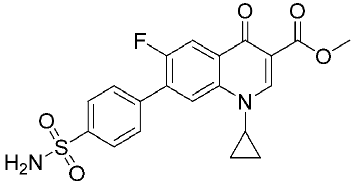 |
| **59** | 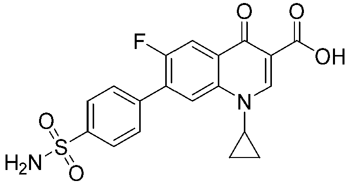 |
| **60** | 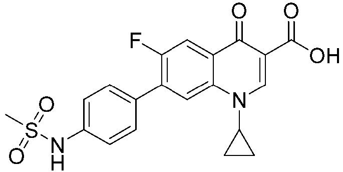 |
| **61** | 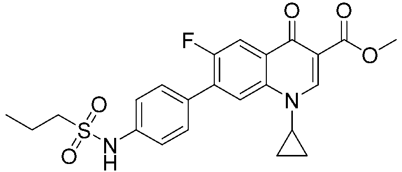 |
| **62** | 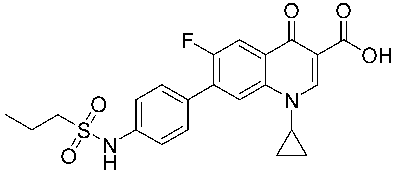 |
| **66** | 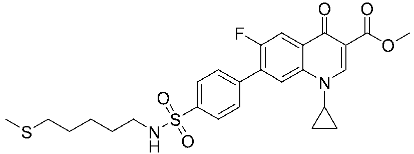 |
| **68** | 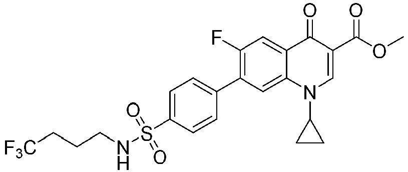 |
| **69** | 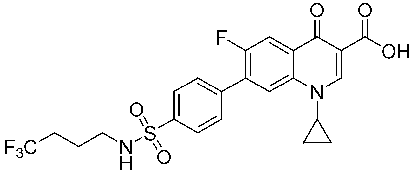 |
| **70** | 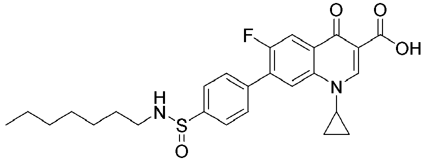 |
| **71** | 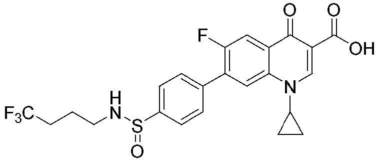 |
| **72** | 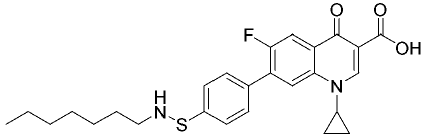 |
| **73** | 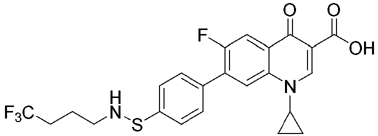 |
| **74** | 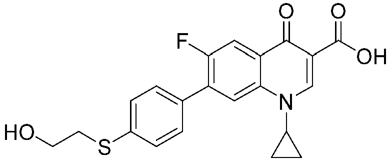 |
| **75** | 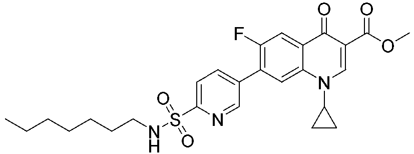 |
| **77** | 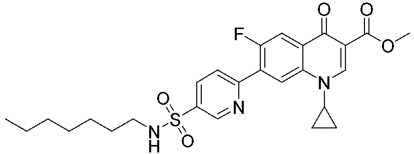 |
| **78** | 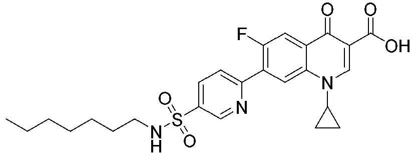 |
| **79** | 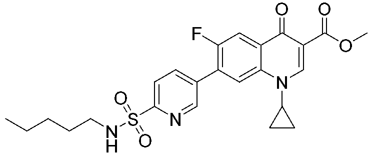 |
| **80** | 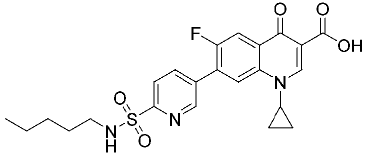 |
| **81** | 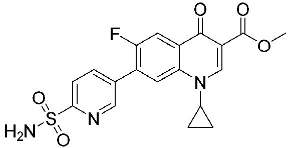 |
| **82** | 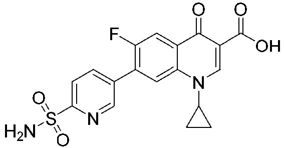 |
| **83** | 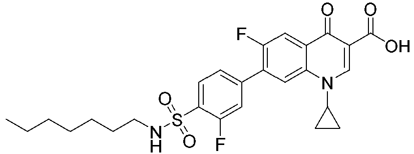 |
| **84** | 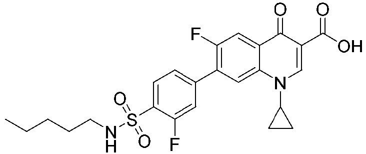 |
| **85** | 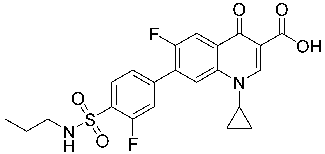 |
| **86** | 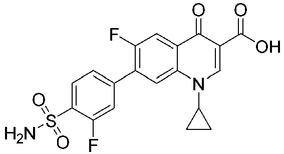 |
| **87** | 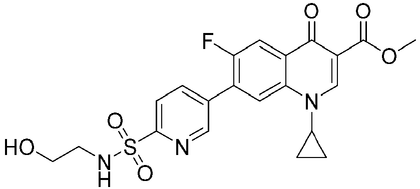 |
| **88** | 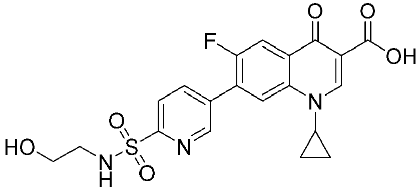 |
| **89** | 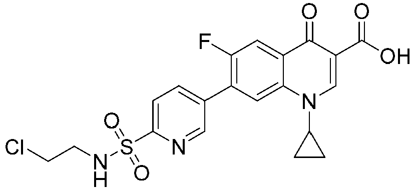 |
| **90** | 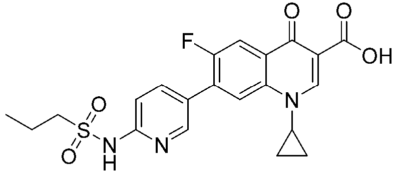 |
| **91** | 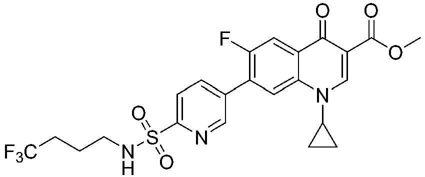 |
| **92** | 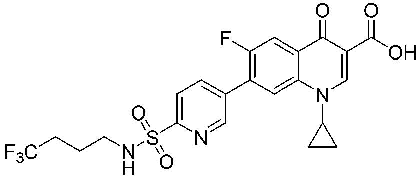 |
| **95** | 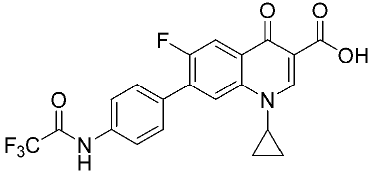 |
| **96** | 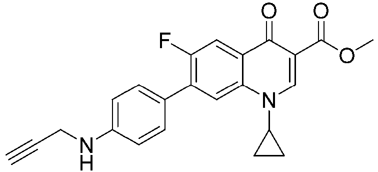 |
| **98** | 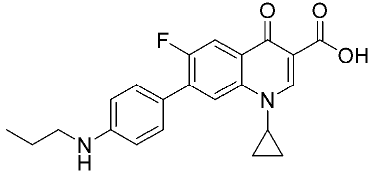 |
| **99** | 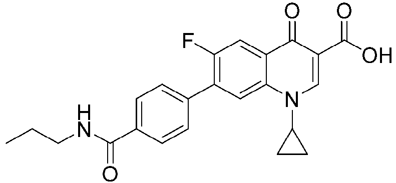 |
| **100** | 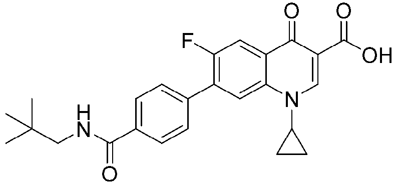 |
| **101** | 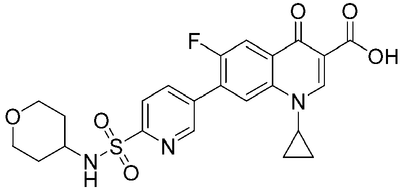 |
| **102** | 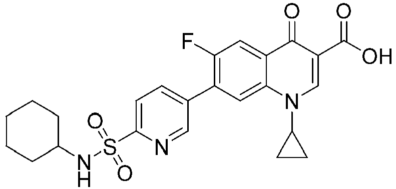 |
| **103** | 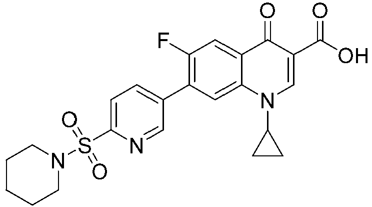 |
| **104** | 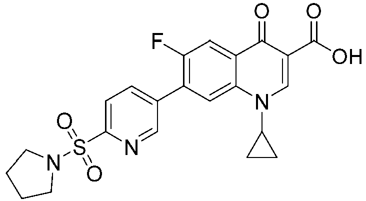 |
| **105** | 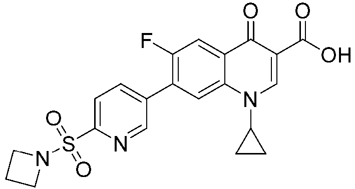 |
| **106** | 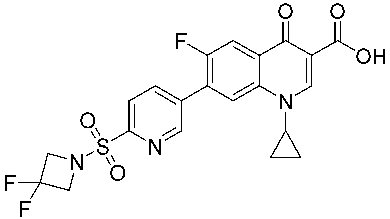 |
| **107** | 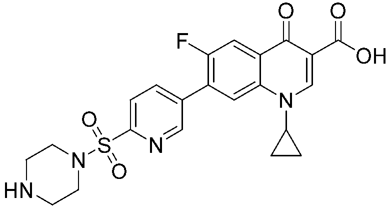 |
| **108** | 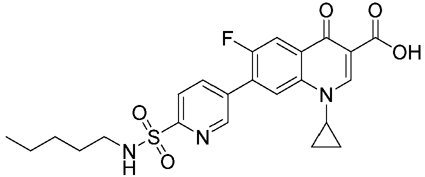 |
| **109** | 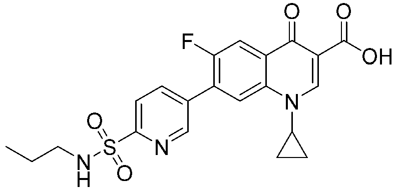 |
| **110** | 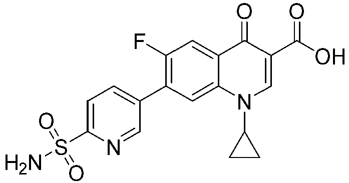 |
| **111** | 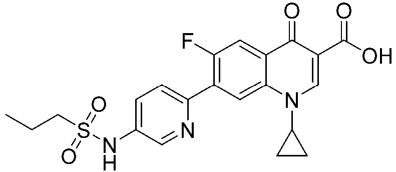 |
| **112** | 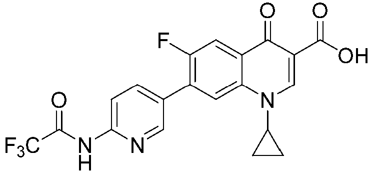 |
| **113** | 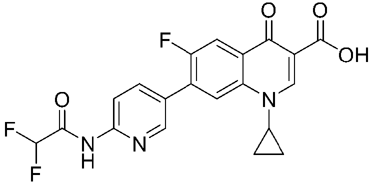 |
| **114** | 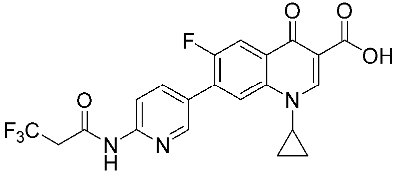 |
| **117** | 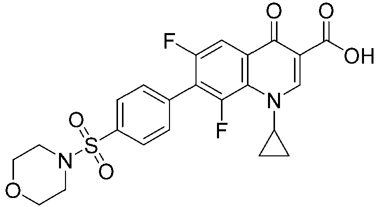 |
| **118** | 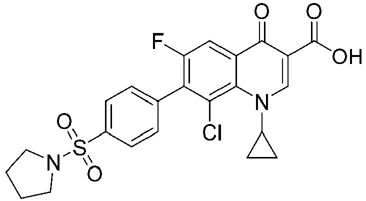 |
| **119** | 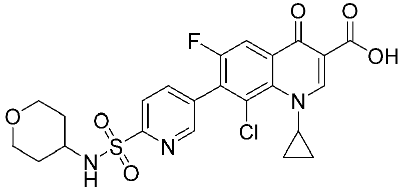 |
| **120** | 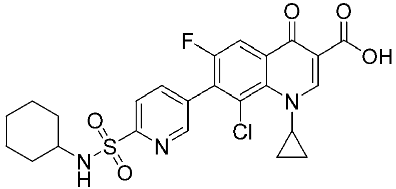 |
| **121** | 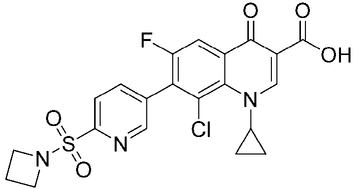 |
| **122** | 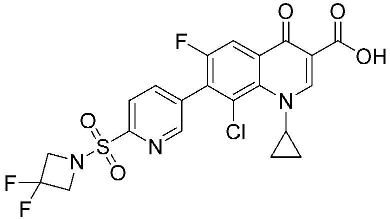 |
| **123** | 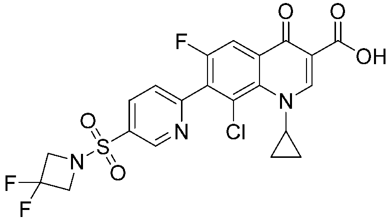 |
| **124** | 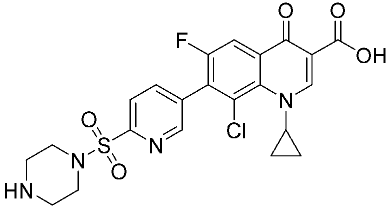 |
| **125** | 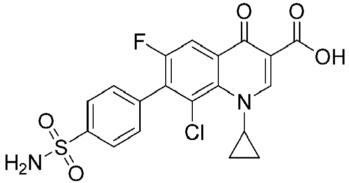 |
| **126** | 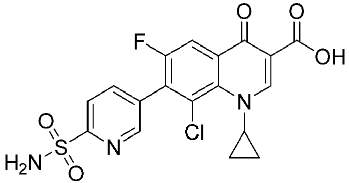 |
| **128** | 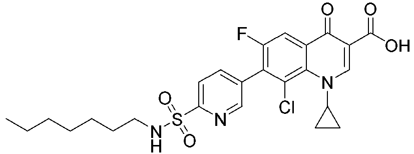 |
| **129** | 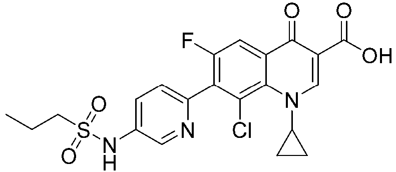 |
| **130** | 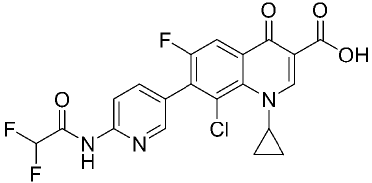 |
| **131** | 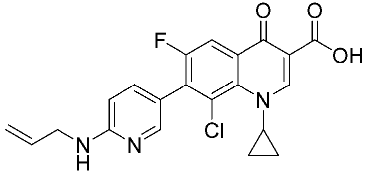 |
| **133** | 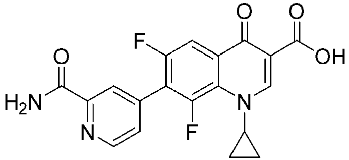 |
| **134** | 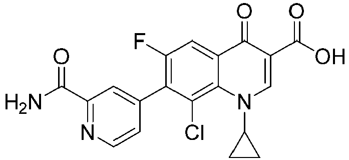 |
| **135** | 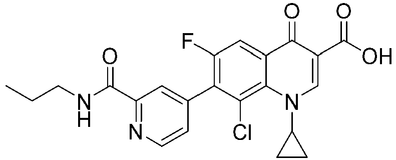 |
| **136** | 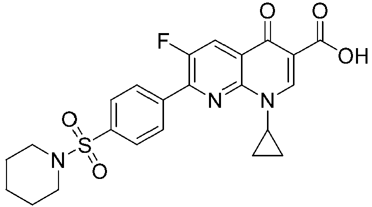 |
| **137** | 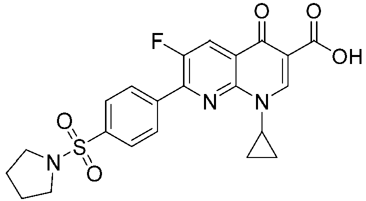 |
| **138** | 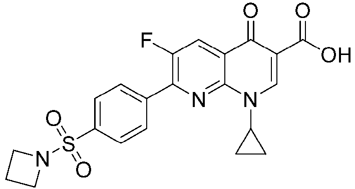 |
| **139** | 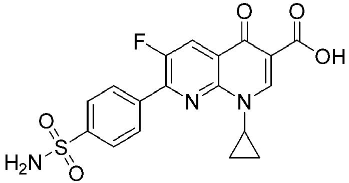 |
| **140** | 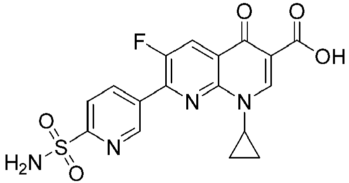 |
| **141** | 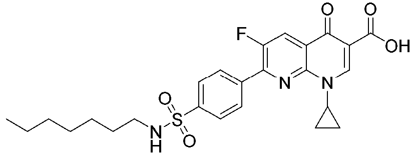 |
| **142** | 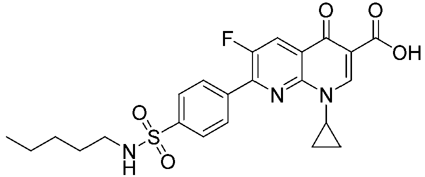 |
| **143** | 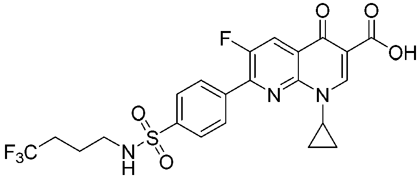 |
| **145** | 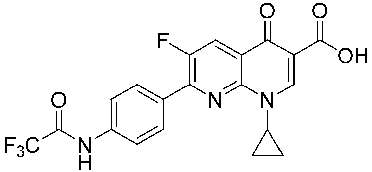 |
| **146** | 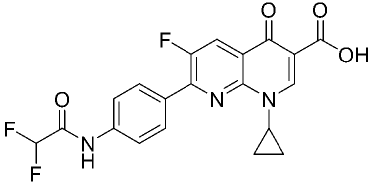 |
| **147** | 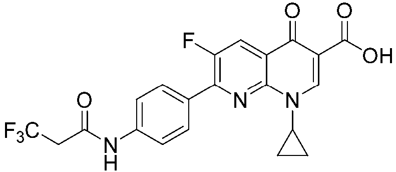 |
| **148** | 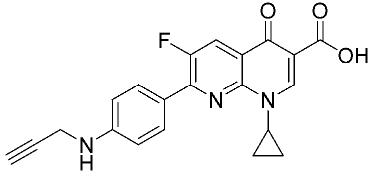 |
| **149** | 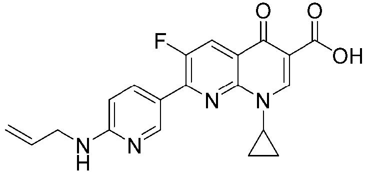 |
| **150** | 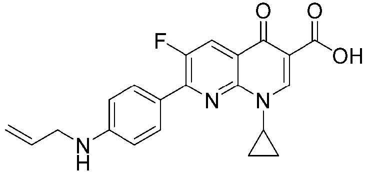 |
| **151** | 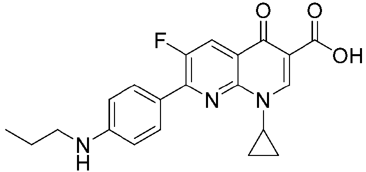 |
| **152** | 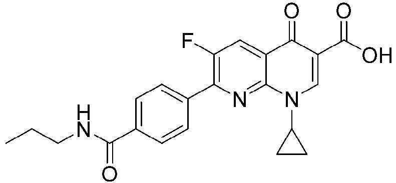 |
| **154** | 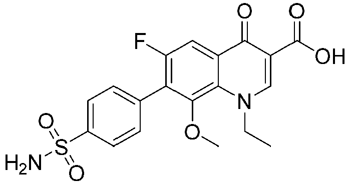 |
| **155** | 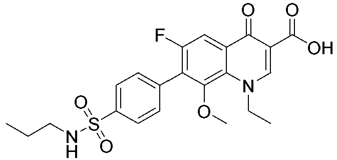 |
| **156** | 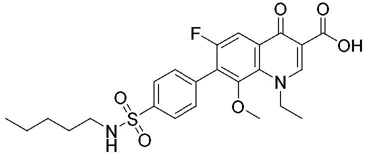 |
| **157** | 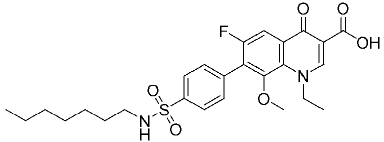 |
| **158** | 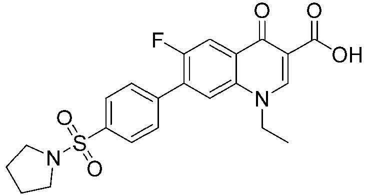 |
| **159** | 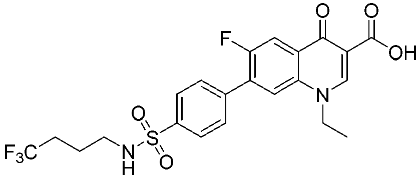 |
| **160** | 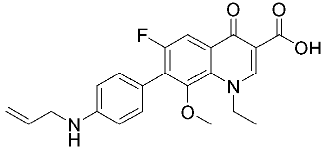 |
| **162** | 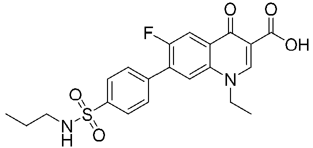 |
| **163** | 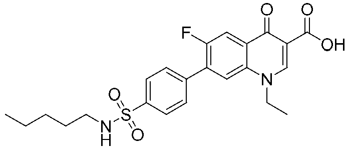 |
| **165** | 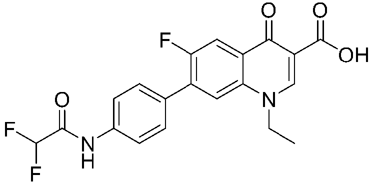 |
| **166** | 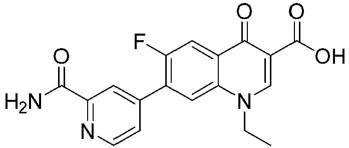 |
| **167** | 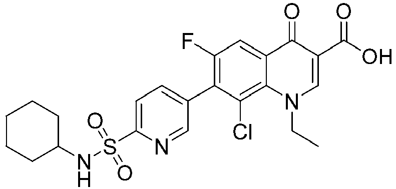 |
| **168** | 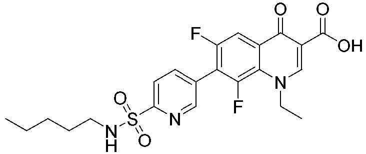 |
| **169** | 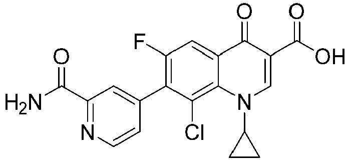 |
| **170** | 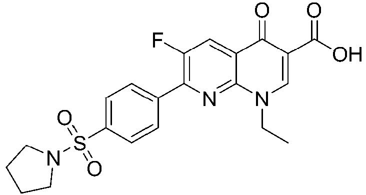 |
| **171** | 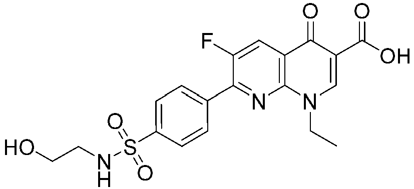 |
| **172** | 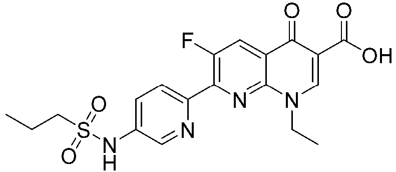 |
| **173** | 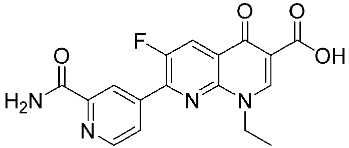 |

or a pharmaceutically acceptable salt, a stereoisomer, a tautomer, a hydrate, or a solvate thereof.

**Embodiment 37.** A pharmaceutical composition, comprising the compound according to any one of embodiments 1-36 or a pharmaceutically acceptable salt, a stereoisomer, a tautomer, a hydrate, or a solvate thereof, and a pharmaceutically acceptable excipient

**Embodiment 38.** The compound according to any one of embodiments 1-36 or a pharmaceutically acceptable salt, a stereoisomer, a tautomer, a hydrate, or a solvate thereof, for use as a medicament.

**Embodiment 39.** The compound according to any one of embodiments 1-36 or a pharmaceutically acceptable salt, a stereoisomer, a tautomer, a hydrate, or a solvate thereof, for use in the treatment or prevention of a disease or disorder mediated by MRGPRX2.

**Embodiment 40.** The compound according to any one of embodiments 1-36 or a pharmaceutically acceptable salt, a stereoisomer, a tautomer, a hydrate, or a solvate thereof, for use in the treatment or prevention of a disease or disorder caused by mast cell degranulation.

**Embodiment 41.** The compound according to any one of embodiments 1-36 or a pharmaceutically acceptable salt, a stereoisomer, a tautomer, a hydrate, or a solvate thereof, for use in the treatment or prevention of autoimmune diseases, allergic diseases, inflammatory diseases, pruritus, and pain, such as inflammatory skin diseases.

**Embodiment 42.** The compound according to any one of embodiments 1-36 or a pharmaceutically acceptable salt, a stereoisomer, a tautomer, a hydrate, or a solvate thereof, for use in the treatment or prevention of psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo. The compound according to any one of embodiments 1-36, or a pharmaceutically acceptable salt, stereoisomer, tautomer, hydrate, or solvate thereof, for use in the treatment or prevention of rosacea, psoriasis, urticaria, atopic dermatitis, inflammatory bowel disease, allergic rhinitis and asthma.

**Embodiment 43.** Use of the compound according to any one of embodiments 1-36 or a pharmaceutically acceptable salt, a stereoisomer, a tautomer, a hydrate, or a solvate thereof in the manufacture of a medicament for use in the treatment or prevention of a disease or disorder mediated by MRGPRX2.

**Embodiment 44.** Use of the compound according to any one of embodiments 1-36 or a pharmaceutically acceptable salt, a stereoisomer, a tautomer, a hydrate, or a solvate thereof in the manufacture of a medicament for use in the treatment or prevention of a disease or disorder caused by mast cell degranulation.

**Embodiment 45.** Use of the compound according to any one of embodiments 1-36 or a pharmaceutically acceptable salt, a stereoisomer, a tautomer, a hydrate, or a solvate thereof in the manufacture of a medicament for use in the treatment or prevention of autoimmune diseases, allergic diseases, inflammatory diseases, pruritus, and pain, such as inflammatory skin diseases.

**Embodiment 46.** Use of the compound according to any one of embodiments 1-36 or a pharmaceutically acceptable salt, a stereoisomer, a tautomer, a hydrate, or a solvate thereof in the manufacture of a medicament for use in the treatment or prevention of psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

**Embodiment 47.** Use of the compound in any one of embodiments 1-36, or a pharmaceutically acceptable salt, stereoisomer, tautomer, hydrate, or solvate thereof in the manufacture of a medicament for use in the treatment or prevention of rosacea, psoriasis, urticaria, atopic dermatitis, inflammatory bowel disease, allergic rhinitis and asthma.

**Embodiment 48.** A method for treating or preventing a disease or disorder in a subject, comprising administering to a subject in need thereof an effective amount of the compound according to any one of embodiments 1-36 or a pharmaceutically acceptable salt, a stereoisomer, a tautomer, a hydrate, or a solvate thereof, wherein the disease or disorder is a disease or disorder mediated by MRGPRX2.

**Embodiment 49.** A method for treating or preventing a disease or disorder in a subject, comprising administering to a subject in need thereof an effective amount of the compound according to any one of embodiments 1-36 or a pharmaceutically acceptable salt, a stereoisomer, a tautomer, a hydrate, or a solvate thereof, wherein the disease or disorder is a disease or disorder caused by mast cell degranulation.

**Embodiment 50.** A method for treating or preventing a disease or disorder in a subject, comprising administering to a subject in need thereof an effective amount of the compound according to any one of embodiments 1-36 or a pharmaceutically acceptable salt, a stereoisomer, a tautomer, a hydrate, or a solvate thereof, wherein the disease or disorder is selected from the group consisting of autoimmune diseases, allergic diseases, inflammatory diseases, pruritus, and pain, such as inflammatory skin diseases.

**Embodiment 51.** A method for treating or preventing a disease or disorder in a subject, comprising administering to a subject in need thereof an effective amount of the compound according to any one of embodiments 1-36 or a pharmaceutically acceptable salt, a stereoisomer, a tautomer, a hydrate, or a solvate thereof, wherein the disease or disorder is selected from the group consisting of psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

**Embodiment 52.** A method for treating or preventing a disease or disorder in a subject, comprising administering to a subject in need thereof an effective amount of the preceding the compound in any one of embodiments 1-36, or a pharmaceutically acceptable salt, stereoisomer, tautomer, hydrate, or solvate thereof, wherein the disease or disorder is selected from the group consisting of rosacea, psoriasis, urticaria, atopic dermatitis, inflammatory bowel disease, allergic rhinitis and asthma.

**Embodiment 53.** Use of the compound according to any one of embodiments 1-36 or a pharmaceutically acceptable salt, a stereoisomer, a tautomer, a hydrate, or a solvate thereof for the treatment or prevention of a disease or disorder mediated by MRGPRX2.

**Embodiment 54.** Use of the compound according to any one of embodiments 1-36 or a pharmaceutically acceptable salt, a stereoisomer, a tautomer, a hydrate, or a solvate thereof for the treatment or prevention of a disease or disorder caused by mast cell degranulation.

**Embodiment 55.** Use of the compound according to any one of embodiments 1-36 or a pharmaceutically acceptable salt, a stereoisomer, a tautomer, a hydrate, or a solvate thereof for the treatment or prevention of autoimmune diseases, allergic diseases, inflammatory diseases, pruritus, and pain, such as inflammatory skin diseases.

**Embodiment 56.** Use of the compound according to any one of embodiments 1-36 or a pharmaceutically acceptable salt, a stereoisomer, a tautomer, a hydrate, or a solvate thereof for the treatment or prevention of psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

**Embodiment 57.** Use of the compound in any one of embodiments 1-36, or a pharmaceutically acceptable salt, stereoisomer, tautomer, hydrate, or solvate thereof for the treatment or prevention of rosacea, psoriasis, urticaria, atopic dermatitis, inflammatory bowel disease, allergic rhinitis and asthma.

**Embodiment 58.** A method for antagonizing MRGPRX2 *in vitro,* comprising utilizing an effective amount of the compound according to any one of embodiments 1-36 or a pharmaceutically acceptable salt, a stereoisomer, a tautomer, a hydrate, or a solvate thereof in an *in vitro* assay for MRGPRX2.

**Embodiment 59.** A pharmaceutical composition for the treatment or prevention of a disease or disorder mediated by MRGPRX2, comprising the compound according to any one of embodiments 1-36 or a pharmaceutically acceptable salt, a stereoisomer, a tautomer, a hydrate, or a solvate thereof.

**Embodiment 60.** A pharmaceutical composition for use in the treatment or prevention of a disease or disorder caused by mast cell degranulation, comprising the compound according to any one of embodiments 1-36 or a pharmaceutically acceptable salt, a stereoisomer, a tautomer, a hydrate, or a solvate thereof.

**Embodiment 61.** A pharmaceutical combination, comprising the compound according to any one of embodiments 1-36 or a pharmaceutically acceptable salt, a stereoisomer, a tautomer, a hydrate, or a solvate thereof, and one or more other therapeutic agents.

### Embodiments of the Present Disclosure - Part C

The present invention specifically provides a use of a class of compounds represented by formula (I) and a pharmaceutically acceptable salt, prodrug, or solvate thereof in the manufacture of a medicament of an MRGPRX2 antagonist.

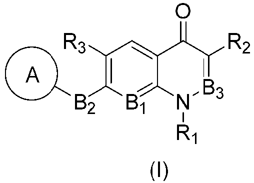

Wherein:
B₁ is selected from the group consisting of -CR₄ or N atom; wherein R₄ is selected from the group consisting of hydrogen atom, C1-C3 alkyl, halogen, C1-C3 alkoxy or nitro;
R₁ is selected from the group consisting of hydrogen atom, C1-C3 alkyl, C1-C3 alkyl substituted with 1 to 3 halogen, C3-C6 cycloalkyl, C3-C6 alkyl substituted with 1 to 3 halogen, or R₁ and B₁ are bonded to form a 6-membered heterocycle group or unsubstituted optionally substituted with a short-chain alkyl;
B₂ is selected from the group consisting of -NH, oxygen atom, -CH=CH- or is absent; when B₂ is absent, 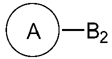 is 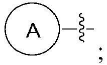 wherein, 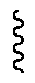 is the point of attachment;
R₂ is selected from the group consisting of hydrogen atom, -C(=O)OR₅ or -C(=O)NHR₅'; wherein R₅ is selected from the group consisting of hydrogen atom, C1-C4 alkyl; R₅' is selected from the group consisting of hydrogen atom or hydroxyl;
B₃ is selected from the group consisting of -CR₆, wherein R₆ is selected from the group consisting of hydrogen atom or a sulfur atom; wherein, when R₆ is a sulfur atom, R₂ is selected from the group consisting of -C(=O)NH₂ and bonds with B₃ to form an isothiazolinone group;
R₃ is selected from the group consisting of halogen, nitro or hydrogen atom;
Ring A is selected from the group consisting of unsubstituted or substituted aryl, wherein the substitution comprises mono-, di-, tri-, or tetra-substitution, wherein the substituent is independently selected from the group consisting of: halogen, hydroxyl, nitro, cyano, C1-C3 alkyl, C1-C3 alkoxy, C1-C3 alkyl substituted with 1 to 3 halogen, C1-C3 alkyl substituted with hydroxyl, C1-C3 alkyl substituted with phenolic hydroxyl, C1-C3 alkyl substituted with amino, C1-C3 alkyl substituted with anilino, unsubstituted or substituted amino, aminosulfonyl, aminosulfinyl, aminocarbonyl, sulfenyl, or alkyliminosulfinyl.

In one embodiment of the present invention, the aryl is phenyl, 2-pyridyl, 3-pyridyl, 4-pyridyl, 2-pyrimidinyl, 5-pyrimidinyl, triazinyl,

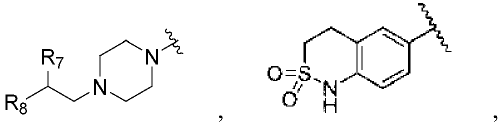

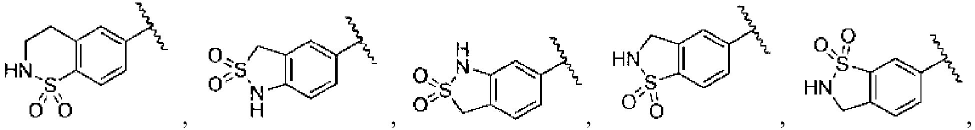

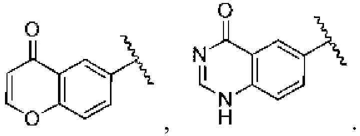

In one embodiment of the present invention:
For unsubstituted or substituted amino-NRₐRₐ', the substitution is mono-substitution or di-substitution, wherein the substituents Rₐ and Rₐ' are each independently selected from the group consisting of H, C1-C9 alkyl, C1-C9 alkyl substituted with 1 to 3 halogen, C3-C25 linear or branched alkyl containing 1 to 10 unsaturated bonds, C3-C6 cycloalkyl, C1-C9 alkyl substituted with hydroxyl, C1-C9 acyl, C2-C9 acyl substituted with 1 to 3 halogen, C2-C9 acyl substituted with hydroxyl, C2-C5 alkoxycarbonyl, C1-C25 alkylsulfonyl;
For unsubstituted or substituted aminosulfonyl -SO₂-NR_{b}R_{b}', the substitution is mono-substitution or di-substitution, the substituents R_{b} and R_{b}' are each independently selected from the group consisting of H, C1-C9 alkyl, C1-C9 alkyl substituted with 1 to 3 halogen, C1-C9 alkyl substituted with hydroxyl, C1-C9 alkyl substituted with amino, C1-C9 alkyl substituted with alkylmercapto (C1-3alkyl-S-), C1-C25 alkylsulfonyl, C3-C25 linear or branched alkyl containing 1 to 10 unsaturated bonds, C3-C6 cycloalkyl, 3- to 6-membered cyclic group containing 1 to 3 heteroatoms, C2-C25 alkenyl, C2-C9 alkynyl, C1-C5 acyl, C2-C5 acyl substituted with 1 to 3 halogen, or C2-C5 alkoxycarbonyl;
For unsubstituted or substituted aminosulfinyl -SO-NR_{c}R_{c}', the substitution is mono-substitution or di-substitution, the substituents R_{c}, R_{c}' are each independently selected from the group consisting of H, C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
For unsubstituted or substituted aminocarbonyl -CO-NR_{d}R_{d}' , the substitution is mono-substitution or di-substitution, the substituents R_{d}, R_{d}' are each independently selected from the group consisting of H, C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
For unsubstituted or substituted sulfenyl -S-Rₑ, the substituent Rₑ is selected from the group consisting of C1-C9 linear or branched alkyl, C3-C6 cycloalkyl, C1-C9 haloalkyl, C1-C9 alkyl substituted with hydroxyl, C1-C5 acyl, C2-C5 haloacyl;
For unsubstituted or substituted alkyliminosulfinyl -S(=O) (=NR_{f})-R_{f}'(i.e., 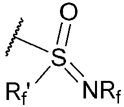 ), the substituent R_{f} is selected from the group consisting of H, C1-C9 linear or branched alkyl, C3-C25 linear or branched alkyl containing 1 to 10 unsaturated bonds, C3-C6 cycloalkyl, C1-C9 haloalkyl, C1-C5 acyl, C2-C5 acyl substituted with 1 to 3 halogen, or C2-C5 alkoxycarbonyl; R_{f}' is selected from the group consisting of C1-3 alkyl.

In one embodiment of the present invention, the aforementioned Rₐ, Rₐ' may both be H, or not both be H, or neither be H.

In one embodiment of the present invention, the aforementioned R_{b}, R_{b}' may both be H, or not both be H, or neither be H.

In one embodiment of the present invention, the aforementioned R_{c}, R_{c}' may both be H, or not both be H, or neither be H.

In one embodiment of the present invention, the aforementioned R_{d}, R_{d}' may both be H, or not both be H, or neither be H.

In one embodiment of the present invention, 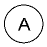 is selected from the group consisting of the following groups:
**(1) phenyl substituted with 0 to 4 substituents, wherein the substituents are selected from the group consisting of:**
   (a) C1-C3 alkyl;
   (b) C1-C3 alkoxy;
   (c) C1-C3 alkyl substituted with 1 to 3 halogen;
   (d) C1-C3 alkyl substituted with hydroxyl;
   (e) C1-C3 alkyl substituted with phenolic hydroxyl;
   (f) C1-C3 alkyl substituted with amino;
   (g) C1-C3 alkyl substituted with anilino;
   (h) halogen;
   (i) hydroxyl;
   (j) nitro;
   (k) cyano;
   (l) unsubstituted or substituted amino, the substitution being mono-substitution or di-substitution, wherein the substituent is independently selected from the group consisting of C1-C9 alkyl, C1-C9 alkyl substituted with 1 to 3 halogen, C3-C25 linear or branched alkyl containing 1 to 10 unsaturated bonds, C3-C6 cycloalkyl, C1-C9 alkyl substituted with hydroxyl, C1-C9 acyl, C2-C9 acyl substituted with 1 to 3 halogen, C2-C9 acyl substituted with hydroxyl, C2-C5 alkoxycarbonyl, C1-C25 alkylsulfonyl;
   (m) unsubstituted or substituted aminosulfonyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C1-C9 alkyl substituted with alkylmercapto (C1-3alkyl-S-), C1-C25 alkylsulfonyl, C3-C25 linear or branched alkyl containing 1 to 10 unsaturated bonds, C3-C6 cycloalkyl, 3- to 6-membered cyclic group containing 1 to 3 heteroatoms, C2-C25 alkenyl, C2-C9 alkynyl, C1-C5 acyl, C2-C5 acyl substituted with 1 to 3 halogen, or C2-C5 alkoxycarbonyl;
   (n) unsubstituted or substituted aminosulfinyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
   (o) unsubstituted or substituted aminocarbonyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
   (p) unsubstituted or substituted sulfenyl, wherein the substituent is selected from the group consisting of C1-C9 linear or branched alkyl, C3-C6 cycloalkyl, C1-C9 haloalkyl, C1-C9 alkyl substituted with hydroxyl, C1-C5 acyl, C2-C5 haloacyl;
   (q) unsubstituted or substituted alkyliminosulfinyl -S(=O) (=NR_{f})-R_{f}'(i.e., 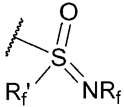 ), the substitution being mono-substitution or di-substitution, the substituent is selected from the group consisting of C1-C9 linear or branched alkyl, C3-C25 linear or branched alkyl containing 1 to 10 unsaturated bonds, C3-C6 cycloalkyl, C1-C9 haloalkyl, C1-C5 acyl, C2-C5 acyl substituted with 1 to 3 halogen, or C2-C5 alkoxycarbonyl; R_{f}' is selected from the group consisting of C1-3alkyl;
**(2) 2-pyridyl substituted with 0 to 3 substituents, wherein the substituents are selected from the group consisting of:**
   (a) C1-C3 alkyl;
   (b) C1-C3 alkoxy;
   (c) C1-C3 alkyl substituted with 1 to 3 halogen;
   (d) C1-C3 alkyl substituted with hydroxyl;
   (e) C1-C3 alkyl substituted with phenolic hydroxyl;
   (f) C1-C3 alkyl substituted with amino;
   (g) C1-C3 alkyl substituted with anilino;
   (h) halogen;
   (i) hydroxyl;
   (j) nitro;
   (k) cyano;
   (l) unsubstituted or substituted amino, the substitution being mono-substitution or di-substitution, wherein the substituent is independently selected from the group consisting of C1-C9 alkyl, C1-C9 alkyl substituted with 1 to 3 halogen, C3-C25 linear or branched alkyl containing 1 to 10 unsaturated bonds, C3-C6 cycloalkyl, C1-C9 alkyl substituted with hydroxyl, C1-C9 acyl, C2-C9 acyl substituted with 1 to 3 halogen, C2-C9 acyl substituted with hydroxyl, C2-C5 alkoxycarbonyl, C1-C25 alkylsulfonyl;
   (m) unsubstituted or substituted aminosulfonyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C1-C9 alkyl substituted with alkylmercapto (C1-3alkyl-S-), C1-C25 alkylsulfonyl, C3-C25 linear or branched alkyl containing 1 to 10 unsaturated bonds, C3-C6 cycloalkyl, 3- to 6-membered cyclic group containing 1 to 3 heteroatoms, C2-C25 alkenyl, C2-C9 alkynyl, C1-C5 acyl, C2-C5 acyl substituted with 1 to 3 halogen, or C2-C5 alkoxycarbonyl;
   (n) unsubstituted or substituted aminosulfinyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
   (o) unsubstituted or substituted aminocarbonyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
   (p) unsubstituted or substituted sulfenyl, wherein the substituent is selected from the group consisting of C1-C9 linear or branched alkyl, C3-C6 cycloalkyl, C1-C9 haloalkyl, C1-C9 alkyl substituted with hydroxyl, C1-C5 acyl, C2-C5 haloacyl;
   (q) unsubstituted or substituted alkyliminosulfinyl -S(=O) (=NR_{f})-R_{f}'(i.e., 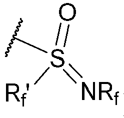 ), the substitution being mono-substitution or di-substitution, the substituent is selected from the group consisting of C1-C9 linear or branched alkyl, C3-C25 linear or branched alkyl containing 1 to 10 unsaturated bonds, C3-C6 cycloalkyl, C1-C9 haloalkyl, C1-C5 acyl, C2-C5 acyl substituted with 1 to 3 halogen, or C2-C5 alkoxycarbonyl; R_{f}' is selected from the group consisting of C1-3alkyl;
(3) **3-pyridyl substituted with 0 to 3 substituents, wherein the substituents are selected from the group consisting of:**
   (a) C1-C3 alkyl;
   (b) C1-C3 alkoxy;
   (c) C1-C3 alkyl substituted with 1 to 3 halogen;
   (d) C1-C3 alkyl substituted with hydroxyl;
   (e) C1-C3 alkyl substituted with phenolic hydroxyl;
   (f) C1-C3 alkyl substituted with amino;
   (g) C1-C3 alkyl substituted with anilino;
   (h) halogen;
   (i) hydroxyl;
   (j) nitro;
   (k) cyano;
   (l) unsubstituted or substituted amino, the substitution being mono-substitution or di-substitution, wherein the substituent is independently selected from the group consisting of C1-C9 alkyl, C1-C9 alkyl substituted with 1 to 3 halogen, C3-C25 linear or branched alkyl containing 1 to 10 unsaturated bonds, C3-C6 cycloalkyl, C1-C9 alkyl substituted with hydroxyl, C1-C9 acyl, C2-C9 acyl substituted with 1 to 3 halogen, C2-C9 acyl substituted with hydroxyl, C2-C5 alkoxycarbonyl, C1-C25 alkylsulfonyl;
   (m) unsubstituted or substituted aminosulfonyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C1-C9 alkyl substituted with alkylmercapto (C1-3alkyl-S-), C1-C25 alkylsulfonyl, C3-C25 linear or branched alkyl containing 1 to 10 unsaturated bonds, C3-C6 cycloalkyl, 3- to 6-membered cyclic group containing 1 to 3 heteroatoms, C2-C25 alkenyl, C2-C9 alkynyl, C1-C5 acyl, C2-C5 acyl substituted with 1 to 3 halogen, or C2-C5 alkoxycarbonyl;
   (n) unsubstituted or substituted aminosulfinyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
   (o) unsubstituted or substituted aminocarbonyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
   (p) unsubstituted or substituted sulfenyl, wherein the substituent is selected from the group consisting of C1-C9 linear or branched alkyl, C3-C6 cycloalkyl, C1-C9 haloalkyl, C1-C9 alkyl substituted with hydroxyl, C1-C5 acyl, C2-C5 haloacyl;
   (q) unsubstituted or substituted alkyliminosulfinyl -S(=O) (=NR_{f})-R_{f}' (i.e., 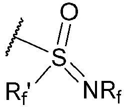 ), the substitution being mono-substitution or di-substitution, the substituent is selected from the group consisting of C1-C9 linear or branched alkyl, C3-C25 linear or branched alkyl containing 1 to 10 unsaturated bonds, C3-C6 cycloalkyl, C1-C9 haloalkyl, C1-C5 acyl, C2-C5 acyl substituted with 1 to 3 halogen, or C2-C5 alkoxycarbonyl; R_{f}' is selected from the group consisting of C1-3alkyl;
**(4) 4-pyridyl substituted with 0 to 3 substituents, wherein the substituents are selected from the group consisting of:**
   (a) C1-C3 alkyl;
   (b) C1-C3 alkoxy;
   (c) C1-C3 alkyl substituted with 1 to 3 halogen;
   (d) C1-C3 alkyl substituted with hydroxyl;
   (e) C1-C3 alkyl substituted with phenolic hydroxyl;
   (f) C1-C3 alkyl substituted with amino;
   (g) C1-C3 alkyl substituted with anilino;
   (h) halogen;
   (i) hydroxyl;
   (j) nitro;
   (k) cyano;
   (l) unsubstituted or substituted amino, the substitution being mono-substitution or di-substitution, wherein the substituent is independently selected from the group consisting of C1-C9 alkyl, C1-C9 alkyl substituted with 1 to 3 halogen, C3-C25 linear or branched alkyl containing 1 to 10 unsaturated bonds, C3-C6 cycloalkyl, C1-C9 alkyl substituted with hydroxyl, C1-C9 acyl, C2-C9 acyl substituted with 1 to 3 halogen, C2-C9 acyl substituted with hydroxyl, C2-C5 alkoxycarbonyl, C1-C25 alkylsulfonyl;
   (m) unsubstituted or substituted aminosulfonyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C1-C9 alkyl substituted with alkylmercapto (C1-3alkyl-S-), C1-C25 alkylsulfonyl, C3-C25 linear or branched alkyl containing 1 to 10 unsaturated bonds, C3-C6 cycloalkyl, 3- to 6-membered cyclic group containing 1 to 3 heteroatoms, C2-C25 alkenyl, C2-C9 alkynyl, C1-C5 acyl, C2-C5 acyl substituted with 1 to 3 halogen, or C2-C5 alkoxycarbonyl;
   (n) unsubstituted or substituted aminosulfinyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
   (o) unsubstituted or substituted aminocarbonyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
   (p) unsubstituted or substituted sulfenyl, wherein the substituent is selected from the group consisting of C1-C9 linear or branched alkyl, C3-C6 cycloalkyl, C1-C9 haloalkyl, C1-C9 alkyl substituted with hydroxyl, C1-C5 acyl, C2-C5 haloacyl;
   (q) unsubstituted or substituted alkyliminosulfinyl -S(=O) (=NR_{f})-R_{f}' (i.e., 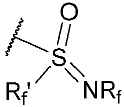 ), the substitution being mono-substitution or di-substitution, the substituent is selected from the group consisting of C1-C9 linear or branched alkyl, C3-C25 linear or branched alkyl containing 1 to 10 unsaturated bonds, C3-C6 cycloalkyl, C1-C9 haloalkyl, C1-C5 acyl, C2-C5 acyl substituted with 1 to 3 halogen, or C2-C5 alkoxycarbonyl; R_{f}' is selected from the group consisting of C1-3alkyl;
**(5) 2-pyrimidinyl substituted with 0 to 2 substituents, wherein the substituents are selected from the group consisting of:**
   (a) C1-C3 alkyl;
   (b) C1-C3 alkoxy;
   (c) C1-C3 alkyl substituted with 1 to 3 halogen;
   (d) C1-C3 alkyl substituted with hydroxyl;
   (e) C1-C3 alkyl substituted with phenolic hydroxyl;
   (f) C1-C3 alkyl substituted with amino;
   (g) C1-C3 alkyl substituted with anilino;
   (h) halogen;
   (i) hydroxyl;
   (j) nitro;
   (k) cyano;
   (l) unsubstituted or substituted amino, the substitution being mono-substitution or di-substitution, wherein the substituent is independently selected from the group consisting of C1-C9 alkyl, C1-C9 alkyl substituted with 1 to 3 halogen, C3-C25 linear or branched alkyl containing 1 to 10 unsaturated bonds, C3-C6 cycloalkyl, C1-C9 alkyl substituted with hydroxyl, C1-C9 acyl, C2-C9 acyl substituted with 1 to 3 halogen, C2-C9 acyl substituted with hydroxyl, C2-C5 alkoxycarbonyl, C1-C25 alkylsulfonyl;
   (m) unsubstituted or substituted aminosulfonyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C1-C9 alkyl substituted with alkylmercapto (C1-3alkyl-S-), C1-C25 alkylsulfonyl, C3-C25 linear or branched alkyl containing 1 to 10 unsaturated bonds, C3-C6 cycloalkyl, 3- to 6-membered cyclic group containing 1 to 3 heteroatoms, C2-C25 alkenyl, C2-C9 alkynyl, C1-C5 acyl, C2-C5 acyl substituted with 1 to 3 halogen, or C2-C5 alkoxycarbonyl;
   (n) unsubstituted or substituted aminosulfinyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
   (o) unsubstituted or substituted aminocarbonyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
   (p) unsubstituted or substituted sulfenyl, wherein the substituent is selected from the group consisting of C1-C9 linear or branched alkyl, C3-C6 cycloalkyl, C1-C9 haloalkyl, C1-C9 alkyl substituted with hydroxyl, C1-C5 acyl, C2-C5 haloacyl;
   (q) unsubstituted or substituted alkyliminosulfinyl -S(=O) (=NR_{f})-R_{f}' (i.e., 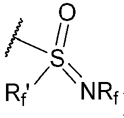 ), the substitution being mono-substitution or di-substitution, the substituent is selected from the group consisting of C1-C9 linear or branched alkyl, C3-C25 linear or branched alkyl containing 1 to 10 unsaturated bonds, C3-C6 cycloalkyl, C1-C9 haloalkyl, C1-C5 acyl, C2-C5 acyl substituted with 1 to 3 halogen, or C2-C5 alkoxycarbonyl; R_{f}' is selected from the group consisting of C1-3alkyl;
**(6) 5-pyrimidinyl substituted with 0 to 2 substituents, wherein the substituents are selected from the group consisting of:**
   (a) C1-C3 alkyl;
   (b) C1-C3 alkoxy;
   (c) C1-C3 alkyl substituted with 1 to 3 halogen;
   (d) C1-C3 alkyl substituted with hydroxyl;
   (e) C1-C3 alkyl substituted with phenolic hydroxyl;
   (f) C1-C3 alkyl substituted with amino;
   (g) C1-C3 alkyl substituted with anilino;
   (h) halogen;
   (i) hydroxyl;
   (j) nitro;
   (k) cyano;
   (l) unsubstituted or substituted amino, the substitution being mono-substitution or di-substitution, wherein the substituent is independently selected from the group consisting of C1-C9 alkyl, C1-C9 alkyl substituted with 1 to 3 halogen, C3-C25 linear or branched alkyl containing 1 to 10 unsaturated bonds, C3-C6 cycloalkyl, C1-C9 alkyl substituted with hydroxyl, C1-C9 acyl, C2-C9 acyl substituted with 1 to 3 halogen, C2-C9 acyl substituted with hydroxyl, C2-C5 alkoxycarbonyl, C1-C25 alkylsulfonyl;
   (m) unsubstituted or substituted aminosulfonyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C1-C9 alkyl substituted with alkylmercapto (C1-3alkyl-S-), C1-C25 alkylsulfonyl, C3-C25 linear or branched alkyl containing 1 to 10 unsaturated bonds, C3-C6 cycloalkyl, 3- to 6-membered cyclic group containing 1 to 3 heteroatoms, C2-C25 alkenyl, C2-C9 alkynyl, C1-C5 acyl, C2-C5 acyl substituted with 1 to 3 halogen, or C2-C5 alkoxycarbonyl;
   (n) unsubstituted or substituted aminosulfinyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
   (o) unsubstituted or substituted aminocarbonyl, the substitution being mono-substitution or di-substitution, wherein the substituent is independently selected from the group consisting of C1-C9 alkyl, C1-C9 alkyl substituted with 1 to 3 halogen, C1-C9 alkyl substituted with hydroxyl, C1-C9 alkyl substituted with amino, C3-C25 linear or branched alkyl containing 1 to 10 unsaturated bonds, C3-C6 cycloalkyl, C2-C25 alkenyl, C2-C9 alkynyl, C1-C5 acyl, C2-C5 acyl substituted with 1 to 3 halogen, or C2-C5 alkoxycarbonyl;
   (p) unsubstituted or substituted sulfenyl, wherein the substituent is selected from the group consisting of C1-C9 linear or branched alkyl, C3-C6 cycloalkyl, C1-C9 haloalkyl, C1-C9 alkyl substituted with hydroxyl, C1-C5 acyl, C2-C5 haloacyl;
   (q) unsubstituted or substituted alkyliminosulfinyl -S(=O) (=NR_{f})-R_{f}' (i.e., 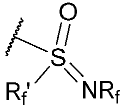 ), the substitution being mono-substitution or di-substitution, the substituent is selected from the group consisting of C1-C9 linear or branched alkyl, C3-C25 linear or branched alkyl containing 1 to 10 unsaturated bonds, C3-C6 cycloalkyl, C1-C9 haloalkyl, C1-C5 acyl, C2-C5 acyl substituted with 1 to 3 halogen, or C2-C5 alkoxycarbonyl; R_{f}' is selected from the group consisting of C1-3alkyl;
**(7) triazinyl;**
**(8) a group of the following formula;** 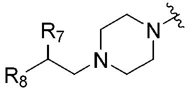
   wherein R₇ is selected from the group consisting of hydrogen atom, hydroxyl, or amino;
   wherein R₈ is selected from the group consisting of -(CH2)ₙ-XR₉; wherein n is selected from the group consisting of 0-3; X is selected from the group consisting of a heteroatom; R₉ is selected from the group consisting of a benzene ring, fused ring, or fused heterocycle;
**(9) a group of the following formula:**

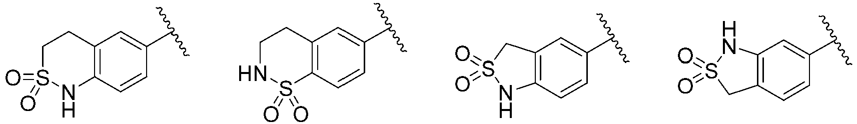

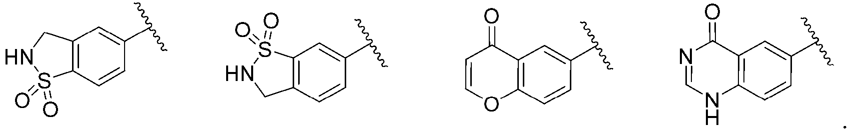

In one embodiment of the present invention, Ⓐ is selected from the group consisting of unsubstituted or substituted phenyl, wherein the substitution is mono-substitution to tetra-substitution, and the substituents are as defined above, wherein at least one substituent is selected from the group consisting of (a) unsubstituted or substituted amino, wherein the substituent is selected from the same range as above; (b) aminosulfonyl, wherein amino may be substituted, the substitution being mono-substitution or di-substitution, the substituent is selected from the same range as above; (c) aminosulfinyl, wherein amino may be substituted, the substitution being mono-substitution or di-substitution, the substituent is selected from the same range as above; (d) aminocarbonyl, wherein amino may be substituted, the substitution being mono-substitution or di-substitution, the substituent is selected from the same range as above; (e) alkyliminosulfinyl, wherein amino may be substituted, the substitution being mono-substitution or di-substitution, the substituent is selected from the same range as above.

In one embodiment of the present invention, Ⓐ is selected from the group consisting of unsubstituted or substituted 2-pyridyl, wherein the substitution is mono-substitution to tri-substitution, and the substituents are as defined above, wherein at least one substituent is selected from the group consisting of (a) unsubstituted or substituted amino, wherein the substituent is selected from the same range as above; (b) aminosulfonyl, wherein amino may be substituted, the substitution being mono-substitution or di-substitution, the substituent is selected from the same range as above; (c) aminosulfinyl, wherein amino may be substituted, the substitution being mono-substitution or di-substitution, the substituent is selected from the same range as above; (d) aminocarbonyl, wherein amino may be substituted, the substitution being mono-substitution or di-substitution, the substituent is selected from the same range as above; (e) alkyliminosulfinyl, wherein amino may be substituted, the substitution being mono-substitution or di-substitution, the substituent is selected from the same range as above.

In one embodiment of the present invention, Ⓐ is selected from the group consisting of unsubstituted or substituted 3-pyridyl, wherein the substitution is mono-substitution to tri-substitution, and the substituents are as defined above, wherein at least one substituent is selected from the group consisting of (a) unsubstituted or substituted amino, wherein the substituent is selected from the same range as above; (b) aminosulfonyl, wherein amino may be substituted, the substitution being mono-substitution or di-substitution, the substituent is selected from the same range as above; (c) aminosulfinyl, wherein amino may be substituted, the substitution being mono-substitution or di-substitution, the substituent is selected from the same range as above; (d) aminocarbonyl, wherein amino may be substituted, the substitution being mono-substitution or di-substitution, the substituent is selected from the same range as above; (e) alkyliminosulfinyl, wherein amino may be substituted, the substitution being mono-substitution or di-substitution, the substituent is selected from the same range as above.

In one embodiment of the present invention, Ⓐ is selected from the group consisting of unsubstituted or substituted 4-pyridyl, wherein the substitution is mono-substitution to tri-substitution, and the substituents are as defined above, wherein at least one substituent is preferably selected from the group consisting of (a) C1-C3 alkyl; (b) C1-C3 alkoxy; (c) unsubstituted or substituted amino, wherein the substituent is selected from the same range as above; (d) aminosulfonyl, wherein amino may be substituted, the substitution being mono-substitution or di-substitution, the substituent is selected from the same range as above; (e) aminosulfinyl, wherein amino may be substituted, the substitution being mono-substitution or di-substitution, the substituent is selected from the same range as above; (f) aminocarbonyl, wherein amino may be substituted, the substitution being mono-substitution or di-substitution, the substituent is selected from the same range as above; (g) alkyliminosulfinyl, wherein amino may be substituted, the substitution being mono-substitution or di-substitution, the substituent is selected from the same range as above.

In one embodiment of the present invention, the amino may also be selected from the group consisting of

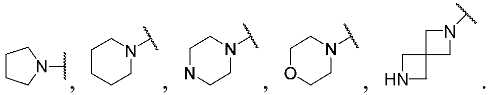

In one embodiment of the present invention, a C atom in the alkyl carbon chain in the definition of Ⓐ may be substituted with a heteroatom at any position permitted by normal valency.

In one embodiment of the present invention, a unsaturated bond comprises carbon-carbon double bonds and/or carbon-carbon triple bond.

In one embodiment of the present invention, the C2-C25 alkenyl refers to C2-C25 alkenyl containing 1-10 unsaturated double bonds, for example: (H or C1-C23 alkyl containing 0-9 unsaturated double bonds)-CH=CH-.

In one embodiment of the present invention, the C2-C9 alkynyl refers to C2-C9 alkynyl containing 1-3 unsaturated bonds, for example: (H or C1-C7alkyl containing 0-2 unsaturated bonds)-C=C-. The unsaturated bond comprises carbon-carbon double bond and/or carbon-carbon triple bond.

In one embodiment of the present invention, the C1-C5 acyl refers to (H or C1-C4 alkyl)-C(=O)-. The C1-C9 acyl refers to (H or C1-C8 alkyl)-C(=O)-.

In one embodiment of the present invention, the C2-C5 acyl substituted with 1 to 3 halogen refers to (C1-C4 alkyl substituted with 1 to 3 halogen)-C(=O)-. C2-C9 acyl substituted with 1 to 3 halogen refers to (C1-C8 alkyl substituted with 1 to 3 halogen)-C(=O)-.

In one embodiment of the present invention, the C2-C5 alkoxycarbonyl refers to (C2-C5 alkyl)-O-C(=O)-.

In one embodiment of the present invention, the C1-C25 acyl refers to (H or C1-C24 alkyl containing 0 to 10 unsaturated double bonds)-C(=O)-.

In one embodiment of the present invention, the C1-C25 alkylsulfonyl refers to (C1-C25 alkyl containing 0 to 10 unsaturated bonds)-SO₂-.

The present invention also provides the use of the compound represented by the above formula (I) and a pharmaceutically acceptable salt, prodrug, or solvate thereof as an immunomodulator in the manufacture of a medicament for an autoimmune disease.

The present invention also provides an immunomodulator, comprising the compound represented by the above formula (I) and a pharmaceutically acceptable salt, prodrug, or solvate thereof.

The present invention also provides the use of the compound represented by the above formula (I) and a pharmaceutically acceptable salt, prodrug, or solvate thereof in the manufacture of a medicament for treating or preventing rosacea.

The present invention also provides the use of the compound represented by the above formula (I) and a pharmaceutically acceptable salt, prodrug, or solvate thereof in the manufacture of a medicament for treating or preventing urticaria.

The present invention also provides the use of the compound represented by the above formula (I) and a pharmaceutically acceptable salt, prodrug, or solvate thereof in the manufacture of a medicament for treating or preventing atopic dermatitis.

The present invention also provides the use of the compound represented by the above formula (I) and a pharmaceutically acceptable salt, prodrug, or solvate thereof in the manufacture of a medicament for treating or preventing psoriasis.

The present invention also provides the use of the compound represented by the above formula (I) and a pharmaceutically acceptable salt, prodrug, or solvate thereof in the manufacture of a medicament for treating or preventing radiation dermatitis.

The present invention also provides the use of the compound represented by the above formula (I) and a pharmaceutically acceptable salt, prodrug, or solvate thereof in the manufacture of a medicament for treating or preventing vitiligo.

The present invention also provides the use of the compound represented by the above formula (I) and a pharmaceutically acceptable salt, prodrug, or solvate thereof in the manufacture of a medicament for treating or preventing alopecia areata.

The present invention also provides the use of the compound represented by the above formula (I) and a pharmaceutically acceptable salt, prodrug, or solvate thereof in the manufacture of a medicament for treating or preventing inflammatory bowel disease.

The present invention also provides a pharmaceutical composition, comprising the compound represented by the above formula (I) and a pharmaceutically acceptable salt, prodrug, or solvate thereof as an active ingredient, as well as a pharmaceutically acceptable carrier.

The present invention also provides a use of the compound represented by the above formula (I) and a pharmaceutically acceptable salt, prodrug, or solvate thereof, and the aforementioned pharmaceutical composition thereof in the manufacture of a medicament related to an MRGPRX2 receptor antagonist.

The present invention also provides the medicament related to an MRGPRX2 receptor antagonist above suitable for treating an autoimmune disease.

The present invention also provides the compound represented by the above formula (I) and a pharmaceutically acceptable salt, prodrug, or solvate thereof, which can be used for treating or preventing a disease or disorder mediated by MRGPRX2.

In one embodiment of the present invention, the compound represented by the above formula (I) and a pharmaceutically acceptable salt, prodrug, or solvate thereof may treat or prevent a disease or disorder mediated by MRGPRX2 through the route of oral administration;

Alternatively, it may treat or prevent a disease or disorder mediated by MRGPRX2 through the route of topical application/external application.

In certain embodiments, the present invention comprises a compound of formula (I), wherein B₁ and R₁ are bonded to form a 6-membered heterocycle group optionally substituted with an alkyl. Examples of such compounds include, but are not limited to, compounds of formula (II) to formula (III). 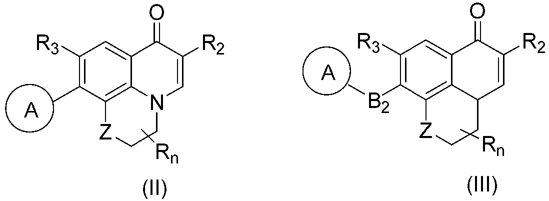 wherein, Z is O or S; Rₙ is selected from the group consisting of C1-C4 alkyl, halogen.

In certain embodiments, the present invention comprises a compound of formula (I), wherein B₃ is selected from the group consisting of CR₆, wherein R₆ is selected from the group consisting of hydrogen atom or a sulfur atom; wherein, when R₆ is a sulfur atom, R₂ is selected from the group consisting of (C=O)NH₂ and bonds with B₃ to form an isothiazolinone group. Examples of such compounds include, but are not limited to, compounds of formula (IV) to formula (V).

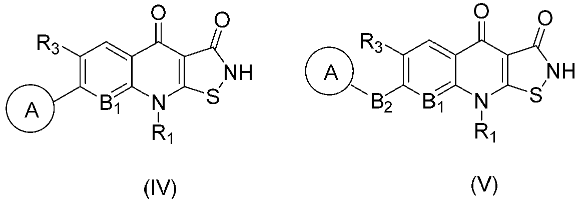

More specifically, the present disclosure provides the following embodiments:
1. Use of a compound represented by formula (I) and a pharmaceutically acceptable salt, prodrug, or solvate thereof in the manufacture of a medicament of an MRGPRX2 antagonist 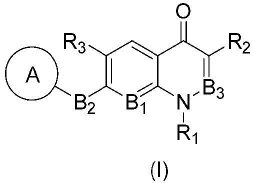 wherein:
   B₁ is selected from the group consisting of -CR₄ or N atom; wherein R₄ is selected from the group consisting of hydrogen atom, C1-C3 alkyl, halogen, C1-C3 alkoxy or nitro;
   R₁ is selected from the group consisting of hydrogen atom, C1-C3 alkyl, C1-C3 alkyl substituted with 1 to 3 halogen, C3-C6 cycloalkyl, C3-C6 alkyl substituted with 1 to 3 halogen, or R₁ and B₁ are bonded to form a 6-membered heterocycle group or unsubstituted optionally substituted with a short-chain alkyl;
   B₂ is selected from the group consisting of -NH, oxygen atom, -CH=CH- or is absent;
   R₂ is selected from the group consisting of hydrogen atom, -C(=O)OR₅ or -C(=O)NHR₅'; wherein R₅ is selected from the group consisting of hydrogen atom, C1-C4 alkyl; R₅' is selected from the group consisting of hydrogen atom or hydroxyl;
   B₃ is selected from the group consisting of -CR₆, wherein R₆ is selected from the group consisting of hydrogen atom or a sulfur atom; wherein, when R₆ is a sulfur atom, R₂ is selected from the group consisting of -C(=O)NH₂ and bonds with B₃ to form an isothiazolinone group;
   R₃ is selected from the group consisting of halogen, nitro or hydrogen atom;
   Ring A is selected from the group consisting of unsubstituted or substituted aryl, wherein the substitution comprises mono-, di-, tri-, or tetra-substitution, wherein the substituent is independently selected from the group consisting of: halogen, hydroxyl, nitro, cyano, C1-C3 alkyl, C1-C3 alkoxy, C1-C3 alkyl substituted with 1 to 3 halogen, C1-C3 alkyl substituted with hydroxyl, C1-C3 alkyl substituted with phenolic hydroxyl, C1-C3 alkyl substituted with amino, C1-C3 alkyl substituted with anilino, unsubstituted or substituted amino, aminosulfonyl, aminosulfinyl, aminocarbonyl, sulfenyl, or alkyliminosulfinyl.
2. The compound according to embodiment 1 and a pharmaceutically acceptable salt, prodrug, or solvate thereof, characterized in that the aryl is phenyl, 2-pyridyl, 3-pyridyl, 4-pyridyl, 2-pyrimidinyl, 5-pyrimidinyl, triazinyl, 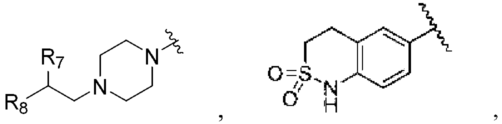 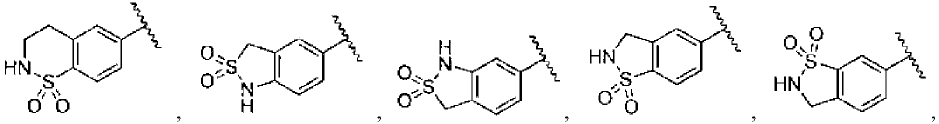 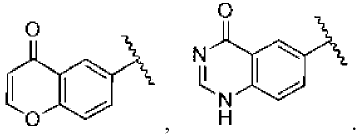
3. The compound according to embodiment 1 and a pharmaceutically acceptable salt, prodrug, or solvate thereof, characterized in that Ring A is selected from the group consisting of unsubstituted or substituted aryl, wherein the substitution comprises mono-, di-, tri-, or tetra-substitution, and at least one substituent is selected from the group consisting of: unsubstituted or substituted amino, aminosulfonyl, aminosulfinyl, aminocarbonyl, sulfenyl, or alkyliminosulfinyl.
4. Use of the compound represented by formula (I) according to embodiment 1 and a pharmaceutically acceptable salt, prodrug, or solvate thereof as an immunomodulator in the manufacture of a medicament for an autoimmune disease.
5. Use of the compound represented by formula (I) according to embodiment 1 and a pharmaceutically acceptable salt, prodrug, or solvate thereof in the manufacture of an immunomodulator.
6. Use of the compound represented by formula (I) according to embodiment 1 and a pharmaceutically acceptable salt, prodrug, or solvate thereof in the manufacture of a medicament for treating or preventing rosacea.
7. Use of the compound represented by formula (I) according to embodiment 1 and a pharmaceutically acceptable salt, prodrug, or solvate thereof in the manufacture of a medicament for treating or preventing urticaria.
8. Use of the compound represented by formula (I) according to embodiment 1 and a pharmaceutically acceptable salt, prodrug, or solvate thereof in the manufacture of a medicament for treating or preventing atopic dermatitis.
9. Use of the compound represented by formula (I) according to embodiment 1 and a pharmaceutically acceptable salt, prodrug, or solvate thereof in the manufacture of a medicament for treating or preventing psoriasis.
10. Use of the compound represented by formula (I) according to embodiment 1 and a pharmaceutically acceptable salt, prodrug, or solvate thereof in the manufacture of a medicament for treating or preventing radiation dermatitis.
11. Use of the compound represented by formula (I) according to embodiment 1 and a pharmaceutically acceptable salt, prodrug, or solvate thereof in the manufacture of a medicament for treating or preventing vitiligo.
12. Use of the compound represented by formula (I) according to embodiment 1 and a pharmaceutically acceptable salt, prodrug, or solvate thereof in the manufacture of a medicament for treating or preventing alopecia areata.
13. Use of the compound represented by formula (I) according to embodiment 1 and a pharmaceutically acceptable salt, prodrug, or solvate thereof in the manufacture of a medicament for treating or preventing inflammatory bowel disease.
14. Use of the compound represented by formula (I) according to embodiment 1 and a pharmaceutically acceptable salt, prodrug, or solvate thereof in the manufacture of a medicament for treating or preventing a disease related to the MRGPRX2 target.
15. Use of the compound represented by formula (I) according to embodiment 1 and a pharmaceutically acceptable salt, prodrug, or solvate thereof in the manufacture of a medicament for treating or preventing radiation stomatitis.
16. Use of the compound represented by formula (I) according to embodiment 1 and a pharmaceutically acceptable salt, prodrug, or solvate thereof in the manufacture of a medicament for treating or preventing inflammatory response.
17. Use of the compound represented by formula (I) according to embodiment 1 and a pharmaceutically acceptable salt, prodrug, or solvate thereof in the manufacture of a medicament for treating or preventing anaphylactoid purpura.
18. Use of the compound represented by formula (I) according to embodiment 1 and a pharmaceutically acceptable salt, prodrug, or solvate thereof in the manufacture of a medicament for treating or preventing irritable bowel syndrome.
19. Use of the compound represented by formula (I) according to embodiment 1 and a pharmaceutically acceptable salt, prodrug, or solvate thereof in the manufacture of a medicament for treating or preventing allergic rhinitis.
20. Use of the compound represented by formula (I) according to embodiment 1 and a pharmaceutically acceptable salt, prodrug, or solvate thereof in the manufacture of a medicament for treating or preventing asthma.
21. Use of the compound represented by formula (I) according to embodiment 1 and a pharmaceutically acceptable salt, prodrug, or solvate thereof in the manufacture of a medicament for treating or preventing multiple sclerosis.
22. Use of the compound represented by formula (I) according to embodiment 1 and a pharmaceutically acceptable salt, prodrug, or solvate thereof in the manufacture of a medicament for treating or preventing systemic lupus erythematosus.
23. Use of the compound represented by formula (I) according to embodiment 1 and a pharmaceutically acceptable salt, prodrug, or solvate thereof in the manufacture of a medicament for treating or preventing rheumatoid arthritis.
24. Use of the compound represented by formula (I) according to embodiment 1 and a pharmaceutically acceptable salt, prodrug, or solvate thereof in the manufacture of a medicament for treating or preventing autoimmune liver disease.
25. Use of the compound represented by formula (I) according to embodiment 1 and a pharmaceutically acceptable salt, prodrug, or solvate thereof in the manufacture of a medicament for treating or preventing autoimmune nephropathy.

### Definitions

The following words, phrases, and symbols used in the present disclosure have the meanings as set forth below, unless the context indicates otherwise.

As used herein, the singular forms "a", "an", "the"and "said" are intended to include the plural forms as well, unless the context clearly indicates otherwise.

As used herein, "comprising", "containing", "including", "having", and variations thereof are intended to indicate the presence of the stated features, components, or steps, but they do not preclude the presence or addition of one or more additional features, components, or steps. Unless otherwise specified, "comprising", "containing", "including", or "having" as used herein encompasses the situation consisting of the stated features, components, or steps.

In accordance with the conventions in this art, 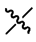 used in structural formulas in the present application, is used to depict the bond at the point of attachment of a moiety or substituent to the core or backbone structure.

The term "halogen" as used herein represents F, Cl, Br, and I, preferably F or Cl.

The term "heteroatom" as used herein represents N, O, and S.

As used herein, the term "alkyl" refers to a linear or branched saturated hydrocarbon group, which is, for example, a linear or branched saturated hydrocarbon chain group containing 1 to 20 carbon atoms (C₁₋₂₀), 1 to 15 carbon atoms (C₁₋₁₅), 1 to 12 carbon atoms (C₁₋₁₂), 1 to 10 carbon atoms (C₁₋₁₀), 1 to 9 carbon atoms (C₁₋₉), 3 to 9 carbon atoms (C₃₋₉), 1 to 6 carbon atoms (C₁₋₆) or 1 to 3 carbon atoms (C₁₋₃). Wherein the term "C_{x~y}alkyl" refers to a linear or branched saturated hydrocarbon group containing x to y carbon atoms. For example, "C₁₋₁₂ alkyl" or "C₁₋₁₂ linear or branched alkyl" refers to a linear or branched saturated hydrocarbon group containing 1 to 12 carbon atoms, and "C₁₋₆ alkyl" or "C₁₋₆ linear or branched alkyl" refers to a linear or branched saturated hydrocarbon containing 1 to 6 (1, 2, 3, 4, 5 or 6) carbon atoms. Examples of alkyl include, but are not limited to: methyl, ethyl, n-propyl, isopropyl, n-butyl, sec-butyl, isobutyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, and n-decyl.

As used herein, the term "alkenyl" refers to a linear or branched unsaturated hydrocarbon group containing one or more carbon-carbon double bonds, such as a linear or branched unsaturated hydrocarbon group having 2 to 25 carbon atoms , 2 to 20 carbon atoms (C₂₋₂₀), 2 to 15 carbon atoms (C₂₋₁₅), 2 to 12 carbon atoms (C₂₋₁₂), 2 to 10 carbon atoms (C₂₋₁₀), 2 to 9 carbon atoms (C₂₋₉), 3 to 9 carbon atoms (C₃₋₉), 2 to 6 carbon atoms (C₂₋₆) or 2 to 3 carbon atoms (C₂₋₃). For example, these groups may comprise 1, 2, 3, or 4 C=C double bonds. For example, "C₂₋₇ alkenyl" or "C₂₋₇ linear or branched alkenyl" refers to a linear or branched alkenyl containing 2-7 carbon atoms, "C₂₋₆alkenyl" or "C₂₋₆ linear or branched alkenyl" refers to a linear or branched alkenyl containing 2-6 carbon atoms. Examples of alkenyl include, but are not limited to, vinyl, allyl, hexenyl, and the like.

As used herein, the term "alkynyl" refers to a linear or branched unsaturated hydrocarbon group containing one or more carbon-carbon triple bonds, such as a linear or branched unsaturated hydrocarbon group having 2 to 20 carbon atoms (C₂₋₂₀), 2 to 15 carbon atoms (C₂₋₁₅), 2 to 12 carbon atoms (C₂₋₁₂), 2 to 10 carbon atoms (C₂₋₁₀), 2 to 9 carbon atoms (C₂₋₉), 3 to 9 carbon atoms (C₃₋₉), 2 to 6 carbon atoms (C₂₋₆) or 2 to 3 carbon atoms (C₂₋₃). For example, these groups may comprise 1, 2, 3, or 4 C=C double bonds. For example, "C₂₋₆alkynyl" or "C₂₋₆ linear or branched alkynyl" represents a linear or branched alkynyl having 2-6 carbon atoms. Examples of alkynyl include, but are not limited to, ethynyl, 2-propynyl, and 2-butynyl.

As used herein, the term "alkoxy" refers to -O-alkyl, wherein the alkyl is as defined above, for example C₁₋₆alkoxy, C₁₋₃alkoxy. For example, C₂ alkoxy is -OCH₂CH₃. Examples of alkoxy include, but are not limited to, methoxy, ethoxy, n-propoxy, isopropoxy, n-butoxy, s-butoxy, t-butoxy, n-pentoxy, s-pentoxy, n-heptoxy, and n-octoxy.

As used herein, the term "haloalkyl" refers to a linear or branched alkyl as defined herein in which one or more hydrogen atoms, for example, 1, 2, 3, 4, or 5 hydrogen atoms, are replaced by halogen atoms, and when more than one hydrogen atom is replaced by halogen atoms, the halogen atoms may be the same or different from each other. Examples of haloalkyl include, but are not limited to, -(CH₂)₀₋₈CF₃, -CF₃, -CHF₂, -CH₂F, -CH₂CF₃, -(CH₂)₂CF₃, -(CH₂)₃CF₃ and -(CH₂)₄CF₃, -(CH₂)₂CH₂Cl. The haloalkyl is preferably C₁₋₆ trifluoroalkyl, more preferably -CF₃.

As used herein, the term "cycloalkyl" refers to a saturated cyclic hydrocarbon group having 3-10 ring atoms (3- to 10-membered), such as 3-9 ring atoms (3- to 9-membered), 3-7 ring atoms (3- to 7-membered), 3-6 ring atoms (3- to 6-membered) or 5-6 ring atoms (5- to 6-membered), which may comprise one or more rings, for example, 1 or 2 rings. For example, the cycloalkyl is a monocyclic cycloalkyl, preferably a monocyclic C₃₋₇ cycloalkyl, a monocyclic C₃₋₆ cycloalkyl or a monocyclic C₅₋₆ cycloalkyl, such as cyclopropyl, cyclobutyl, cyclopentyl, cyclohexyl. For example, the cycloalkyl is a bicyclic cycloalkyl, such as a bicyclic C₆-C₁₀ cycloalkyl. Bicyclic cycloalkyl includes fused ring, bridged ring, or spiro ring. Examples of bridged cycloalkyl include, but are not limited to: bicyclo[3.1.1]heptane, bicyclo[2.2.1]heptane, bicyclo[2.2.2]octane, bicyclo[3.2.2]nonane, bicyclo[3.3.1]nonane, bicyclo[4.2.1]nonane, tricyclo[3.3.1.03,7]nonane(octahydro-2,5_ methanopentalene or noradamantane) and tricyclo[3.3.1.13,7]decane (adamantane).

As used herein, the term "heterocycloalkyl" refers to a saturated ring having 4-10 ring atoms (4- to 10-membered), such as 4-9 ring atoms (4- to 9-membered), 4-7 ring atoms (4- to 7-membered), 4-6 ring atoms (4- to 6-membered), 5-7 ring atoms (5- to 7-membered), 5-6 ring atoms (5- to 6-membered) or 7-9 ring atoms (7- to 9-membered) , wherein one or more, such as 1, 2, or 3, preferably 1 or 2 of the ring atoms are heteroatoms independently selected from N, O, and S, and the remaining ring atoms are carbon atoms; it may comprise one or more rings, for example 1, 2, or 3, or 1 or 2 rings, wherein the N and S heteroatoms can optionally be oxidized to various oxidation states. The point of attachment of the heterocyclic group may be at the N heteroatom or on the C atom. The heterocyclic group also includes fused ring, bridged ring, or spiro ring. Examples of "heterocycloalkyl" include, but are not limited to: azetidinyl, oxetanyl, pyrrolidinyl, imidazolidinyl, oxazolidinyl, tetrahydrofuranyl, oxazolidinyl, thiazolidinyl, piperidinyl, piperazinyl, hexahydropyrimidinyl, tetrahydropyranyl, morpholinyl and thiomorpholinyl. For example, a 4- to 6-membered heterocycloalkyl contains 1, 2, or 3, for example 1 or 2 heteroatoms selected from N, O, or S (e.g., selected from N or O; or selected from N), which is optionally substituted with one or more halogens, for example, substituted with 2 F on the same C atom. Examples of 4- to 6-membered heterocycloalkyl are, for example, azetidinyl, pyrrolidinyl, piperidinyl, piperazinyl, morpholinyl, difluoroazetidinyl or 3,3-difluoroazetidinyl. For example, 5- to 6-membered heterocycloalkyl contains 1, 2, or 3, for example 1 or 2 heteroatoms selected from N, O, or S (e.g., selected from N or O; or selected from O). Examples of 5- to 6-membered heterocycloalkyl are, for instance, tetrahydropyranyl (e.g., tetrahydrofuran-4-yl).

The term "aryl" as used herein refers to a monocyclic or polycyclic fused aromatic carbocyclic group, such as phenyl or naphthyl.

The term "heteroaryl" as used herein refers to an aromatic ring group having 5-6 ring atoms (5-to 6-membered), 5 ring atoms (5-membered), or 6 ring atoms (6-membered), wherein one or more, for example 1, 2, or 3, preferably 1 or 2 ring atoms are heteroatoms independently selected from N, O, and S, and the remaining ring atoms are carbon, wherein the N or S heteroatom is optionally oxidized to various oxidation states. Examples of heteroaryl include, but are not limited to: furanyl (including but not limited to: furan-2-yl), imidazolyl (including but not limited to: *1H*-imidazol-1-yl), isoxazolyl, isothiazolyl, oxadiazolyl, 1,3-oxazolyl, pyridyl (e.g., pyridin-4-yl, pyridin-2-yl and pyridin-3-yl), pyridazinyl, pyrimidinyl, pyrazinyl, pyrazolyl, pyrrolyl, tetrazolyl, thiadiazolyl, 1,3-thiazolyl, thienyl (including but not limited to: thiophen-2-yl and thiophen-3-yl), triazolyl, and triazinyl, for example:

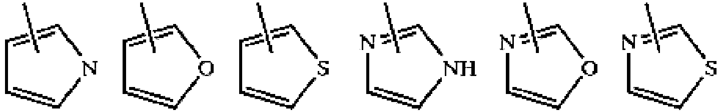

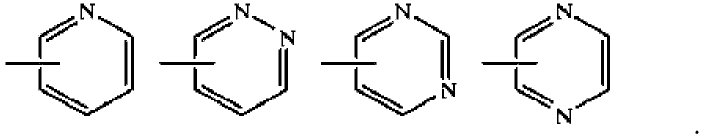

The terms "optional", or "optionally" as used herein mean that the subsequently described event or circumstance may or may not occur, and that the description includes instances where the event or circumstance occurs and instances where it does not. For example, "optionally substituted" includes "unsubstituted" and "substituted with 1, 2, 3, or more...". It is understood by those skilled in the art with respect to any group containing one or more substituents, such group does not include any substitution or substitution patterns that are sterically impractical, chemically incorrect, synthetically non-feasible, and/or inherently unstable.

The term "substituted" or "substituted with..." as used herein means that one or more hydrogen atoms on a given atom or group are replaced by one or more substituents independently selected from the specified group of substituents, provided that the normal valence of the given atom is not exceeded. The term "substituted with 1, 2, or 3..." means that 1, 2, or 3 hydrogens on a given atom or group are replaced by 1, 2, or 3 substituents independently selected from the specified group of substituents, provided that the normal valence of the specified atom is not exceeded. Such combinations are permissible only if the combination of each substituent and/or each variable results in a chemically correct and stable compound. A chemically correct and stable compound means a compound that is robust enough to be isolated from a reaction mixture.

The compounds of the present disclosure may exist in an isotopically labeled or enriched form, comprising one or more atoms having an atomic mass or mass number different from the atomic mass or mass number predominantly found in nature. Isotopes can be radioactive or non-radioactive isotopes. Isotopes of atoms such as hydrogen, carbon, phosphorus, sulfur, fluorine, chlorine, and iodine include, but are not limited to: ²H, ³H, ¹³C, ¹⁴C, ¹⁵N, ¹⁸O, ³²P, ³⁵S, ¹⁸F, ³⁶Cl and ¹²⁵I. Compounds comprising these isotopes and/or other isotopes of other atoms are within the scope of the present invention.

It is understood by those skilled in the art that some of the compounds disclosed herein may contain one or more chiral centers or rings, and thus exist as two or more stereoisomers. Racemic mixtures, individual isomers and enantiomerically enriched mixtures of these isomers, as well as diastereomers and specific diastereomerically partially enriched mixtures when two chiral centers are present, are all within the scope of the present disclosure. It should also be understood by those skilled in the art that the present disclosure encompasses all individual stereoisomers (e.g., enantiomers, diastereomers, cis- or trans-isomers (e.g., the configuration of substituents on divalent saturated or partially saturated cyclic groups), or atropisomers, as long as chemically feasible) of the compounds of formula (I), racemates of the compounds of the present disclosure, mixtures thereof, and, if appropriate, their individual tautomeric forms.

The term "pharmaceutically acceptable salt" as used herein includes, but is not limited to: acid addition salts formed by the compounds disclosed herein with inorganic acids, such as hydrochloride, hydrobromide, carbonate, bicarbonate, phosphate, sulfate, sulfite, nitrate, and the like; and acid addition salts formed by the compounds disclosed herein with organic acids, such as formate, acetate, malate, maleate, fumarate, tartrate, succinate, citrate, lactate, methanesulfonate, p-toluenesulfonate, 2-hydroxyethanesulfonate, benzoate, salicylate, stearate, and salts formed with alkanedicarboxylic acids of formula HOOC-(CH₂)ₙ-COOH (wherein n is 0-4), and the like. "Pharmaceutically acceptable salt" also includes base addition salts formed by the compounds of the present disclosure having acidic groups with pharmaceutically acceptable cations, such as sodium, potassium, calcium, aluminum, lithium, and ammonium.

The term "subject" or "individual" as used herein denotes mammals and non-mammals. Mammals denote any member of Mammalian class, including but not limited to humans; non-human primates such as chimpanzees and other apes and monkey species; farm animals such as cattle, horses, sheep, goats, and swine; domestic animals such as rabbits, dogs, and cats; laboratory animals including rodents such as rats, mice, and guinea pigs; and the like. In some embodiments, the subject is a human.

All numerical ranges herein are to be understood as disclosing each and every value within the range, as well as each and every subset of values within the range, whether or not they are otherwise specifically disclosed. For example, reference to any numerical range should be considered as referring to each and every numerical value within the numerical range, for example, every integer within the numerical range. The present disclosure encompasses all values falling within these ranges, all narrower ranges, and the upper or lower limits of the range.

Technical and scientific terms not specifically defined herein have the meanings commonly understood by those skilled in the art to which the present invention pertains.

The various embodiments described in the present disclosure and the features in the various embodiments should be understood as being capable of being combined with each other in any manner. The resulting individual technical solutions obtained from such combination are included within the scope of the present disclosure as if these combined solutions were specifically and individually set forth herein, unless the context clearly indicates otherwise.

All patents, patent applications, and other documents cited or mentioned herein are incorporated by reference in their entirety to the extent permitted by law. The discussion of these references is intended merely to summarize the assertions made therein. No admission is made that any of the patents, patent applications, or documents, or any portion thereof, constitutes relevant material or prior art. The right to challenge the accuracy and relevanceof any assertions regarding such patents, patent applications, and other documents constitute relevant material or prior art is specifically reserved.

### Administration and Therapy

The compounds of the present disclosure (e.g., any compound in the Examples described herein) alone or in combination with one or more additional therapeutic agents, can be formulated into a pharmaceutical composition. The pharmaceutical composition comprises: (a) a compound of the present disclosure; (b) a pharmaceutically acceptable carrier (e.g., one or more pharmaceutically acceptable carriers); and optionally (c) at least one additional therapeutic agent.

Pharmaceutically acceptable carrier refers to an excipient or auxiliary material that is compatible with the active ingredients in the composition (in some embodiments, capable of stabilizing the active ingredients) and not deleterious to the subject being treated. Suitable pharmaceutically acceptable carriers are disclosed in standard reference texts in this art (e.g., Remington's Pharmaceutical Sciences, Remington: The Science and Practice of Pharmacy), and includ one or more of buffers, stabilizers, surfactants, wetting agents, lubricants, emulsifiers, suspending agents, preservatives, antioxidants, opacifiers, glidants, processing aids, colorants, sweeteners, perfuming agents, flavoring agents, diluents, and other known additives, to provide an elegant presentation of the drug (i.e., a compound of the present disclosure or the pharmaceutical composition thereof) or aid in the manufacturing of the pharmaceutical product (i.e., medicament).

The compounds of the present disclosure can be administered in various known manners, such as orally, topically, parenterally, by inhalation, or by implantation. The term "parenteral" as used herein includes subcutaneous, intradermal, intravenous, intramuscular, intraarticular, intra-arterial, intrasynovial, intrasternal, intrathecal, intralesional, and intracranial injection or infusion. Oral or topical administration is preferred, such as, topical administration to the skin or mucous membranes, for example, by external application.

The compounds of the present disclosure can be administered in any convenient formulation, such as tablets, capsules, ointments, creams, gels, pastes, emulsions, powders, patches, solutions, dispersions, suspensions, syrups, sprays, suppositories, and the like.

The exact dosage of the compound of the present disclosure capable of treating or preventing a disease or disorder mediated by MRGPRX2 depends on multiple factors, including the host, the nature and severity of the condition to be treated, and the mode of administration.

The beneficial technical effects achieved by the present invention include at least:
(1) Currently commercially available fluoroquinolone drugs, such as ciprofloxacin, are generally considered to be MRGPRX2 agonists, which causes some patients to experience side effects such as pruritus and rash after using fluoroquinolone compounds. However, the inventors of the present application have surprisingly found that the compounds provided in the present disclosure can act as MRGPRX2 antagonists for treating or preventing autoimmune diseases, allergic diseases, inflammatory diseases, pruritus, and pain, etc.
(2) Target MRGPRX2 is predominantly localized in barrier tissues. Through topical administration, such as topical administration to the skin, more targeted therapy against localized lesions can be brought, while the side effects caused by systemic absorption of the drug can also be reduced, thereby improving the therapeutic experience of patients.

### General Synthetic Methods

Different synthetic routes were applied in the preparation of the compounds of the present invention. The compounds of formulas (IA), (IB), and (I) can be synthesized using the following synthetic methods.

The variables therein are as defined in formula (IA), (IB), or (I). For example, for formula (IA), R₉ is correspondingly C₁₋₆ linear or branched alkyl or hydrogen, preferably C₁₋₃ linear or branched alkyl, R₃ is halogen; Ring A is further substituted with R₄ and (R₅)ₙ (not shown in the scheme), and B₂ is absent; for instance, for formula (IB), R₉ is correspondingly C₁₋₆ linear or branched alkyl or hydrogen, preferably C₁₋₃ linear or branched alkyl, R₃ is halogen; Ring A is further substituted with R₄ (not shown in the scheme), and B₂ is absent.

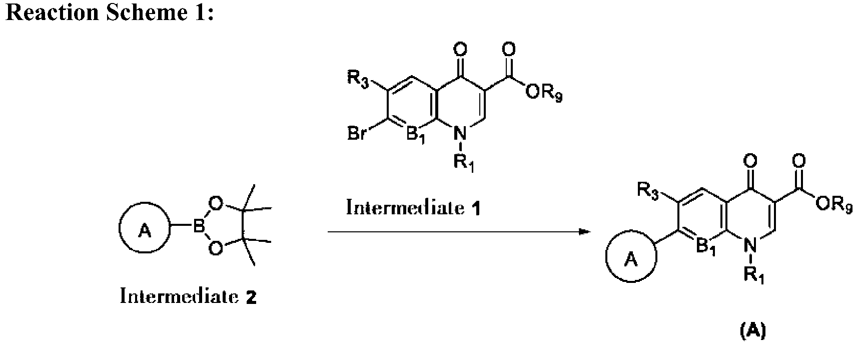

The synthesis is carried out by reacting Intermediate 1 and Intermediate 2 via a Suzuki reaction in the presence of a base such as K₂CO₃, Na₂CO₃, Cs₂CO₃, etc., and a palladium catalyst such as 1,1-bis(diphenylphosphino)ferrocene palladium (II) dichloride, palladium (II) chloride and palladium (II) acetate, in a solvent such as DMF, DMSO, THF, dichloromethane, acetonitrile, etc. The reaction can be carried out under atmospheric pressure in an inert gas atmosphere (including nitrogen, argon, etc.). The reaction is generally carried out at a temperature ranging from room temperature to about 150°C for about 16 to 24 hours.

Reaction Scheme 1 also corresponds to the case for the compound of formula (I), wherein B₂ is absent (i.e., not present) and B₃ is CH.

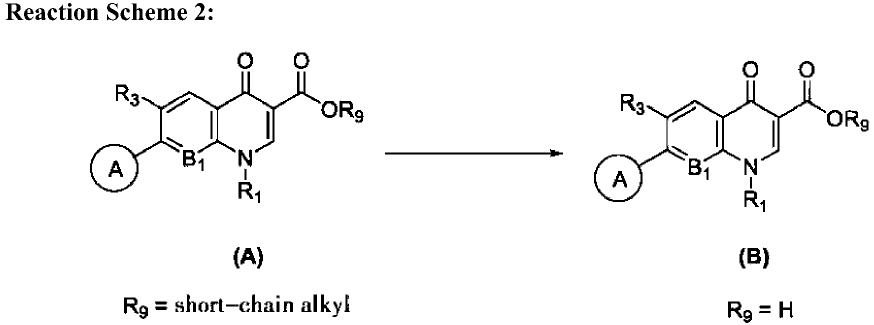

The compound of general formula (B) can be prepared by hydrolyzing the compound of formula (A). The hydrolysis of the compound of formula (A) can be carried out under conventional conditions for hydrolysis reaction, for example, in the presence of a basic compound such as LiOH, NaOH, KOH, etc., in a solvent including dichloromethane, an alcohol such as methanol, DMF, DMSO, THF, etc. The reaction is generally carried out at a temperature ranging from room temperature to about 60 °C for about 0.1 to 24 hours.

Reaction Scheme 2 also corresponds to the case for the compound of formula (I), wherein B₂ is absent (i.e., not present) and B₃ is CH.

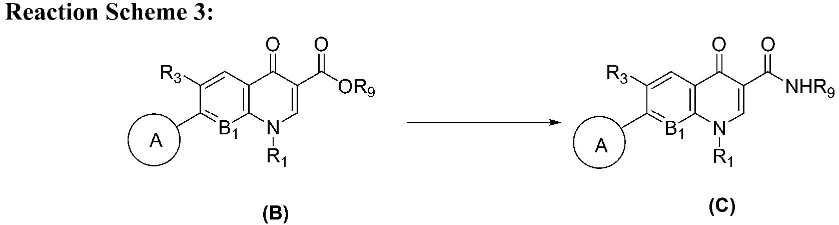

The compound of general formula (C) can be synthesized from the compound of general formula (B) through condensation reaction. The compound of formula (B) can react with an appropriate amine in the presence of a condensing agent (e.g., DMAP, HATU, etc.) and in a solvent including dichloromethane, DMF, THF, etc. The reaction is generally carried out at room temperature for about 3 to 12 hours.

Reaction Scheme 3 corresponds to the case for the compound of formula (I), wherein B₂ is absent (i.e., not present) and B₃ is CH.

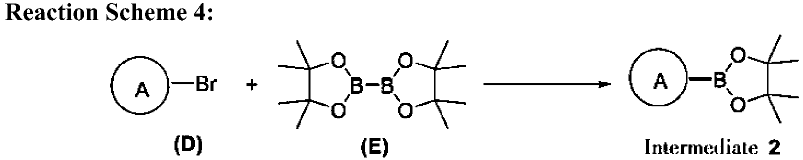

Intermediate 2 can be prepared by reacting the compound of formula (D) with a bis(pinacolato)diboron compound (E) in the presence of a palladium catalyst and a base in a typical inert solvent. The reaction is carried out under atmospheric pressure in an inert gas atmosphere including nitrogen, argon, etc. The reaction can generally be carried out at a temperature ranging from room temperature to about 150 °C for about 16 to 24 hours. 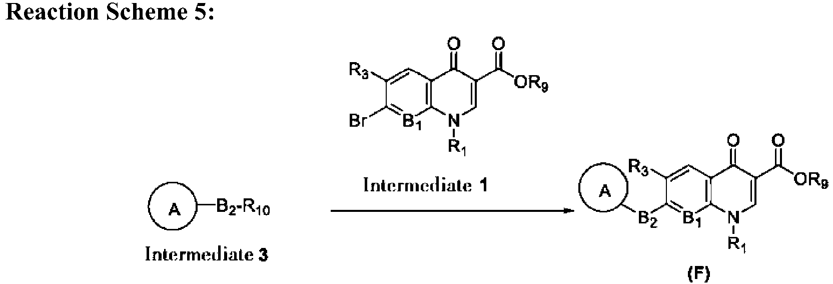 wherein R₇ is hydrogen or halogen.

The compound of formula (F) can be synthesized by reacting Intermediate 3 and Intermediate 1 via a Buchwald reaction, Kumada reaction, Negishi reaction, or Ullmann reaction in the presence of a base such as K₂CO₃, Na₂CO₃, Cs₂CO₃, LDA, NaH, etc., and a copper catalyst such as CuI, Cu(I), or a palladium catalyst such as 1,1-bis(diphenylphosphino)ferrocene palladium (II) dichloride, palladium (II) chloride, and palladium (II) acetate, etc., in a solvent such as DMF, DMSO, THF, toluene, and the like. The reaction can be carried out under atmospheric pressure in an inert gas atmosphere including nitrogen, argon, etc. The reaction is generally carried out at a temperature ranging from room temperature to about 80-150 °C for about 16-24 hours.

Reaction Scheme 5 corresponds to the case for the compound of formula (I), wherein B₂ is absent (i.e., not present) and B₃ is CH.

### Examples

In the present application, in the case of a chemical name is inconsistent with a structural formula, the structural formula shall prevail, unless it can be inferred from the context that the chemical name rather than the structural formula, is correct.

All non-aqueous reactions were carried out using oven-dried glassware under a dry argon environment. The progress of the reaction was monitored using thin-layer chromatography on glass plates coated with general reagent silica gel 60. Flash column chromatography was performed on general reagent silica gel. Mass spectra of the compounds were recorded using an Agilent G7115A liquid chromatography-mass spectrometer. NMR spectra at ambient temperature were recorded using a Bruker Avance 400 spectrometer. ¹H chemical shifts are reported in parts per million (δ) relative to external tetramethylsilane, and are referenced to the signal of residual protons in the deuterated solvent.

Unless otherwise specified, the experimental materials and reagents used in the following examples are all commercially available.

### Preparation of Intermediate Compound 7:

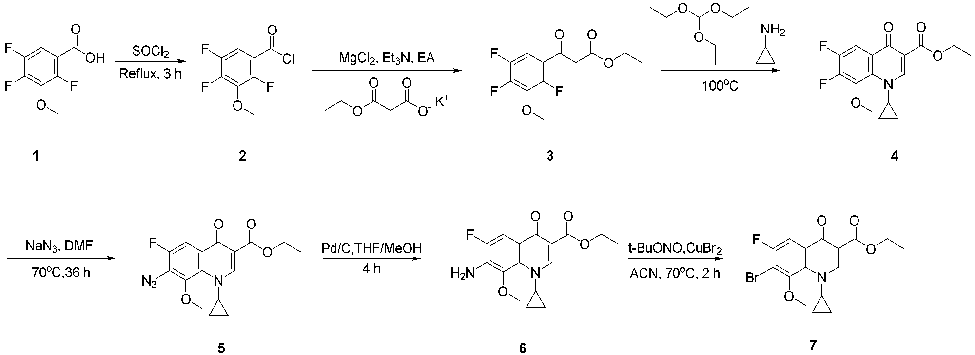

### Preparation of 2,4,5-trifluoro-3-methoxybenzoyl chloride (2)

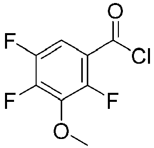

To 2,4,5-trifluoro-3-methoxybenzoic acid (1) (30.0 g, 145.5 mmol) was added a solution of SOCl₂ (180 mL), and the mixture was stirred at 85 °C for 3 hours. After cooling to room temperature, the solvent was removed by evaporation. The residue was dried under vacuum to dryness, to yield a pale yellow oil (32.0 g, 142.5 mmol, yield 97.9%), which was directly used in the next step without further purification.

### Preparation of ethyl 3-oxo-3-(2,4,5-trifluoro-3-methoxyphenyl)propanoate (3)

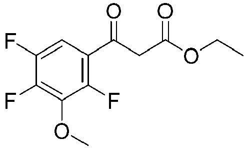

To a solution of potassium 3-ethoxy-3-oxopropanoate (2) (48.5 g, 284.9 mmol) in EA (300 mL) was added MgCl₂(33.9 g, 356.1 mmol). The mixture was stirred at room temperature under a nitrogen protection for 30 minutes. Trimethylamine (28.8 g, 284.6 mmol) was then added to the mixture, and the mixture was stirred at room temperature for another 30 minutes. 2,4,5-Trifluoro-3-methoxybenzoyl chloride (32.0 g, 142.5 mmol) was added dropwise to the mixture, and the resulting mixture was stirred at 85°C for 2 hours. After the mixture was cooled to room temperature, water (150 ml) was added, and the mixture acidified with HCl (2M) (pH=1-2). The mixture was extracted with EA (200 mL), and the organic phase was dried over Na₂SO₄, filtered, and the solvent was removed under reduced pressure to obtain the crude product (58.0 g) as a brown oil.

### Preparation of ethyl 1-cyclopropyl-6,7-difluoro-8-methoxy-4-oxo-1,4-dihydroquinoline-3-carboxylate (4)

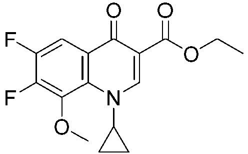

To a solution of ethyl 3-oxo-3-(2,4,5-trifluoro-3-methoxyphenyl)propanoate (3) (58.0 g, 210.0 mmol) in Ac₂O(64.3 g, 630.0 mmol) was added triethoxymethane (46.6 g, 314.4 mmol), and the mixture was stirred at 100°C for 2 hours. The solvent was removed under reduced pressure to yield a residue. The residue was dissolved in DMSO (400 ml), and cyclopropanamine (14.4 g, 252.2 mmol) was added. The mixture was stirred at room temperature for 2 hours. K₂CO₃ (43.4 g, 314.8 mmol) was then added to the mixture, which was stirred at 100°C for another 1 hour. After cooling to room temperature, water (500 mL) was added to the mixture, and the mixture was stirred for 10 minutes. The suspension was filtered to remove the filtrate, and the filtered solid was washed with water (50 mL) and dried *in vacuo* to yield the target product (21.0 g, 65.0 mmol, 45.6% yield, over two steps) as a white solid.

### Preparation of ethyl 7-azido-1-cyclopropyl-6-fluoro-8-methoxy-4-oxo-1,4-dihydroquinoline-3-carboxylate (5)

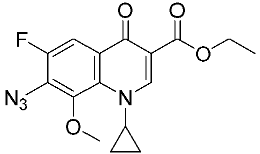

To a solution of ethyl 1-cyclopropyl-6,7-difluoro-8-methoxy-4-oxo-1,4-dihydroquinoline-3-carboxylate(4) (10.0 g, 30.9 mmol) in DMF (50 mL) was added NaN₃(4.02 g, 61.8 mmol), and the mixture was stirred at 70°C for 36 hours. After the reaction was complete and cooled to room temperature, the mixture was filtered through Celite. Water (50 mL) was added to the filtrate, and the mixture was extracted with EA (70 mL x3). The organic phase was washed with water (50 ml x3) and brine (50 mL x3), dried over Na₂SO₄, filtered, and concentrated under reduced pressure to yield the crude product (5.6 g) as a brown solid.

### Preparation of ethyl 7-amino-1-cyclopropyl-6-fluoro-8-methoxy-4-oxo-1,4-dihydroquinoline-3-carboxylate (6):

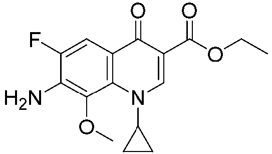

To a solution of ethyl 7-azido-1-cyclopropyl-6-fluoro-8-methoxy-4-oxo-1,4-dihydroquinoline-3-carboxylate (5) (5.6 g, crude product) in THF/MeOH (50 mL/10 mL) was added Pd/C (2.8 g, 10% wt). The mixture was purged with hydrogen for three times, and stirred at room temperature under hydrogen (balloon) for 4 hours. The mixture was filtered, and the solvent was removed under reduced pressure. The residue was purified on silica gel by column chromatography (DCM/MeOH=50/1-20/1) to yield a white solid (3.8 g, 11.86 mmol, 38.3% yield, over two steps).

### Preparation of ethyl 7-bromo-1-cyclopropyl-6-fluoro-8-methoxy-4-oxo-1,4-dihydroquinoline-3-carboxylate (7):

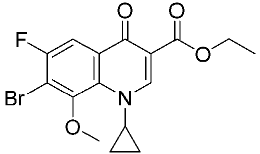

To a solution of ethyl 7-amino-1-cyclopropyl-6-fluoro-8-methoxy-4-oxo-1,4-dihydroquinoline-3-carboxylate (6) (3.80 g, 11.86 mmol) in ACN (50 mL) were added tert-butanol (1.84 g, 17.84 mmol) and CuBr₂ (5.30 g, 23.73 mmol), and the mixture was stirred at 70 °C for 2 hours. After the mixture was cooled to room temperature, it was diluted with saturated NH₄Cl solution (25 mL), and extracted with EA (50 mL x3). The organic phase was washed with brine (30 mL x3), dried over Na₂SO₄, filtered and concentrated under reduced pressure to yield a residue. The residue was dissolved in DCM/MTBE (10mL/610ml), and the mixture was stirred at room temperature for 10 minutes. The suspension was filtered to remove the filtrate, and the filtered solid was dried *in vacuo* to yield the target product (2.0 g, 5.21 mmol, 43.9%) as a yellow solid.

LCMS: 386.1[M+H]⁺. ¹H NMR (400 MHz, MeOD) δ 8.77 (s, 1H), 7.92 (d, *J =* 8.5 Hz, 1H), 4.33 (q, *J =* 7.1 Hz, 2H), 4.14 - 4.10 (m, 1H), 3.94 (s, 3H), 1.37 (t, *J =* 7.1 Hz, 3H), 1.26 - 1.16 (m, 2H), 1.09 - 0.99 (m, 2H).

### Preparation of Intermediate Compound 9:

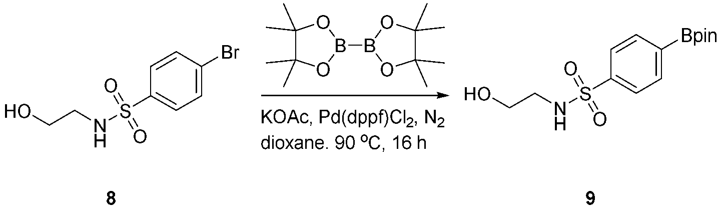

### Preparation of N-(2-hydroxyethyl)-4-(4,4,5,5-tetramethyl-1,3,2-dioxaborolan-2-yl)benzenesulfonamide (9)

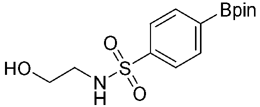

To a mixture of 4-bromo-N-(2-hydroxyethyl)benzenesulfonamide (8) (1 g, 3.57 mmol) in 1,4-dioxane (10 mL) were added B₂Pin₂ (1.2 g, 4.28 mmol), KOAc (1.05 g, 10.71 mmol) and Pd(dppf)Cl₂ (259.04 mg, 356.97 mmol) at room temperature. The mixture was then heated to 90°C and stirred for 16 hours. After the reaction was complete, it was cooled to room temperature and concentrated under reduced pressure. The crude product was purified on silica gel by column chromatography (DCM:MeOH=20:1) to obtain the title product (582 mg, 312.24 mmol, 58.2%) as a brown solid. LCMS: 327.9[M+H]⁺.

A series of derivatives can be obtained by replacing the starting materials with the corresponding ones, through the same preparative route as described above.

### Example 1 Ethyl 1-cyclopropyl-6-fluoro-7-(4-(N-(2-hydroxyethyl)aminosulfonyl)phenyl)-8-methoxy-4-oxo-1,4-d ihydroquinoline-3-carboxylate

The synthetic route is as shown below:

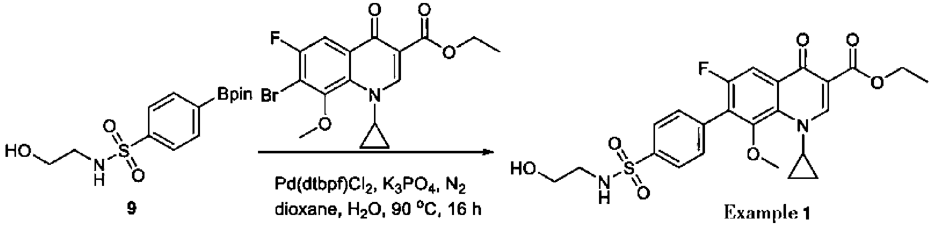

To the mixture of N-(2-hydroxyethyl)-4-(4,4,5,5-tetramethyl-1,3,2-dioxaborolan-2-yl)benzenesulfonamide (9) (146 mg, 312.24 umol) in 1,4-dioxane (3 mL) and H₂O (1 mL) were added ethyl 7-bromo-1-cyclopropyl-6-fluoro-8-methoxy-4-oxo-1,4-dihydroquinoline-3-carboxylate (7) (100 mg, 260.28 umol), K₃PO₄ (165.74 mg, 780.84 umol) and Pd(dtbpf)Cl₂ (16.81 mg, 26.03 umol) at room temperature. The mixture was then heated to 90°C and stirred for 16 hours. The reaction mixture was then cooled to room temperature and concentrated under reduced pressure. The crude was purified on silica gel by column chromatography, eluting with a gradient of DCM:MeOH - 1:0 to 20:1 to obtain the product (66 mg, 130.81 umol, 35.18%) as a brown solid.

LCMS: 505.1[M+H]⁺. ¹H NMR (400 MHz, DMSO-d6) δ 8.58 (s, 1H), 7.96 (d, *J =* 8.3 Hz, 2H), 7.86 - 7.68 (m, 3H), 4.72 (t*, J =* 5.6 Hz, 1H), 4.25 (q, *J =* 7.1 Hz, 2H), 4.05 (dt, *J =* 6.4, 2.5 Hz, 1H), 3.47 - 3.23 (m, 5H), 2.88 (t, *J =* 6.1 Hz, 2H), 1.29 (t, *J =* 7.1 Hz, 3H), 1.36 - 1.03 (m, 7H).

### Example 2 1-Cyclopropyl-6-fluoro-7-(4-(N-(2-hydroxyethyl)aminosulfonyl)phenyl)-8-methoxy-4-oxo-1,4-dihydroquinoline-3-carboxylic acid

The synthetic route is as shown below:

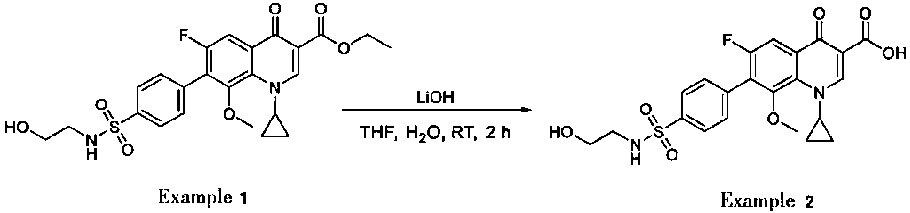

To a solution of ethyl 1-cyclopropyl-6-fluoro-7-(4-(N-(2-hydroxyethyl)aminosulfonyl)phenyl)-8-methoxy-4-oxo-1,4-dihy droquinoline-3-carboxylate (66 mg, 130.81 umol) in THF (3 mL) were added H₂O(1 mL) and LiOH (12.53 mg, 523.26 umol). The reaction solution was stirred at room temperature for 2 h. After completion of the reaction, the pH was adjusted to neutral. The reaction solution was diluted with DCM (10 mL), and then washed with water (10 mL X 3). The organic phase was dried over anhydrous sodium sulfate, filtered, and concentrated under reduced pressure to yield the product (22 mg, 46.17 umol, 35.30%) as a white solid.

LCMS: 477.2 [M+H]⁺. ¹H NMR (400 MHz, DMSO-d6) δ 14.61 (s, 1H), 8.82 (s, 1H), 7.98 (dd, *J* = 8.7, 4.7 Hz, 3H), 7.77 (dd, *J =* 13.3, 6.9 Hz, 3H), 4.72 (t, *J =* 5.2 Hz, 1H), 4.28 - 4.17 (m, 1H), 3.44 - 3.36 (m, 5H), 2.88 (q, *J =* 6.0 Hz, 2H), 1.23 - 1.12 (m, 4H).

### Example 3 7-(4-(N-(2-Chloroethyl)aminosulfonyl)phenyl)-1-cyclopropyl-6-fluoro-8-methoxy-4-oxo-1,4-di hydroquinoline-3-carboxylic acid

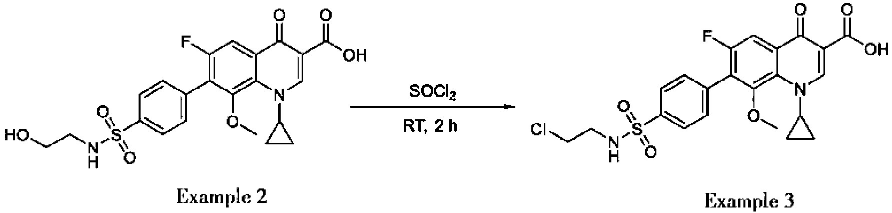

To a solution of 1-cyclopropyl-6-fluoro-7-(4-(N-(2-hydroxyethyl)aminosulfonyl)phenyl)-8-methoxy-4-oxo-1,4-dihy droquinoline-3-carboxylic acid (**Example 2**) (22 mg, 130.81 umol) in THF (3 mL) were added H₂O(1 mL) and SOCl₂(5 mL) at room temperature. After completion of the reaction detected by MS, the reaction mixture was concentrated under reduced pressure. The crude was purified by preparative liquid chromatography (0.1% FA, H₂O) to obtain the product (13.70 mg, 36.37 umol, 78.77%) as a white solid.

LCMS: 495.1 [M+H]⁺. ¹H NMR (400 MHz, DMSO-d6) δ 14.63 (s, 1H), 8.82 (s, 1H), 8.16 (d, *J* = 10.6 Hz, 1H), 7.98 (d, *J =* 11.1 Hz, 3H), 7.80 (d, *J =* 7.9 Hz, 2H), 4.21 (q, *J =* 5.8 Hz, 1H), 3.61 (t, *J =* 6.1 Hz, 2H), 3.39 (s, 3H), 3.21 - 3.17 (m, 2H), 1.20-1.13 (m, 4H).

### Example 4 7-(3-Cyano-4-(2,2,2-trifluoroacetamido)phenyl)-1-cyclopropyl-6-fluoro-8-methoxy-4-oxo-1,4-d ihydroquinoline-3-carboxylic acid

### Preparation of ethyl 7-(4-amino-3-cyanophenyl)-1-cyclopropyl-6-fluoro-8-methoxy-4-oxo-1,4-dihydroquinoline-3-carb oxylate

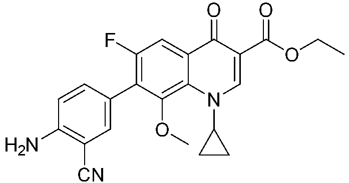

A mixture of ethyl 7-bromo-1-cyclopropyl-6-fluoro-8-methoxy-4-oxo-1,4-dihydroquinoline-3-carboxylate (200 mg, 561.57 umol), 2-amino-5-(4,4,5,5-tetramethyl-1,3,2-dioxaborolan-2-yl)benzonitrile (205.62 mg, 842.35 umol), Pd(dtbpf)Cl₂(36.26 mg, 56.16 umol), and K₃PO₄(357.6 mg, 1.68 mmol) in 1,4-dioxane (5 mL) and H₂O (1 mL) was stirred under nitrogen atmosphere at 90°C for 16 h. The reaction was cooled to room temperature, and then concentrated under reduced pressure. The crude product was purified on silica gel by column chromatography, eluting with DCM:MeOH = 20:1, to obtain the product (130 mg, 561.57 ummol, 54.93%) as a white solid. LCMS: 422.0[M+H]⁺.

### Preparation of 7-(4-amino-3-cyanophenyl)-1-cyclopropyl-6-fluoro-8-methoxy-4-oxo-1,4-dihydroquinoline-3-carb oxylic acid

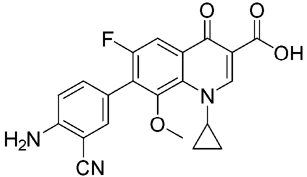

A mixture of ethyl 7-(4-amino-3-cyanophenyl)-1-cyclopropyl-6-fluoro-8-methoxy-4-oxo-1,4-dihydroquinoline-3-carb oxylate (130 mg, 308.47 umol), LiOH(29.55 mg, 1.23 umol) in THF(3 mL) and H₂O (1 mL) was stirred at room temperature for 2 hours. After completion of the reaction, the mixture was concentrated under reduced pressure. The crude product was purified by preparative liquid chromatography (0.1% NH₃-H₂O, H₂O) to obtain the product (22.73 mg, 308.47 umol, 18.73%) as a white solid. LCMS: 394.1[M+H]⁺.

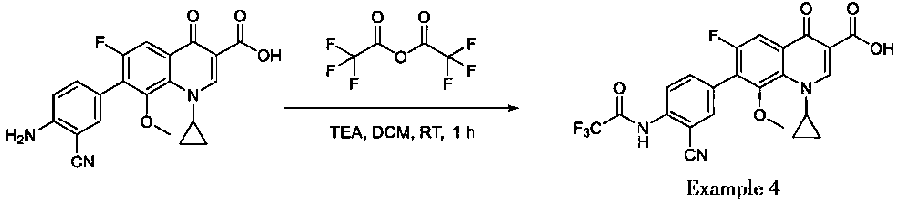

To a solution of 7-(4-amino-3-cyanophenyl)-1-cyclopropyl-6-fluoro-8-methoxy-4-oxo-1,4-dihydroquinoline-3-carb oxylic acid (35 mg, 88.97 umol) in DCM (3 mL) were added 2,2,2-trifluoroacetic anhydride (28.03 mg, 133.46 umol) and TEA (13.51 mg, 133.46 umol) at room temperature. The mixture was stirred at room temperature for 1 h and then concentrated under reduced pressure. The crude was purified by preparative high-performance liquid chromatography (0.1% formic acid in water) to obtain the product (17.35 mg, 88.97 umol, 39.85%) as a white solid.

LCMS: 489.8 [M+H]⁺. ¹H NMR (400 MHz, DMSO-d6) δ 14.60 (s, 1H), 11.94 (s, 1H), 8.82 (s, 1H), 8.21 (s, 1H), 8.06 - 7.91 (m, 2H), 7.81 (d, *J =* 8.4 Hz, 1H), 4.33 - 4.10 (m, 1H), 3.43 (s, 3H), 1.27 - 1.09 (m, 4H).

### Example 5

### 1-Cyclopropyl-6-fluoro-8-methoxy-4-oxo-7-(4-(N-(tetrahydro-2H-pyran-4-yl)aminosulfonyl)p henyl)-1,4-dihydroquinoline-3-carboxylic acid

The target product with the following structure was obtained according to the preparative processes described in Examples 1-4, replacing the starting materials with the corresponding ones:

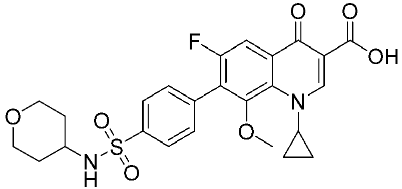

LCMS: 517.2 [M+H]⁺. ¹H NMR (400 MHz, DMSO-d6) δ 14.65 (s, 1H), 8.51 (s, 1H), 7.71 (d, J = 8.2 Hz, 1H), 7.69 - 7.62 (m, 2H), 7.52 - 7.44 (m, 2H), 6.88 (d, J = 11.7 Hz, 1H), 4.25 - 4.15 (m, 1H), 3.84 (s, 3H), 3.72 (dt, J = 12.1, 7.2 Hz, 2H), 3.57 (dt, J = 12.5, 7.0 Hz, 2H), 3.22 - 3.16 (m, 1H), 2.07 - 1.89 (m, 4H), 1.27 - 1.19 (m, 2H), 1.16 - 1.05 (m, 2H).

### Example 6 7-(3-Cyano-4-(N-cyclohexylaminosulfonyl)phenyl)-1-cyclopropyl-6-fluoro-8-methoxy-4-oxo-1, 4-dihydroquinoline-3-carboxylic acid

The target product with the following structure was obtained according to the preparative processes described in Examples 1-4, replacing the starting materials with the corresponding ones:

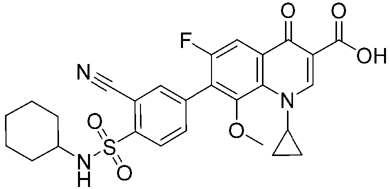

LCMS: 540.3 [M+H]⁺. ¹H NMR (400 MHz, DMSO-d6) δ 14.85 (s, 1H), 8.71 (s, 1H), 8.12 - 7.85 (m, 4H), 7.71 (dt, J = 7.4, 1.7 Hz, 1H), 7.26 (d, J = 12.1 Hz, 1H), 4.13 - 3.80 (m, 4H), 3.36 - 3.15 (m, 1H), 1.75 - 0.88 (m, 14H).

### Example 7 1-Cyclopropyl-6-fluoro-8-methoxy-4-oxo-7-(4-(piperidin-1-ylsulfonyl)phenyl)-1,4-dihydroquin oline-3-carboxylic acid

The target product with the following structure was obtained according to the preparative processes described in Examples 1-4, replacing the starting materials with the corresponding ones:

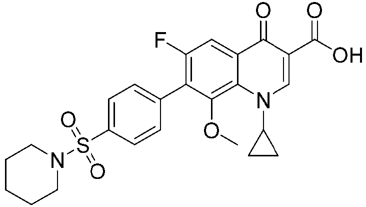

LCMS: 501.1 [M+H]⁺. ¹H NMR (400 MHz, DMSO-d6) δ 14.80 (s, 1H), 8.61 (s, 1H), 8.04 (d, J = 7.8 Hz, 1H), 7.74 - 7.67 (m, 2H), 7.47 - 7.35 (m, 2H), 4.20 - 4.10 (m, 1H), 3.85 (s, 3H), 2.96 - 2.80 (m, 4H), 1.67 - 1.46 (m, 6H), 1.29 - 1.05 (m, 4H).

### Example 8 1-Cyclopropyl-6-fluoro-8-methoxy-4-oxo-7-(4-(pyrrolidin-1-ylsulfonyl)phenyl)-1,4-dihydroqui noline-3-carboxylic acid

The target product with the following structure was obtained according to the preparative processes described in Examples 1-4, replacing the starting materials with the corresponding ones:

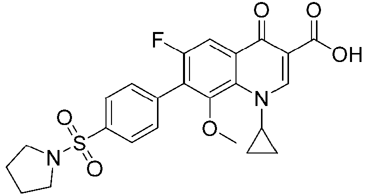

LCMS: 487.1 [M+H]⁺. ¹H NMR (400 MHz, DMSO-d6) δ 14.79 (s, 1H), 8.56 (s, 1H), 7.94 (d, J = 7.8 Hz, 1H), 7.73 - 7.65 (m, 2H), 7.51 - 7.43 (m, 2H), 4.21 - 4.11 (m, 1H), 3.81 (s, 3H), 3.21 - 3.11 (m, 4H), 1.80 - 1.66 (m, 4H), 1.39 - 1.24 (m, 2H), 1.18 - 1.02 (m, 2H).

### Example 9 7-(4-(Azetidin-1-ylsulfonyl)phenyl)-1-cyclopropyl-6-fluoro-8-methoxy-4-oxo-1,4-dihydroquino line-3-carboxylic acid

The target product with the following structure was obtained according to the preparative processes described in Examples 1-4, replacing the starting materials with the corresponding ones:

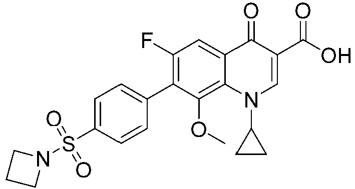

LCMS: 473.1 [M+H]⁺. 1H NMR (400 MHz, DMSO-d6) δ 14.77 (s, 1H), 8.62 (s, 1H), 7.94 (d, J = 7.8 Hz, 1H), 7.72 - 7.65 (m, 2H), 7.51 - 7.43 (m, 2H), 4.26 - 4.10 (m, 1H), 3.85 (s, 3H), 3.39 - 3.24 (m, 4H), 1.96 - 1.87 (m, 2H), 1.29 - 1.14 (m, 2H), 1.08 - 1.02 (m, 2H).

### Example 10 1-Cyclopropyl-7-(4-((3,3-difluoroazetidin-1-yl)sulfonyl)phenyl)-6-fluoro-8-methoxy-4-oxo-1,4-dihydroquinoline-3-carboxylic acid

The target product with the following structure was obtained according to the preparative processes described in Examples 1-4, replacing the starting materials with the corresponding ones:

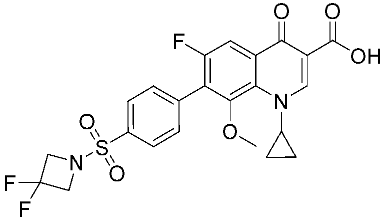

LCMS: 509.1 [M+H]⁺. ¹H NMR (400 MHz, DMSO-d6) δ 14.75 (s, 1H), 8.61 (s, 1H), 7.91 (d, J = 8.2 Hz, 1H), 7.76 - 7.68 (m, 2H), 7.51 - 7.43 (m, 2H), 4.23 - 4.13 (m, 1H), 3.83 - 3.64 (m, 7H), 1.29 - 1.02 (m, 4H).

Accroding to a similar processes, a series of compounds with structures shown below were synthesized in the present invention:

| **Ex.** | **Structure** | **Characterization Data** |
|---|---|---|
| **11** | 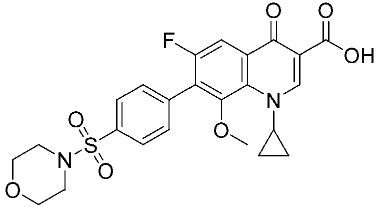 | LCMS: 503.1 [M+H]⁺ |
| | | ¹H NMR (400 MHz, DMSO-d6) δ 14.79 (s, 1H), 8.60 (s, 1H), 7.91 - 7.69 (m, 3H), 7.57 - 7.50 (m, 2H), 4.22 - 4.12 (m, 1H), 3.75 - 3.64 (m, 7H), 3.05 (t, J = 7.1 Hz, 3H), 1.29 - 1.20 (m, 2H), 1.14 - 1.00 (m, 2H). |
| **12** | 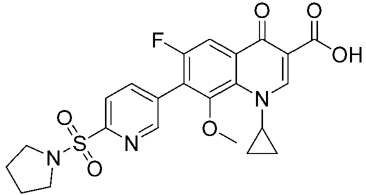 | LCMS: 488.1 [M+H]⁺ |
| | | ¹H NMR (400 MHz, DMSO-d6) δ 14.80 (s, 1H), 8.62 (s, 1H), 8.28 - 8.10 (m, 3H), 7.88 - 7.78 (m, 1H), 4.25 - 4.15 (m, 1H), 3.76 (s, 3H), 3.24 - 3.11 (m, 4H), 1.80 - 1.66 (m, 4H), 1.27 - 1.02 (m, 4H). |
| **13** | 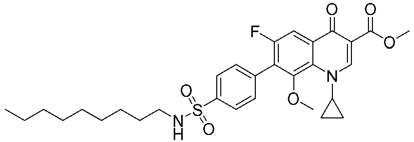 | LCMS: 573.3 [M+H]⁺ |
| | | ¹H NMR (400 MHz, DMSO-d6) δ 8.54 (s, 1H), 7.89 - 7.75 (m, 3H), 7.46 - 7.27 (m, 3H), 4.22 - 4.12 (m, 1H), 3.73 - 3.61 (m, 6H), 2.75 - 2.68 m, 2H), 1.55 - 1.02 (m, 18H), 0.95 - 0.83 (m, 3H). |
| **14** | 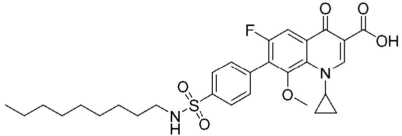 | LCMS: 559.2 [M+H]⁺ |
| | | ¹H NMR (400 MHz, DMSO-d6) δ 14.80 (s, 1H), 8.61 (s, 1H), 7.91 (d, J = 8.2 Hz, 1H), 7.79 - 7.72 (m, 2H), 7.60 - 7.51 (m, 2H), 7.27 - 7.22 (m, 1H), 4.22 - 4.12 (m, 1H), 3.74 (s, 3H), 2.73 (dt, J = 9.7, 7.1 Hz, 2H), 1.50 - 1.00 (m, 20H), 0.95 - 0.83 (m, 3H). |
| **15** | 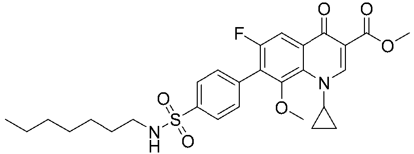 | LCMS: 545.2 [M+H]⁺ |
| | | ¹H NMR (400 MHz, DMSO-d6) δ 8.56 (s, 1H), 7.89 (d, J = 8.2 Hz, 1H), 7.79 - 7.72 (m, 2H), 7.58 - 7.51 (m, 2H), 7.27 - 7.22 (m, 1H), 4.22 - 4.12 (m, 1H), 3.73 (d, J = 9.4 Hz, 6H), 2.63 (dt, J = 9.7, 7.1 Hz, 2H), 1.51 - 1.14 (m, 13H), 1.08 - 0.84 (m, 5H). |
| **16** | 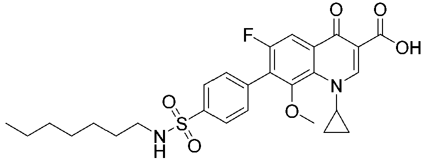 | LCMS: 530.9 [M+H]⁺ |
| | | ¹H NMR (400 MHz, DMSO-d6) δ 14.62 (s, 1H), 8.81 (s, 1H), 7.97 (d, J = 8.5 Hz, 3H), 7.78 (d, J = 8.1 Hz, 2H), 7.71 (t, J = 5.8 Hz, 1H), 4.27 - 4.17 (m, 1H), 3.39 (s, 3H), 2.87 - 2.77 (m, 2H), 1.40 - 1.32 (m, 2H), 1.26 - 1.12 (m, 12H), 0.83 (t, J = 6.9 Hz, 3H). |
| **17** | 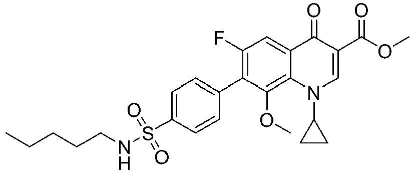 | LCMS: 517.2 [M+H]⁺ |
| | | ¹H NMR (400 MHz, DMSO-d6) δ 8.56 (s, 1H), 7.90 (d, J = 8.2 Hz, 1H), 7.79 - 7.72 (m, 2H), 7.50 - 7.42 (m, 2H), 7.19 (t, J = 9.4 Hz, 1H), 4.25 - 4.15 (m, 1H), 3.75 - 3.65 (m, 6H), 2.77 - 2.70 (m, 2H), 1.52 - 1.18 (m, 8H), 1.11 - 1.02 (m, 2H), 0.95 - 0.84 (m, 3H). |
| **18** | 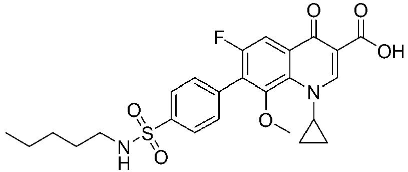 | LCMS: 503.3 [M+H]⁺ |
| | | 1H NMR (400 MHz, DMSO-d6) δ 14.63 (s, 1H), 8.82 (s, 1H), 8.01 - 7.94 (m, 3H), 7.79 (d, J = 8.1 Hz, 2H), 7.72 (t, J = 5.7 Hz, 1H), 4.28 - 4.16 (m, 1H), 3.39 (s, 3H), 2.82 (q, J = 6.8 Hz, 2H), 1.43 - 1.27 (m, 10H), 0.87 - 0.74 (m, 3H). |
| **19** | 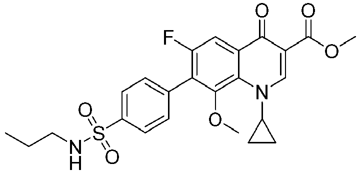 | LCMS: 489.2 [M+H]⁺ |
| | | ¹H NMR (400 MHz, DMSO-d6) δ 8.58 (s, 1H), 7.91 (d, J = 8.2 Hz, 1H), 7.80 - 7.73 (m, 2H), 7.48 - 7.40 (m, 2H), 7.25 - 7.20 (m, 1H), 4.23 - 4.13 (m, 1H), 3.74 - 3.69 (m, 6H), 2.92 - 2.88 (m, 2H), 1.44 - 1.18 (m, 4H), 1.12 - 1.01 (m, 2H), 0.92 (t, J = 8.0 Hz, 3H). |
| **20** | 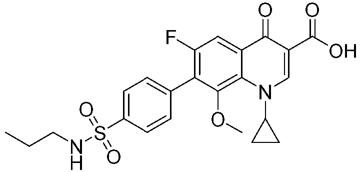 | LCMS: 475.3 [M+H]⁺ |
| | | ¹H NMR (400 MHz, DMSO-d6) δ 14.64 (s, 1H), 8.82 (s, 1H), 7.97 (d, J = 8.4 Hz, 3H), 7.82 - 7.70 (m, 3H), 4.25 - 4.16 (m, 1H), 3.38 (s, 3H), 2.85 - 2.74 (m, 2H), 1.45 - 1.33 (m, 2H), 1.22 - 1.10 (m, 4H), 0.80 (t, J = 7.4 Hz, 3H). |
| **21** | 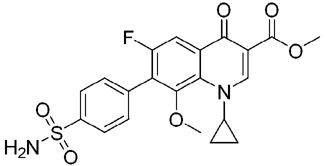 | LCMS: 447.1 [M+H]⁺ |
| | | ¹H NMR (400 MHz, DMSO-d6) δ 8.59 (s, 1H), 7.90 (d, J = 8.2 Hz, 1H), 7.74 - 7.66 (m, 2H), 7.61 - 7.58 (m, 2H), 7.23 (s, 2H), 4.24 - 4.14 (m, 1H), 3.84 - 3.80 (m, 6H), 1.29 - 1.20 (m, 2H), 1.15 - 1.02 (m, 2H). |
| **22** | 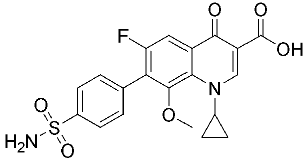 | LCMS: 433.1 [M+H]⁺ |
| | | ¹H NMR (400 MHz, DMSO-d6) δ 14.86 (s, 1H), 8.72 (s, 1H), 8.05 (d, J = 7.8 Hz, 1H), 7.82 - 7.57 (m, 4H), 7.23 (s, 2H), 4.10 - 3.83 (m, 4H), 1.33 - 0.94 (m, 4H). |
| **23** | 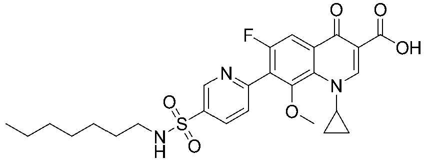 | LCMS: 532.2 [M+H]⁺ |
| | | ¹H NMR (400 MHz, DMSO-d6) δ 14.78 (s, 1H), 8.82 (d, J *=* 1.3 Hz, 1H), 8.60 (s, 1H), 8.10 - 7.99 (m, 2H), 7.67 - 7.60 (m, 1H), 7.25 (t, J = 9.6 Hz, 1H), 4.25 - 4.15 (m, 1H), 3.75 (s, 3H), 2.81 - 2.75 (m, 2H), 1.43 - 1.14(m, 12H), 1.11 - 0.83 (m, 5H). |
| **24** | 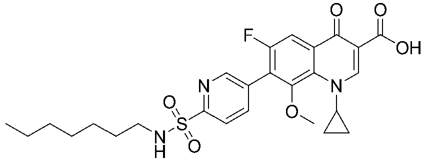 | LCMS: 532.3 [M+H]⁺ |
| | | ¹H NMR (400 MHz, DMSO-d6) δ 14.58 (s, 1H), 8.93 (s, 1H), 8.83 (s, 1H), 8.33 (dd, J = 8.1, 1.2 Hz, 1H), 8.14 (dd, J = 8.1, 0.7 Hz, 1H), 8.02 (d, J = 9.1 Hz, 1H), 7.96 (t, J = 5.8 Hz, 1H), 4.29 - 4.16 (m, 1H), 3.42 (s, 3H), 3.05 - 2.92 (m, 2H), 1.32 - 1.33 (m, 2H), 1.27 - 1.16 (m, 12H), 0.84 (t, J = 7.0 Hz, 3H). |
| **25** | 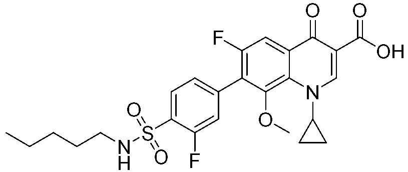 | LCMS: 521.2 [M+H]⁺ |
| | | ¹H NMR (400 MHz, DMSO-d6) δ 14.80 (s, 1H), 8.61 (s, 1H), 7.94 - 7.86 (m, 2H), 7.54 - 7.49 (m, 1H), 7.29 - 7.18 (m, 2H), 4.25 - 4.15 (m, 1H), 3.76 (s, 3H), 2.76 - 2.69 (m, 2H), 1.41 - 1.15 (m, 8H), 1.10 - 0.84 (m, 5H). |
| **26** | 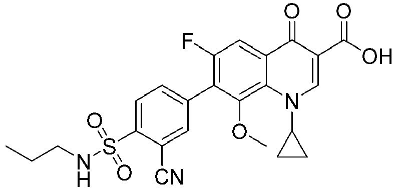 | LCMS: 500.1 [M+H]⁺ |
| | | ¹H NMR (400 MHz, DMSO-d6) δ 14.79 (s, 1H), 8.61 (s, 1H), 7.98 (d, J = 8.2 Hz, 1H), 7.88 - 7.78 (m, 2H), 7.60 (dt, J = 7.4, 1.8 Hz, 1H), 7.20 - 7.10 (m, 1H), 4.25 - 4.11(m, 1H), 3.75 (s, 3H), 2.84 (dt, J = 9.4, 7.1 Hz, 2H), 1.41- 1.14(m, 4H), 1.08 - 0.98 (m, 2H), 0.93 (t, J = 7.8 Hz, 3H). |
| **27** | 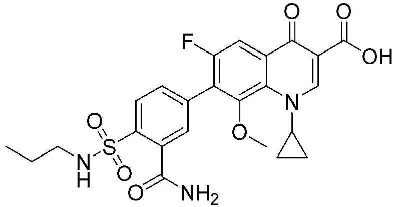 | LCMS: 518.1 [M+H]⁺ |
| | | ¹H NMR (400 MHz, DMSO-d6) δ 14.80 (s, 1H), 8.65 (s, 1H), 8.51 (d, J = 12.1 Hz, 1H), 8.37 (d, J = 12.5 Hz, 1H), 8.20 (t, J = 1.8 Hz, 1H), 8.04 (d, J = 7.8 Hz, 1H), 7.91 (d, J = 7.4 Hz, 1H), 7.61 - 7.58 (m, 1H), 7.07 (t, J = 9.4 Hz, 1H), 4.22 - 4.12 (m, 1H), 3.78 (s, 3H), 2.93 - 2.88 (m, 2H), 1.58 - 1.43 (m, 2H), 1.24 - 1.09 (m, 2H), 1.08 - 0.98 (m, 2H), 0.93 (t, J = 7.9 Hz, 3H). |
| **28** | 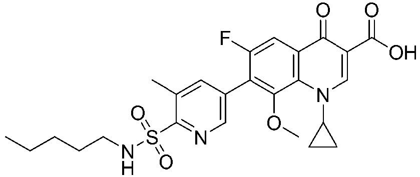 | LCMS:518.4 [M+H]⁺ |
| | | ¹H NMR (400 MHz, DMSO-d6) δ 14.58 (s, 1H), 8.83 (s, 1H), 8.67 (s, 1H), 8.15 (s, 1H), 8.03 (dd, J = 15.7, 7.5 Hz, 2H), 4.27 - 4.17 (m, 1H), 3.44 (s, 3H), 3.10 (dd, J = 13.1, 6.7 Hz, 2H), 2.66 (s, 3H), 1.53 - 1.43 (m, 2H), 1.29 - 1.15 (m, 8H), 0.85 (t, J = 6.5 Hz, 3H). |
| **29** | 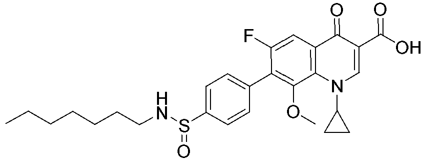 | LCMS: 515.3 [M+H]⁺ |
| | | ¹H NMR (400 MHz, DMSO-d6) δ 14.85 (s, 1H), 8.71 (s, 1H), 8.01 (d, J = 8.0 Hz, 1H), 7.76 - 7.42 (m, 4H), 4.93 - 4.69 (m, 1H), 4.16 - 3.81 (m, 4H), 3.13 - 2.87 (m, 2H), 1.66 - 1.44 (m, 2H), 1.40 - 0.80 (m, 15H). |
| **30** | 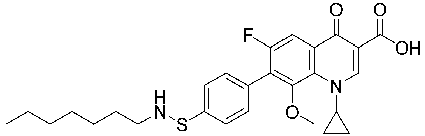 | LCMS: 499.2 [M+H]⁺ |
| | | ¹H NMR (400 MHz, DMSO-d6) δ 14.85 (s, 1H), 8.71 (s, 1H), 8.03 (d, J = 7.8 Hz, 2H), 7.61 - 7.32 (m, 4H), 4.62 - 4.40 (m, 2H), 4.16 - 3.79 (m, 4H), 2.87 - 2.66 (m, 2H), 1.68 - 0.79 (m, 17H). |
| **31** | 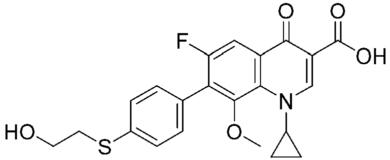 | LCMS:430.1 [M+H]⁺ |
| | | ¹H NMR (400 MHz, DMSO-d6) δ 14.69 (s, 1H), 8.80 (s, 1H), 7.92 (d, J = 9.2 Hz, 1H), 7.63 - 7.20 (m, 4H), 5.01 (t, J = 5.6 Hz, 1H), 4.32 - 4.05 (m, 1H), 3.64 (dd, J = 12.4, 6.8 Hz, 2H), 3.39 (s, 3H), 3.14 (t, J = 6.8 Hz, 2H), 1.27 - 1.12 (m, 4H). |
| **32** | 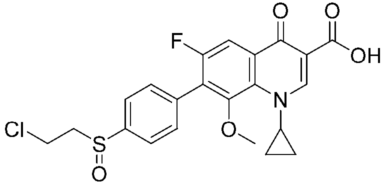 | LCMS:464.1 [M+H]⁺ |
| | | ¹H NMR (400 MHz, DMSO-d6) δ 14.65 (s, 1H), 8.82 (s, 1H), 7.97 (d, J = 9.2 Hz, 1H), 7.91 (d, J = 8.4 Hz, 2H), 7.79 (d, J = 8.0 Hz, 2H), 4.27 - 4.17 (m, 1H), 4.03 - 3.94 (m, 1H), 3.89 (dt, J = 11.2, 5.2 Hz, 1H), 3.58 - 3.46 (m, 1H), 3.39 (s, 3H), 3.36 (d, J = 5.2 Hz, 1H), 1.25 - 1.14 (m, 4H). |
| **33** | 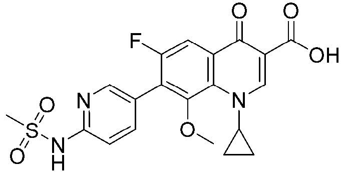 | LCMS:448.2[M+H]⁺ |
| | | ¹H NMR (400 MHz, DMSO-d6) δ 14.66 (s, 1H), 8.81 (s, 1H), 8.42 (s, 1H), 7.93 (dd, J = 14.3, 8.8 Hz, 2H), 7.09 (d, J = 8.5 Hz, 1H), 4.26 - 4.16 (m, 1H), 3.44 (s, 3H), 3.30 (s, 3H), 1.21 - 1.11 (m, 4H). |
| **34** | 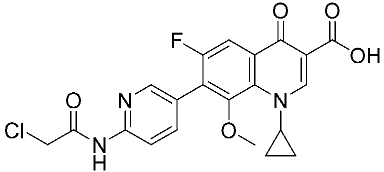 | LCMS:446.2[M+H]⁺ |
| | | ¹H NMR (400 MHz, DMSO-d6) δ 14.65 (s, 1H), 11.07 (s, 1H), 8.82 (s, 1H), 8.54 (s, 1H), 8.25 (d, J = 8.6 Hz, 1H), 8.06 (d, J = 8.6 Hz, 1H), 7.97 (d, J = 9.1 Hz, 1H), 4.41 (s, 2H), 4.27 - 4.19 (m, 1H), 3.42 (s, 3H), 1.18 (d, J = 6.7 Hz, 4H). |
| **35** | 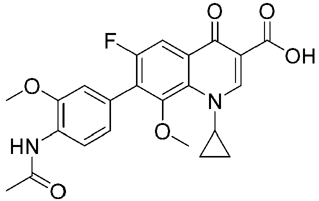 | LCMS:441.1 [M+H]⁺ |
| | | ¹H NMR (400 MHz, DMSO-d6) δ 14.70 (s, 1H), 9.31 (s, 1H), 8.80 (s, 1H), 8.16 (d, J = 8.5 Hz, 1H), 7.91 (d, J = 8.9 Hz, 1H), 7.20 (s, 1H), 7.08 (d, J = 8.0 Hz, 1H), 4.28 - 4.19 (m, 1H), 3.87 (s, 3H), 3.43 (s, 3H), 2.14 (s, 3H), 1.19 - 1.13 (m, 4H). |
| **36** | 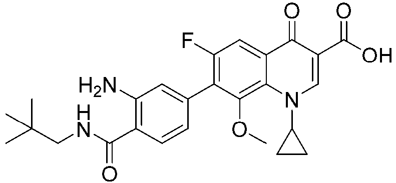 | LCMS:482.2 [M+H]⁺ |
| | | ¹H NMR (400 MHz, DMSO-d6) δ 14.68 (s, 1H), 8.79 (s, 1H), 8.31 - 8.24 (m, 1H), 7.96 - 7.83 (m, 1H), 7.65 (d, J = 8.1 Hz, 1H), 6.84 (s, 1H), 6.67 (d, J = 7.8 Hz, 1H), 6.44 (s, 2H), 4.26 - 4.10 (m, 2H), 3.48 (s, 3H), 3.10 (d, J = 6.3 Hz, 2H), 1.19 - 1.10 (m, 4H), 0.92 (s, 9H). |
| **37** | 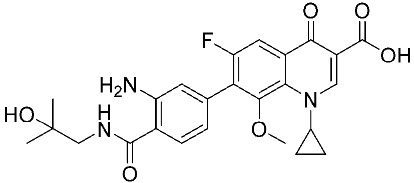 | LCMS:484.7 [M+H]⁺ |
| | | ¹H NMR (400 MHz, DMSO-d6) δ 14.68 (s, 1H), 8.81 (s, 1H), 8.12 (t, J = 6.0 Hz, 1H), 7.91 (d, J = 9.0 Hz, 1H), 7.67 (d, J = 8.2 Hz, 1H), 6.85 (s, 1H), 6.67 (d, J = 8.0 Hz, 1H), 6.48 (s, 2H), 4.56 (s, 1H), 4.27 - 4.14 (m, 1H), 3.49 (s, 3H), 3.25 (d, J = 6.1 Hz, 2H), 1.19 - 1.10 (m, 10H). |
| **38** | 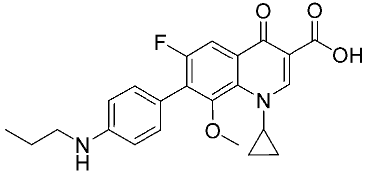 | LCMS: 411.2 [M+H]⁺ |
| | | ¹H NMR (400 MHz, DMSO-d6) δ 14.80 (s, 1H), 8.62 (s, 1H), 7.91 (d, J = 7.8 Hz, 1H), 7.37 (dd, J = 7.6, 2.1 Hz, 2H), 6.53 - 6.46 (m, 2H), 6.16 (t, J = 5.5 Hz, 1H), 4.26 - 4.16 (m, 1H), 3.66 (s, 3H), 3.15 (td, J = 7.1, 5.5 Hz, 2H), 1.72 - 1.58 (m, 2H), 1.29 - 1.19 (m, 2H), 1.08 - 0.92 (m, 5H). |
| **39** | 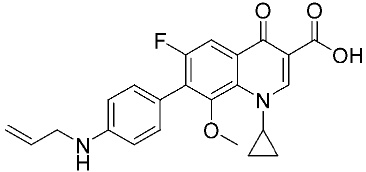 | LCMS: 409.2 [M+H]⁺ |
| | | ¹H NMR (400 MHz, DMSO-d6) δ 14.77 (s, 1H), 8.72 (s, 1H), 8.01 (d, J = 7.8 Hz, 1H), 7.51 - 7.44 (m, 2H), 6.53 - 6.46 (m, 2H), 6.08 (t, J = 3.5 Hz, 1H), 6.00 - 5.86 (m, 1H), 5.25 - 5.16 (m, 2H), 4.21 - 3.99 (m, 3H), 3.86 (s, 3H), 1.27 - 1.19 (m, 2H), 1.08 - 0.92 (m, 2H). |
| **40** | 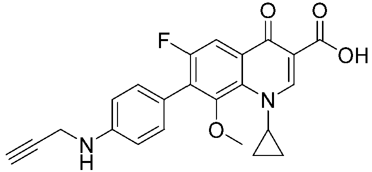 | LCMS: 407.1 [M+H]⁺ |
| | | ¹H NMR (400 MHz, DMSO-d6) δ 14.81 (s, 1H), 8.67 (s, 1H), 7.99 (d, J = 7.8 Hz, 1H), 7.40 - 7.33 (m, 2H), 6.74 - 6.67 (m, 2H), 5.99 (t, J = 4.9 Hz, 1H), 4.24 - 4.00 (m, 3H), 3.86 (s, 3H), 2.41 - 2.38 (m, 1H), 1.29 - 1.15 (m, 2H), 1.08 - 0.92 (m, 2H). |
| **41** | 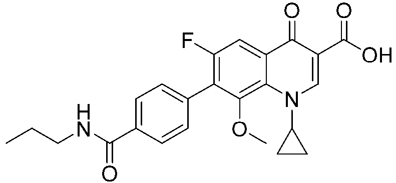 | LCMS: 439.2 [M+H]⁺ |
| | | ¹H NMR (400 MHz, DMSO-d6) δ 14.80 (s, 1H), 8.62 (s, 1H), 7.98 - 7.81 (m, 4H), 7.68 (dd, J = 7.6, 2.1 Hz, 2H), 4.25 - 4.15 (m, 1H), 3.45 (s, 3H), 3.08 - 2.99 (m, 2H), 1.46 - 1.32 (m, 2H), 1.29 - 1.04 (m, 4H), 0.80 (t, J = 8.0 Hz, 3H). |
| **42** | 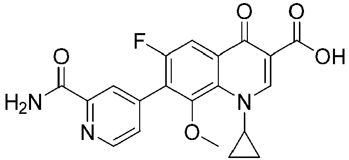 | LCMS: 398.1 [M+H]⁺ |
| | | ¹H NMR (400 MHz, DMSO-d6) δ 14.88 (s, 1H), 8.70 (s, 1H), 8.54 (d, J = 7.6 Hz, 1H), 8.08 - 7.90 (m, 2H), 7.69 (d, J = 9.4 Hz, 1H), 7.57 - 7.50 (m, 2H), 4.26 - 4.16 (m, 1H), 3.56 (s, 3H), 1.26 - 1.14 (m, 2H), 1.08 - 0.96 (m, 2H). |
| **43** | 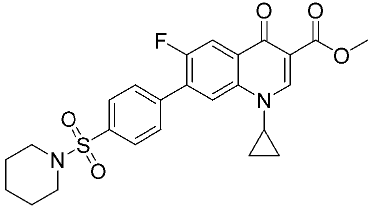 | LCMS: 485.2 [M+H]⁺ |
| | | ¹H NMR (400 MHz, DMSO-d6) δ 8.56 (s, 1H), 8.05 (d, J = 7.8 Hz, 1H), 7.74 - 7.66 (m, 2H), 7.53 - 7.45 (m, 2H), 7.28 (d, J = 5.1 Hz, 1H), 4.18 - 4.05 (m, 1H), 3.81 (s, 3H), 2.97 - 2.86 (m, 4H), 1.55 - 1.32 (m, 8H), 1.26 - 1.10 (m, 2H). |
| **44** | 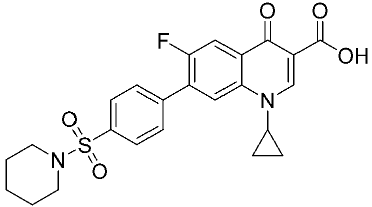 | LCMS: 471.2 [M+H]⁺ |
| | | ¹H NMR (400 MHz, DMSO-d6) δ 14.88 (s, 1H), 8.68 (s, 1H), 8.26 (d, J = 8.0 Hz, 1H), 7.87 - 7.54 (m, 4H), 7.39 (d, J = 5.1 Hz, 1H), 4.01 - 3.91 (m, 1H), 2.97 (t, J = 7.0 Hz, 4H), 1.75 - 1.02 (m, 10H). |
| **45** | 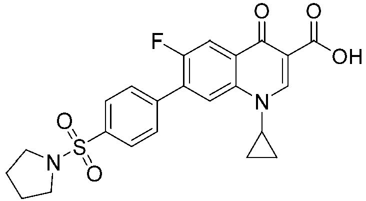 | LCMS: 457.1 [M+H]⁺ |
| | | ¹H NMR (400 MHz, DMSO-d6) δ 14.88 (s, 1H), 8.43 (s, 1H), 8.16 (d, J = 8.0 Hz, 1H), 7.69 - 7.55 (m, 4H), 7.38 (d, J = 5.1 Hz, 1H), 4.08 - 3.95 (m, 1H), 3.22 - 3.10 (m, 4H), 1.80 - 1.66 (m, 4H), 1.48 - 1.32 (m, 2H), 1.26 - 1. 10 (m, 2H). |
| **46** | 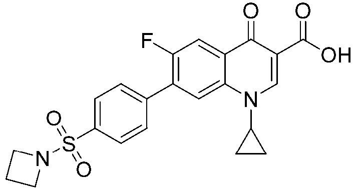 | LCMS: 443.1 [M+H]⁺ |
| | | ¹H NMR (400 MHz, DMSO-d6) δ 14.78 (s, 1H), 8.69 (s, 1H), 8.26 (d, J = 8.0 Hz, 1H), 7.80 - 7.73 (m, 2H), 7.66 - 7.60 (m, 2H), 7.49 (d, J = 5.1 Hz, 1H), 4.08 - 3.98 (m, 1H), 3.48 - 3.40 (m, 4H), 2.01 - 1.95 (m, 2H), 1.48 - 1.32 (m, 2H), 1.26 - 1.10 (m, 2H). |
| **47** | 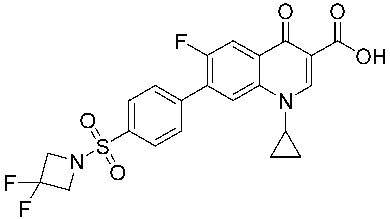 | LCMS: 479.1 [M+H]⁺ |
| | | ¹H NMR (400 MHz, DMSO-d6) δ 14.81 (s, 1H), 8.66 (s, 1H), 8.26 (d, J = 8.0 Hz, 1H), 7.80 - 7.73 (m, 2H), 7.62 - 7.54 (m, 2H), 7.39 (d, J = 5.1 Hz, 1H), 4.05 - 3.82 (m, 5H), 1.42 - 1.32 (m, 2H), 1.26 - 1.10 (m, 2H). |
| **48** | 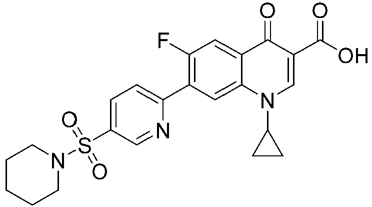 | LCMS: 472.1 [M+H]⁺ |
| | | ¹H NMR (400 MHz, DMSO-d6) δ 14.80 (s, 1H), 8.83 (d, J = 1.3 Hz, 1H), 8.58 (s, 1H), 8.15 - 8.00 (m, 2H), 7.74 (dd, J = 7.5, 2.1 Hz, 1H), 7.53 (d, J = 5.1 Hz, 1H), 4.08 - 3.90 (m, 1H), 3.20 - 3.10 (m, 4H), 1.60 - 1.32 (m, 8H), 1.26 - 1.10 (m, 2H). |
| **49** | 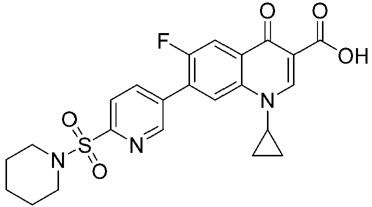 | LCMS: 472.1 [M+H]⁺ |
| | | ¹H NMR (400 MHz, DMSO-d6) δ 14.80 (s, 1H), 8.68 (s, 1H), 8.43 - 8.36 (m, 2H), 8.01 - 7.95 (m 2H), 7.43 (d, J = 5.1 Hz, 1H), 4.03 - 3.89 (m, 1H), 3.20 - 3.10 (m, 4H), 1.66 - 1.54 (m, 4H), 1.50 - 1.32 (m, 4H), 1.26 - 1.10 (m, 2H). |
| **50** | 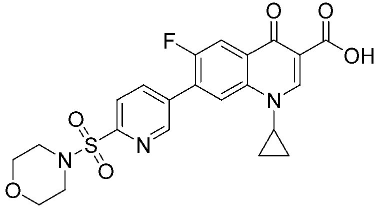 | LCMS: 474.1 [M+H]⁺ |
| | | ¹H NMR (400 MHz, DMSO-d6) δ 14.81 (s, 1H), 8.60 - 8.53 (m, 2H), 8.36 (d, J = 8.0 Hz, 1H), 8.09 - 7.95 (m, 2H), 7.43 (d, J = 5.1 Hz, 1H), 4.01 - 3.90 (m, 1H), 3.69 (t, J = 7.1 Hz, 4H), 3.08 (t, J = 7.0 Hz, 4H), 1.40 - 1.16 (m, 4H). |
| **51** | 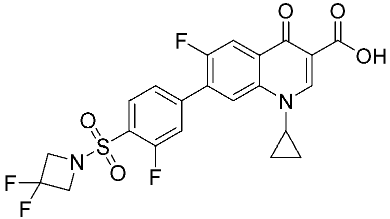 | LCMS: 497.1 [M+H]⁺ |
| | | ¹H NMR (400 MHz, DMSO-d6) δ 14.85 (s, 1H), 8.66 (s, 1H), 8.27 (d, J = 8.0 Hz, 1H), 7.93 - 7.85 (m, 1H), 7.59 - 7.51 (m, 1H), 7.44 - 7.38 (m, 2H), 4.04 - 3.80 (m, 5H), 1.48 - 1.32 (m, 2H), 1.26 - 1.10 (m, 2H). |
| **52** | 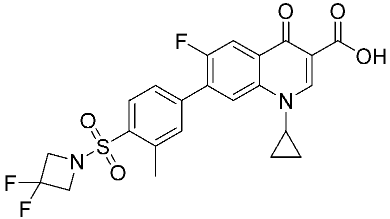 | LCMS: 493.1 [M+H]⁺ |
| | | ¹H NMR (400 MHz, DMSO-d6) δ 14.85 (s, 1H), 8.67 (s, 1H), 8.25 (d, J = 8.2 Hz, 1H), 7.87 (d, J = 7.4 Hz, 1H), 7.64 - 7.50 (m, 2H), 7.38 (d, J = 5.1 Hz, 1H), 4.01 - 3.82 (m, 5H), 2.69 (s, 3H), 1.42 - 1.32 (m, 2H), 1.26 - 1. 10 (m, 2H). |
| **53** | 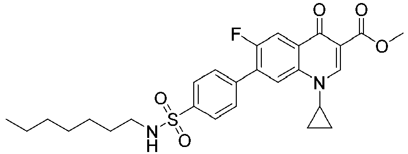 | LCMS:515.1[M+H]⁺ |
| | | ¹H NMR (400 MHz, DMSO-d6) δ 8.56 (s, 1H), 8.23 (d, J = 6.3 Hz, 1H), 8.04 - 7.86 (m, 5H), 7.72 (t, J = 5.4 Hz, 1H), 3.77 (s, 4H), 2.80 (q, J = 6.4 Hz, 2H), 1.38 (d, J = 6.3 Hz, 2H), 1.30 - 1.10 (m, 12H), 0.82 (t, J = 6.7 Hz, 3H). |
| **54** | 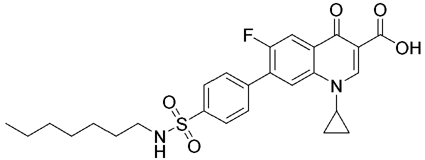 | LCMS:501.0 [M+H]⁺ |
| | | ¹H NMR (400 MHz, DMSO-d6) δ 14.78 (s, 1H), 8.80(s, 1H), 8.44 (d, J = 10.4 Hz 1H), 8.19 (d, J = 10.4 Hz 1H), 8.00-7.94 (m, 4H), 7.77-7.72 (m, 1H),4.00-3.93 (m, 1H), 2.81 (q, J = 13.0 Hz, J =6.8 Hz, 2H), 1.43-1.30(m, 4H), 1.26-1.16 (m, 10H), 0.82 (t, J = 6.9 Hz, 3H). |
| **55** | 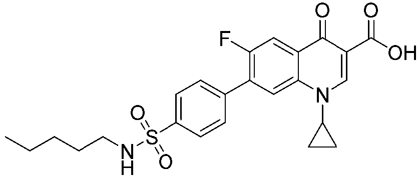 | LCMS:473.1[M+H]⁺ |
| | | ¹H NMR (400 MHz, DMSO-d6) δ 14.76 (s, 1H), 8.80 (s, 1H), 8.43 (d, J = 6.3 Hz, 1H), 8.18 (d, J = 10.3 Hz, 1H), 8.01 - 7.93 (m, 4H), 7.75 (t, J = 5.8 Hz, 1H), 4.00 - 3.93 (m, 1H), 2.81 (q, J = 6.7 Hz, 2H), 1.35 (ddd, J = 19.3, 13.4, 7.0 Hz, 4H), 1.28 - 1.16 (m, 6H), 0.81 (t, J = 7.0 Hz, 3H). |
| **56** | 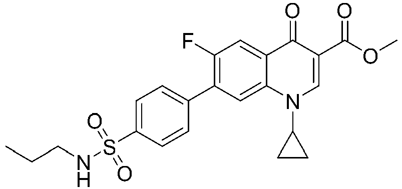 | LCMS: 459.1 [M+H]⁺ |
| | | ¹H NMR (400 MHz, DMSO-d6) δ 8.66 (s, 1H), 8.25 (d, J = 8.0 Hz, 1H), 7.88 - 7.81 (m, 2H), 7.59 (dd, J = 7.4, 1.9 Hz, 2H), 7.39 (d, J = 5.1 Hz, 1H), 7.20 (t, J = 9.4 Hz, 1H), 4.02 - 3.91 (m, 1H), 3.82 (s, 3H), 2.92 - 2.81 (m, 2H), 1.54 - 1.32 (m, 4H), 1.26 - 1.10 (m, 2H), 0.82 (t, J = 8.0 Hz, 3H). |
| **57** | 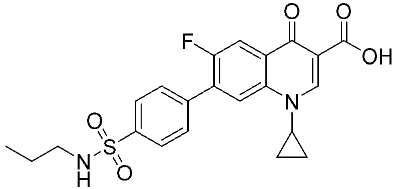 | LCMS:445.1[M+H]⁺ |
| | | ¹H NMR (400 MHz, DMSO-d6) δ 14.79 (s, 1H), 8.80 (s, 1H), 8.44 (d, J = 6.4 Hz, 1H), 8.19 (d, J = 10.3 Hz, 1H), 8.02 - 7.93 (m, 4H), 7.77 (t, J = 5.8 Hz, 1H), 4.02 - 3.92 (m, 1H), 2.78 (q, J = 6.8 Hz, 2H), 1.50 - 1.38 (m, 2H), 1.37 - 1.29 (m, 2H), 1.25 (d, J = 3.7 Hz, 2H), 0.83 (t, J = 7.4 Hz, 3H). |
| **58** | 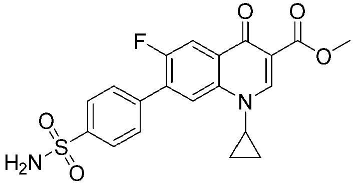 | LCMS: 417.1 [M+H]⁺ |
| | | ¹H NMR (400 MHz, DMSO-d6) δ 8.68 (s, 1H), 8.25 (d, J = 7.8 Hz, 1H), 7.89 - 7.82 (m, 2H), 7.67 - 7.60 (m, 2H), 7.31 - 7.22 (m, 3H), 4.02 - 3.88 (m, 1H), 3.83 (s, 3H), 1.38 - 1.22 (m, 2H), 1.26 - 1.10 (m, 2H). |
| **59** | 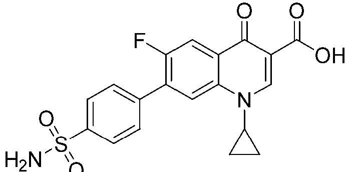 | LCMS:403.1 [M+H]⁺ |
| | | ¹H NMR (400 MHz, DMSO-*d*₆) δ 14.78 (s, 1H), 8.80 (s, 1H), 8.42 (d, *J =* 6.2 Hz, 1H), 8.18 (d, *J* = 10.3 Hz, 1H), 8.02 (d, *J =* 8.3 Hz, 2H), 7.94 (d, *J* = 7.8 Hz, 2H), 7.52 (s, 2H), 3.98 - 3.94 (m, 1H), 1.36 - 1.21 (m, 4H). |
| **60** | 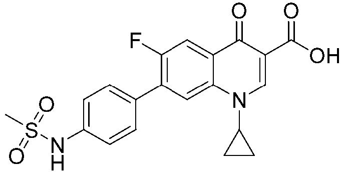 | LCMS: 417.1 [M+H]⁺ |
| | | ¹H NMR (400 MHz, DMSO-d6) δ 14.89 (s, 1H), 9.09 (s, 1H), 8.68 (s, 1H), 8.25 (d, J = 8.0 Hz, 1H), 7.51 (dq, J = 7.8, 1.6 Hz, 2H), 7.45 - 7.33 (m, 3H), 3.87 (p, J = 7.0 Hz, 1H), 2.97 (s, 3H), 1.32 - 1.15 (m, 4H). |
| **61** | 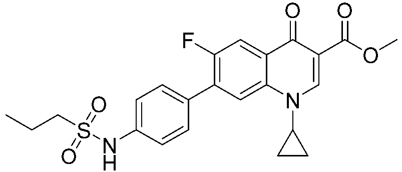 | LCMS: 459.1 [M+H]⁺ |
| | | ¹H NMR (400 MHz, DMSO-d6) δ 9.67 (s, 1H), 8.76 (s, 1H), 8.35 (d, J = 8.2 Hz, 1H), 7.68 (dq, J = 7.8, 1.6 Hz, 2H), 7.53 - 7.46 (m, 2H), 7.33 (d, J = 5.1 Hz, 1H), 3.98 - 3.91 (m, 1H), 3.83 (s, 3H), 3.15 - 2.94 (m, 2H), 1.81 - 1.67 (m, 2H), 1.48 - 1.26 (m, 4H), 1.16 - 0.97 (m, 3H). |
| **62** | 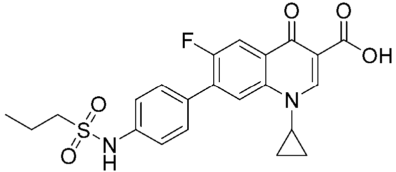 | LCMS: 445.1 [M+H]⁺ |
| | | ¹H NMR (400 MHz, DMSO-d6) δ 14.88 (s, 1H), 9.77 (s, 1H), 8.59 (s, 1H), 8.16 (d, J = 8.0 Hz, 1H), 7.48 - 7.37 (m, 5H), 4.05 - 3.91 (m, 1H), 3.15 - 2.95 (m, 2H), 1.85 - 1.64 (m, 2H), 1.48 - 1.38 (m, 2H), 1.26 - 1.16 (m, 2H), 1.16 - 0.97 (m, 3H). |
| **63** | 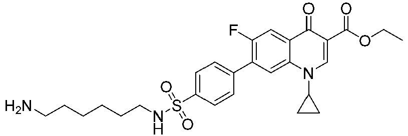 | LCMS: 530.2 [M+H]⁺ |
| | | ¹H NMR (400 MHz, DMSO) δ 8.55 (s, 1H), 8.42 (s, 1H), 8.23 (d, J = 6.5 Hz, 1H), 7.97 (dt, J = 14.7, 9.6 Hz, 5H), 4.25 (q, J = 7.0 Hz, 2H), 3.77 (ddd, J = 10.8, 6.4, 4.3 Hz, 1H), 2.80 (t, J = 7.0 Hz, 2H), 2.71 - 2.63 (m, 2H), 1.48 - 1.34 (m, 4H), 1.32 - 1.22 (m, 9H), 1.16 (t, J = 8.0 Hz, 2H). |
| **64** | 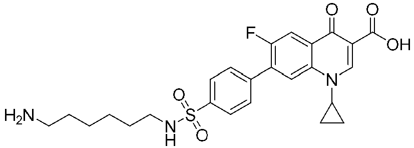 | LCMS: 502.6 [M+H]⁺ |
| | | ¹H NMR (400 MHz, DMSO) δ 8.81 (s, 1H), 8.43 (d, J = 6.3 Hz, 1H), 8.20 (d, J = 9.7 Hz, 1H), 7.97 (s, 4H), 3.97 (dd, J = 7.4, 3.6 Hz, 1H), 2.81 (t, J = 7.0 Hz, 2H), 2.71 (d, J = 7.7 Hz, 2H), 1.44 (s, 4H), 1.33 (d, J = 6.6 Hz, 2H), 1.26 (s, 6H). |
| **65** | 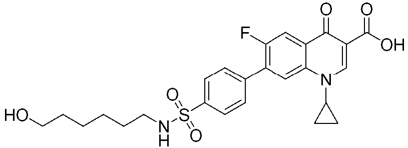 | LCMS:503.3 [M+H]⁺ |
| | | ¹H NMR (400 MHz, DMSO-d6) δ 14.75 (s, 1H), 8.80 (s, 1H), 8.43 (d, *J =* 6.1 Hz, 1H), 8.18 (d, *J* = 10.2 Hz, 1H), 8.03 - 7.91 (m, 4H), 7.74 (t, *J =* 5.6 Hz, 1H), 4.30 (s, 1H), 3.97 (s, 1H), 3.34 (s, 2H), 2.91 - 2.70 (m, 2H), 1.43 - 1.18 (m, 12H). |
| **66** | 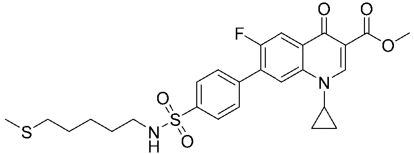 | LCMS: 533.2 [M+H]⁺ |
| | | ¹H NMR (400 MHz, DMSO-d6) δ 14.80 (s, 1H), 8.83 (s, 1H), 8.42 (d, J = 6.4 Hz, 1H), 8.19 (d, J = 10.3 Hz, 1H), 8.04 - 7.94 (m, 4H), 7.78 - 7.74 (m, 1H), 4.00 - 3.88 (m, 2H), 2.82 (d, J = 6.4 Hz, 2H), 2.43 - 2.37 (m, 2H), 2.00 (s, 3H), 1.62 - 1.06 (m, 10H). |
| **67** | 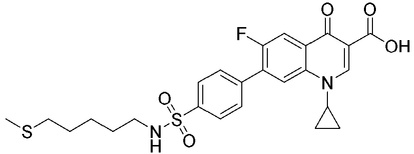 | LCMS:519.3 [M+H]⁺ |
| | | ¹H NMR (400 MHz, DMSO-d6) δ 14.78 (s, 1H), 8.81 (s, 1H), 8.44 (d, J = 6.4 Hz, 1H), 8.19 (d, J = 10.3 Hz, 1H), 8.00 - 7.94 (m, 4H), 7.76 (t, J = 5.8 Hz, 1H), 4.00 - 3.94 (m, 1H), 2.82 (d, J = 6.4 Hz, 2H), 2.40 (t, J = 7.2 Hz, 2H), 2.00 (s, 3H), 1.65 - 1.09 (m, 10H). |
| **68** | 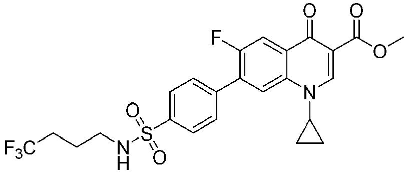 | LCMS: 527.1 [M+H]⁺ |
| | | ¹H NMR (400 MHz, DMSO-d6) δ 8.64 (s, 1H), 8.24 (d, J = 8.0 Hz, 1H), 7.91 - 7.84 (m, 2H), 7.63 - 7.42 (m, 3H), 7.36 (d, J = 4.8 Hz, 1H), 4.01 - 3.96 (m, 1H), 3.78 (s, 3H), 2.76 - 2.32 (m, 4H), 1.71 - 1.32 (m, 4H), 1.26 - 1.10 (m, 2H). |
| **69** | 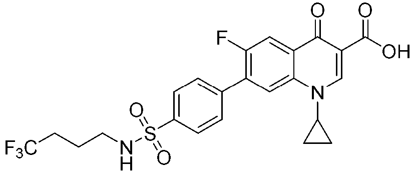 | LCMS: 513.1 [M+H]⁺ |
| | | 1H NMR (400 MHz, DMSO-d6) δ 14.85 (s, 1H), 8.64 (s, 1H), 8.26 (d, J = 8.0 Hz, 1H), 7.92 - 7.85 (m, 2H), 7.63 - 7.47 (m, 3H), 7.36 (d, J = 4.8 Hz, 1H), 4.02 - 3.96 (m, 1H), 2.76 - 2.32 (m, 4H), 1.71 - 1.32 (m, 4H), 1.28 - 1. 10 (m, 2H). |
| **70** | 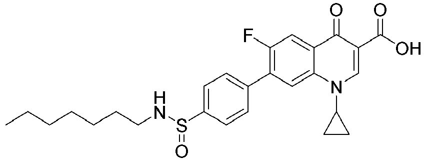 | LCMS: 485.2 [M+H]⁺ |
| | | ¹H NMR (400 MHz, DMSO-d6) δ 14.77 (s, 1H), 8.57 (s, 1H), 8.16 (d, J = 8.0 Hz, 1H), 7.74 - 7.67 (m, 2H), 7.58 - 7.50 (m, 2H), 7.40 (d, J = 5.1 Hz, 1H), 4.92 - 4.87 (m, 1H), 4.02 - 3.87 (m, 1H), 3.05 - 2.97 (m, 2H), 1.57 - 1.11 (m, 15H), 0.97 - 0.86 (m, 3H). |
| **71** | 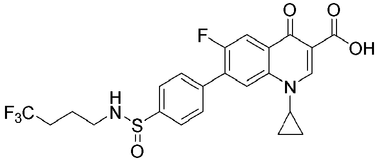 | LCMS: 497.1 [M+H]⁺ |
| | | ¹H NMR (400 MHz, DMSO-d6) δ 14.86 (s, 1H), 8.56 (s, 1H), 8.18 (d, J = 8.0 Hz, 1H), 7.71 - 7.54 (m, 4H), 7.40 (d, J = 5.1 Hz, 1H), 4.95 (t, J = 6.8 Hz, 1H), 4.01 - 3.94 (m, 1H), 3.13 - 3.04 (m, 2H), 2.17 - 1.80 (m, 4H), 1.42 - 1.32 (m, 2H), 1.26 - 1.10 (m, 2H). |
| **72** | 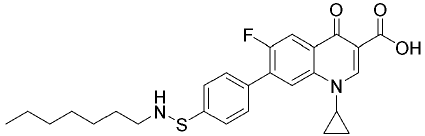 | LCMS: 469.2 [M+H]⁺ |
| | | ¹H NMR (400 MHz, DMSO-d6) δ 14.81 (s, 1H), 8.62 (s, 1H), 8.18 (d, J = 8.0 Hz, 1H), 7.52 - 7.42 (m, 4H), 7.30 (d, J = 4.8 Hz, 1H), 4.52 (t, J = 5.3 Hz, 1H), 4.02 - 3.92 (m, 1H), 2.73 - 2.66 (m, 2H), 1.52 - 1.04 (m, 16H), 0.95 - 0.82 (m, 3H). |
| **73** | 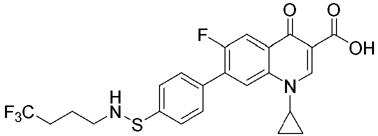 | LCMS: 481.1 [M+H]⁺ |
| | | ¹H NMR (400 MHz, DMSO-d6) δ 14.81 (s, 1H), 8.61 (s, 1H), 8.19 (d, J = 8.0 Hz, 1H), 7.52 - 7.41 (m, 4H), 7.28 (d, J = 4.8 Hz, 1H), 4.00 -3.88 (m, 2H), 2.87 (td, J = 7.0, 4.7 Hz, 2H), 2.33 - 2.26 (m, 2H), 1.80 - 1.73 (m, 2H), 1.48 - 1.38 (m, 2H), 1.286- 1.12 (m, 2H). |
| **74** | 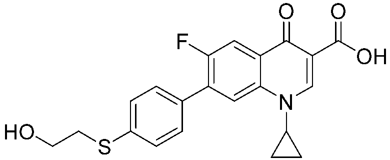 | LCMS: 400.1 [M+H]⁺ |
| | | ¹H NMR (400 MHz, DMSO-d6) δ 14.86 (s, 1H), 8.66 (s, 1H), 8.24 (d, J = 8.0 Hz, 1H), 7.59 - 7.52 (m, 2H), 7.34 - 7.25 (m, 3H), 4.61 (t, J = 6.6 Hz, 1H), 3.95 - 3.85 (m, 1H), 3.65 - 3.58 (m, 2H), 3.11 (t, J = 7.2 Hz, 2H), 1.46 - 1.36 (m, 2H), 1.28 - 1.12 (m, 2H). |
| **75** | 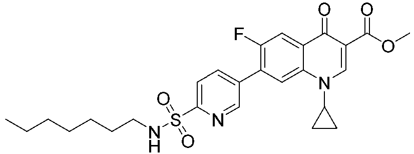 | LCMS: 516.2 [M+H]⁺ |
| | | 1H NMR (400 MHz, DMSO-d6) δ 9.09 (s, 1H), 8.81 (s, 1H), 8.51 (d, J = 40.6 Hz, 2H), 8.20 (d, J = 10.1 Hz, 1H), 8.13 (d, J = 7.7 Hz, 1H), 7.98 (t, J = 5.6 Hz, 1H), 3.99 - 3.91 (m, 1H), 3.81 (s, 3H), 2.98 (dd, J = 12.8, 6.5 Hz, 2H), 1.46 - 1.34 (m, 4H), 1.27 - 1.18 (m, 10H), 0.83 (t, J = 6.8 Hz, 3H). |
| **76** | 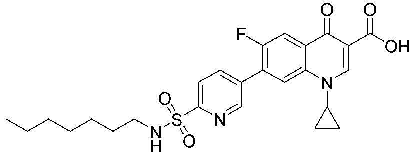 | LCMS:502.3 [M+H]⁺ |
| | | ¹H NMR (400 MHz, DMSO-d6) δ 14.74 (s, 1H), 9.09 (s, 1H), 8.81 (s, 1H), 8.50 (d, J = 40.6 Hz, 2H), 8.22 (d, J = 10.1 Hz, 1H), 8.13 (d, J = 7.7 Hz, 1H), 7.98 (t, J = 5.6 Hz, 1H), 3.98 - 3.94 (m, 1H), 2.98 (dd, J = 12.8, 6.5 Hz, 2H), 1.46 - 1.34 (m, 4H), 1.27 - 1.18 (m, 10H), 0.83 (t, J = 6.8 Hz, 3H). |
| **77** | 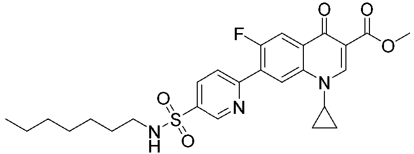 | LCMS: 516.2 [M+H]⁺ |
| | | 1H NMR (400 MHz, DMSO-d6) δ 9.11 (d, J = 2.0 Hz, 1H), 8.83 - 8.80 (m, 2H), 8.39 (dd, J = 8.0 Hz, 2.0 Hz, 1H), 8.22 (d, J = 10.8 Hz, 2H), 7.97 (t, J = 5.6 Hz, 1H), 4.02 - 3.90 (m, 1H), 3.80 (s, 3H), 2.89 - 2.85 (m, 2H), 1.43-1.37 (m, 2H), 1.35-1.29 (m, 2H), 1.26-1.17 (m, 10H), 0.82 (t, J = 7.2 Hz, 3H). |
| **78** | 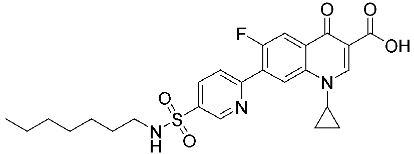 | LCMS:502.2 [M+H]⁺ |
| | | ¹H NMR (400 MHz, DMSO-d6) δ 14.71 (s, 1H), 9.15 (d, *J =* 2.0 Hz, 1H), 8.86 (d, *J =* 6.4 Hz, 1H), 8.82 (s, 1H), 8.39 (dd, *J =* 8.0 Hz, 2.0 Hz, 1H), 8.22 (d, *J* = 10.8 Hz, 2H), 7.97 (t, *J* = 5.6 Hz, 1H), 4.02 - 3.90 (m, 1H), 2.87 (q, *J* = 6.8 Hz, 2H), 1.43-1.37 (m, 2H), 1.35-1.29 (m, 2H), 1.26-1.17 (m, 10H), 0.82 (t*, J =* 7.2 Hz, 3H). |
| **79** | 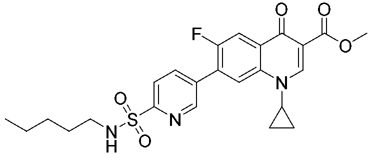 | LCMS: 488.2 [M+H]⁺ |
| | | ¹H NMR (400 MHz, DMSO-d6) δ 8.71 (s, 1H), 8.56 (t, J = 2.0 Hz, 1H), 8.41 (d, J = 8.0 Hz, 1H), 8.29 (d, J = 7.4 Hz, 1H), 8.02 (dt, J = 7.8, 2.0 Hz, 1H), 7.53 (t, J = 10.6 Hz, 1H), 7.47 (d, J = 5.1 Hz, 1H), 4.02 - 3.94 (m, 1H), 3.83 (s, 3H), 2.94 - 2.87 (m, 2H), 1.59 - 1.53 (m, 2H), 1.47 - 1.26 (m, 8H), 0.96 - 0.81 (m, 3H). |
| **80** | 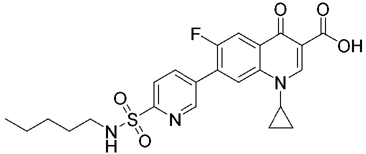 | LCMS: 474.2 [M+H]⁺ |
| | | ¹H NMR (400 MHz, DMSO-d6) δ 14.78 (s, 1H), 8.68 (s, 1H), 8.50 (t, J = 2.0 Hz, 1H), 8.36 (d, J = 8.0 Hz, 1H), 8.22 (d, J = 7.5 Hz, 1H), 7.95 (dt, J = 7.4, 1.6 Hz, 1H), 7.47 (t, J = 10.5 Hz, 1H), 7.43 (d, J = 5.1 Hz, 1H), 4.00 - 3.94 (m, 1H), 2.99 - 2.78 (m, 2H), 1.53 - 1.46 (m, 2H), 1.41 - 1.26 (m, 9H), 0.95 - 0.83 (m, 3H). |
| **81** | 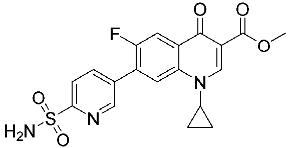 | LCMS: 418.1 [M+H]⁺ |
| | | ¹H NMR (400 MHz, DMSO-d6) δ 8.69 (s, 1H), 8.61 - 8.56 (m, 1H), 8.35 (d, J = 8.2 Hz, 1H), 8.13 (d, J = 7.6 Hz, 1H), 7.96 (dt, J = 7.8, 1.6 Hz, 1H), 7.44 (d, J = 5.0 Hz, 1H), 7.32 (s, 2H), 3.98 - 3.94 (m, 1H), 3.83 (s, 3H), 1.48 - 1.22 (m, 4H). |
| **82** | 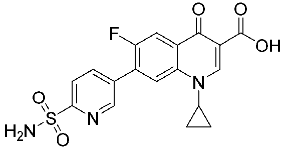 | LCMS: 404.1 [M+H]⁺ |
| | | ¹H NMR (400 MHz, DMSO-d6) δ 14.82 (s, 1H), 8.71 (s, 1H), 8.59 (t, J = 1.8 Hz, 1H), 8.38 (d, J = 7.8 Hz, 1H), 8.17 (d, J = 7.4 Hz, 1H), 7.98 (dt, J = 7.8, 1.8 Hz, 1H), 7.47 (d, J = 5.1 Hz, 1H), 7.34 (s, 2H), 4.01 - 3.94 (m, 1H), 1.48 - 1.33 (m, 2H), 1.29 - 1.19 (m, 2H). |
| **83** | 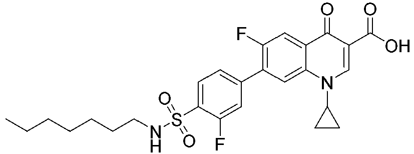 | LCMS: 519.4 [M+H]⁺ |
| | | ¹H NMR (400 MHz, DMSO-d6) δ 14.75 (s, 1H), 8.81 (s, 1H), 8.46 (d, J = 6.4 Hz, 1H), 8.19 (d, J = 10.3 Hz, 1H), 8.06 (t, J = 5.7 Hz, 1H), 7.97 (t, J = 7.9 Hz, 1H), 7.89 (d, J *=* 11.0 Hz, 1H), 7.76 (d, J = 8.1 Hz, 1H), 4.02 - 3.92 (m, 1H), 2.92 (dd, J = 12.8, 6.8 Hz, 2H), 1.45 - 1.38 (m, 2H), 1.36 - 1.31 (m, 2H), 1.22 (dd, J = 17.6, 8.1 Hz, 10H), 0.82 (t, J = 7.0 Hz, 3H). |
| **84** | 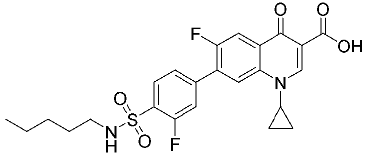 | LCMS: 491.2 [M+H]⁺ |
| | | ¹H NMR (400 MHz, DMSO-d6) δ 14.76 (s, 1H), 8.75 (s, 1H), 8.32 (d, J = 8.0 Hz, 1H), 8.09 (dd, J = 7.4, 5.1 Hz, 1H), 7.64 (dt, J = 7.8, 1.8 Hz, 1H), 7.53 (dt, J = 7.7, 2.0 Hz, 1H), 7.41 - 7.32 (m, 2H), 3.99 - 3.89 (m, 1H), 2.83 (dt, J = 9.4, 7.0 Hz, 2H), 1.51 - 1.32 (m, 9H), 1.26 - 1.17 (m, 2H), 0.91 - 0.81 (m, 3H). |
| **85** | 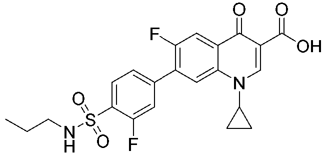 | LCMS: 463.1 [M+H]⁺ |
| | | ¹H NMR (400 MHz, DMSO-d6) δ 14.80 (s, 1H), 8.71 (s, 1H), 8.26 (d, J = 8.2 Hz, 1H), 8.04 (dd, J = 7.5, 5.0 Hz, 1H), 7.60 (dt, J = 7.8, 1.8 Hz, 1H), 7.52 (dt, J = 7.6, 2.0 Hz, 1H), 7.41 (d, J = 5.0 Hz, 1H), 7.23 (t, J = 9.4 Hz, 1H), 4.02 - 3.92 (m, 1H), 2.96 (dt, J = 9.4, 7.0 Hz, 2H), 1.60 - 1.16 (m, 6H), 0.90 (t, J = 8.0 Hz, 3H). |
| **86** | 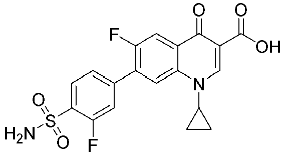 | LCMS: 421.1 [M+H]⁺ |
| | | ¹H NMR (400 MHz, DMSO-d6) δ 14.79 (s, 1H), 8.70 (s, 1H), 8.24 (d, J = 8.2 Hz, 1H), 8.04 (dd, J = 7.4, 5.1 Hz, 1H), 7.64 (dt, J = 8.0, 1.8 Hz, 1H), 7.55 (dt, J = 7.6, 1.9 Hz, 1H), 7.39 (d, J = 5.1 Hz, 1H), 7.11 (s, 2H), 3.98 - 3.88 (m, 1H), 1.46 - 1.38 (m, 2H), 1.29 - 1.13 (m, 2H). |
| **87** | 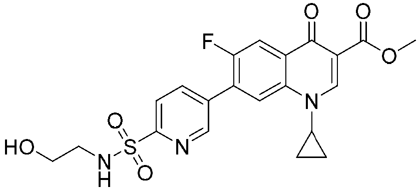 | LCMS: 462.1 [M+H]⁺ |
| | | ¹H NMR (400 MHz, DMSO-d6) δ 8.71 (s, 1H), 8.56 (t, J = 2.0 Hz, 1H), 8.40 (d, J = 7.8 Hz, 1H), 8.23 (d, J = 7.4 Hz, 1H), 8.00 (dt, J = 7.4, 1.7 Hz, 1H), 7.59 (t, J = 11.3 Hz, 1H), 7.46 (d, J = 4.8 Hz, 1H), 4.54 (t, J = 6.1 Hz, 1H), 4.03 - 3.93 (m, 1H), 3.83 (s, 3H), 3.70 (td, J = 7.1, 6.0 Hz, 2H), 3.14 (dt, J = 11.1, 7.1 Hz, 2H), 1.48 - 1.35 (m, 2H), 1.29 - 1.15 (m, 2H). |
| **88** | 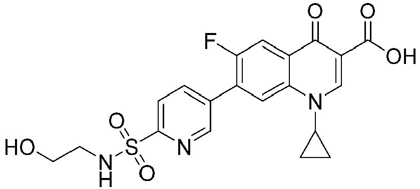 | LCMS: 448.1 [M+H]⁺ |
| | | ¹H NMR (400 MHz, DMSO-d6) δ 14.78 (s, 1H), 8.72 (s, 1H), 8.53 (t, J = 1.7 Hz, 1H), 8.39 (d, J = 8.0 Hz, 1H), 8.21 (d, J = 7.4 Hz, 1H), 7.98 (dt, J = 7.4, 1.8 Hz, 1H), 7.57 (t, J = 11.3 Hz, 1H), 7.46 (d, J = 5.1 Hz, 1H), 4.52 (t, J = 6.1 Hz, 1H), 4.01 - 3.91 (p, J = 7.0 Hz, 1H), 3.68 (td, J = 7.0, 5.8 Hz, 2H), 3.11 (dt, J = 11.3, 7.1 Hz, 2H), 1.50 - 1.40 (m, 2H), 1.29 - 1.13 (m, 2H). |
| **89** | 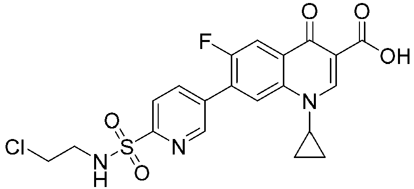 | LCMS: 466.1 [M+H]⁺ |
| | | ¹H NMR (400 MHz, DMSO-d6) δ 14.84 (s, 1H), 8.64 (s, 1H), 8.47 (t, J = 2.0 Hz, 1H), 8.32 (d, J = 8.0 Hz, 1H), 8.17 (d, J = 7.4 Hz, 1H), 7.91 (dt, J = 7.4, 1.6 Hz, 1H), 7.52 (t, J = 10.7 Hz, 1H), 7.39 (d, J = 4.8 Hz, 1H), 3.94 - 3.84 (m, 1H), 3.66 (t, J = 7.0 Hz, 2H), 3.23 (dt, J = 10.5, 7.1 Hz, 2H), 1.48 - 1.38 (m, 2H), 1.26 - 1.14 (m, 2H). |
| **90** | 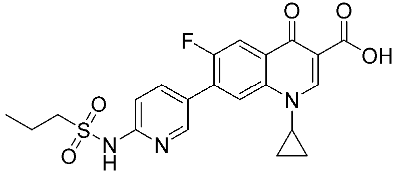 | LCMS:446.3[M+H]⁺ |
| | | ¹H NMR (400 MHz, DMSO-d6) δ 15.08 - 14.64 (m, 1H), 11.92 - 10.78 (m, 1H), 8.78 (s, 1H), 8.58 (t*, J =* 6.2 Hz, 1H), 8.41 (t*, J =* 6.3 Hz, 1H), 8.21 (s, 1H), 8.18 - 8.10 (m, 1H), 8.09 - 8.02 (m, 1H), 7.07 (d, *J* = 8.8 Hz, 1H), 3.95 (ddd, *J =* 11.3, 7.2, 3.8 Hz, 1H), 3.43 (dd, *J* = 13.2, 4.9 Hz, 2H), 1.81 - 1.68 (m, 2H), 1.34 (dd, *J =* 7.4, 5.2 Hz, 2H), 1.25 - 1.23 (m, 2H), 0.99 (t, *J* = 7.4 Hz, 3H). |
| **91** | 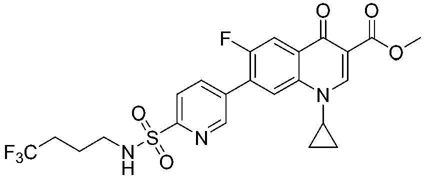 | LCMS: 528.3 [M+H]⁺ |
| | | ¹H NMR (400 MHz, DMSO-d6) δ 8.64 (s, 1H), 8.50 (t, J = 2.0 Hz, 1H), 8.34 (d, J = 7.8 Hz, 1H), 8.22 (d, J = 7.7 Hz, 1H), 7.97 (dt, J = 7.4, 1.7 Hz, 1H), 7.47 (t, J = 9.8 Hz, 1H), 7.41 (d, J = 5.1 Hz, 1H), 3.98 - 3.88 (m, 1H), 3.80 (s, 3H), 3.12 (dt, J = 9.8, 7.1 Hz, 2H), 2.36 - 2.32 (m, 2H), 1.73 (p, J = 7.2 Hz, 2H), 1.48 - 1.38 (m, 2H), 1.30 - 1.15 (m, 2H). |
| **92** | 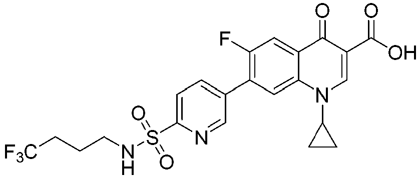 | LCMS: 514.3 [M+H]⁺ |
| | | ¹H NMR (400 MHz, DMSO-d6) δ 14.85 (s, 1H), 8.66 (s, 1H), 8.50 (t, J = 2.0 Hz, 1H), 8.36 (d, J = 7.8 Hz, 1H), 8.22 (d, J = 7.7 Hz, 1H), 7.97 (dt, J = 7.4, 1.7 Hz, 1H), 7.47 (t, J = 9.8 Hz, 1H), 7.42 (d, J = 4.8 Hz, 1H), 3.99 - 3.89 (m, 1H), 3.12 (dt, J = 9.8, 7.1 Hz, 2H), 2.35 - 2.29 (m, 2H), 1.80 - 1.73 (m, 2H), 1.46 - 1.38 (m, 2H), 1.32 - 1.18 (m, 2H). |
| **93** | 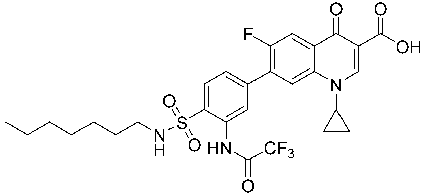 | LCMS:612.3 [M+H]⁺ |
| | | ¹H NMR (400 MHz, DMSO-d6) δ 14.75 (s, 1H), 10.96 (s, 1H), 8.81 (s, 1H), 8.44 (d, J = 6.0 Hz, 1H), 8.32 (s, 1H), 8.20 (d, J = 10.4 Hz, 1H), 8.09 (d, J = 8.4 Hz, 1H), 8.05 (t, J = 5.6 Hz, 1H), 7.90 (d, J = 8.4 Hz, 1H), 4.01-3.94 (m, 1H), 2.87 (dd, J = 12.8 Hz, 6.8 Hz, 2H), 1.45-1.37 (m, 2H), 1.35-1.30 (m, 2H), 1.27-1.16 (m, 10H), 0.82 (t, J = 6.8 Hz, 3H). |
| **94** | 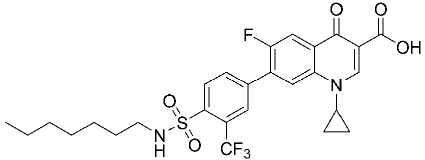 | LCMS:569.3 [M+H]⁺ |
| | | ¹H NMR (400 MHz, DMSO-d6) δ 14.75 (s, 1H), 8.75 (s, 1H), 8.48 (d, J = 8.0 Hz, 1H), 8.19 - 8.12 (m, 1H), 7.79 (d, J = 7.4 Hz, 1H), 7.58 - 7.39 (m, 3H), 4.12 - 4.06 (m, 1H), 2.83 - 2.68 (m, 2H), 1.56 - 1.10 (m, 14H), 0.99 - 0.87 (m, 3H). |
| **95** | 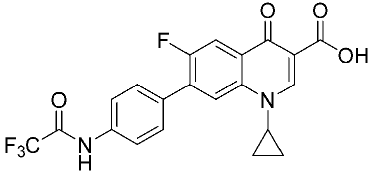 | LCMS:435.1 [M+H]⁺ |
| | | ¹H NMR (400 MHz, DMSO-d6) δ 14.83 (s, 1H), 9.70 (s, 1H), 8.64 (s, 1H), 8.20 (d, J = 8.0 Hz, 1H), 7.67 - 7.60 (m, 2H), 7.32 - 7.24 (m, 3H), 4.03 - 3.93 (m, 1H), 1.48 - 1.38 (m, 2H), 1.31 - 1.15 (m, 2H). |
| **96** | 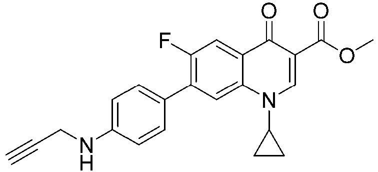 | LCMS: 391.2 [M+H]⁺ |
| | | ¹H NMR (400 MHz, DMSO-d6) δ 8.71 (s, 1H), 8.26 (d, J = 7.8 Hz, 1H), 7.56 - 7.48 (m, 2H), 7.34 (d, J = 5.1 Hz, 1H), 6.58 - 6.50 (m, 2H), 6.02 (t, J = 4.7 Hz, 1H), 4.08 - 3.88 (m, 3H), 3.81 (s, 3H), 2.41 (t, J = 2.9 Hz, 1H), 1.48 - 1.40 (m, 2H), 1.28 - 1.12 (m, 2H). |
| **97** | 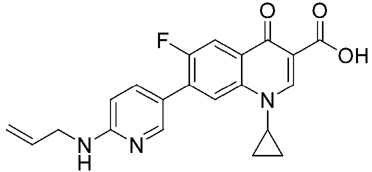 | LCMS:380.3 [M+H]⁺ |
| | | ¹H NMR (400 MHz, DMSO-d6) δ 8.56 (s, 1H), 8.31 (s, 1H), 8.18 (d, J = 6.5 Hz, 1H), 8.06 (s, 1H), 7.98 (d, J = 10.7 Hz, 1H), 7.54 (s, 1H), 6.94 (d, J = 9.0 Hz, 1H), 6.10 - 5.97 (m, 1H), 5.47 (d, J = 17.3 Hz, 1H), 5.26 (d, J = 12.0 Hz, 1H), 4.74 (d, J = 5.1 Hz, 2H), 3.76 - 3.73 (m, 1H), 1.30 - 1.26 (m, 2H), 1.17 - 1.12 (m, 2H). |
| **98** | 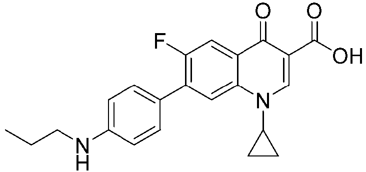 | LCMS: 381.3 [M+H]⁺ |
| | | ¹H NMR (400 MHz, DMSO-d6) δ 14.81 (s, 1H), 8.62 (s, 1H), 8.22 (d, J = 8.2 Hz, 1H), 7.50 - 7.32 (m, 3H), 6.86 - 6.79 (m, 2H), 6.11 (t, J = 5.7 Hz, 1H), 4.01 - 3.91 (m, 1H), 3.20 (td, J = 7.0, 5.5 Hz, 2H), 1.76 - 1.62 (m, 2H), 1.48 - 1.41 (m, 2H), 1.30 - 1.14 (m, 2H), 0.90 (t, J = 8.0 Hz, 3H). |
| **99** | 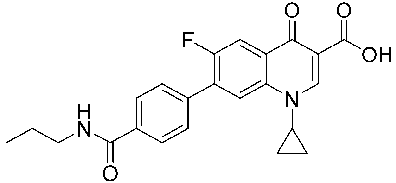 | LCMS: 409.2 [M+H]⁺ |
| | | ¹H NMR (400 MHz, DMSO-d6) δ 14.85 (s, 1H), 8.66 (s, 1H), 8.22 (d, J = 8.0 Hz, 1H), 7.93 - 7.87 (m, 3H), 7.67 - 7.62 (m, 2H), 7.35 (d, J = 5.0 Hz, 1H), 3.97 - 3.87 (m, 1H), 3.08 (q, J = 7.0 Hz, 2H), 1.48 - 1.16 (m, 6H), 0.79 (t, J = 8.0 Hz, 3H). |
| **100** | 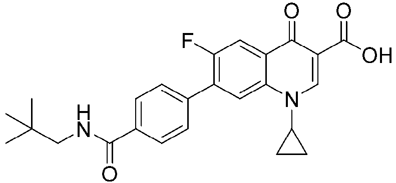 | LCMS: 437.2 [M+H]⁺ |
| | | ¹H NMR (400 MHz, DMSO-d6) δ 14.83 (s, 1H), 8.64 (s, 1H), 8.23 - 8.13 (m, 2H), 7.91 - 7.83 (m, 2H), 7.66 (dq, J = 7.8, 1.6 Hz, 2H), 7.33 (d, J = 5.1 Hz, 1H), 4.03 - 3.93 (m, 1H), 3.31 (d, J = 6.6 Hz, 2H), 1.51 - 1.35 (m, 2H), 1.29 - 1.13 (m, 11H). |
| **101** | 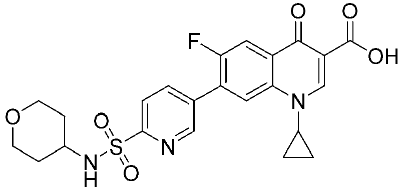 | LCMS: 488.1 [M+H]⁺ |
| | | ¹H NMR (400 MHz, DMSO-d6) δ 14.81 (s, 1H), 8.62 (s, 1H), 8.45 (t, J = 2.0 Hz, 1H), 8.31 (d, J = 8.0 Hz, 1H), 8.18 (d, J = 7.8 Hz, 1H), 7.93 - 7.86 (m, 1H), 7.37 (d, J = 4.8 Hz, 1H), 7.10 (d, J = 12.5 Hz, 1H), 3.99 - 3.89 (m, 1H), 3.77 (dt, J = 12.5, 7.0 Hz, 2H), 3.61 (dt, J = 12.1, 7.0 Hz, 2H), 3.49 - 3.44 (m, 1H), 1.94 - 1.89 (m, 4H), 1.48 - 1.34 (m, 2H), 1.32 - 1.16 (m, 2H). |
| **102** | 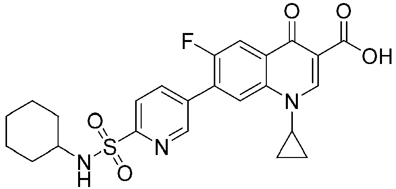 | LCMS: 486.2 [M+H]⁺ |
| | | ¹H NMR (400 MHz, DMSO-d6) δ 14.80 (s, 1H), 8.61 (s, 1H), 8.46 (t, J = 2.0 Hz, 1H), 8.30 (d, J = 8.0 Hz, 1H), 8.16 (d, J = 7.5 Hz, 1H), 7.89 (dt, J = 7.4, 1.8 Hz, 1H), 7.36 (d, J = 4.8 Hz, 1H), 7.11 (d, J = 12.5 Hz, 1H), 4.05 - 3.95 (m, 1H), 3.38 - 3.33 (m, 1H), 1.79 - 1.64 (m, 2H), 1.59 - 1.15 (m, 13H). |
| **103** | 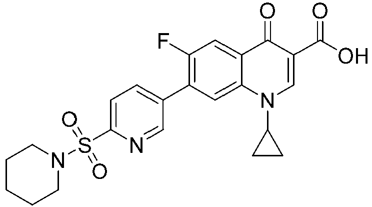 | LCMS: 472.1 [M+H]⁺ |
| | | ¹H NMR (400 MHz, DMSO-d6) δ 14.88 (s, 1H), 8.68 (s, 1H), 8.53 (t, J = 2.0 Hz, 1H), 8.36 (d, J = 8.0 Hz, 1H), 8.11 (d, J = 7.3 Hz, 1H), 7.98 - 7.94 (m, 1H), 7.43 (d, J = 5.1 Hz, 1H), 3.99 - 3.89 (m, 1H), 3.09 (t, J = 7.0 Hz, 4H), 1.66 - 1.14 (m, 10H). |
| **104** | 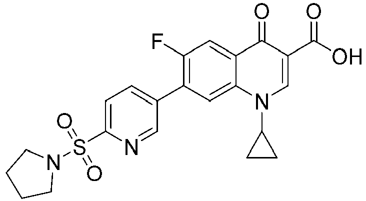 | LCMS: 458.1 [M+H]⁺ |
| | | ¹H NMR (400 MHz, DMSO-d6) δ 14.78 (s, 1H), 8.78 (s, 1H), 8.63 (t, J = 2.0 Hz, 1H), 8.46 (d, J = 8.0 Hz, 1H), 8.21 (d, J = 7.5 Hz, 1H), 8.11 - 8.04 (m, 1H), 7.42 (d, J = 5.1 Hz, 1H), 3.99 - 3.89 (m, 1H), 3.34 - 3.21 (m, 4H), 1.90 - 1.76 (m, 4H), 1.48 - 1.16 (m, 4H). |
| **105** | 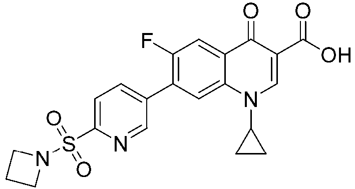 | LCMS: 444.1 [M+H]⁺ |
| | | ¹H NMR (400 MHz, DMSO-d6) δ 14.79 (s, 1H), 8.58 (s, 1H), 8.46 (t, J = 1.9 Hz, 1H), 8.27 (d, J = 8.0 Hz, 1H), 8.09 (d, J = 7.5 Hz, 1H), 7.97 - 7.95 (m, 1H), 7.34 (d, J = 5.1 Hz, 1H), 3.98 - 3.89 (m, 1H), 3.44 - 3.39 (m, 4H), 1.98 - 1.93 (m, 2H), 1.48 - 1.38 (m, 2H), 1.30 - 1.14 (m, 2H). |
| **106** | 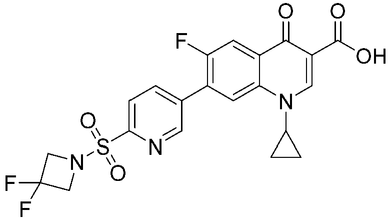 | LCMS: 480.2 [M+H]⁺ |
| | | ¹H NMR (400 MHz, DMSO-d6) δ 14.81 (s, 1H), 8.65 (s, 1H), 8.52 - 8.48 (m, 1H), 8.33 (d, J = 8.0 Hz, 1H), 8.06 (d, J = 7.3 Hz, 1H), 7.96 - 7.92 (m, 1H), 7.40 (d, J = 5.1 Hz, 1H), 4.11 - 3.83 (m, 5H), 1.48 - 1.38 (m, 2H), 1.32 - 1.16 (m, 2H). |
| **107** | 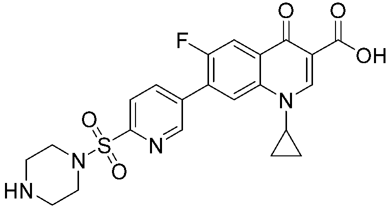 | LCMS: 473.1 [M+H]⁺ |
| | | ¹H NMR (400 MHz, DMSO-d6) δ 14.82 (s, 1H), 8.62 (s, 1H), 8.47 (t, J = 2.0 Hz, 1H), 8.30 (d, J = 8.0 Hz, 1H), 8.04 (d, J = 7.3 Hz, 1H), 7.95 - 7.89 (m, 1H), 7.37 (d, J = 5.1 Hz, 1H), 3.95 - 3.89 (m, 1H), 3.24 (t, J = 7.0 Hz, 4H), 2.93 - 2.79 (m, 5H), 1.40 - 1.15 (m, 4H). |
| **108** | 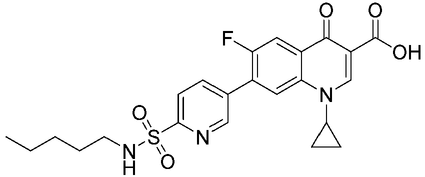 | LCMS: 474.2 [M+H]⁺ |
| | | ¹H NMR (400 MHz, DMSO-d6) δ 14.80 (s, 1H), 8.71 (s, 1H), 8.53 (t, J = 2.0 Hz, 1H), 8.39 - 8.30 (m, 2H), 8.01 - 7.95 (m, 1H), 7.47 - 7.43 (m, 2H), 4.02 - 3.92 (m, 1H), 2.99 - 2.78 (m, 2H), 1.53 - 1.16 (m, 11H), 0.95 - 0.83 (m, 3H). |
| **109** | 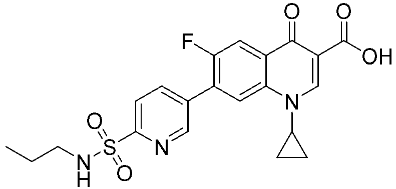 | LCMS: 446.1 [M+H]⁺ |
| | | ¹H NMR (400 MHz, DMSO-d6) δ 14.78 (s, 1H), 8.69 (s, 1H), 8.54 - 8.49 (m, 1H), 8.36 (d, J = 8.0 Hz, 1H), 8.22 (d, J = 7.5 Hz, 1H), 7.97 - 7.95 (m, 1H), 7.50 - 7.40 (m, 2H), 4.00-3.93 (m, 1H), 2.87 - 2.81 (m, 2H), 1.46 - 1.38 (m, 4H), 1.26 - 1.10 (m, 2H), 0.90 (t, J = 8.0 Hz, 3H). |
| **110** | 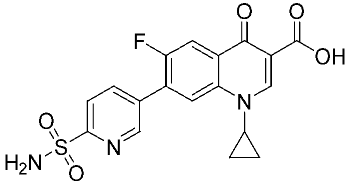 | LCMS: 404.1 [M+H]⁺ |
| | | ¹H NMR (400 MHz, DMSO-d6) δ 14.80 (s, 1H), 8.72 (s, 1H), 8.50 (t, J = 1.8 Hz, 1H), 8.29 (d, J = 7.8 Hz, 1H), 8.08 - 7.95 (m, 2H), 7.38 - 7.31 (m, 3H), 3.98-3.88 (m, 1H), 1.48 - 1.33 (m, 2H), 1.26 - 1.16 (m, 2H). |
| **111** | 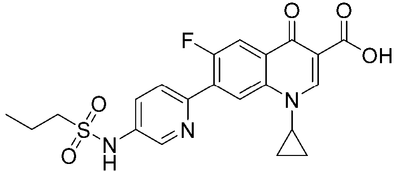 | LCMS: 446.1 [M+H]⁺ |
| | | ¹H NMR (400 MHz, DMSO-d6) δ 14.81 (s, 1H), 9.80 (s, 1H), 8.69 (s, 1H), 8.35 (d, J = 8.0 Hz, 1H), 8.29 (d, J = 1.7 Hz, 1H), 7.70 - 7.62 (m, 2H), 7.55 (d, J = 5.0 Hz, 1H), 3.97 - 3.87 (m, 1H), 3.02 (t, J = 7.1 Hz, 2H), 1.81 - 1.67 (m, 2H), 1.48 - 1.32 (m, 2H), 1.26 - 1.16 (m, 2H), 1.01 (t, J = 8.0 Hz, 3H). |
| **112** | 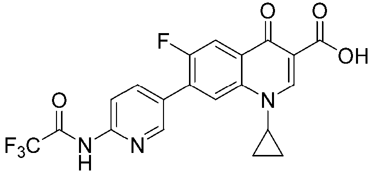 | LCMS: 436.1 [M+H]⁺ |
| | | ¹H NMR (400 MHz, DMSO-d6) δ 14.78 (s, 1H), 10.75 (s, 1H), 8.59 (s, 1H), 8.27 (d, J = 7.8 Hz, 1H), 8.19 - 7.99 (m, 2H), 7.81 - 7.75 (m, 1H), 7.38 (d, J = 5.1 Hz, 1H), 3.98 - 3.88 (m, 1H), 1.48 - 1.36 (m, 2H), 1.26 - 1.17 (m, 2H). |
| **113** | 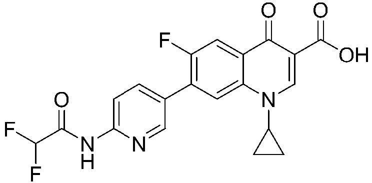 | LCMS: 418.1 [M+H]⁺ |
| | | ¹H NMR (400 MHz, DMSO-d6) δ 14.81 (s, 1H), 10.58 (s, 1H), 8.63 (s, 1H), 8.30 (d, J = 7.8 Hz, 1H), 8.23 (t, J = 1.7 Hz, 1H), 7.89 - 7.82 (m, 2H), 7.40 (d, J = 4.8 Hz, 1H), 6.50 - 6.40 (m, 1H), 3.99 - 3.89 (m, 1H), 1.42 - 1.32 (m, 2H), 1.28 - 1.16 (m, 2H). |
| **114** | 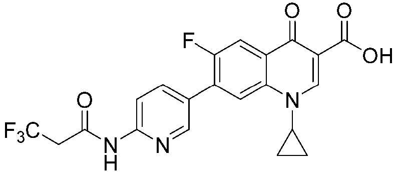 | LCMS: 450.1 [M+H]⁺ |
| | | ¹H NMR (400 MHz, DMSO-d6) δ 14.81 (s, 1H), 10.31 (s, 1H), 8.59 (s, 1H), 8.33 - 8.28 (m, 2H), 7.90 - 7.82 (m, 2H), 7.38 (d, J = 4.8 Hz, 1H), 3.99 - 3.89 (m, 1H), 3.11 - 3.02 (m, 2H), 1.48 - 1.32 (m, 2H), 1.26 - 1.16 (m, 2H). |
| **115** | 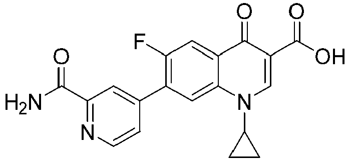 | LCMS:368.2 [M+H]+. |
| | | ¹H NMR (400 MHz, DMSO) δ 14.73 (s, 1H), 8.85 (d, J = 5.0 Hz, 1H), 8.82 (s, 1H), 8.51 (d, J = 6.3 Hz, 1H), 8.35 (s, 1H), 8.28 - 8.19 (m, 2H), 7.97 (d, J = 4.8 Hz, 1H), 7.81 (s, 1H), 4.05 - 3.95 (m, 1H), 1.37 - 1.29 (m, 2H), 1.28 - 1.22 (m, 2H). |
| **116** | 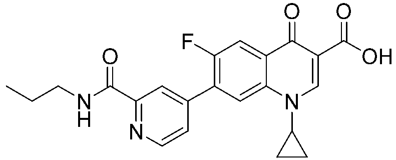 | LCMS:410.5 [M+H]⁺ |
| | | ¹H NMR (400 MHz, DMSO-d6) δ 14.74 (s, 1H), 8.94 (t, J = 6.0 Hz, 1H), 8.86 (d, J = 5.0 Hz, 1H), 8.82 (s, 1H), 8.51 (d, J = 6.1 Hz, 1H), 8.34 (s, 1H), 8.22 (d, J = 10.4 Hz, 1H), 7.97 (d, J = 5.0 Hz, 1H), 4.07 - 3.93 (m, 1H), 3.30 - 3.27 (m, 2H), 1.58 (dd, J = 14.6, 7.2 Hz, 2H), 1.36 - 1.21 (m, 4H), 0.90 (t, J = 7.4 Hz, 3H). |
| **117** | 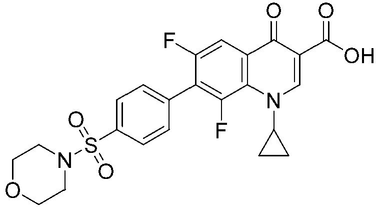 | LCMS: 491.1 [M+H]⁺ |
| | | ¹H NMR (400 MHz, DMSO-d6) δ 14.85 (s, 1H), 8.66 (s, 1H), 8.05 (d, J = 8.2 Hz, 1H), 7.83 - 7.76 (m, 2H), 7.55 - 7.48 (m, 2H), 4.12 - 4.02 (m, 1H), 3.62 - 3.57 (m, 4H), 3.15 - 3.05 (m, 4H), 1.43 - 1.28 (m, 2H), 1.22 - 1.16 (m, 2H). |
| **118** | 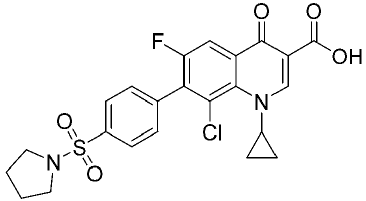 | LCMS: 491.1 [M+H]⁺ |
| | | ¹H NMR (400 MHz, DMSO-d6) δ 14.81 (s, 1H), 8.70 (s, 1H), 8.12 (d, J = 8.2 Hz, 1H), 7.82 - 7.75 (m, 2H), 7.60 - 7.52 (m, 2H), 4.25 - 4.15 (m, 1H), 3.22 - 3.10 (m, 4H), 1.80 - 1.66 (m, 4H), 1.47 - 1.11 (m, 4H). |
| **119** | 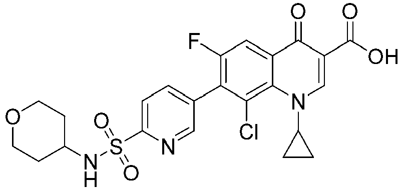 | LCMS: 522.1 [M+H]⁺ |
| | | ¹H NMR (400 MHz, DMSO-d6) δ 14.85 (s, 1H), 8.66 (s, 1H), 8.47 (t, J = 2.0 Hz, 1H), 8.18 - 7.91 (m, 3H), 7.15 (d, J = 12.5 Hz, 1H), 4.26 - 4.16 (m, 1H), 3.71 - 3.55 (m, 4H), 3.44 - 3.38 (m, 1H), 2.00 - 1.85 (m, 4H), 1.37 - 1.26 (m, 2H), 1.15 - 1.00 (m, 2H). |
| **120** | 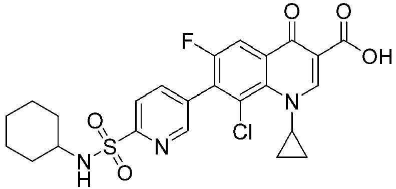 | LCMS: 520.1 [M+H]⁺ |
| | | ¹H NMR (400 MHz, DMSO-d6) δ 14.85 (s, 1H), 8.63 (s, 1H), 8.42 (t, J = 2.0 Hz, 1H), 8.22 - 8.14 (m, 2H), 7.91 - 7.86 (m, 1H), 7.17 (d, J = 12.5 Hz, 1H), 4.30 - 4.16 (m, 1H), 3.33 - 3.28 (m, 1H), 1.68 - 1.21 (m, 14H), 1.15 - 1.00 (m, 2H). |
| **121** | 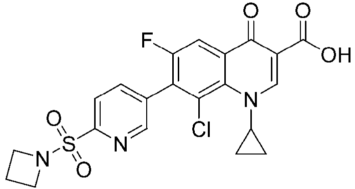 | LCMS: 478.1 [M+H]⁺ |
| | | ¹H NMR (400 MHz, DMSO-d6) δ 14.82 (s, 1H), 8.70 (s, 1H), 8.45 (t, J = 1.9 Hz, 1H), 8.16 (d, J = 7.8 Hz, 1H), 8.17 (d, J = 7.4 Hz, 1H), 7.92 - 7.88 (m, 1H), 4.29 - 4.19 (m, 1H), 3.45 - 3.38 (m, 4H), 1.98 - 1.86 (m, 2H), 1.37 - 1.27 (m, 2H), 1.15 - 0.99 (m, 2H). |
| **122** | 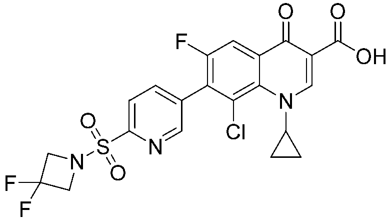 | LCMS: 514.1 [M+H]⁺ |
| | | ¹H NMR (400 MHz, DMSO-d6) δ 14.81 (s, 1H), 8.69 (s, 1H), 8.46 (t, J = 1.8 Hz, 1H), 8.17 (d, J = 7.8 Hz, 1H), 8.04 (d, J = 7.6 Hz, 1H), 7.94 - 7.90 (m, 1H), 4.29 - 4.20 (m, 1H), 4.09 - 3.88 (m, 6H), 1.37 - 1.27 (m, 2H), 1.15 - 1.05 (m, 2H). |
| **123** | 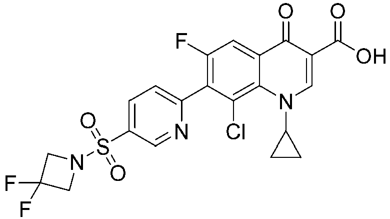 | LCMS: 514.1 [M+H]⁺ |
| | | ¹H NMR (400 MHz, DMSO-d6) δ 14.82 (s, 1H), 8.96 (d, J = 1.7 Hz, 1H), 8.68 (s, 1H), 8.16 (d, J = 7.8 Hz, 1H), 8.11 (dd, J = 7.4, 1.6 Hz, 1H), 7.82 - 7.75 (m, 1H), 4.31 - 4.21 (m, 1H), 3.92 - 3.81 (m, 4H), 1.34 - 1.26 (m, 2H), 1.14 - 1.04 (m, 2H). |
| **124** | 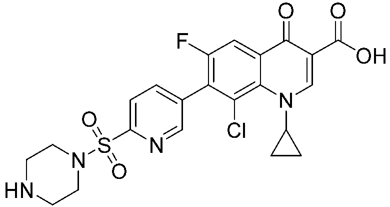 | LCMS: 507.1 [M+H]⁺ |
| | | ¹H NMR (400 MHz, DMSO-d6) δ 14.81 (s, 1H), 8.64 (s, 1H), 8.47 (t, J = 2.0 Hz, 1H), 8.17 (d, J = 7.8 Hz, 1H), 8.05 (d, J = 7.3 Hz, 1H), 7.94 - 7.88 (m, 1H), 4.30 - 4.20 (m, 1H), 3.30 - 3.20 (m, 4H), 2.97 - 2.79 (m, 5H), 1.37 - 1.27 (m, 2H), 1.18 - 1.06 (m, 2H). |
| **125** | 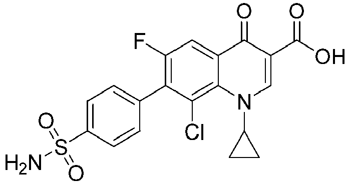 | LCMS: 437.1 [M+H]⁺ |
| | | ¹H NMR (400 MHz, DMSO-d6) δ 14.85 (s, 1H), 8.70 (s, 1H), 8.12 (d, J = 8.2 Hz, 1H), 7.88 - 7.71 (m, 4H), 7.60 (dd, J = 7.4, 1.9 Hz, 2H), 4.30 - 4.20 (m, 1H), 1.36 - 1.21 (m, 2H), 1.13 - 0.98 (m, 2H). |
| **126** | 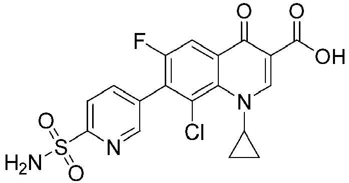 | LCMS: 438.1 [M+H]⁺ |
| | | ¹H NMR (400 MHz, DMSO-d6) δ 14.80 (s, 1H), 8.70 (s, 1H), 8.46 - 8.31 (m, 2H), 8.19 (d, J = 7.5 Hz, 1H), 7.91 (dt, J = 7.4, 1.7 Hz, 1H), 7.32 (s, 2H), 4.31 - 4.21 (m, 1H), 1.36 - 1.26 (m, 2H), 1.13 - 0.98 (m, 2H). |
| **127** | 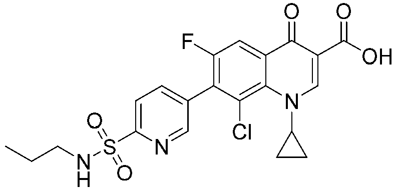 | LCMS:479.9 [M+H] ⁺ |
| | | ¹H NMR (400 MHz, DMSO) δ 14.19 (s, 1H), 8.94 (s, 1H), 8.87 (s, 1H), 8.27 (dd, *J* = 8.0, 1.8 Hz, 1H), 8.22 (d, *J =* 8.6 Hz, 1H), 8.15 (d, *J* = 8.1 Hz, 1H), 8.00 (t, *J* = 5.8 Hz, 1H), 4.54 - 4.21 (m, 1H), 2.97 (dd, *J* = 13.1, 6.7 Hz, 2H), 1.48 - 1.38 (m, 2H), 1.26 - 1.19 (m, 2H), 1.18 - 1.12 (m, 2H), 0.82 (t, *J* = 7.4 Hz, 3H). |
| **128** | 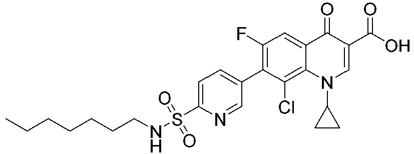 | LCMS:536.1 [M+H]+. |
| | | ¹H NMR (400 MHz, DMSO) δ 14.17 (s, 1H), 8.94 (s, 1H), 8.87 (d, J = 1.8 Hz, 1H), 8.27 (dd, J = 8.1, 2.1 Hz, 1H), 8.22 (d, J = 8.5 Hz, 1H), 8.14 (d, J = 8.0 Hz, 1H), 7.98 (t, J = 5.6 Hz, 1H), 4.54 - 4.21 (m, 1H), 3.00 (dd, J = 12.6, 6.7 Hz, 2H), 1.38 (dd, J = 13.5, 7.0 Hz, 2H), 1.25 - 1.14 (m, 12H), 0.84 (t, J = 7.0 Hz, 3H). |
| **129** | 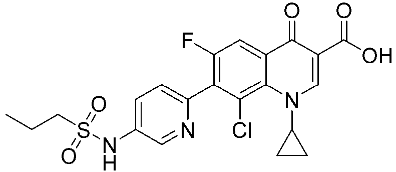 | LCMS: 479.9 [M+H]⁺ |
| | | ¹H NMR (400 MHz, DMSO-d6) δ 14.81 (s, 1H), 9.77 (s, 1H), 8.70 (s, 1H), 8.38 (d, J = 1.6 Hz, 1H), 8.16 (d, J = 7.8 Hz, 1H), 7.70 - 7.55 (m, 2H), 4.30 - 4.20 (m, 1H), 3.05 (t, J = 7.1 Hz, 2H), 1.81 - 1.67 (m, 2H), 1.34 - 1.26 (m, 2H), 1.30 - 1.21 (m, 2H), 1.08 - 0.98 (m, 4H). |
| **130** | 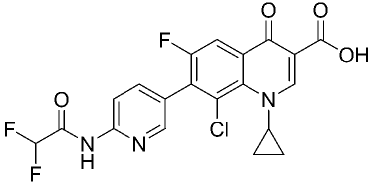 | LCMS: 452.1 [M+H]⁺ |
| | | ¹H NMR (400 MHz, DMSO-d6) δ 14.87 (s, 1H), 10.45 (s, 1H), 8.70 (s, 1H), 8.26 - 8.19 (m, 2H), 7.92 - 7.81 (m, 2H), 6.50 - 6.35 (m, 1H), 4.31 - 4.21 (m, 1H), 1.36 - 1.26 (m, 2H), 1.14 - 0.98 (m, 2H). |
| **131** | 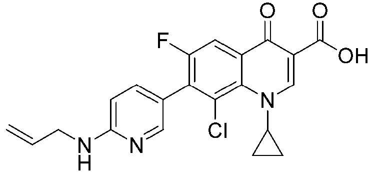 | LCMS: 414.1 [M+H]⁺ |
| | | ¹H NMR (400 MHz, DMSO-d6) δ 14.88 (s, 1H), 8.70 (s, 1H), 8.20 - 8.09 (m, 2H), 7.67 (dt, J = 7.4, 1.8 Hz, 1H), 7.07 - 6.98 (m, 2H), 5.99 - 5.85 (m, 1H), 5.24 - 5.14 (m, 2H), 4.32 - 4.22 (m, 1H), 4.10 - 4.05 (m, 2H), 1.36 - 1.21 (m, 2H), 1.13 - 0.98 (m, 2H). |
| **132** | 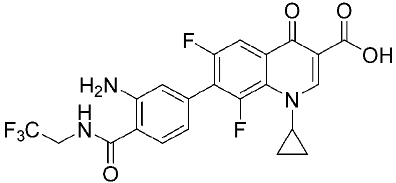 | LCMS:482.3 [M+H]⁺ |
| | | ¹H NMR (400 MHz, DMSO-d6) δ 14.50 (s, 1H), 8.95 (s, 1H), 8.78 (s, 1H), 8.03 (dd, J = 9.1, 1.5 Hz, 1H), 7.70 (d, J = 8.3 Hz, 1H), 6.90 (s, 1H), 6.69 (d, J = 11.1 Hz, 3H), 4.21 - 3.96 (m, 3H), 1.32 - 1.07 (m, 4H). |
| **133** | 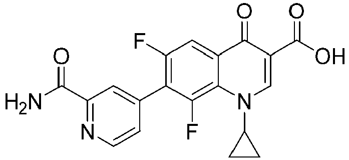 | LCMS: 386.2 [M+H]⁺ |
| | | ¹H NMR (400 MHz, DMSO-d6) δ 14.81 (s, 1H), 8.91 (d, J = 7.6 Hz, 1H), 8.65 (s, 1H), 8.18 - 8.08 (m, 2H), 7.79 (d, J = 9.4 Hz, 1H), 7.67 - 7.62 (m, 2H), 4.12 - 4.02 (m, 1H), 1.43 - 1.27 (m, 2H), 1.20 - 1.10 (m, 2H). |
| **134** | 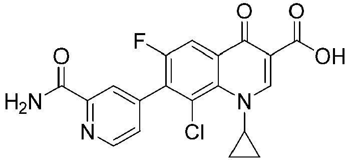 | LCMS: 402.2 [M+H]⁺ |
| | | ¹H NMR (400 MHz, DMSO-d6) δ 14.88 (s, 1H), 8.92 (d, J = 7.4 Hz, 1H), 8.69 (s, 1H), 8.23 - 8.17 (m, 2H), 7.79 - 7.68 (m, 3H), 4.30 - 4.20 (m, 1H), 1.36 - 1.21 (m, 2H), 1.13 - 0.98 (m, 2H). |
| **135** | 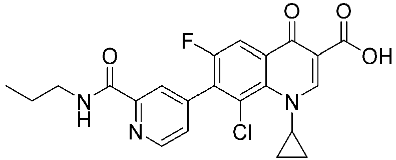 | LCMS: 444.2 [M+H]⁺ |
| | | ¹H NMR (400 MHz, DMSO-d6) δ 14.81 (s, 1H), 8.91 (d, J = 7.4 Hz, 1H), 8.70 (s, 1H), 8.25 - 8.12 (m, 3H), 7.74 (dt, J = 7.4, 1.8 Hz, 1H), 4.30 - 4.20 (m, 1H), 3.30 - 3.25 (m, 2H), 1.58 - 1.44 (m, 2H), 1.36 - 1.21 (m, 2H), 1.14 - 1.04 (m, 2H), 0.91 (t, J = 8.0 Hz, 3H). |
| **136** | 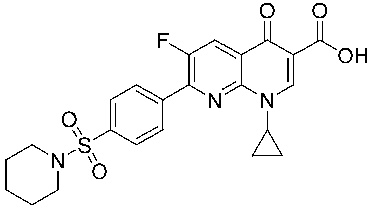 | LCMS: 472.1 [M+H]⁺ |
| | | ¹H NMR (400 MHz, DMSO-d6) δ 14.86 (s, 1H), 9.05 (s, 1H), 7.96 - 7.77 (m, 5H), 4.00 - 3.90 (m, 1H), 2.97 - 2.92 (m, 4H), 1.59 - 1.34 (m, 8H), 1.31 - 1.16 (m, 2H). |
| **137** | 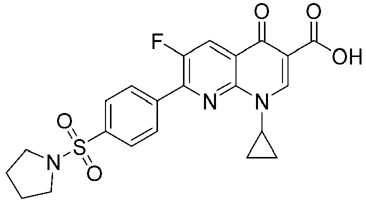 | LCMS: 458.1 [M+H]⁺ |
| | | ¹H NMR (400 MHz, DMSO-d6) δ 14.83 (s, 1H), 9.05 (s, 1H), 7.88 - 7.72 (m, 5H), 4.06 - 3.96 (m, 1H), 3.22 - 3.10 (m, 4H), 1.80 - 1.66 (m, 4H), 1.47 - 1.17 (m, 4H). |
| **138** | 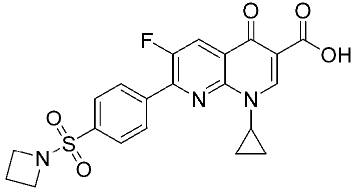 | LCMS: 444.1 [M+H]⁺ |
| | | ¹H NMR (400 MHz, DMSO-d6) δ 14.87 (s, 1H), 9.05 (s, 1H), 7.86 - 7.72 (m, 5H), 4.06 - 3.96 (m, 1H), 3.43 - 3.36 (m, 4H), 2.03 - 1.95 (m, 2H), 1.50 - 1.34 (m, 2H), 1.34 - 1.19 (m, 2H). |
| **139** | 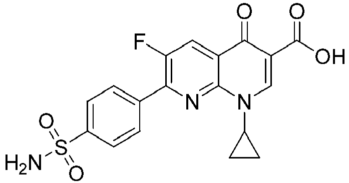 | LCMS: 404.1 [M+H]⁺ |
| | | ¹H NMR (400 MHz, DMSO-d6) δ 14.80 (s, 1H), 9.05 (s, 1H), 7.94 - 7.76 (m, 5H), 7.22 (s, 2H), 4.04 - 3.94 (m, 1H), 1.50 - 1.34 (m, 2H), 1.32 - 1.17 (m, 2H). |
| **140** | 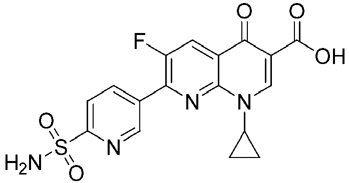 | LCMS: 405.1 [M+H]⁺ |
| | | ¹H NMR (400 MHz, DMSO-d6) δ 14.88 (s, 1H), 9.05 (s, 1H), 8.74 (dt, J = 1.9, 0.8 Hz, 1H), 8.25 (t*, J =* 1.3 Hz, 2H), 7.80 (d, J = 8.2 Hz, 1H), 7.31 (s, 2H), 4.05 - 3.95 (m, 1H), 1.50 - 1.34 (m, 2H), 1.32 - 1.17 (m, 2H). |
| **141** | 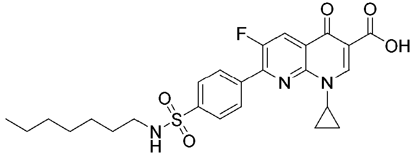 | LCMS:502.3[M+H]⁺ |
| | | ¹H NMR (400 MHz, DMSO-d6) δ 14.39 (s, 1H), 8.90 (s, 1H), 8.68 (d, J = 10.4 Hz, 1H), 8.38 (d, J = 7.2 Hz, 2H), 8.02 (d, J = 8.4 Hz, 2H), 7.79 (t, J = 5.8 Hz, 1H), 3.98 - 3.93 (m, 1H), 2.86 - 2.76 (m, 2H), 1.42 - 1.35 (m, 2H), 1.33 - 1.27 (m, 2H), 1.24 - 1.15 (m, 10H), 0.81 (t, J = 6.9 Hz, 3H). |
| **142** | 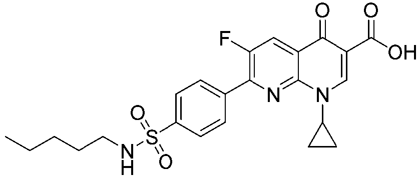 | LCMS: 474.2 [M+H]⁺ |
| | | ¹H NMR (400 MHz, DMSO-d6) δ 14.86 (s, 1H), 9.05 (s, 1H), 7.92 - 7.86 (m, 4H), 7.77 (d, J = 8.2 Hz, 1H), 7.29 (t, J = 9.6 Hz, 1H), 4.06 - 3.96 (m, 1H), 2.75 - 2.70 (m, 2H), 1.53 - 1.17 (m, 11H), 0.95 - 0.84 (m, 3H). |
| **143** | 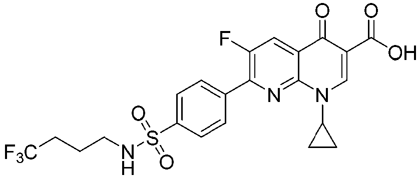 | LCMS: 514.2 [M+H]⁺ |
| | | ¹H NMR (400 MHz, DMSO-d6) δ 14.84 (s, 1H), 9.05 (s, 1H), 8.02 - 7.87 (m, 4H), 7.79 (d, J = 7.8 Hz, 1H), 7.47 (t, J = 9.0 Hz, 1H), 4.05 - 3.95 (m, 1H), 2.86 - 2.80 (m, 2H), 2.32 - 2.26 (m, 2H), 1.68 - 1.17 (m, 6H). |
| **144** | 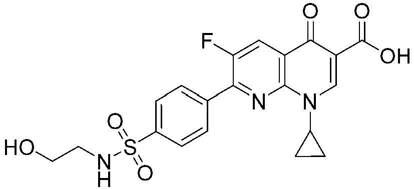 | LCMS: 448.1 [M+H]⁺ |
| | | ¹H NMR (400 MHz, DMSO-d6) δ 14.81 (s, 1H), 9.01 (s, 1H), 7.87 - 7.76 (m, 5H), 7.58 (t, J = 10.2 Hz, 1H), 4.49 (t, J = 6.1 Hz, 1H), 4.02 - 3.92 (m, 1H), 3.64 - 3.58 (m, 2H), 3.19 - 3.10 (m, 2H), 1.50 - 1.34 (m, 2H), 1.34 - 1.20 (m, 2H). |
| **145** | 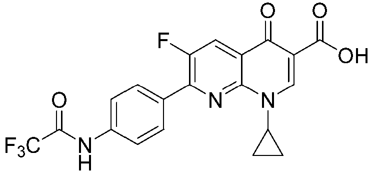 | LCMS: 436.1 [M+H]⁺ |
| | | ¹H NMR (400 MHz, DMSO-d6) δ 14.81 (s, 1H), 10.19 (s, 1H), 9.05 (s, 1H), 7.79 (d, J = 8.2 Hz, 1H), 7.69 - 7.58 (m, 4H), 4.05 - 3.95 (m, 1H), 1.50 - 1.34 (m, 2H), 1.34 - 1.24 (m, 2H). |
| **146** | 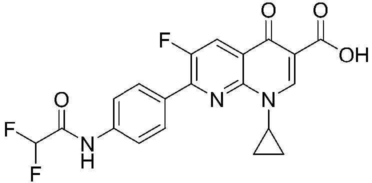 | LCMS: 418.1 [M+H]⁺ |
| | | ¹H NMR (400 MHz, DMSO-d6) δ 14.81 (s, 1H), 10.30 (s, 1H), 9.05 (s, 1H), 7.79 (d, J = 8.2 Hz, 1H), 7.66 - 7.50 (m, 4H), 6.50 - 6.42 (s, 1H), 4.05 - 3.95 (m, 1H), 1.48 - 1.40 (m, 2H), 1.32 - 1.17 (m, 2H). |
| **147** | 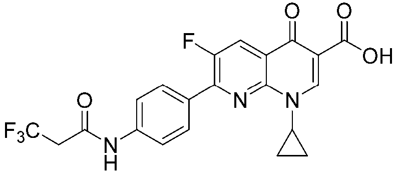 | LCMS: 450.3 [M+H]⁺ |
| | | ¹H NMR (400 MHz, DMSO-d6) δ 14.87 (s, 1H), 10.18 (s, 1H), 9.05 (s, 1H), 7.79 (d, J = 8.2 Hz, 1H), 7.63 - 7.59 (m, 4H), 4.06 - 3.96 (m, 1H), 3.06 - 2.96 (m, 2H), 1.50 - 1.34 (m, 2H), 1.32 - 1.22 (m, 2H). |
| **148** | 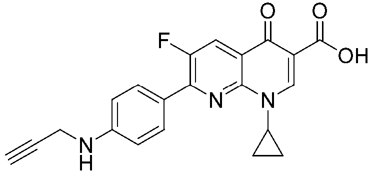 | LCMS: 378.1 [M+H]⁺ |
| | | ¹H NMR (400 MHz, DMSO-d6) δ 14.88 (s, 1H), 9.05 (s, 1H), 8.00 - 7.79 (m, 3H), 6.58 - 6.51 (m, 2H), 6.00 (t, J = 4.7 Hz, 1H), 4.11 - 3.99 (m, 3H), 2.39 (t, J = 2.9 Hz, 1H), 1.49 - 1.33 (m, 2H), 1.31 - 1.15 (m, 2H). |
| **149** | 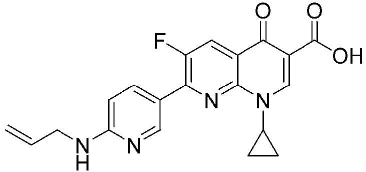 | LCMS:381.3 [M+H]⁺ |
| | | ¹H NMR (400 MHz, DMSO-d6) δ 8.56 (s, 1H), 8.31 (s, 1H), 8.18 (d, J = 6.5 Hz, 1H), 8.06 (s, 1H), 7.98 (d, J = 10.7 Hz, 1H), 7.54 (s, 1H), 6.94 (d, J = 9.0 Hz, 1H), 6.10 - 5.97 (m, 1H), 5.47 (d, J = 17.3 Hz, 1H), 5.26 (d, J = 12.0 Hz, 1H), 4.74 (d, J = 5.1 Hz, 2H), 3.76 - 3.73 (m, 1H), 1.30 - 1.26 (m, 2H), 1.17 - 1.12 (m, 2H). |
| **150** | 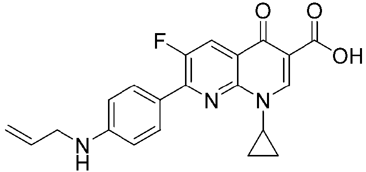 | LCMS: 380.1 [M+H]⁺ |
| | | ¹H NMR (400 MHz, DMSO-d6) δ 14.79 (s, 1H), 9.05 (s, 1H), 8.05 - 7.79 (m, 3H), 6.61 - 6.46 (m, 2H), 6.17 - 5.81 (m, 2H), 5.20 (dd, J = 13.6, 1.2 Hz, 2H), 4.16 - 3.92 (m, 3H), 1.54 - 1.17 (m, 4H). |
| **151** | 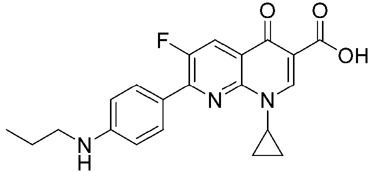 | LCMS: 382.2 [M+H]⁺ |
| | | ¹H NMR (400 MHz, DMSO-d6) δ 14.81 (s, 1H), 9.02 (s, 1H), 8.12 - 7.93 (m, 2H), 7.79 (d, J = 8.2 Hz, 1H), 7.43 - 7.23 (m, 2H), 6.23 (t, J = 5.5 Hz, 1H), 4.05 - 3.95 (m, 1H), 3.16 (td, J = 7.0, 5.5 Hz, 2H), 1.71 - 1.16 (m, 6H), 0.96 (t, J = 8.0 Hz, 3H). |
| **152** | 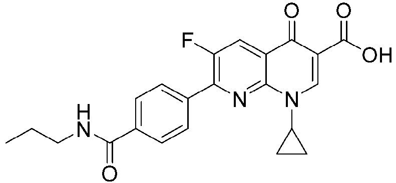 | LCMS: 410.2 [M+H]⁺ |
| | | ¹H NMR (400 MHz, DMSO-d6) δ 14.81 (s, 1H), 8.99 (s, 1H), 8.04 - 7.53 (m, 6H), 4.04 - 3.94 (m, 1H), 3.11 - 3.06 (m, 2H), 1.54 - 1.12 (m, 7H), 0.79 (t, J = 8.0 Hz, 3H). |
| **153** | 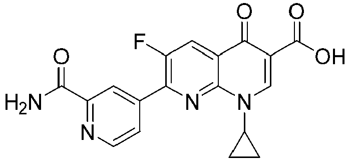 | LCMS: 369.1 [M+H]⁺ |
| | | ¹H NMR (400 MHz, DMSO-d6) δ 14.71 (s, 1H), 9.12 - 8.91 (m, 2H), 8.22 (t, J = 1.8 Hz, 1H), 8.08 - 7.70 (m, 4H), 4.07 - 3.99 (m, 1H), 1.51 - 1.12 (m, 4H). |
| **154** | 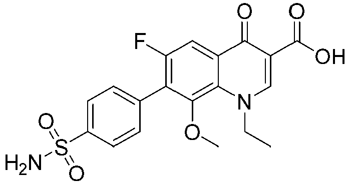 | LCMS: 421.1 [M+H]⁺ |
| | | ¹H NMR (400 MHz, DMSO-d6) δ 14.88 (s, 1H), 8.85 (s, 1H), 8.05 (d, J = 7.8 Hz, 1H), 7.81 - 7.54 (m, 4H), 7.22 (s, 2H), 4.39 - 4.31 (m, 2H), 3.85 (s, 3H), 1.41 (t, J = 8.0 Hz, 3H). |
| **155** | 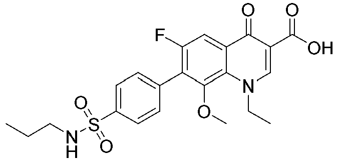 | LCMS: 463.1 [M+H]⁺ |
| | | ¹H NMR (400 MHz, DMSO-d6) δ 14.88 (s, 1H), 8.83 (s, 1H), 8.05 - 7.79 (m, 3H), 7.58 - 7.53 (m, 2H), 7.22 (t, J = 9.4 Hz, 1H), 4.38 - 4.21 (m, 2H), 3.84 (s, 3H), 2.93 - 2.86 (m, 2H), 1.71 - 1.35 (m, 5H), 0.92 (t, J = 8.0 Hz, 3H). |
| **156** | 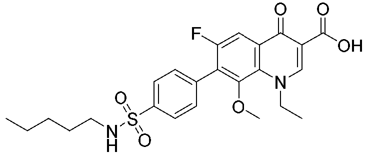 | LCMS: 491.2 [M+H]⁺ |
| | | ¹H NMR (400 MHz, DMSO-d6) δ 14.87 (s, 1H), 8.82 (s, 1H), 8.05 (d, J = 7.8 Hz, 1H), 7.90 - 7.81 (m, 2H), 7.56 - 7.48(m, 2H), 7.32 (t, J = 9.4 Hz, 1H), 4.36 - 4.21 (m, 2H), 3.85 (s, 3H), 2.80 - 2.75 (m, 2H), 1.59 - 1.53 (m, 2H), 1.41 - 1.25 (m, 7H), 0.99 - 0.86 (m, 3H). |
| **157** | 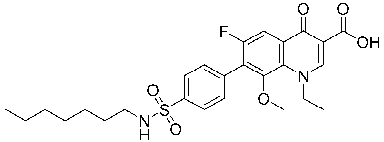 | LCMS: 519.2 [M+H]⁺ |
| | | ¹H NMR (400 MHz, DMSO-d6) δ 14.86 (s, 1H), 8.77 (s, 1H), 8.01 7.84 (m, 3H), 7.60 - 7.52 (m, 2H), 7.29 (t, J = 9.4 Hz, 1H), 4.40 - 4.35 (m, 2H), 3.85 (s, 3H), 2.72 - 2.68 (m, 2H), 1.62 - 1.23 (m, 13H), 0.95 - 0.85 (m, 3H). |
| **158** | 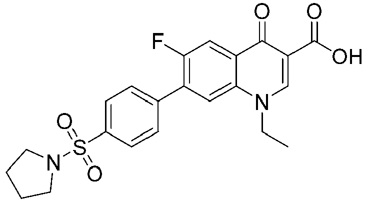 | LCMS: 445.2 [M+H]⁺ |
| | | ¹H NMR (400 MHz, DMSO-d6) δ 14.88 (s, 1H), 8.86 (s, 1H), 8.26 (d, J = 8.0 Hz, 1H), 7.80 - 7.49 (m, 4H), 7.32 (d, J = 5.1 Hz, 1H), 4.44 - 4.29 (m, 2H), 3.28 - 3.04 (m, 4H), 1.87 - 1.62 (m, 4H), 1.38 (t, J = 8.0 Hz, 3H). |
| **159** | 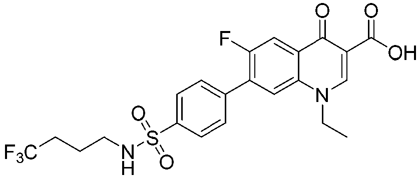 | LCMS: 501.1 [M+H]⁺ |
| | | ¹H NMR (400 MHz, DMSO-d6) δ 14.86 (s, 1H), 8.92 (s, 1H), 8.28 (d, J = 8.0 Hz, 1H), 7.98 - 7.82 (m, 2H), 7.65 - 7.27 (m, 4H), 4.48 - 4.36 (m, 2H), 2.86 - 2.81 (, 2H), 2.32 - 3.26 (, 2H), 1.72 - 1.65 (m, 2H), 1.37 (t, J = 8.0 Hz, 3H). |
| **160** | 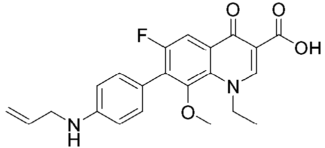 | LCMS: 397.2 [M+H]⁺ |
| | | ¹H NMR (400 MHz, DMSO-d6) δ 14.89 (s, 1H), 8.85 (s, 1H), 8.01 (d, J = 7.8 Hz, 1H), 7.60 - 7.36 (m, 2H), 6.56 - 6.43 (m, 2H), 6.14 - 5.80 (m, 2H), 5.29 - 5.10 (m, 2H), 4.40 - 4.17 (m, 2H), 4.11 - 3.80 (m, 5H), 1.42 (t, J = 8.0 Hz, 3H). |
| **161** | 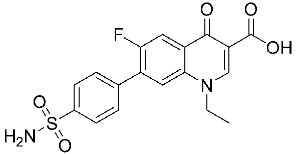 | LCMS: 391.1 [M+H]⁺ |
| | | ¹H NMR (400 MHz, DMSO-d6) δ 14.87 (s, 1H), 8.86 (s, 1H), 8.26 (d, J = 8.2 Hz, 1H), 7.85 - 7.68 (m, 4H), 7.32 (d, J = 5.1 Hz, 1H), 7.22 (s, 2H), 4.43 - 4.38 (m, 2H), 1.38 (t, J = 8.0 Hz, 3H). |
| **162** | 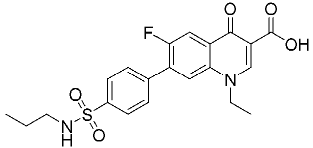 | LCMS: 433.2 [M+H]⁺ |
| | | ¹H NMR (400 MHz, DMSO-d6) δ 14.87 (s, 1H), 8.84 (s, 1H), 8.25 (d, J = 8.2 Hz, 1H), 7.86 - 7.77 (m, 2H), 7.63 - 7.58 (m, 2H), 7.34 - 7.25 (m, 2H), 4.48 - 4.38 (m, 2H), 2.99 - 2.88 (m, 2H), 1.57 - 1.35 (m, 5H), 0.94 (t, J = 8.0 Hz, 3H). |
| **163** | 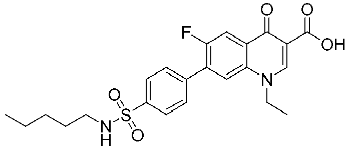 | LCMS: 461.2 [M+H]⁺ |
| | | ¹H NMR (400 MHz, DMSO-d6) δ 14.87 (s, 1H), 8.84 (s, 1H), 8.25 (d, J = 8.0 Hz, 1H), 7.90 - 7.82 (m, 2H), 7.65 - 7.57 (m, 2H), 7.35 - 7.26 (m, 2H), 4.47 - 4.37 (m, 2H), 2.82 - 2.72 (m, 2H), 1.57 - 1.23 (m, 9H), 0.96 - 0.84 (m, 3H). |
| **164** | 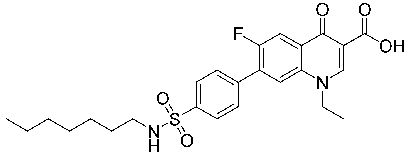 | LCMS: 489.2 [M+H]⁺ |
| | | ¹H NMR (400 MHz, DMSO-d6) δ 14.87 (s, 1H), 8.87 (s, 1H), 8.28 (d, J = 8.0 Hz, 1H), 7.97 - 7.75 (m, 2H), 7.61 - 7.56 (m, 2H), 7.43 - 7.23 (m, 2H), 4.44 - 4.38 (m, 2H), 2.73 - 2.68 (m, 2H), 1.65 - 1.24 (m, 13H), 0.96 - 0.81 (m, 3H). |
| **165** | 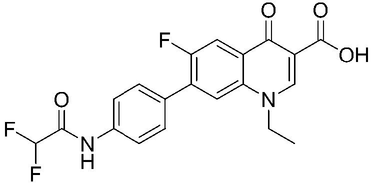 | LCMS: 405.1 [M+H]⁺ |
| | | ¹H NMR (400 MHz, DMSO-d6) δ 14.88 (s, 1H), 10.01 (s, 1H), 8.85 (s, 1H), 8.24 (d, J = 7.8 Hz, 1H), 7.74 - 7.60 (m, 2H), 7.43 - 7.17 (m, 3H), 6.45 (t, J = 57.2 Hz, 1H), 4.42 - 4.32 (m, 2H), 1.39 (t, J = 8.0 Hz, 3H). |
| **166** | 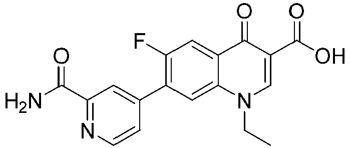 | LCMS: 356.1 [M+H]⁺ |
| | | ¹H NMR (400 MHz, DMSO-d6) δ 14.88 (s, 1H), 9.02 - 8.79 (m, 2H), 8.39 - 8.09 (m, 2H), 7.89 - 7.57 (m, 3H), 7.29 (d, J = 5.1 Hz, 1H), 4.44 - 4.36 (m, 2H), 1.38 (t, J = 8.0 Hz, 3H). |
| **167** | 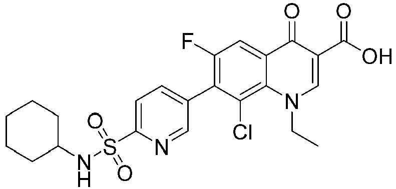 | LCMS: 508.1 [M+H]⁺ |
| | | ¹H NMR (400 MHz, DMSO-d6) δ 14.86 (s, 1H), 8.90 (s, 1H), 8.47 (t, J = 1.8 Hz, 1H), 8.17 - 8.13 (m, 2H), 7.92 - 7.85 (m, 1H), 7.17 (d, J = 12.5 Hz, 1H), 4.45 - 4.37 (m, 2H), 3.33 - 3.28 (m, 1H), 1.75 - 1.33 (m, 15H). |
| **168** | 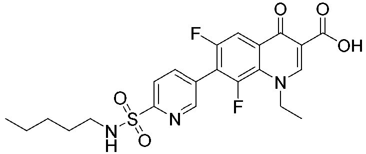 | LCMS: 480.2 [M+H]⁺ |
| | | ¹H NMR (400 MHz, DMSO-d6) δ 14.87 (s, 1H), 8.81 (s, 1H), 8.41 (t, J = 1.8 Hz, 1H), 8.17 - 8.13 (m, 2H), 7.91 - 7.88 (m, 1H), 7.47 - 7.43 (m, 1H), 4.49 - 4.33 (m, 2H), 3.02 - 2.65 (m, 2H), 1.59 - 1.23 (m, 9H), 0.95 - 0.79 (m, 3H). |
| **169** | 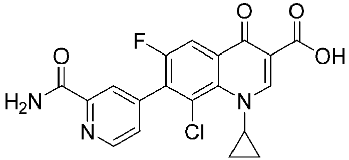 | LCMS: 402.1 [M+H]⁺ |
| | | ¹H NMR (400 MHz, DMSO-d6) δ 14.87 (s, 1H), 8.81 (s, 1H), 8.41 (t, J = 1.8 Hz, 1H), 8.17 - 8.13 (m, 2H), 7.91 - 7.86 (m, 1H), 7.46 (d, J = 10.5 Hz, 1H), 4.39 - 4.32 (m, 2H), 3.02 - 2.65 (m, 2H), 1.59 - 1.23 (m, 9H), 0.95 - 0.79 (m, 3H). |
| **170** | 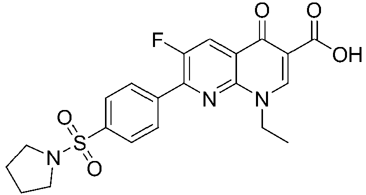 | LCMS: 446.1 [M+H]⁺ |
| | | ¹H NMR (400 MHz, DMSO-d6) δ 14.87 (s, 1H), 8.91 (s, 1H), 7.90 - 7.69 (m, 5H), 4.53 - 4.48 (m, 2H), 3.29 - 3.05 (m, 4H), 1.73 - 1.35 (m, 7H). |
| **171** | 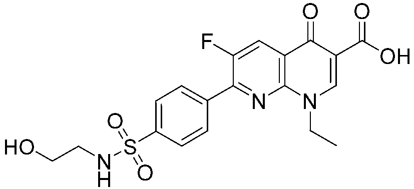 | LCMS: 436.1 [M+H]⁺ |
| | | ¹H NMR (400 MHz, DMSO-d6) δ 14.88 (s, 1H), 8.91 (s, 1H), 7.97 - 7.69 (m, 5H), 7.60 (t, J = 10.2 Hz, 1H), 4.62 - 4.39 (m, 3H), 3.64 - 3.60 (m, 2H), 3.19 - 3.15 (m, 2H), 1.45 (t, J = 8.0 Hz, 3H). |
| **172** | 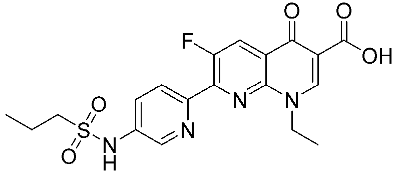 | LCMS: 435.1 [M+H]⁺ |
| | | ¹H NMR (400 MHz, DMSO-d6) δ 14.88 (s, 1H), 9.81 (s, 1H), 8.91 (s, 1H), 8.34 (d, J = 1.7 Hz, 1H), 8.04 - 7.62 (m, 3H), 4.53 - 4.43 (m, 2H), 3.04 (t, J = 7.1 Hz, 2H), 1.84 - 1.38 (m, 5H), 1.01 (t, J = 8.0 Hz, 3H). |
| **173** | 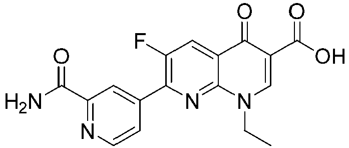 | LCMS: 357.1 [M+H]⁺ |
| | | ¹H NMR (400 MHz, DMSO-d6) δ 14.71 (s, 1H), 9.05 - 8.79 (m, 2H), 8.19 (t, J = 1.8 Hz, 1H), 8.02 - 7.66 (m, 4H), 4.53 - 4.44 (m, 2H), 1.38 (t, J = 8.0 Hz, 3H). |

### Biological Evaluation:

### 1. In vitro Target Binding Assay:

Bioluminescence Resonance Energy Transfer (BRET) is an important biophysical technique for monitoring molecular interactions, and has been widely used to study protein-protein interactions in living cells. This technique requires the relevant proteins to be linked to an energy donor (such as luciferase) and an acceptor (such as a fluorescent protein). When a relevant drug molecule interacts with the protein, the protein structure alters, causing a change in the distance between the energy donor and the energy acceptor. The luminescence energy induced by the chemical reaction is transferred to the corresponding acceptor, resulting in increased acceptor emission, and generating a BRET signal. In this work, a DNA-synthesized target human MRGPRX2 and a donor luciferase Nluc (NanoLuc^{®}Promega) (Supplier: Beijing Tsingke, Lot No.: SZ0171228-1), as well as an acceptor fluorescent protein Venus and the relevant test protein, namely human β-arrestin1 (ARRB1) (Supplier: Guangzhou Dahong Biotechnology, Lot No. HY22694-4396), were utilized, and were inserted into the eukaryotic expression vector plasmid pcDNA3.1(+) (Supplier: Qiyun Bio, Lot No.: QY2310) as fusion genes via a Linker sequence (GGSG). The cell line selected for the experiment was human embryonic kidney cells HEK-293T. The human embryonic kidney cell line HEK-293T was routinely cultured in 10 cm cell culture dishes using complete culture medium consisting of high-glucose DMEM medium supplemented with 10% (V/V) fetal bovine serum. Cells were seeded at 2×10⁶ cells/10 cm dish on the first day of culture, and passaged every 72 h with the same number of cells. One day prior to transfection, cells were resuspended, counted, and seeded at a density of 2×10⁶ cells/6 cm dish or 4×10⁶ cells/10 cm dish. Transfection was carried out 16-24 h after seeding. Plasmids constructed with the fusion protein gene were transfected into the expression cell line, and the transfected cells were cultured under appropriate conditions for a period of time to allow sufficient expression of the fusion proteins. Transfection was performed by encapsulating plasmid DNA with PEI reagent, and the encapsulation process should to be carried out in Opti-MEM medium. The ratio of plasmid, PEI (Supplier: Yeasen Biotechnology, Lot No.: WP5430010), and Opti-MEM (Supplier: Gibco, Lot No.: 2883564) was 1:4:14 (W: W: V). The encapsulated plasmid was added to an appropriate amount of complete culture medium, amd gently mixed, which was then used to replace the existing culture medium of the previously plated cells. Cells were harvested 24 h after transfection. Subsequently, cells were resuspended using HBSS (Source: Biosharp, Lot No.: 24179687) and counted with a cell counter. The cells were diluted to a density of (3~5) ×10⁵ cells/mL, and then seeded into a flat-bottom white 96-well microplate at 100 µ L/well, resulting in (3~5) ×10⁴ cells per well. In each well, 10 µ L of antagonist compounds diluted with HBSS a different concentrations were added firstly, then the agonist compound at a specific concentration (EC80) was added. After reacting for 2 min, the substrate Furimazine (Supplier: TargetMol, Lot No.: 362434) at a final concentration of 10 µM was added. After reacting for another 2 min, the 96-well plate was placed in a PHERAstar FSX microplate reader (Supplier: BMG Labtech) to simultaneously measure the readings at wavelengths of 440 nm and 530 nm. Based on the fluorescence readings at 440 nm and 530 nm, the Ratio and net BRET were calculated. Ratio = Em530 nm / Em440 nm, and net BRET = Ratio _{co-transfection} - Ratio single-transfection. The calculated net BRET values were inputted into GraphPad Prism 9, and curve fitting via nonlinear regression (four-parameter equation) and IC₅₀ calculation were performed.

R-ZINC-3573 (Supplier: MCE, Cat. No.: HY-118069) has been reported to exhibit an agonistic effect on MRGPRX2 and was used as an agonist in this experiment. The IC₅₀ values of the compounds of the present invention are shown in Table 1.

**Table 1**

| **Example** | IC₅₀ |
|---|---|
| **3** | + |
| **5** | ++ |
| **6** | ++ |
| **7** | ++ |
| **10** | +++ |
| **13** | +++ |
| **14** | +++ |
| **16** | +++ |
| **18** | +++ |
| **19** | ++ |
| **23** | +++ |
| **31** | + |
| **35** | + |
| **41** | ++ |
| **45** | ++ |
| **53** | +++ |
| **54** | +++ |
| **56** | + |
| **64** | + |
| **69** | ++ |
| **76** | +++ |
| **78** | ++ |
| **79** | ++ |
| **82** | ++ |
| **85** | + |
| **86** | + |
| **90** | ++ |
| **100** | + |
| **101** | + |
| **103** | ++ |
| **104** | ++ |
| **105** | ++ |
| **106** | ++ |
| **108** | ++ |
| **109** | + |
| **110** | ++ |
| **113** | + |
| **115** | ++ |
| **116** | ++ |
| **118** | ++ |
| **120** | ++ |
| **123** | ++ |
| **127** | ++ |
| **129** | + |
| **131** | ++ |
| **133** | ++ |
| **134** | + |
| **143** | ++ |
| **150** | + |
| **153** | ++ |
| **158** | ++ |
| **163** | ++ |
| **164** | +++ |
| **168** | ++ |

| | |
|---|---|
| Note: + indicates 80 µM >IC₅₀>10 µM , ++ indicates 10 µM >IC₅₀>1 µM, +++ indicates 1 µM >IC₅₀>0.1 µM. | |

### 2, In vivo Efficacy Experiments:

### 2.1 Rosacea

Experimental animals: BALB/c mice, from Guangdong Vital River Laboratory Animal Technology Co., Ltd.

### Model establishment:

The hair on the back of the mice was shaved, and the modeling area was marked on the back skin, avoiding positions with abundant subcutaneous blood vessels. LL-37 (an antimicrobial peptide used for rosacea modeling) (Supplier: QYAOBlO (ChinaPeptides), Lot No.: 20240319) solution was drawn with a microsyringe and injected intradermally along the intradermal path. The needle was withdrawn slowly after injection. The modeling continued for 5 to 7 days, with injections administered twice a day.

### Animal Grouping and Administration:

Administration was initiated on the day of grouping, which was designated as D1, and was performed once daily for 7 consecutive days. The normal control group and the model control group were administered with vehicle alone under the same conditions. The changes in skin lesions at the injection site on the back of mice in each group were observed, and scores ranging from 0 to 4 were assigned based on the degree of erythema, wherein, a score of 0 indicates no erythema, a score of 1 indicates barely visible erythema, a score of 2 indicates pale erythema with blurred boundaries, a score of 3 indicates erythema with clear boundaries, and a score of 4 indicates dark erythema with clear boundaries. The administration regimen for the mouse rosacea model was as follows:

| Group | **Drug** | No. of Animals | Dose | Administration Route | Dosing Period |
|---|---|---|---|---|---|
| G1 | Normal control group | 6 | - | Topical application | 7 days |
| G2 | Model control group | 6 | - | Topical application | 7 days |
| G3 | Example 10 | 6 | 40mg/kg | Oral | 7 days |
| G4 | Example 10 | 6 | 4mg/g | Topical application | 7 days |
| G5 | Example 41 | 6 | 40mg/kg | Oral | 7 days |
| G6 | Example 41 | 6 | 4mg/g | Topical application | 7 days |
| G7 | Example 123 | 6 | 40mg/kg | Oral | 7 days |
| G8 | Example 123 | 6 | 4mg/g | Topical application | 7 days |

Experimental results showed that the model group (administrated with vehicle) developed typical rosacea-like skin lesions with marked erythema, whereas the blank group (administrated with vehicle) showed no abnormal skin symptoms. The Example groups showed significant improvement in erythema symptoms during the experimental period, with statistical significance. The erythema scores for each group are shown in Table 2, and the data are presented as mean ± standard error of the mean (SEM).

**Table 2**

| Group | D1 | D2 | D3 | D4 | D5 | D6 | D7 |
|---|---|---|---|---|---|---|---|
| G1 | 0 | 0 | 0 | 0 | 0 | 0 | 0 |
| G2 | 1.6±0.2 | 3.1±0.3 | 3.3±0.2 | 3.0±0.2 | 2.4±0.2 | 2.1±0.3 | 1.7±0.3 |
| G3 | 1.6±0.2 | 2.1±0.3* | 2.5±0.2* | 1.8±0.2** | 1.9±0.2* | 1.6±0.2 | 1.5±0.3 |
| G4 | 1.6±0.3 | 2.7±0.2 | 2.7±0.2* | 2.4±0.2* | 1.7±0.2* | 1.6±0.3 | 1.5±0.3 |
| G5 | 1.6±0.2 | 2.2±0.2* | 2.2±0.1* | 1.740.2%** | 1.7±0.2* | 1.3±0.3* | 1.2±0.2 |
| G6 | 1.6±0.3 | 2.6±0.2 | 2.6±0.2* | 2.3±0.3* | 1.8±0.2* | 1.5±0.3 | 1.2±0.3 |
| G7 | 1.7±0.2 | 2.2±0.2* | 2.5±0.2* | 1.8±0.2** | 1.7±0.2* | 1.3±0.2* | 1.2±0.2 |
| G8 | 1.6±0.3 | 2.3±0.3* | 2.6±0.3* | 2.2±0.3* | 1.7±0.2* | 1.3±0.2* | 1.3±0.2 |

| | | | | | | | |
|---|---|---|---|---|---|---|---|
| * indicates P<0.05, compared with model control group; ** indicates P<0.01, compared with model control group. | | | | | | | |

### 2.2 Psoriasis

Experimental animals: BALB/c mice, from Guangdong Vital River Laboratory Animal Technology Co., Ltd.

The clinical severity of psoriasis was assessed using the Psoriasis Area and Severity Index (PASI) score. The PASI scoring criteria for mice are as follows:

| **Score** | 0 | 1 | 2 | 3 | 4 |
|---|---|---|---|---|---|
| Erythema | No visible erythema | Light red | Red | Dark red | Very dark red |
| Scaling | No visible scaling | Partial lesions covered with scales, predominantly characterized by fine scales | Most lesions are completely or partially covered with scales, with scaling appearing in flakes | Almost all lesions covered with relatively thick scales | All lesions are covered with thick scales, with scaling accumulated in layers |
| Thickening | Flush with normal skin | Lesions slightly elevated relative to normal skin surface | Lesions are moderately elevated, with plaque edges being rounded or sloped | Lesions are thickened and significantly elevated | Lesions are severely thickened and prominently elevated |

### Model establishment:

The hair on the back of the mice was shaved, and the application area was marked with a marker pen. Imiquimod cream (Supplier: Sichuan Mingxin Pharmaceutical, Lot No.: 24054002) was applied to the back of the mice for several consecutive days to establish a mouse model of psoriasis. Before daily application, the residual cream was removed using pure water.

Animal Grouping and Administration:

Administration was initiated on the day of grouping, which was designated as D1, and was performed once daily for 8 consecutive days. The normal control group and the model control group were administered with vehicle alone under the same conditions. Mice were scored according to the PASI (Psoriasis area and severity index). The administration regimen for the mouse psoriasis model was as follows:

| Group | **Drug** | No. of Animals | Dose | Administration Route | Dosing Period |
|---|---|---|---|---|---|
| G1 | Normal control group | 6 | - | Topical application | 8 days |
| G2 | Model control group | 6 | - | Topical application | 8 days |
| G3 | Example 10 | 6 | 4mg/g | Topical application | 8 days |
| G4 | Example 18 | 6 | 4mg/g | Topical application | 8 days |
| G5 | Example 69 | 6 | 4mg/g | Topical application | 8 days |
| G6 | Example 90 | 6 | 4mg/g | Topical application | 8 days |
| G7 | Example 106 | 6 | 4mg/g | Topical application | 8 days |
| G8 | Example 133 | 6 | 4mg/g | Topical application | 8 days |

Experimental results showed that the model group developed typical psoriasis-like skin lesions, including erythema, scaling, and skin thickening, whereas no abnormal symptoms were observed in the skin of the blank group. The Example groups showed significant improvement in psoriasis-like skin lesions, at the end of the experiment with statistical significance. The PASI scores of each group are shown in Table 3, and the data are presented as mean ± standard error of the mean (SEM).

**Table 3**

| Group | D1 | D2 | D3 | D4 | D5 | D6 | D7 | D8 |
|---|---|---|---|---|---|---|---|---|
| G1 | 0 | 0 | 0 | 0 | 0 | 0 | 0 | 0 |
| G2 | 0 | 0.6±0.3 | 1.9±0.3 | 3.4±0.4 | 4.3±0.4 | 5.5±0.4 | 6.8±0.5 | 9.1±0.5 |
| G3 | 0 | 0.5±0.3 | 1.7±0.3 | 2.8±0.3 | 3.6±0.4 | 4.3±0.4** | 5.3±0.4** | 6.6±0.4* |
| G4 | 0 | 0.5±0.3 | 1.6±0.3 | 3.0±0.4 | 3.7±0.3 | 4.6±0.4* | 5.5±0.4* | 6.7±0.4* |
| G5 | 0 | 0.6±0.3 | 1.7±0.3 | 3.1±0.3 | 3.8±0.3 | 4.6±0.4* | 5.5±0.4* | 6.6±0.4* |
| G6 | 0 | 0.6±0.3 | 1.9±0.3 | 3.1±0.3 | 3.9±0.4 | 4.7±0.3* | 5.7±0.3* | 6.6±0.5 * |
| G7 | 0 | 0.5±0.3 | 1.8±0.3 | 2.6±0.3* | 3.3±0.3* | 4.4±0.4** | 5.1±0.3** | 6.4±0.5** |
| G8 | 0 | 0.6±0.3 | 1.6±0.3 | 2.7±0.4 | 3.4±0.3* | 4.6±0.4* | 5.6±0.4* | 6.7±0.5 * |

| | | | | | | | | |
|---|---|---|---|---|---|---|---|---|
| * indicates P<0.05, compared with model control group; ** indicates P<0.01, compared with model control group. | | | | | | | | |

### 2.3 Urticaria

Experimental animals: BALB/c mice, from Hangzhou Ziyuan Laboratory Animal Technology Co., Ltd

### Model establishment:

After random grouping, the back of the mice was shaved in advance, with a shaving area of about 2 cm × 2 cm. Except for the normal control group, mice in the remaining groups were sensitized via tail vein injection of an anti-dinitrophenyl monoclonal antibody (Supplier: Sigma-Aldrich, Lot No.: 0000241402) (0.2 ng/mL) at a dose of 0.5 mL per mouse. After 24 h, the mice in each sensitization modeling group were challenged by topically applying 50 µL of 0.15% dinitrofluorobenzene (Supplier: Shanghai Macklin, Lot No.: C14969320) to the dorsal side of both ears. The normal control group was injected and topically applied with normal saline under the same conditions.

The administration regimen for the mouse urticaria model was as follows (the normal control group and the model control group were administered with vehicle alone under the same conditions) (once daily):

| Group | **Drug** | No. of Animals | Dose | Administration Route | Dosing Period |
|---|---|---|---|---|---|
| G1 | Normal control group | 6 | - | - | 6 days |
| G2 | Model control group | 6 | - | - | 6 days |
| G3 | Example 10 | 6 | 4mg/kg | Topical application | 6 days |
| G4 | Example 10 | 6 | 25mg/kg | Oral | 6 days |
| G5 | Example 54 | 6 | 4mg/kg | Topical application | 6 days |
| G6 | Example 54 | 6 | 25mg/kg | Oral | 6 days |
| G7 | Example 69 | 6 | 4mg/kg | Topical application | 6 days |
| G8 | Example 69 | 6 | 25mg/kg | Oral | 6 days |
| G9 | Example 164 | 6 | 4mg/kg | Topical application | 6 days |
| G10 | Example 164 | 6 | 25mg/kg | Oral | 6 days |

Experimental results showed that the model group developed typical urticaria wheal symptoms, whereas no abnormal symptoms were observed in the skin of the blank group. The Example groups showed significant improvement in the number of wheals at the end of the experiment with statistical significance. In addition, the test results after the experiment of serum IgE and histamine showed significant improvement with statistical significance. The number of wheals, serum IgE level, and histamine level in each group are shown in Table 4, and the data are presented as mean ± standard error of the mean (SEM).

Mouse IgE ELISA detection kit: purchased from Shanghai Enzyme-linked Biotechnology Co., Ltd., Lot No.: ml1037602.

Mouse histamine ELISA detection kit: purchased from Shanghai Enzyme-linked Biotechnology Co., Ltd., Lot No.: ml1001877.

Microplate reader, manufacturer BioTek, model: Epoch.

**Table 4**

| Group | **Number of Wheals** | **IgE Level** (ug/mL) | **Histamine Level** (ng/mL) |
|---|---|---|---|
| G1 | 1.2±1.7 | 6.4±0.9 | 4.2±0.9 |
| G2 | 15.8±1.5 | 23.0±2.5 | 9.7±1.0 |
| G3 | 10.6±2.0* | 12.2±2.0** | 6.3±1.0** |
| G4 | 8.4±1.0** | 11.5±2.0** | 5.8±1.0** |
| G5 | 10.6±1.5* | 14.8±1.0 | 6.4±1.0 |
| G6 | 6.5±1.5** | 17.3±1.5* | 6.9±1.0** |
| G7 | 11.4±2.0* | 15.2±2.0** | 7.4±1.0* |
| G8 | 10.0±2.0* | 11.5±2.0** | 5.8±1.0** |
| G9 | 9.7±2.0* | 14.9±2.0** | 6.5±1.0** |
| G10 | 8.8±2.0** | 10.5±2.0 | 5.6±1.0** |

| | | | |
|---|---|---|---|
| * indicates P<0.05, compared with model control group; ** indicates P<0.01, compared with model control group. | | | |

### 2.4 Atopic Dermatitis

Experimental animals: BALB/c mice, from Guangdong Vital River Laboratory Animal Technology Co., Ltd.

### Model establishment:

MC903 (calcipotriol) (Supplier: TargetMol, Lot No.: 235760) was applied to the right ear of mice, once a day, for 14 consecutive days.

### Animal Grouping and Administration:

The animals were randomly divided into 6 groups before induction on D1. The model control group was administered with vehicle alone under the same conditions. Administration was carried out according to the following groups:

| Group | Description | Dose (mg/kg) | Administration Frequency (times per day) | Route | No. of Animals |
|---|---|---|---|---|---|
| G1 | Model control group | - | - | - | 5 |
| G2 | Example 10 | 50 | BID | p.o. | 5 |
| G3 | Example 41 | 50 | BID | p.o. | 5 |
| G4 | Example 54 | 50 | BID | p.o. | 5 |
| G5 | Example 85 | 50 | BID | p.o. | 5 |
| G6 | Example 116 | 50 | BID | p.o. | 5 |

| | | | | | |
|---|---|---|---|---|---|
| Note: BID, twice a day; p.o., *oral administration.* | | | | | |

During the experiment period, the thickness of the right ear of the mice was measured. The results showed that compared with the model group, the thickness of the right ear of the mice in all Example groups decreased, with statistical significance. The data are presented as mean ± standard error of the mean (SEM).

| Group | Right Ear Thickness of Mice (mm) | | | | | | |
|---|---|---|---|---|---|---|---|
| | D2 | D4 | D6 | D8 | D10 | D12 | D14 |
| G1 | 0.22±0.03 | 0.32±0.04 | 0.44±0.06 | 0.55±0.06 | 0.65±0.04 | 0.62±0.03 | 0.67±0.04 |
| G2 | 0.19±0.03 | 0.28+0.04 | 0.40+0.04 | 0.48+0.04* | 0.52+0.04* | 0.60+0.04 | 0.57+0.02** |
| G3 | 0.19±0.04 | 0.28+0.02 | 0.36+0.04* | 0.48+0.04* | 0.56+0.04* | 0.52+0.04** | 0.58+0.04** |
| G4 | 0.19±0.04 | 0.28+0.04 | 0.35+0.04* | 0.48+0.04* | 0.52+0.04* | 0.54+0.04** | 0.59+0.03** |
| G5 | 0.19±0.05 | 0.27+0.04 | 0.34+0.02* | 0.44+0.04* | 0.50+0.04* | 0.52+0.04** | 0.54+0.04** |
| G6 | 0.19±0.04 | 0.28+0.02 | 0.42+0.04 | 0.50+0.04 | 0.52+0.04* | 0.60+0.04 | 0.58+0.02** |

| | | | | | | | |
|---|---|---|---|---|---|---|---|
| * indicates P<0.05, compared with model control group; ** indicates P<0.01, compared with model control group. | | | | | | | |

### 2.5 Inflammatory Bowel Disease (IBD)

Experimental animals: C57BL/6 mice, from Guangdong Vital River Laboratory Animal Technology Co., Ltd.

### Model Establishment and Grouping:

C57BL/6 mice were used. 3% DSS (Dextran Sulfate Sodium) (Supplier: MPBIO, Lot No.: YD11024) in water was prepared as drinking water for the mice.

| Group | Description | Dose | Treatment | Route | No. of Animals |
|---|---|---|---|---|---|
| G1 | Normal control group | - | Drinking water without DSS | - | 6 |
| G2 | Model control group | - | 3% DSS drinking water, 7 consecutive days | - | 6 |
| G3 | Example 7 | 20mg/kg | 3% DSS drinking water+QD, 7 consecutive days | p.o. | 6 |
| G4 | Example 13 | 20mg/kg | 3% DSS drinking water+QD, 7 consecutive days | p.o. | 6 |
| G5 | Example 19 | 20mg/kg | 3% DSS drinking water+QD, 7 consecutive days | p.o. | 6 |
| G6 | Example 54 | 20mg/kg | 3% DSS drinking water+QD, 7 consecutive days | p.o. | 6 |
| G7 | Example 105 | 20mg/kg | 3% DSS drinking water+QD, 7 consecutive days | p.o. | 6 |
| G8 | Example 164 | 20mg/kg | 3% DSS drinking water+QD, 7 consecutive days | p.o. | 6 |

| | | | | | |
|---|---|---|---|---|---|
| Note: QD, once a day; p.o., oral administration; vehicle is the administration vehicle for formulating the Examples. | | | | | |

During the experiment, the animals were weighed at a fixed time daily, and their status (including feces, mortality) was observed. The efficacy was evaluated by the DAI score. The details of the DAI scoring criteria are as follows: 0 point for normal stool, 1 point for soft but formed stool, 2 points for soft stool, 3 points for soft and wet stool, 4 points for loose stool; 0-1 point for no occult blood, 2 points for occult blood positive, 3 points for visible blood in stool, 4 points for severe fecal blood; 0 point for no weight loss or weight gain, 1 point for weight loss of 1%-5%, 2 points for weight loss of 6%-10%, 3 points for weight loss of 11%-15%, 4 points for weight loss > 15%. The sum of the scores for 3 items is the total DAI score, and the weighing and scoring were carried out every other day. The experimental results showed that the administration groups exhibited significant efficacy with statistical significance. The DAI scores are shown in Table 5, and the data are presented as mean + standard error of the mean (SEM):

**Table 5**

| Group | D1 | D2 | D3 | D4 | D5 | D6 | D7 |
|---|---|---|---|---|---|---|---|
| G1 | 0.00±0.00 | 0.2±0.5 | 0.5±0.5 | 0.0±0.0 | 0.0±0.0 | 0.0±0.0 | 0.1±0.4 |
| G2 | 0.00±0.00 | 1.5±1.0 | 2.4±1.1 | 3.4±1.0 | 4.9±1.1 | 7.5±1.2 | 9.4±1.3 |
| G3 | 0.00±0.00 | 0.9±1.0 | 2.0±1.2 | 2.8±1.8 | 3.3±1.2 | 5.2±1.6 | 6.0±1.6** |
| G4 | 0.00±0.00 | 1.1±1.0 | 1.9±1.3 | 2.9±1.1 | 3.5±1.3 | 4.7±1.0* | 5.9±1.3** |
| G5 | 0.00±0.00 | 1.0±1.0 | 1.6±1.2 | 2.8±1.4 | 3.3±1.2 | 5.0±1.6 | 6.0±1.6** |
| G6 | 0.00±0.00 | 0.8±1.0 | 0.9±0.6 | 2.4±0.8 | 2.3±0.8* | 3.3±0.8** | 3.8±0.9*** |
| G7 | 0.00±0.00 | 1.0±1.0 | 1.9±1.2 | 2.8±1.6 | 3.3±1.1 | 4.8±1.6 | 5.0±1.4*** |
| G8 | 0.00±0.00 | 1.2±1.0 | 2.0±1.2 | 3.1±1.0 | 3.7±1.0 | 4.7±1.4 | 6.2±1.4* |

| | | | | | | | |
|---|---|---|---|---|---|---|---|
| * indicates P<0.05, compared with model control group; ** indicates P<0.01, compared with model control group; *** indicates P<0.001, compared with model control group. | | | | | | | |

### 2.6 Allergic Rhinitis

Experimental animals: BALB/c mice, from Guangdong Vital River Laboratory Animal Technology Co., Ltd.

### Model establishment

Modeling was initiated on the day of grouping, which was designated as M1. Except for the normal group, mice in all modeling groups were intraperitoneally injected with a sensitization solution of (250 µg OVA (Ovalbumin) (Supplier: Shanghai Macklin, Lot No.: C16255388) mixed with 5 mg aluminum hydroxide/mL) per mouse, while the normal control group was given an equal volume of sterile PBS solution. The injection cycle was M1, M7, and M14. Starting from M21, challenge was carried out by nasal instillation once daily for 7 consecutive days. The challenge solution was a 50 mg/mL OVA solution, and a volume of 10 µL was instilled into each nostril per time. The normal control group was not subjected to nasal instillation.

### Animal Grouping and Administration

Administration was initiated on the day of the challenge by nasal instillation, which was designated as D1, and was performed once daily for 7 consecutive days. The normal control group and the model control group were administered with vehicle alone under the same conditions:

| Group | **Drug** | No. of Animals | Dose | Administration Route | Dosing Period |
|---|---|---|---|---|---|
| G1 | Normal control group | 6 | - | p.o. | 7 days |
| G2 | Model control group | 6 | - | p.o. | 7 days |
| G3 | Example 7 | 6 | 5mg/kg | p.o. | 7 days |
| G4 | Example 10 | 6 | 5mg/kg | p.o. | 7 days |
| G5 | Example 23 | 6 | 5mg/kg | p.o. | 7 days |
| G6 | Example 69 | 6 | 5mg/kg | p.o. | 7 days |
| G7 | Example 79 | 6 | 5mg/kg | p.o. | 7 days |
| G8 | Example 116 | 6 | 5mg/kg | p.o. | 7 days |

### Evaluation Indicators

### Rhinitis Symptom Score

The number of sneezes in each group was recorded within 10 min after the nasal challenge every day during the administration period.

During the experiment, the allergic rhinitis model mice developed typical symptoms, including shortness of breath, ear and nose scratching, and sneezing, etc. Compared with the model group, the number of sneezes was significantly reduced in the Example groups. The counting results of each group are shown in Table 6, and the unit expressed as (number of sneezes/mouse). The data are presented as mean ± standard error of the mean (SEM).

**Table 6**

| Group | D1 | D2 | D3 | D4 | D5 | D6 | D7 |
|---|---|---|---|---|---|---|---|
| G1 | 0 | 0 | 0 | 0 | 0 | 0 | 0 |
| G2 | 1.7 | 4.0 | 13.0 | 14.7 | 22.2 | 27.7 | 20.0 |
| G3 | 2.5 | 3.3 | 10.2 | 14.5 | 11.3 | 15.8 | 13.2 |
| G4 | 2.0 | 1.4 | 14.6 | 7.8 | 9.1 | 13.0 | 10.4 |
| G5 | 1.5 | 4.7 | 15.2 | 13.5 | 9.8 | 14.0 | 12.0 |
| G6 | 2.8 | 3.1 | 10.2 | 14.9 | 10.7 | 16.1 | 13.4 |
| G7 | 2.4 | 1.3 | 14.5 | 7.6 | 7.2 | 13.4 | 14.8 |
| G8 | 1.5 | 3.7 | 15.2 | 12.5 | 10.8 | 13.0 | 15.2 |

| | | | | | | | |
|---|---|---|---|---|---|---|---|
| Note: *The unit is number of sneezes*/*mouse.* | | | | | | | |

### IgE Assay

Prior to necropsy, blood was collected from the eyeball. The collected mouse blood was then centrifuged at 1800 r/min at 4 °C for 15 min, and the supernatant serum was aliquoted and stored at -80 °C for testing. At the same time, nasal lavage fluid and tracheal tissues were collected. The IgE levels in the above three samples was detected using an ELISA method (Supplier: Shanghai Enzyme-linked Biotechnology Co., Ltd., Lot No.: ml1037602).

IgE Assay (the manufacturer of Microplate reader: BioTek, model: Epoch): compared with the model group, the IgE levels in peripheral blood, nasal lavage fluid, and tracheal tissue were significantly reduced in the Example groups. The results for the peripheral blood, lavage fluid, and tracheal tissue of each group are shown in Table 7.

**Table 7**

| Group | Peripheral Blood IgE Concentration (ng/mL) | Nasal Lavage Fluid IgE Concentration (ng/mL) | Tracheal Tissue IgE Concentration (ng/mL) |
|---|---|---|---|
| G1 | 4.1±1.7 | 4.5±0.7 | 11.6±3.4 |
| G2 | 30.9 ±9.5 | 17.1±2.9 | 24.5±5.8 |
| G3 | 9.6±2.2* | 9.6±3.8 * | 10.9±4.9* |
| G4 | 6.8±1.5** | 11.0±1.5* | 11.9±2.9* |
| G5 | 9.6±2.2* | 10.3±1.5* | 10.0±5.3* |
| G6 | 9.8±1.9* | 10.2±2.3* | 10.3±4.4* |
| G7 | 9.7+2.9* | 10.6+1.8* | 9.6+4.2* |
| G8 | 9.1+2.9* | 10.4+2.2* | 10.5+3.4* |

| | | | |
|---|---|---|---|
| * indicates P<0.05, compared with model control group; ** indicates P<0.01, compared with model control group. | | | |

### 2.7 Asthma

Experimental animals: BALB/c mice, from Guangdong Vital River Laboratory Animal Technology Co., Ltd.

### Model establishment

Modeling was initiaed on the day of grouping, which was designated as M1. Except for the normal group, mice in all modeling groups were intraperitoneally injected with a sensitization solution of (500 µg OVA (Ovalbumin) (Supplier: Shanghai Macklin, Lot No.: C16255388) mixed with 5 mg aluminum hydroxide/mL) per mouse, while the normal control group was given an equal volume of sterile PBS solution. The injection cycle was M1, M7, and M14. Starting from M21, challenge was carried out by aerosolization once daily for 7 consecutive days. The challenge solution was a 20 mg/mL OVA (Ovalbumin) solution, and each aerosolization challenge lasted for 30 min. The normal control group was not subjected to aerosolization.

### Animal Grouping and Administration

Administration was initiated on day M21, which was designated as D1, and was performed once daily for 7 consecutive days. The normal control group and the model control group were administered with vehicle alone under the same conditions. The administration regimen for the mouse asthma model was as follows:

| Group | **Drug** | No. of Animals | Dose | Administration Route | Dosing Period |
|---|---|---|---|---|---|
| G1 | Normal control group | 6 | - | PO | 7 days |
| G2 | Model control group | 6 | - | PO | 7 days |
| G3 | Example 5 | 6 | 5mg/kg | PO | 7 days |
| G4 | Example 37 | 6 | 5mg/kg | PO | 7 days |
| G5 | Example 117 | 6 | 5mg/kg | PO | 7 days |
| G6 | Example 144 | 6 | 5mg/kg | PO | 7 days |

### Evaluation Indicators

### Bronchoalveolar Lavage Fluid (BALF) Cell Differential count

The lungs were gently lavaged twice with normal saline to collect BALF. The recovered BALF was stored at 4 °C, and the cell differential count was detected using an automated hematology analyzer (Manufacturer: SYSMEX, Model: XN-1000). During the experiment, the asthma model mice developed typical symptoms, including shortness of breath, and ear and nose scratching, etc. Compared with the model group, the number of inflammatory cells in the alveolar lavage fluid was significantly reduced in the Example groups. The results of the bronchoalveolar lavage fluid cell differential count in each group are shown in Table 8, and the data are presented as mean ± standard error of the mean (SEM).

**Table 8**

| Group | **Leukocyte Count** WBC (10^9/L) | **Neutrophil Count** (10^9/L) | **Lymphocyte Count** (10^9/L) | **Monocyte Count** (10^9/L) | **Eosinophil Count** (10^9/L) |
|---|---|---|---|---|---|
| G1 | 0.03 ±0.10 | 0 | 0 | 0 | 0 |
| G2 | 4.7±0.4 | 0.3 ±0.04 | 0.4±0.1 | 0.2±0.06 | 3.8±0.4 |
| G3 | 2.6 ±0.7** | 0.2±0.05* | 0.2±0.06* | 0.1±0.04* | 2.0 ±0.7** |
| G4 | 2.5 ±0.5** | 0.2±0.04** | 0.2±0.06* | 0.1±0.02* | 2.0±0.5** |
| G5 | 3.0 ±0.8* | 0.2 ±0.05* | 0.2±0.04* | 0.1±0.04* | 2.4±0.6* |
| G6 | 1.9±0.5*** | 0.1±0.08** | 0.1±0.06** | 0.08±0.02** | 1.6±0.4*** |

| | | | | | |
|---|---|---|---|---|---|
| * indicates P<0.05, compared with model control group; ** indicates P<0.01, compared with model control group; *** indicates P<0.001, compared with model control group. | | | | | |

### Serum Assay IgE Assay

Prior to necropsy, blood was collected from the eyeball. The collected mouse blood was then centrifuged at 1800 r/min at 4 °C for 15 min, and the supernatant serum was aliquoted and stored at -80 °C for testing. At the same time, alveolar lavage fluid and lung tissues were collected. The IgE levels in the above three samples was detected using an ELISA method (Supplier: Shanghai Enzyme-linked Biotechnology Co., Ltd., Lot No.: ml1037602). Compared with the model group, the IgE levels in peripheral blood, alveolar lavage fluid, and lung tissue were significantly reduced in the Example groups. The results of the IgE levels in peripheral blood, lavage fluid, and lung tissue of each group are shown in Table 9, and the data are presented as mean ± standard error of the mean (SEM).

**Table 9**

| Group | Peripheral Blood IgE Concentration (ng/mL) | Alveolar Lavage Fluid IgE Concentration (ng/mL) | Lung Tissue IgE Concentration (ng/mL) |
|---|---|---|---|
| G1 | 10.3±3.0 | 1.9±0.4 | 7.1±1.9 |
| G2 | 22.6±2.0 | 33.5±1.7 | 58.3±3.0 |
| G3 | 15.6±5.8* | 12.8±0.9** | 39.8±2.4* |
| G4 | 14.7±4.3* | 12.1±1.8** | 37.2±3.5** |
| G5 | 12.1±3.2** | 14.8±3.5** | 48.9±4.6* |
| G6 | 11.9±2.3** | 6.3±1.7*** | 32.8±4.1*** |

| | | | |
|---|---|---|---|
| Note: * P<0.05, indicates compared with model control group, ** indicates P<0.01, compared with model control group, *** indicates P<0.001, compared with model control group. | | | |

### Microplate reader, manufacturer: BioTek, model: Epoch.

The examples provided above are not intended to limit the scope covered by the present invention, and the described steps are not intended to limit their execution order. Based on the present disclosure, those skilled in the art can make obvious improvements to the present invention in combination with the prior art, which also fall within the protection scope defined by the claims of the present invention.

## Claims

1. A compound of formula (IA), or a pharmaceutically acceptable salt, stereoisomer, tautomer, hydrate, or solvate thereof, for use in the treatment or prevention of a disease or disorder mediated by MRGPRX2, 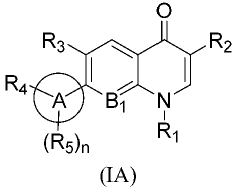 wherein:
R₁ is -C₃₋₆ cycloalkyl or -C₁₋₆ linear or branched alkyl;
R₂ is -COOH, -COOC₁₋₆ linear or branched alkyl, -C(O)NH₂, -C(O)-NH-C₁₋₆ linear or branched alkyl, -C(O)-N (C₁₋₆ linear or branched alkyl)₂ or -C(O)-NH-OH;
R₃ is halogen;
B₁ is -CR₆ or N, wherein R₆ is H, halogen or -O-C₁₋₆ linear or branched alkyl;
A is C₆₋₁₀ aryl or 5-6-membered heteroaryl;
R₄ is selected from the group consisting of -S(O)₂-NH₂, -S(O)₂-NHRₐ, -S(O)₂-NR_{b}R_{c}, -S(O)-NH₂, -S(O)-NH-Rₐ, -S(O)-NR_{b}R_{c}, -S-NH₂, -S-NH-Rₐ, -S-NR_{b}R_{c}, -S(O)₂-Rₐ, -S(O)-Rₐ, -NH-S(O)₂-Rₐ, -C(O)-NH₂, -C(O)-NH-Rₐ, -C(O)-NR_{b}R_{c}, -NH-C(O)-Rₐ, -C(O)-Rₐ, -NHR_{c}, -S-R_{d} and NO₂;
Rₐ and R_{d} are each independently selected from the group consisting of C₁₋₂₀ linear or branched alkyl, C₂₋₂₀ linear or branched alkenyl, C₂₋₂₀ linear or branched alkynyl, C₃₋₁₀-membered cycloalkyl and 4-10-membered heterocycloalkyl, wherein the heterocycloalkyl contains one or more heteroatoms selected from the group consisting of N, O, and S, and the alkyl, alkenyl, alkynyl, cycloalkyl or heterocycloalkyl is optionally substituted with 1, 2, or 3 substituents selected from the group consisting of: halogen, -OH, -NH₂, -CN, -NO₂, -SC₁₋₆ linear or branched alkyl, -OC₁₋₆ linear or branched alkyl, -NHC₁₋₆ linear or branched alkyl and -N (C₁₋₆ linear or branched alkyl)₂;
R_{b} and R_{c}, together with the N atom to which they are attached, form a 4-10-membered heterocycloalkyl group, wherein the heterocycloalkyl contains one or more heteroatoms selected from the group consisting of N, O, and S, and the heterocycloalkyl is optionally substituted with 1, 2, or 3 substituents selected from the group consisting of: halogen, -OH, -NH₂, -CN, -NO₂, -SC₁₋₆ linear or branched alkyl, -OC₁₋₆ linear or branched alkyl, -NHC₁₋₆ linear or branched alkyl and -N (C₁₋₆ linear or branched alkyl)₂;
R₅ is selected from the group consisting of halogen, -CN, -NH₂, -OH, -NH-C₁₋₆ linear or branched alkyl, -N (C₁₋₆ linear or branched alkyl)₂, C₁₋₆ linear or branched alkyl, C₁₋₆ linear or branched alkoxy, -C(O)-NH₂, -C(O)-NH-C₁₋₆ linear or branched alkyl, -C(O)-N (C₁₋₆ linear or branched alkyl)₂ and -NH-C(O)-C₁₋₆ linear or branched haloalkyl; and
n is 0, 1, 2 or 3.

2. The compound, or a pharmaceutically acceptable salt, stereoisomer, tautomer, hydrate or solvate thereof for use according to any one of the preceding claims, wherein R₁ is 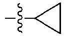 or

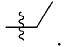

3. The compound, or a pharmaceutically acceptable salt, stereoisomer, tautomer, hydrate or solvate thereof for use according to any one of the preceding claims, wherein R₂ is -COOH, -COOC₁₋₃ linear or branched alkyl or -C(O)NH₂.

4. The compound, or a pharmaceutically acceptable salt, stereoisomer, tautomer, hydrate or solvate thereof for use according to any one of the preceding claims, wherein R₃ is F.

5. The compound, or a pharmaceutically acceptable salt, stereoisomer, tautomer, hydrate or solvate thereof for use according to any one of the preceding claims, wherein B₁ is -CR₆ or N, wherein R₆ is H, halogen or -O-C₁₋₃ linear or branched alkyl.

6. The compound, or a pharmaceutically acceptable salt, stereoisomer, tautomer, hydrate or solvate thereof for use according to any one of the preceding claims, wherein, A is phenyl or pyridyl.

7. The compound, or a pharmaceutically acceptable salt, stereoisomer, tautomer, hydrate or solvate thereof for use according to any one of the preceding claims, wherein R₄ is selected from the group consisting of -S(O)₂-NH₂, -S(O)₂-NHRₐ, -S(O)₂-NR_{b}R_{c}, -S(O)-NH-Rₐ, -S-NH-Rₐ, -S(O)-Rₐ, -NH-S(O)₂-Rₐ, -C(O)-NH₂, -C(O)-NH-Rₐ, -NH-C(O)-Rₐ, -NHR_{d} and -S-R_{d}.

8. The compound, or a pharmaceutically acceptable salt, stereoisomer, tautomer, hydrate or solvate thereof for use according to any one of the preceding claims, wherein R₄ is selected from the group consisting of -S(O)₂-NH₂, -S(O)₂-NHRₐ and -S(O)₂-NR_{b}R_{c}.

9. The compound, or a pharmaceutically acceptable salt, stereoisomer, tautomer, hydrate or solvate thereof for use according to any one of the preceding claims, wherein Rₐ is selected from the group consisting of C₁₋₁₅ linear or branched alkyl, C₃₋₆-membered cycloalkyl and 4-6-membered heterocycloalkyl, wherein the heterocycloalkyl contains 1, 2 or 3 heteroatoms selected from the group consisting of N, O, and S, and the alkyl is optionally substituted with 1, 2, or 3 substituents selected from the group consisting of: halogen, -OH, -NH₂ and -CN; for example, Rₐ is C₁₋₁₅ linear alkyl, and the alkyl is optionally substituted with 1, 2, or 3 halogen.

10. The compound, or a pharmaceutically acceptable salt, stereoisomer, tautomer, hydrate or solvate thereof for use according to any one of the preceding claims, wherein R_{d} is selected from the group consisting of C₁₋₆ linear or branched alkyl, C₂₋₆ linear or branched alkenyl and C₂₋₆ linear or branched alkynyl, wherein the alkyl, alkenyl or alkynyl is optionally substituted with 1, 2, or 3 substituents selected from the group consisting of: halogen, -OH and -CN.

11. The compound, or a pharmaceutically acceptable salt, stereoisomer, tautomer, hydrate or solvate thereof for use according to any one of the preceding claims, wherein R_{b} and R_{c}, together with the N atom to which they are attached, form a 4-6-membered heterocycloalkyl group, wherein the heterocycloalkyl contains 1 or 2 heteroatoms selected from the group consisting of N, O, and S, and the heterocycloalkyl is optionally substituted with 1, 2, or 3 substituents selected from the group consisting of: halogen, -OH, -NH₂ and -CN.

12. The compound, or a pharmaceutically acceptable salt, stereoisomer, tautomer, hydrate or solvate thereof for use according to any one of the preceding claims, wherein R₅ is selected from the group consisting of halogen, -CN, -NH₂, -OH, -NH-C₁₋₆ linear or branched alkyl, C₁₋₆ linear or branched alkyl and C₁₋₆ linear or branched alkoxy.

13. The compound, or a pharmaceutically acceptable salt, stereoisomer, tautomer, hydrate or solvate thereof for use according to any one of the preceding claims, wherein n is 0 or 1.

14. The compound, or a pharmaceutically acceptable salt, stereoisomer, tautomer, hydrate or solvate thereof for use according to claim 1, wherein:
R₁ is 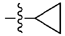 or 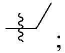
R₂ is -COOH or -COOC₁₋₃ linear or branched alkyl; for example -COOH or -COOCH₃;
R₃ is F;
B₁ is -CR₆ or N, wherein R₆ is H, halogen or -O-C₁₋₃ linear or branched alkyl, for example H, F or -O-CH₃;
A is phenyl or pyridyl;
R₄ is selected from the group consisting of -S(O)₂-NH₂, -S(O)₂-NHRₐ, -S(O)₂-NR_{b}R_{c}, -NH-S(O)₂-Rₐ, -C(O)-NH₂, -C(O)-NH-Rₐ, -C(O)-NR_{b}R_{c} and -NH-C(O)-Rₐ;
Rₐ is selected from the group consisting of C₁₋₁₂ linear or branched alkyl, C₃₋₆-membered cycloalkyl and 4-6-membered heterocycloalkyl, wherein the heterocycloalkyl contains 1 or 2 heteroatoms selected from the group consisting of N, O, and S, and the alkyl is optionally substituted with 1, 2, or 3 substituents selected from the group consisting of: halogen and -OH;
R_{b} and R_{c}, together with the N atom to which they are attached, form a 4-6-membered heterocycloalkyl group, wherein the heterocycloalkyl contains 1 or 2 heteroatoms selected from the group consisting of N, O, and S, and the heterocycloalkyl is optionally substituted with 1, 2, or 3 halogen; and
n is 0.

15. The compound, or a pharmaceutically acceptable salt, stereoisomer, tautomer, hydrate or solvate thereof for use according to any one of the preceding claims, wherein the compound is selected from the group consisting of:
| **NO.** | **Structure** |
|---|---|
| **1** | 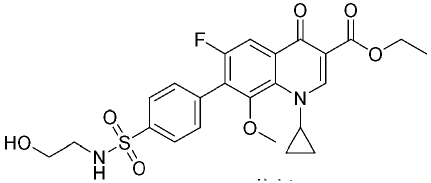 |
| **2** | 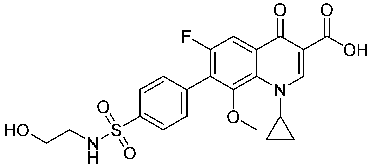 |
| **3** | 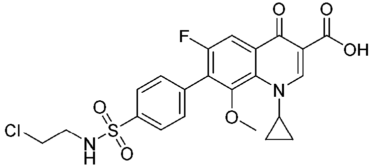 |
| **4** | 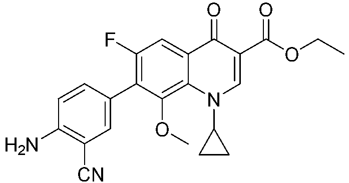 |
| **5** | 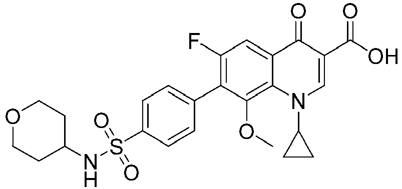 |
| **6** | 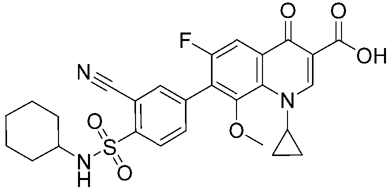 |
| **7** | 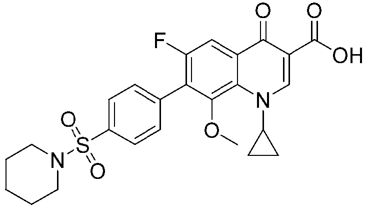 |
| **8** | 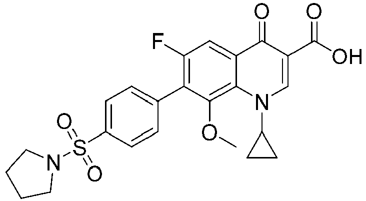 |
| **9** | 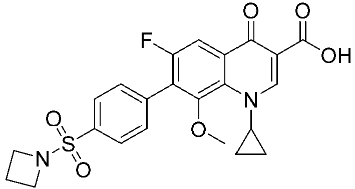 |
| **10** | 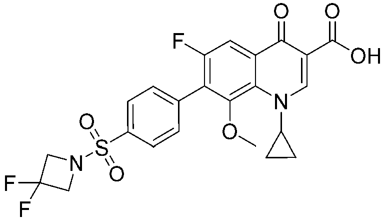 |
| **11** | 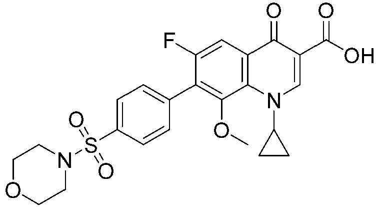 |
| **12** | 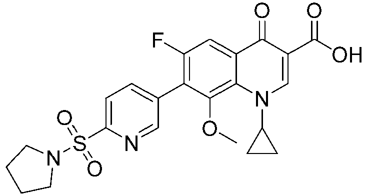 |
| **13** | 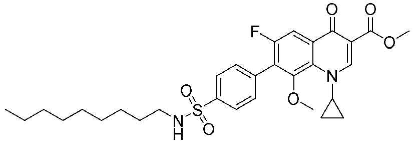 |
| **14** | 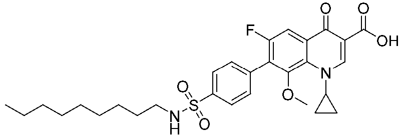 |
| **15** | 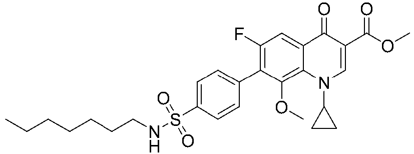 |
| **16** | 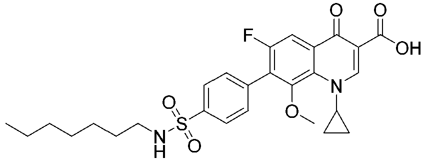 |
| **17** | 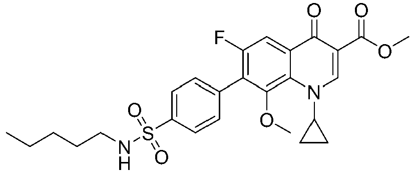 |
| **18** | 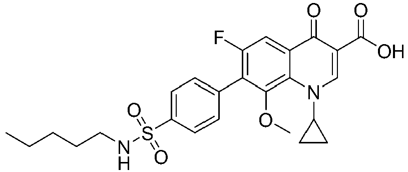 |
| **19** | 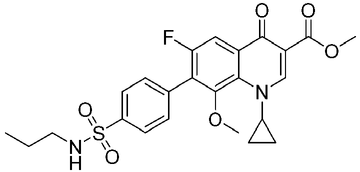 |
| **20** | 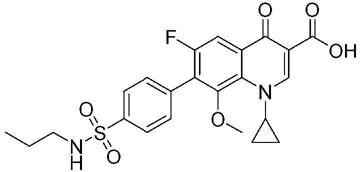 |
| **21** | 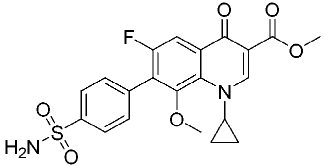 |
| **22** | 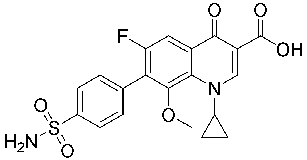 |
| **23** | 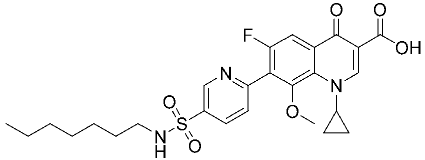 |
| **24** | 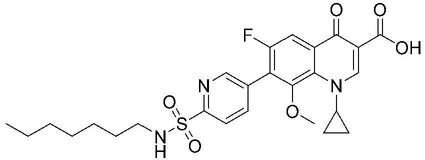 |
| **25** | 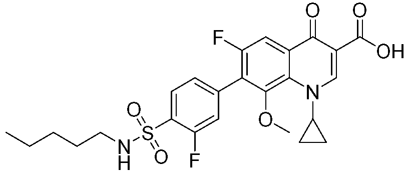 |
| **26** | 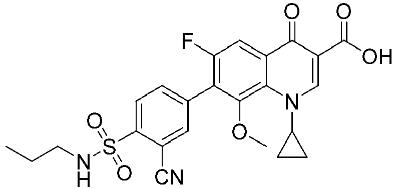 |
| **27** | 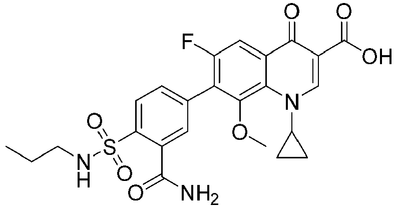 |
| **28** | 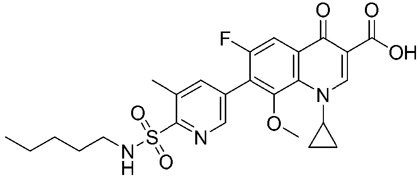 |
| **29** | 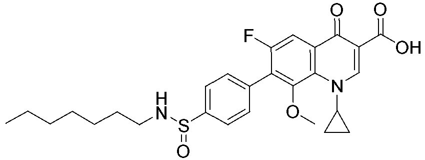 |
| **30** | 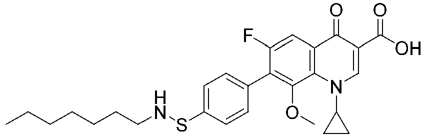 |
| **31** | 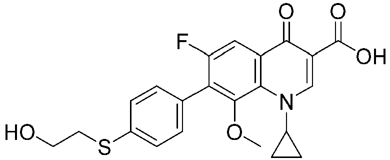 |
| **32** | 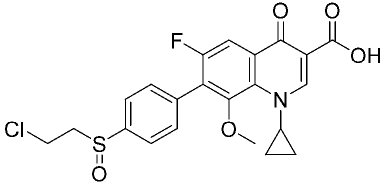 |
| **33** | 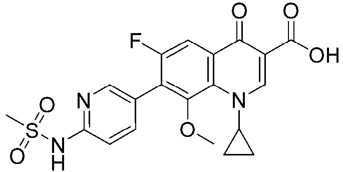 |
| **34** | 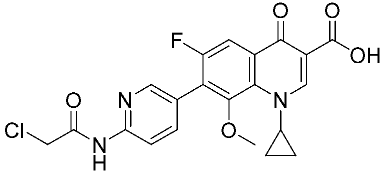 |
| **35** | 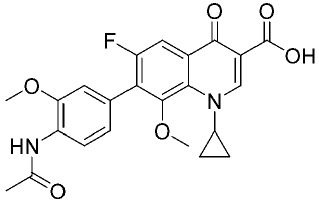 |
| **36** | 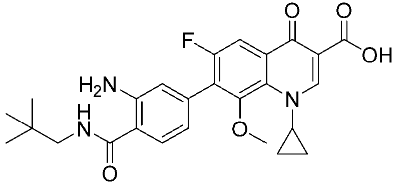 |
| **37** | 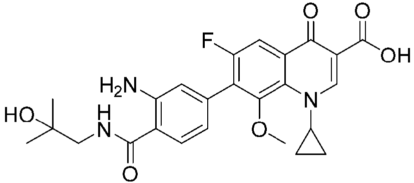 |
| **38** | 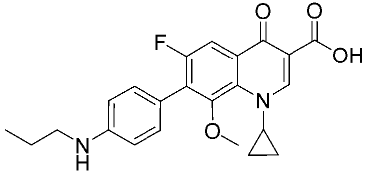 |
| **39** | 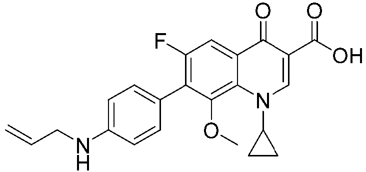 |
| **40** | 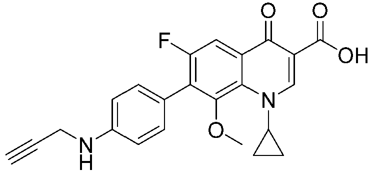 |
| **41** | 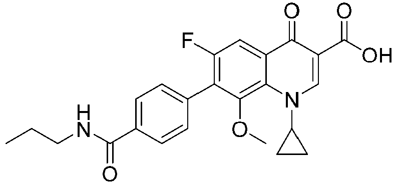 |
| **42** | 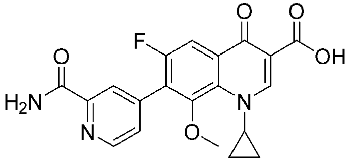 |
| **43** | 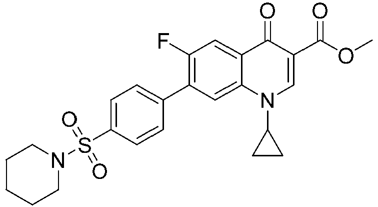 |
| **44** | 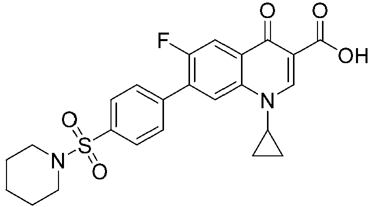 |
| **45** | 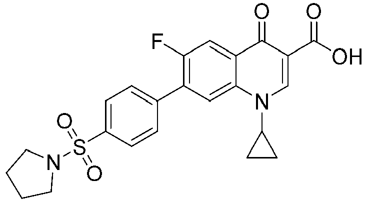 |
| **46** | 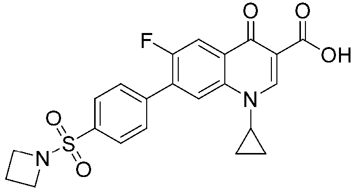 |
| **47** | 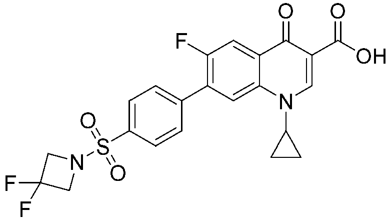 |
| **48** | 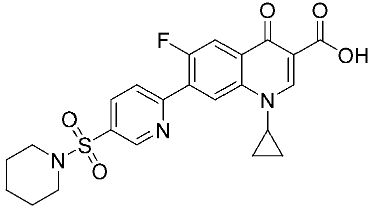 |
| **49** | 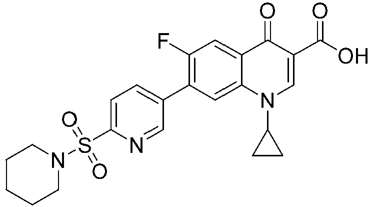 |
| **50** | 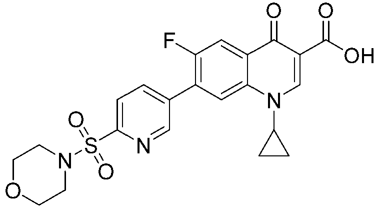 |
| **51** | 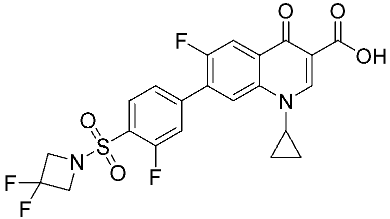 |
| **52** | 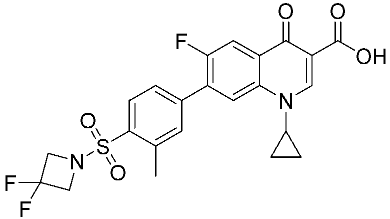 |
| **53** | 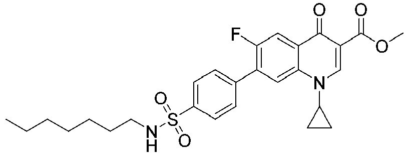 |
| **54** | 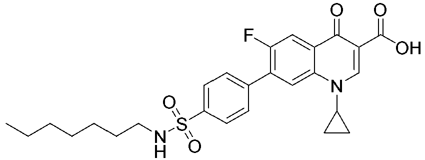 |
| **55** | 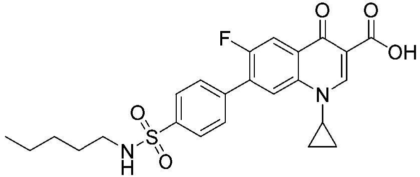 |
| **56** | 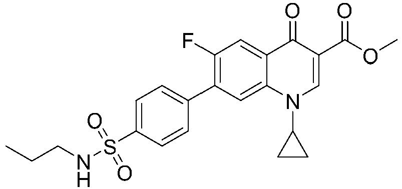 |
| **57** | 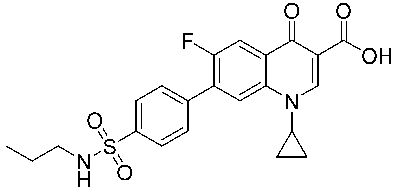 |
| **58** | 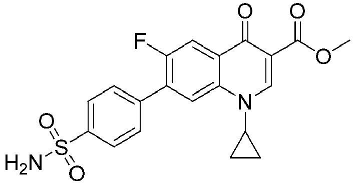 |
| **59** | 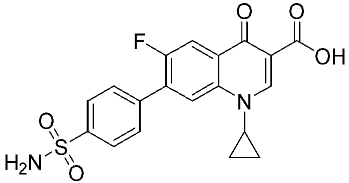 |
| **60** | 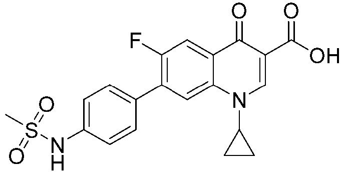 |
| **61** | 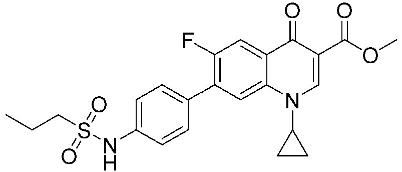 |
| **62** | 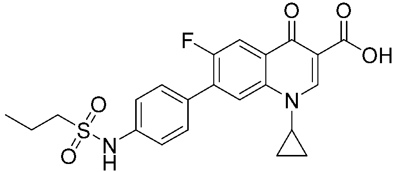 |
| **63** | 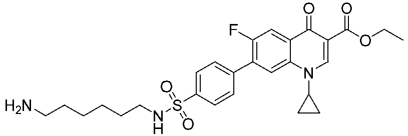 |
| **64** | 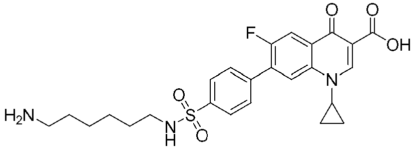 |
| **65** | 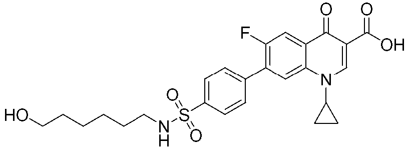 |
| **66** | 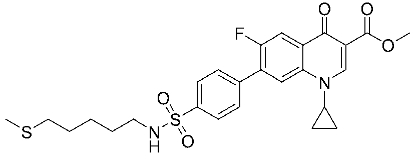 |
| **67** | 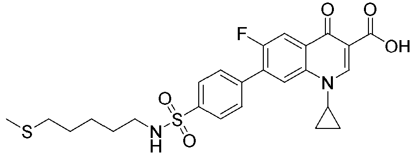 |
| **68** | 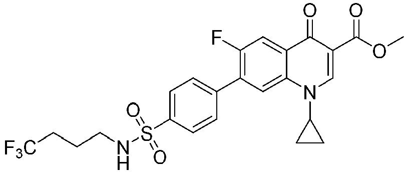 |
| **69** | 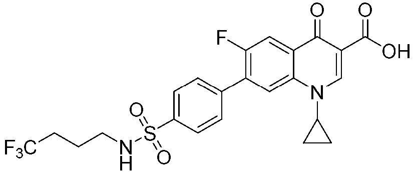 |
| **70** | 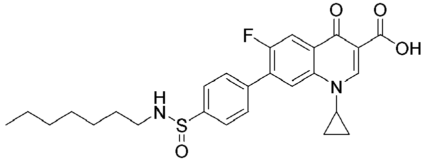 |
| **71** | 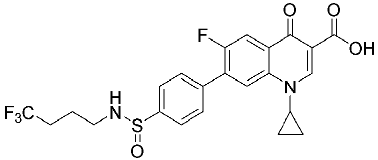 |
| **72** | 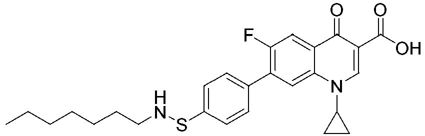 |
| **73** | 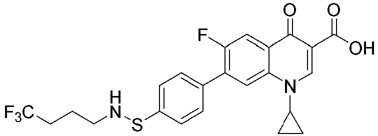 |
| **74** | 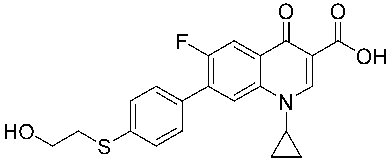 |
| **75** | 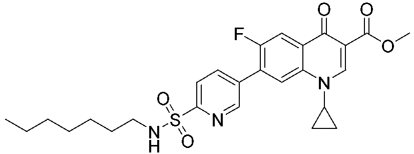 |
| **76** | 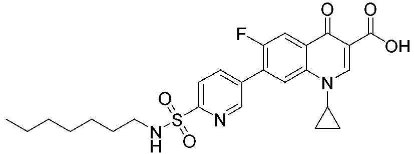 |
| **77** | 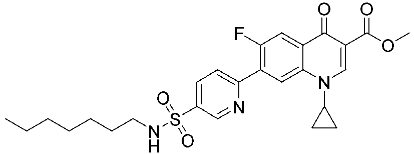 |
| **78** | 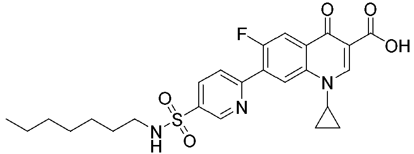 |
| **79** | 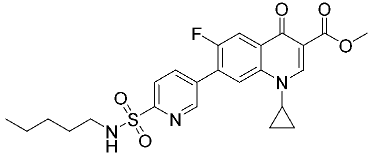 |
| **80** | 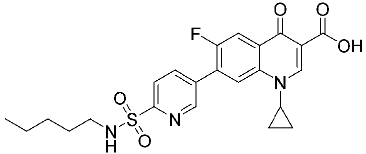 |
| **81** | 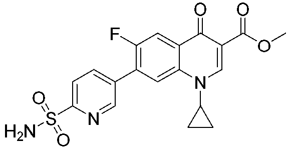 |
| **82** | 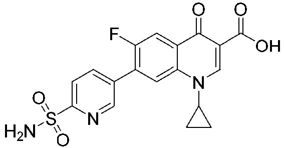 |
| **83** | 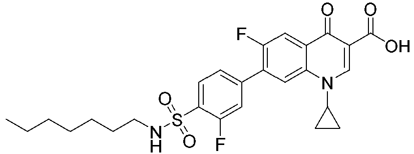 |
| **84** | 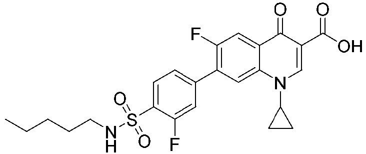 |
| **85** | 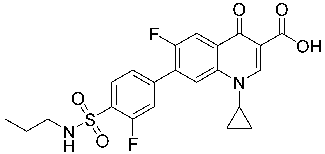 |
| **86** | 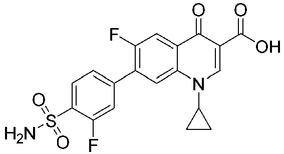 |
| **87** | 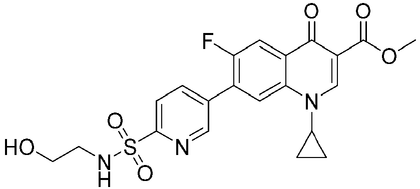 |
| **88** | 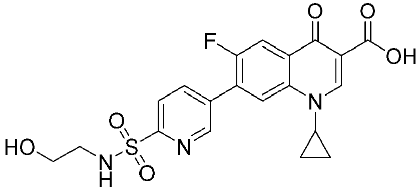 |
| **89** | 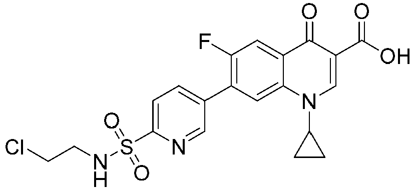 |
| **90** | 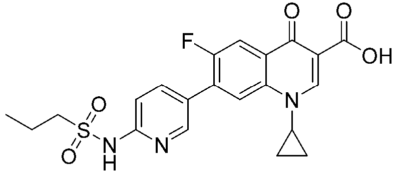 |
| **91** | 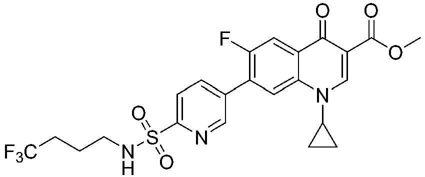 |
| **92** | 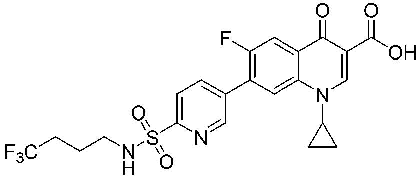 |
| **93** | 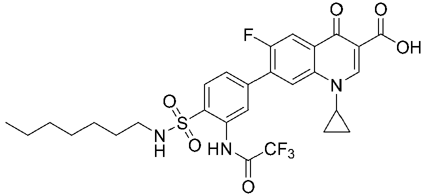 |
| **94** | 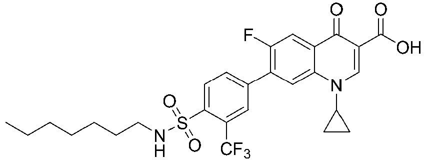 |
| **95** | 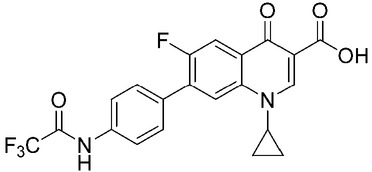 |
| **96** | 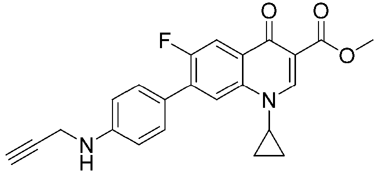 |
| **97** | 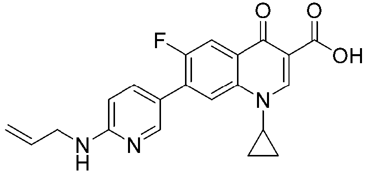 |
| **98** | 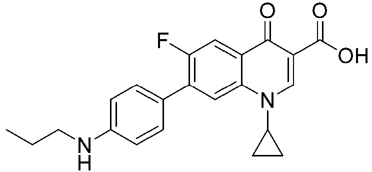 |
| **99** | 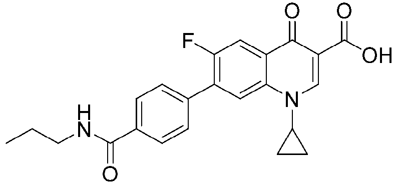 |
| **100** | 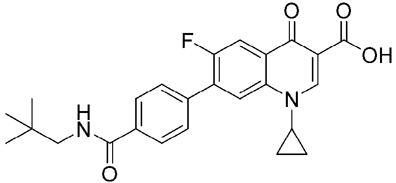 |
| **101** | 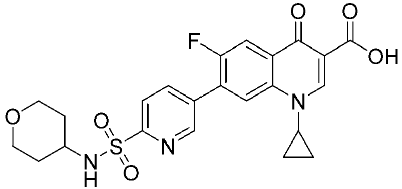 |
| **102** | 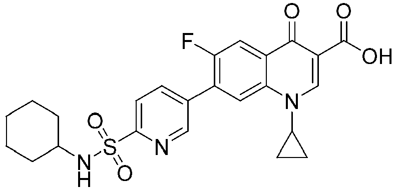 |
| **103** | 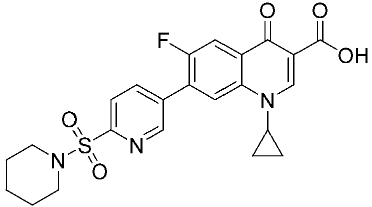 |
| **104** | 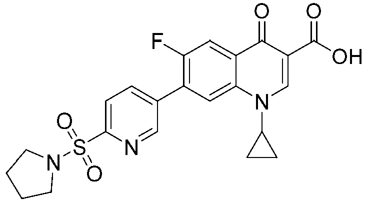 |
| **105** | 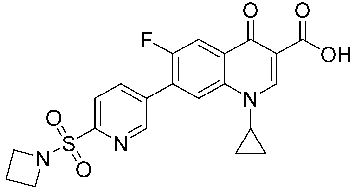 |
| **106** | 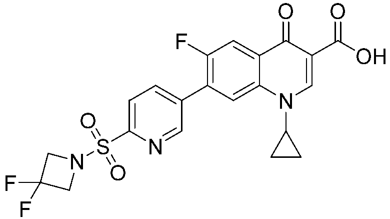 |
| **107** | 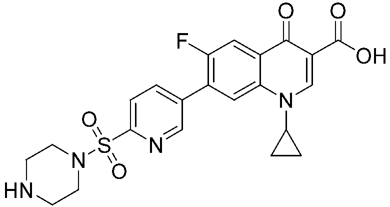 |
| **108** | 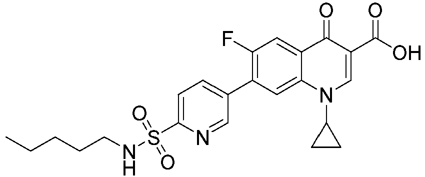 |
| **109** | 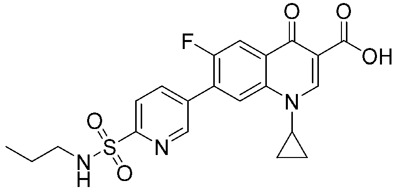 |
| **110** | 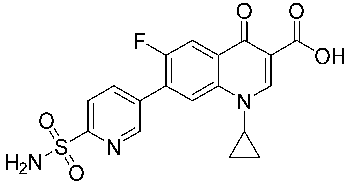 |
| **111** | 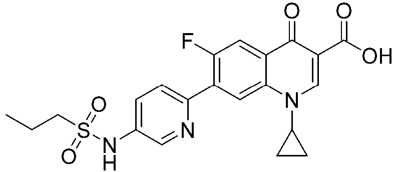 |
| **112** | 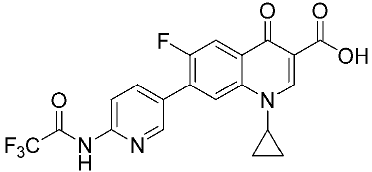 |
| **113** | 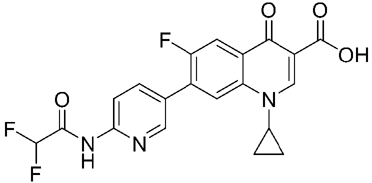 |
| **114** | 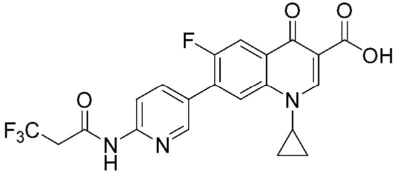 |
| **115** | 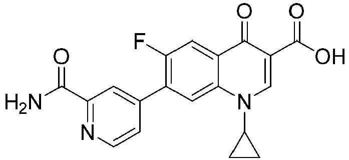 |
| **116** | 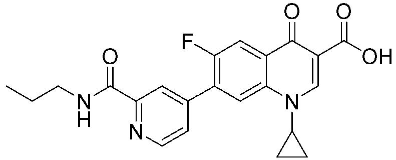 |
| **117** | 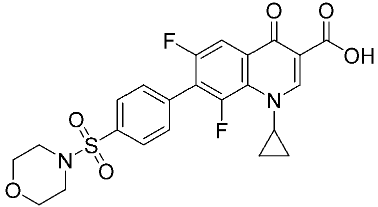 |
| **118** | 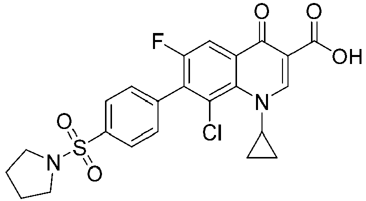 |
| **119** | 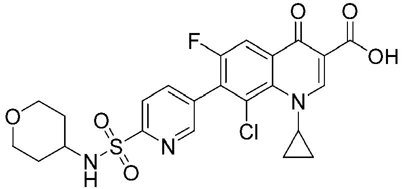 |
| **120** | 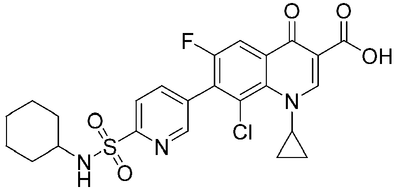 |
| **121** | 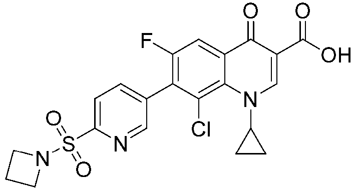 |
| **122** | 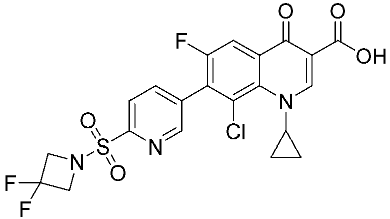 |
| **123** | 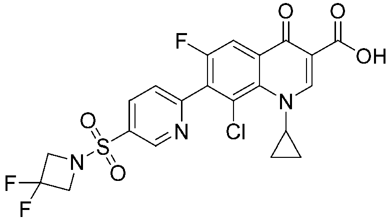 |
| **124** | 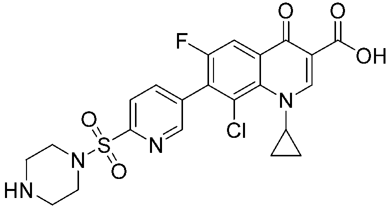 |
| **125** | 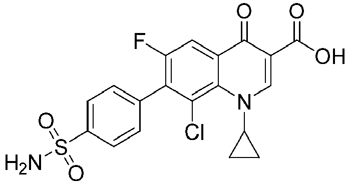 |
| **126** | 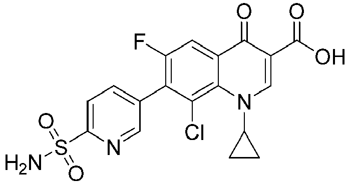 |
| **127** | 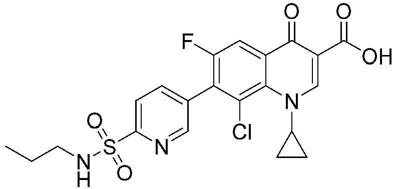 |
| **128** | 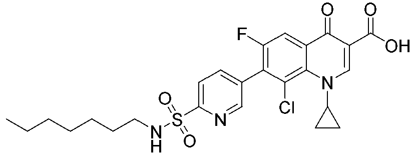 |
| **129** | 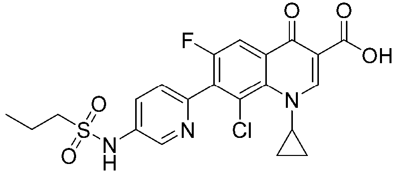 |
| **130** | 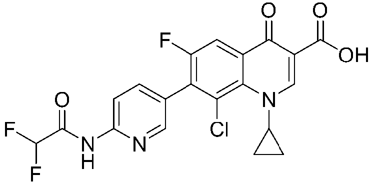 |
| **131** | 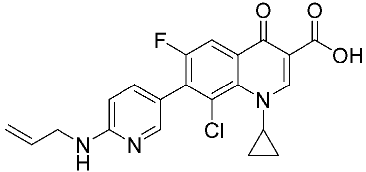 |
| **132** | 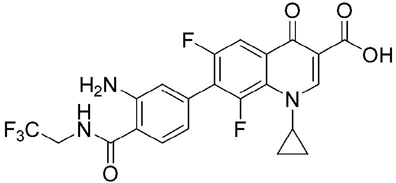 |
| **133** | 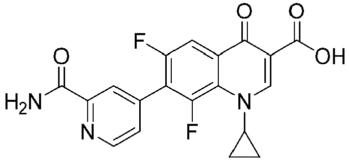 |
| **134** | 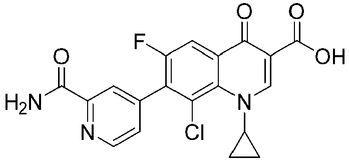 |
| **135** | 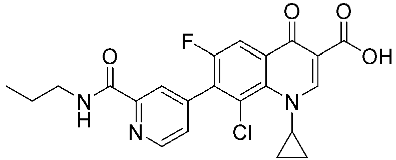 |
| **136** | 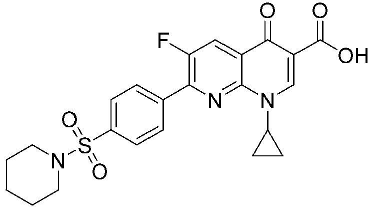 |
| **137** | 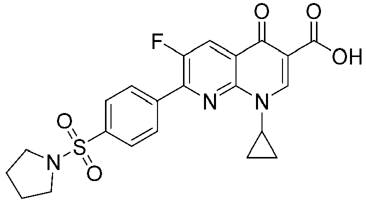 |
| **138** | 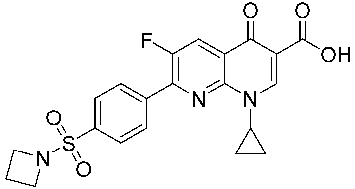 |
| **139** | 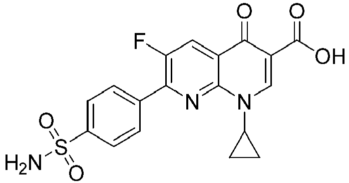 |
| **140** | 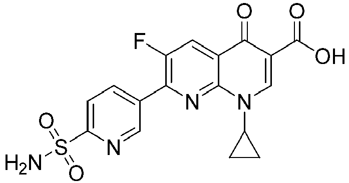 |
| **141** | 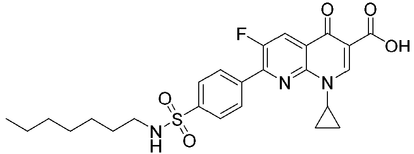 |
| **142** | 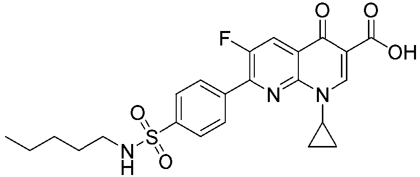 |
| **143** | 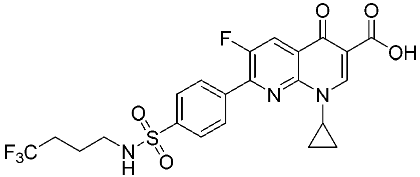 |
| **144** | 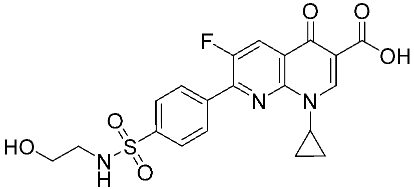 |
| **145** | 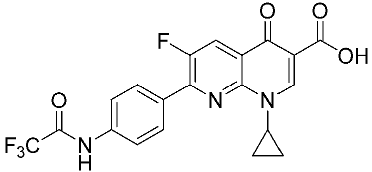 |
| **146** | 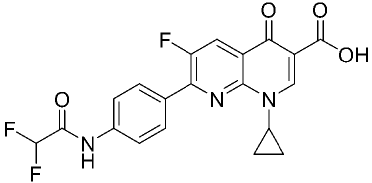 |
| **147** | 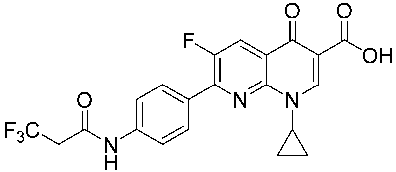 |
| **148** | 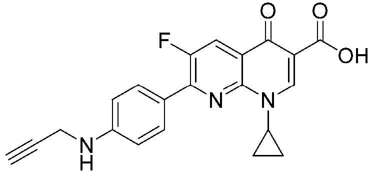 |
| **149** | 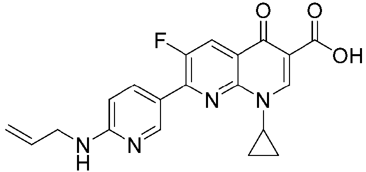 |
| **150** | 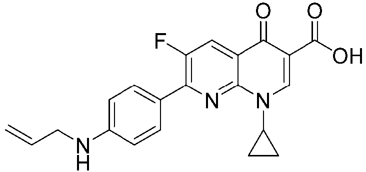 |
| **151** | 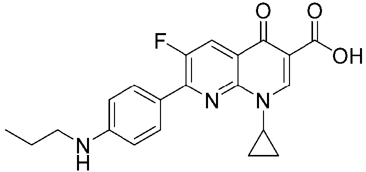 |
| **152** | 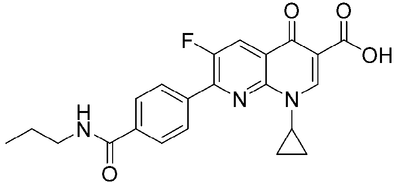 |
| **153** | 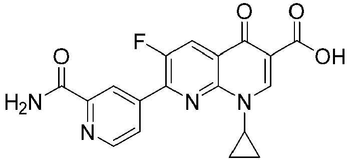 |
| **154** | 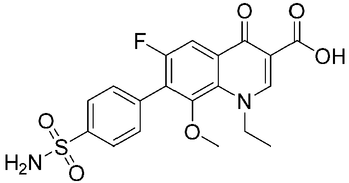 |
| **155** | 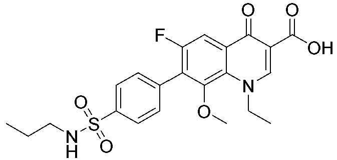 |
| **156** | 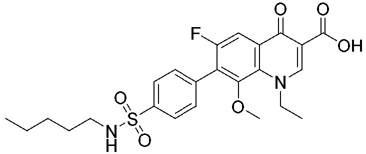 |
| **157** | 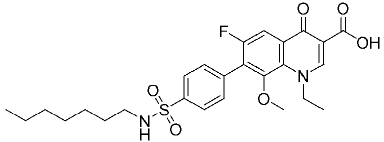 |
| **158** | 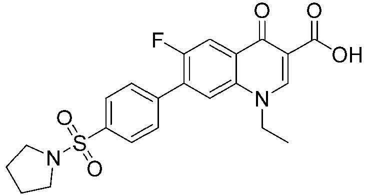 |
| **159** | 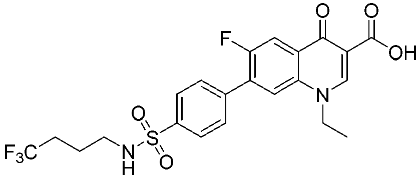 |
| **160** | 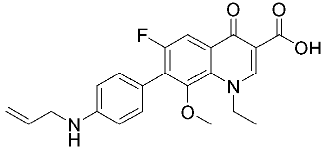 |
| **161** | 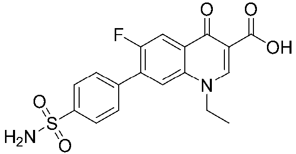 |
| **162** | 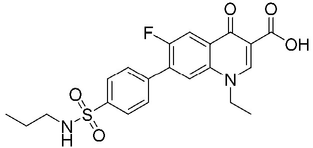 |
| **163** | 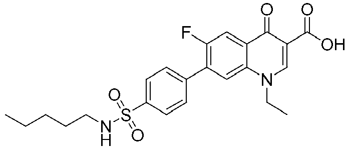 |
| **164** | 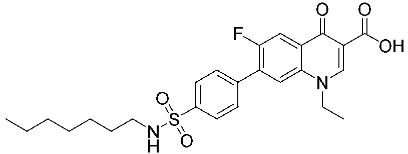 |
| **165** | 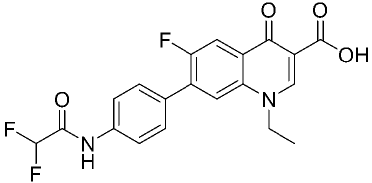 |
| **166** | 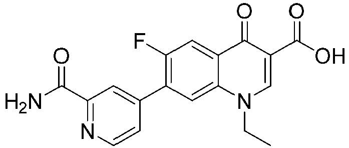 |
| **167** | 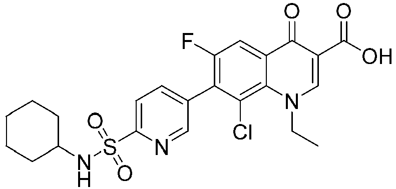 |
| **168** | 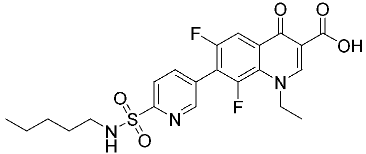 |
| **169** | 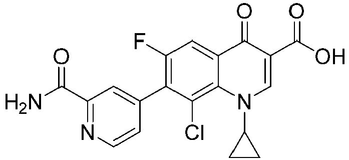 |
| **170** | 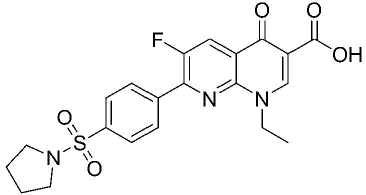 |
| **171** | 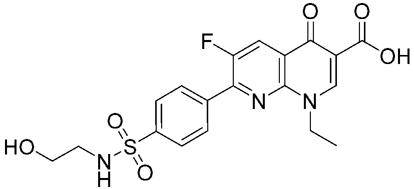 |
| **172** | 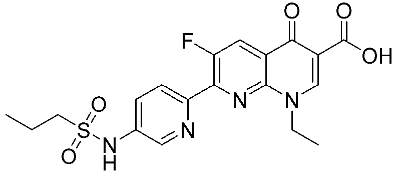 |
| **173** | 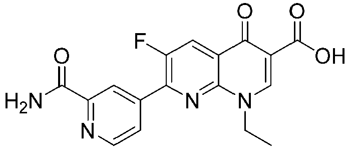 |

16. The compound, or a pharmaceutically acceptable salt, stereoisomer, tautomer, hydrate or solvate thereof for use according to any one of claims 1-15, wherein the disease or disorder is particularly a disease or disorder caused by mast cell degranulation; the disease or disorder is preferably selected from the group consisting of autoimmune diseases, allergic diseases, inflammatory diseases, pruritus, and pain, and the disease or disorder is preferably selected from the group consisting of psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

17. A method for antagonizing MRGPRX2 *in vitro,* comprising utilizing an effective amount of the compound as defined in any one of claims 1-15 or a pharmaceutically acceptable salt, a stereoisomer, a tautomer, a hydrate, or a solvate thereof, in an *in vitro* assay of MRGPRX2.

18. A compound of formula (IB), or a pharmaceutically acceptable salt, stereoisomer, tautomer, hydrate, or solvate thereof, 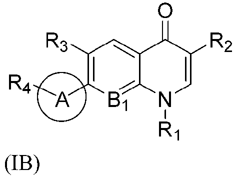 wherein:
R₁ is -C₃₋₆ cycloalkyl or -C₁₋₆ linear or branched alkyl;
R₂ is -COOH or -COOC₁₋₆ linear or branched alkyl;
R₃ is halogen;
B₁ is -CR₆ or N, wherein R₆ is H, halogen or -O-C₁₋₆ linear or branched alkyl; and
A is phenyl; and R₄ is selected from the group consisting of -S(O)₂-NH-C₈₋₁₂ linear or branched alkyl, -S(O)₂-NH-C₁₋₆ linear or branched alkyl-CF₃, -S(O)₂-NH-cyclohexyl, -S(O)₂-NR_{b}R_{c}, -S(O)-NH-C₁₋₆ linear or branched alkyl-CF₃, -S-NH-C₁₋₆ linear or branched alkyl-CF₃, -NH-S(O)₂-C₁₋₆ linear or branched alkyl, -C(O)-NH₂, -C(O)-NH-C₂₋₆ linear or branched alkyl, -C(O)-NR_{b}R_{c}, -NH-C(O)-CF₃, -NH-C(O)-CH₂Cl, -NH-C₁₋₆ linear or branched alkyl, -NH-C₁₋₆ linear or branched alkenyl and -NH-C₁₋₆ linear or branched alkynyl; wherein R_{b} and R_{c}, together with the N atom to which they are attached, form an azetidinyl or difluoroazetidinyl group;
or, A is pyridyl; and R₄ is selected from the group consisting of -S(O)₂-NH₂, -S(O)₂-NH-CH₂CH₂OH, -S(O)₂-NH-CH₂CH₂Cl, -S(O)₂-NH-C₄₋₆ linear or branched alkyl, -S(O)₂-NH-C₈₋₁₂ linear or branched alkyl, -S(O)₂-NH-cyclohexyl, -S(O)₂-NH-tetrahydropyranyl, -S(O)₂-NR_{b}R_{c}, -S(O)-NH-C₁₋₆ linear or branched alkyl, -S-NH-C₁₋₆ linear or branched alkyl-CF₃, -NH-S(O)₂-C₂₋₆ linear or branched alkyl, -C(O)-NH-C₃₋₆ branched alkyl, -C(O)-NR_{b}R_{c}, -NH-C(O)-CF₂, -NH-C(O)-CH₂CF₃, -NH-C₁₋₆ linear or branched alkyl and -S-C₁₋₆ linear or branched hydroxyalkyl; wherein R_{b} and R_{c}, together with the N atom to which they are attached, form an azetidinyl, difluoroazetidinyl, pyrrolidinyl, piperidinyl, piperazinyl or morpholinyl group.

19. The compound according to any one of the preceding claims, or a pharmaceutically acceptable salt, stereoisomer, tautomer, hydrate, or solvate thereof, wherein R₁ is 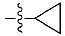 or

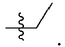

20. The compound according to any one of claims 1-7, or a pharmaceutically acceptable salt, stereoisomer, tautomer, hydrate, or solvate thereof, wherein R₂ is -COOH or -COOCH₃.

21. The compound according to any one of the preceding claims, or a pharmaceutically acceptable salt, stereoisomer, tautomer, hydrate, or solvate thereof, wherein R₃ is F.

22. The compound according to any one of the preceding claims, or a pharmaceutically acceptable salt, stereoisomer, tautomer, hydrate, or solvate thereof, wherein B₁ is -CR₆ or N, wherein R₆ is H, F, Cl or -OCH₃.

23. The compound according to any one of the preceding claims, or a pharmaceutically acceptable salt, stereoisomer, tautomer, hydrate, or solvate thereof, wherein, A is phenyl; and R₄ is selected from the group consisting of -S(O)₂-NH-C₈₋₁₀ linear alkyl, -S(O)₂-NH-C₂₋₄ linear alkyl-CF₃, -S(O)₂-NH-cyclohexyl, -S(O)₂-NR_{b}R_{c}, -NH-S(O)₂-C₂₋₄ linear alkyl, -C(O)-NH₂, -C(O)-NH-C₂₋₆ linear or branched alkyl, -NH-C(O)-CF₃, -NH-C₂₋₄ linear alkyl and -NH-C₂₋₄ linear alkenyl; wherein R_{b} and R_{c}, together with the N atom to which they are attached, form an azetidinyl or difluoroazetidinyl group.

24. The compound according to any one of the preceding claims, or a pharmaceutically acceptable salt, stereoisomer, tautomer, hydrate, or solvate thereof, wherein, A is pyridyl; and R₄ is selected from the group consisting of -S(O)₂-NH₂, -S(O)₂-NH-CH₂CH₂OH, -S(O)₂-NH-CH₂CH₂Cl, -S(O)₂-NH-C₄₋₆ linear alkyl, -S(O)₂-NH-C₈₋₁₀ linear alkyl, -S(O)₂-NH-cyclohexyl, -S(O)₂-NH-tetrahydropyranyl, -S(O)₂-NR_{b}R_{c}, -NH-S(O)₂-C₂₋₄ linear alkyl, -C(O)-NH-C₃₋₆ branched alkyl, -NH-C(O)-CF₂, -NH-C(O)-CH₂CF₃ and -S-C₂₋₄ hydroxy linear alkyl; wherein R_{b} and R_{c}, together with the N atom to which they are attached, form an azetidinyl, difluoroazetidinyl, pyrrolidinyl, piperidinyl, piperazinyl or morpholinyl group; for example, A is pyridyl; and R₄ is selected from the group consisting of -S(O)₂-NH₂, -S(O)₂-NH-cyclohexyl, -S(O)₂-NH-tetrahydropyranyl, -S(O)₂-NR_{b}R_{c}, -NH-C(O)-CF₂ and -NH-C(O)-CH₂CF₃; wherein R_{b} and R_{c}, together with the N atom to which they are attached, form an azetidinyl, difluoroazetidinyl, pyrrolidinyl, piperidinyl, piperazinyl or morpholinyl group.

25. The compound according to any one of the preceding claims, or a pharmaceutically acceptable salt, stereoisomer, tautomer, hydrate, or solvate thereof, wherein,
R₁ is 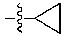 or 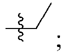 for example 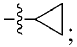
R₂ is -COOH or -COOC₁₋₃ linear or branched alkyl; for example -COOH or -COOCH₃;
R₃ is F;
B₁ is -CR₆ or N, wherein R₆ is H, halogen or -O-C₁₋₃ linear or branched alkyl, for example H, F, Cl or -O-CH₃; and
A is phenyl; and R₄ is selected from the group consisting of -S(O)₂-NH-C₈₋₁₀ linear alkyl, -S(O)₂-NH-C₂₋₄ linear alkyl-CF₃, -S(O)₂-NR_{b}R_{c}, -C(O)-NH-C₂₋₆ linear or branched alkyl, -NH-C(O)-CF₃ and -NH-C₂₋₄ linear alkenyl; wherein R_{b} and R_{c}, together with the N atom to which they are attached, form a difluoroazetidinyl group;
or, A is pyridyl; and R₄ is selected from the group consisting of -S(O)₂-NH₂, -S(O)₂-NH-cyclohexyl, -S(O)₂-NH-tetrahydropyranyl, -S(O)₂-NR_{b}R_{c}, -NH-C(O)-CF₂ and -NH-C(O)-CH₂CF₃; wherein R_{b} and R_{c}, together with the N atom to which they are attached, form an azetidinyl, difluoroazetidinyl, pyrrolidinyl, piperidinyl, piperazinyl or morpholinyl group.

26. A compound, which is selected from the group consisting of:
| **NO.** | **Structure** |
|---|---|
| **7** | 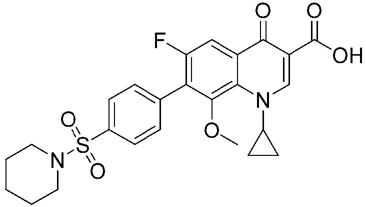 |
| **9** | 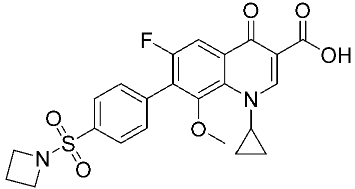 |
| **10** | 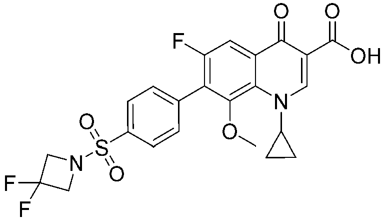 |
| **11** | 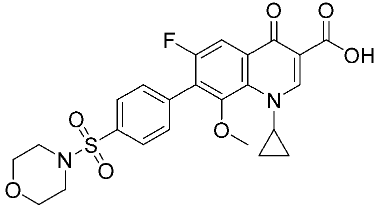 |
| **12** | 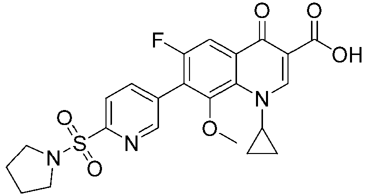 |
| **13** | 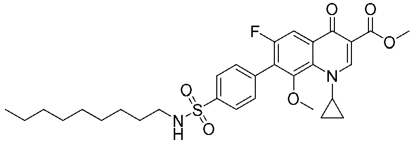 |
| **14** | 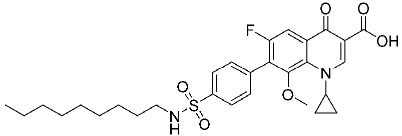 |
| **15** | 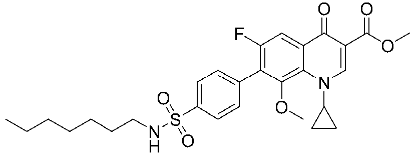 |
| **17** | 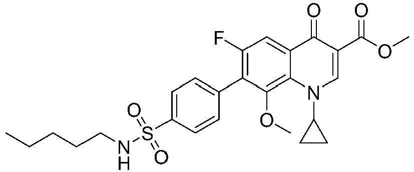 |
| **19** | 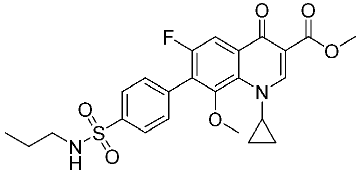 |
| **21** | 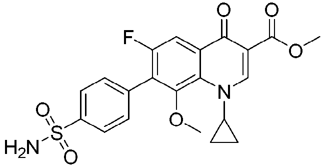 |
| **22** | 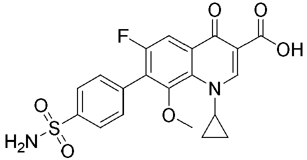 |
| **23** | 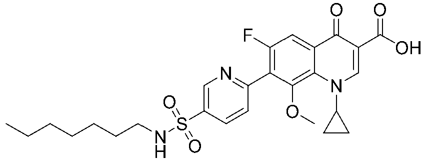 |
| **24** | 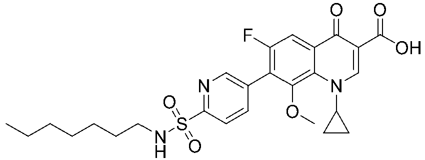 |
| **25** | 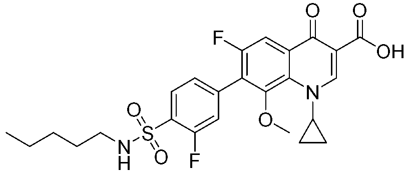 |
| **26** | 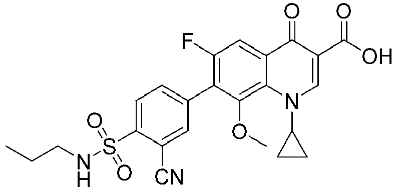 |
| **27** | 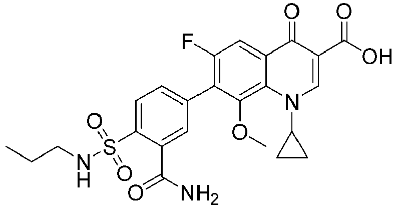 |
| **33** | 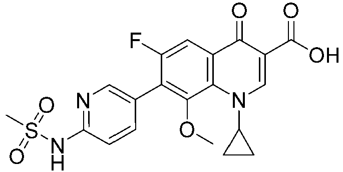 |
| **34** | 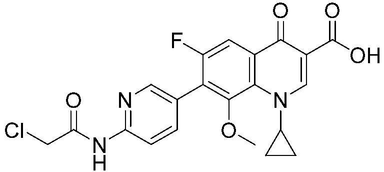 |
| **38** | 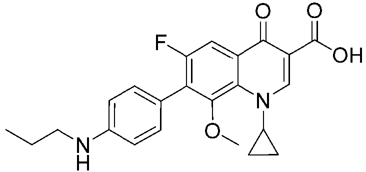 |
| **39** | 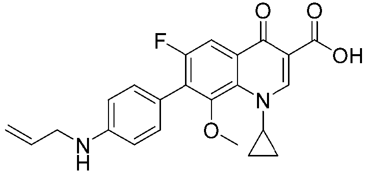 |
| **40** | 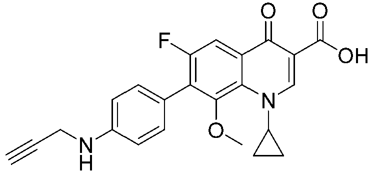 |
| **41** | 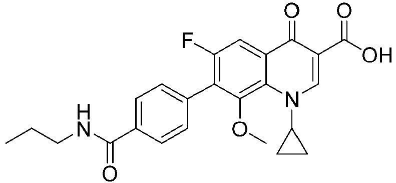 |
| **42** | 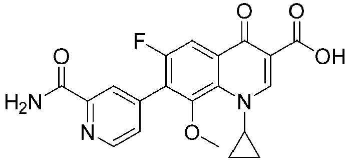 |
| **43** | 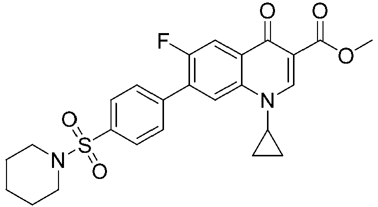 |
| **44** | 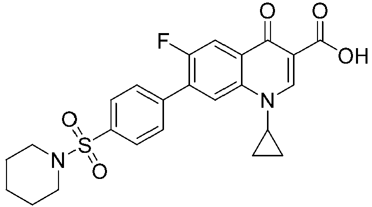 |
| **45** | 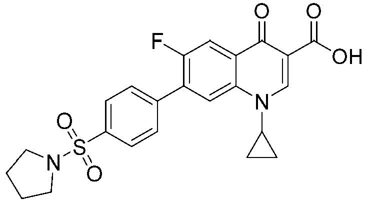 |
| **46** | 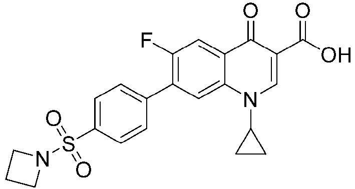 |
| **47** | 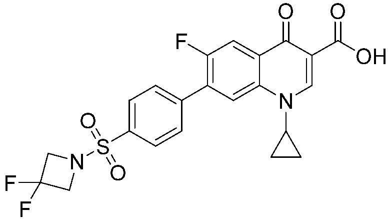 |
| **48** | 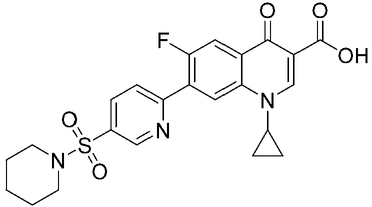 |
| **49** | 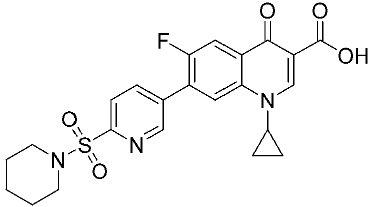 |
| **50** | 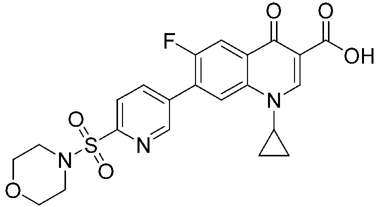 |
| **51** | 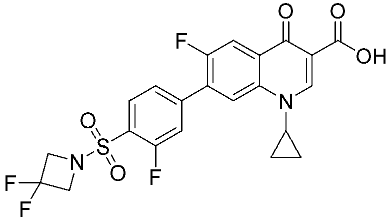 |
| **52** | 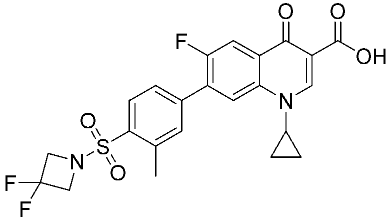 |
| **53** | 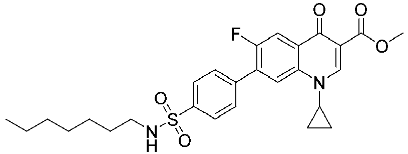 |
| **55** | 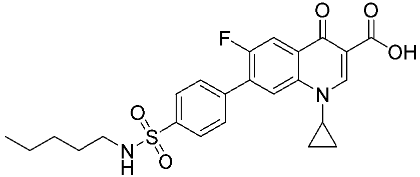 |
| **56** | 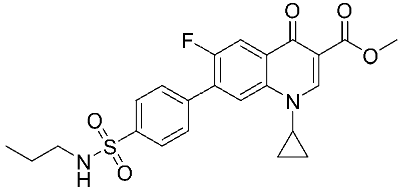 |
| **57** | 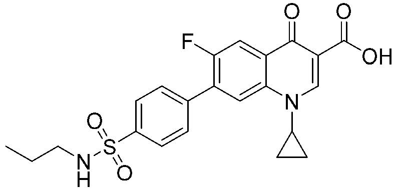 |
| **58** | 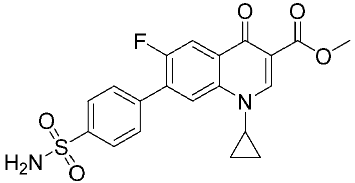 |
| **59** | 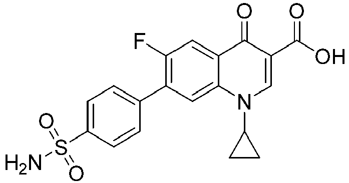 |
| **60** | 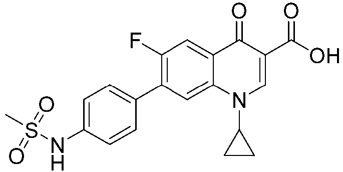 |
| **61** | 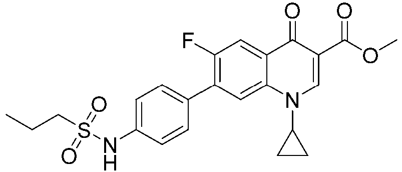 |
| **62** | 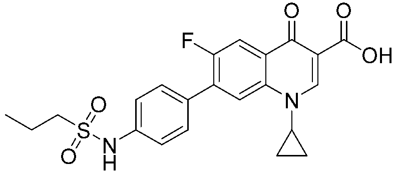 |
| **66** | 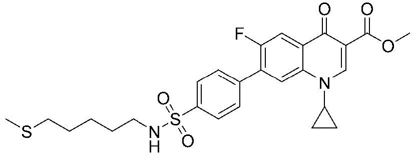 |
| **68** | 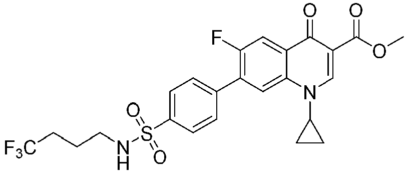 |
| **69** | 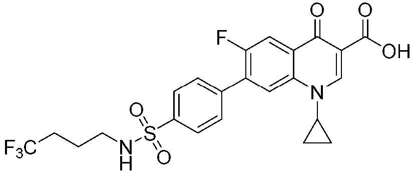 |
| **70** | 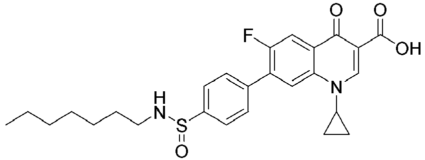 |
| **71** | 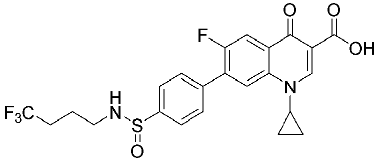 |
| **72** | 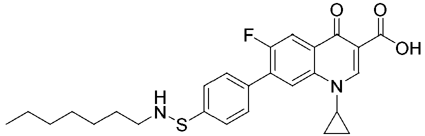 |
| **73** | 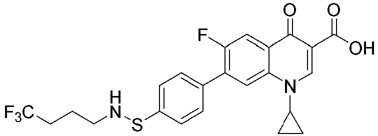 |
| **74** | 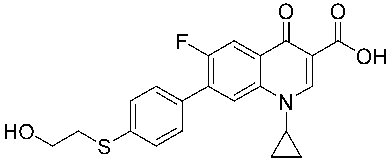 |
| **75** | 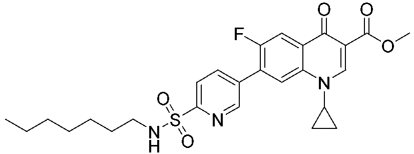 |
| **77** | 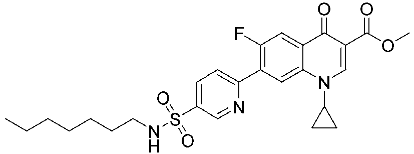 |
| **78** | 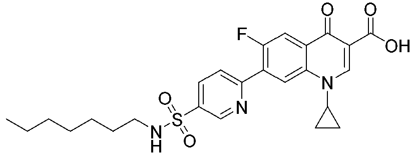 |
| **79** | 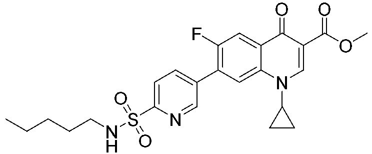 |
| **80** | 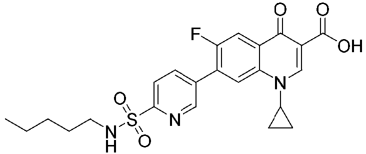 |
| **81** | 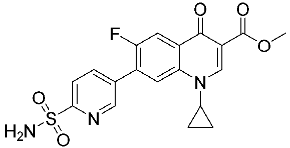 |
| **82** | 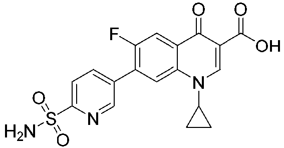 |
| **83** | 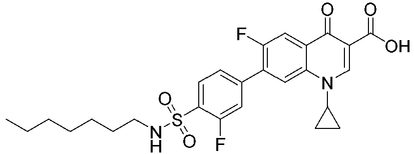 |
| **84** | 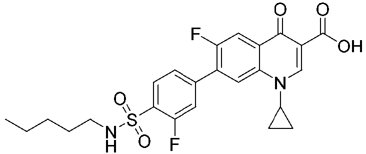 |
| **85** | 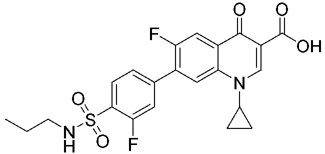 |
| **86** | 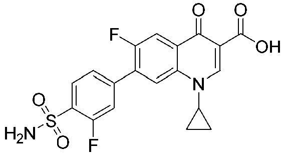 |
| **87** | 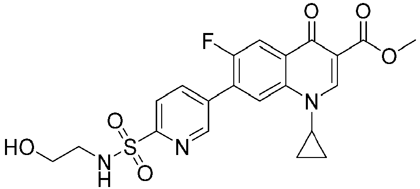 |
| **88** | 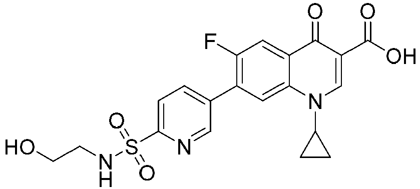 |
| **89** | 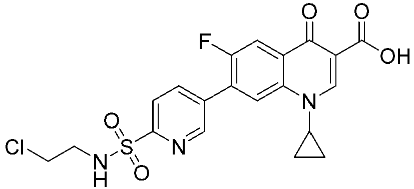 |
| **90** | 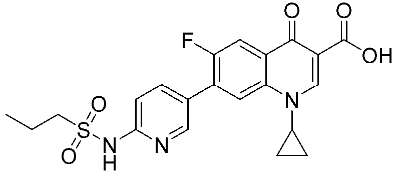 |
| **91** | 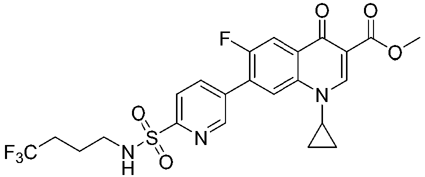 |
| **92** | 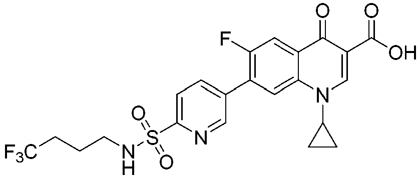 |
| **95** | 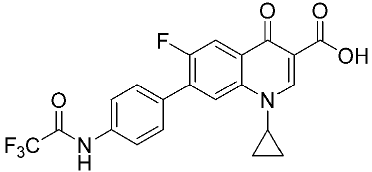 |
| **96** | 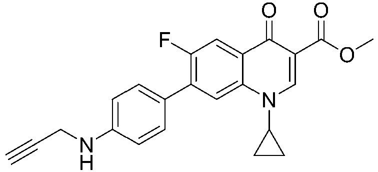 |
| **98** | 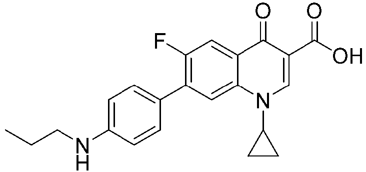 |
| **99** | 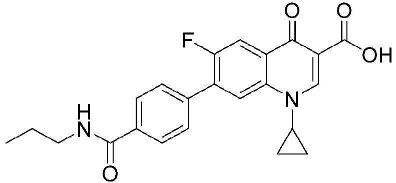 |
| **100** | 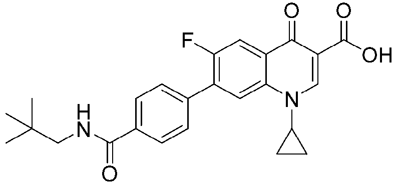 |
| **101** | 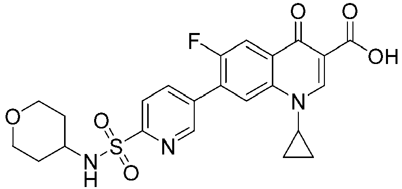 |
| **102** | 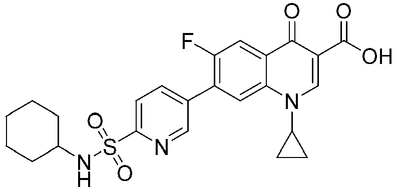 |
| **103** | 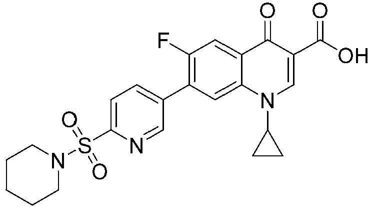 |
| **104** | 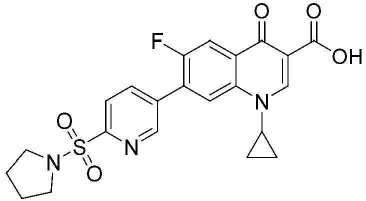 |
| **105** | 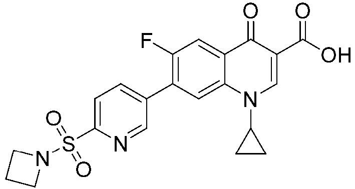 |
| **106** | 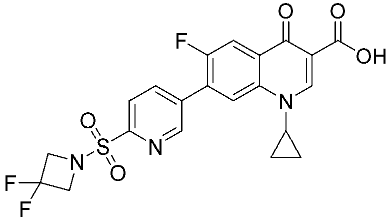 |
| **107** | 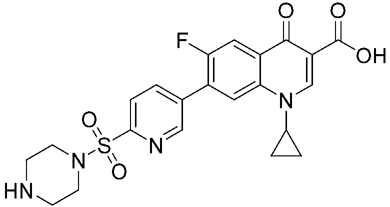 |
| **108** | 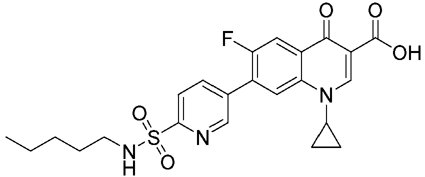 |
| **109** | 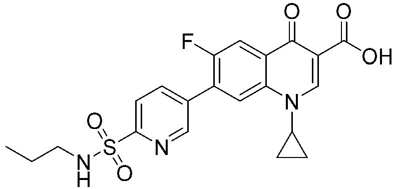 |
| **110** | 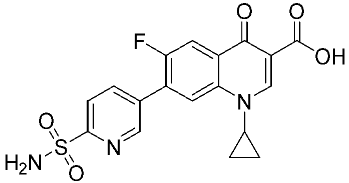 |
| **111** | 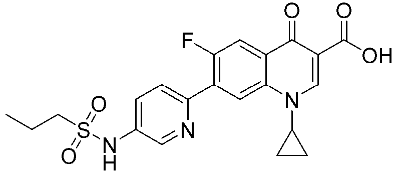 |
| **112** | 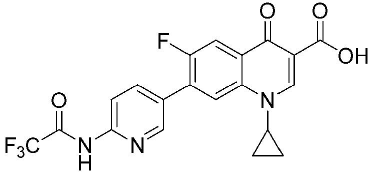 |
| **113** | 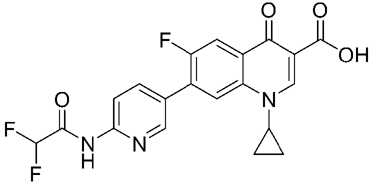 |
| **114** | 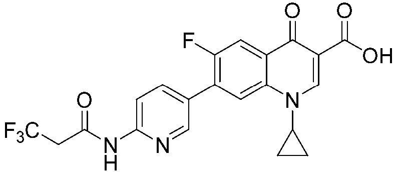 |
| **117** | 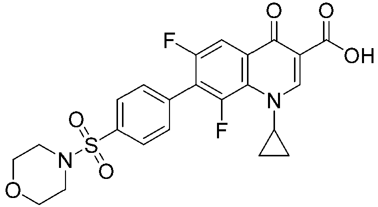 |
| **118** | 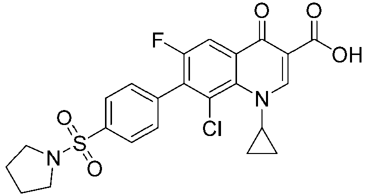 |
| **119** | 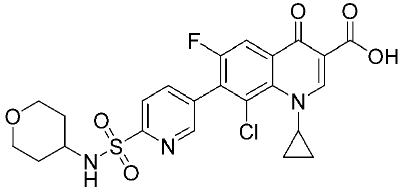 |
| **120** | 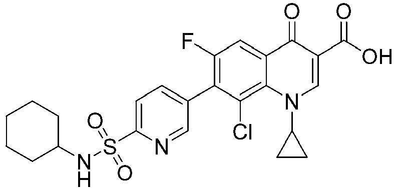 |
| **121** | 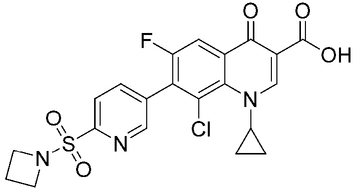 |
| **122** | 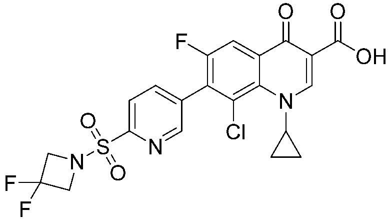 |
| **123** | 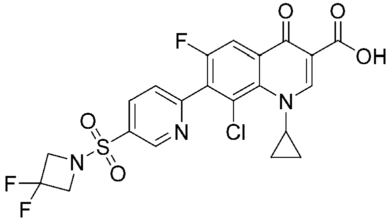 |
| **124** | 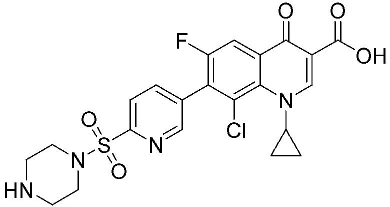 |
| **125** | 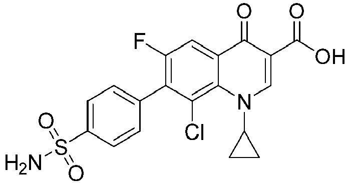 |
| **126** | 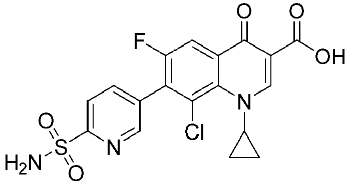 |
| **128** | 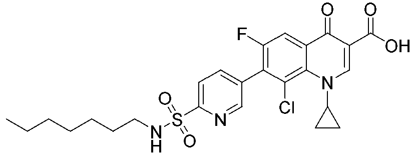 |
| **129** | 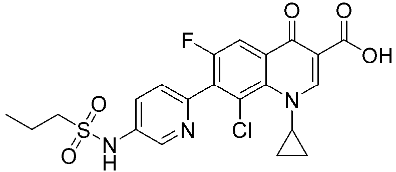 |
| **130** | 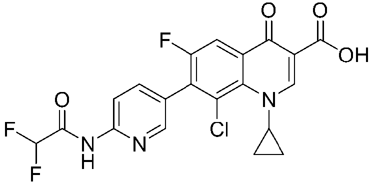 |
| **131** | 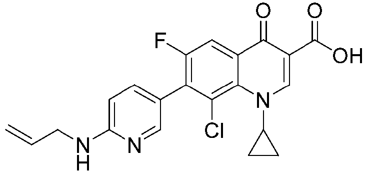 |
| **133** | 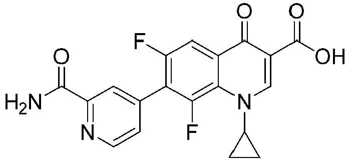 |
| **134** | 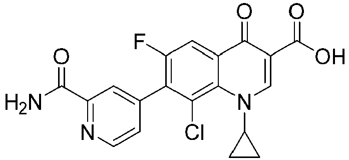 |
| **135** | 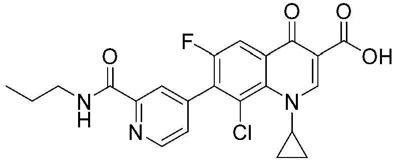 |
| **136** | 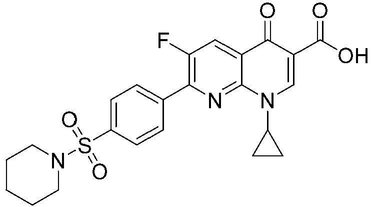 |
| **137** | 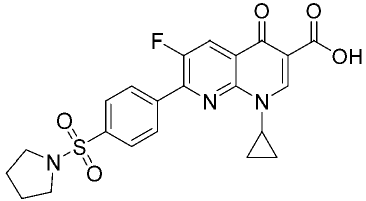 |
| **138** | 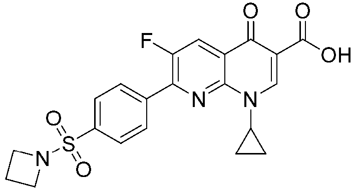 |
| **139** | 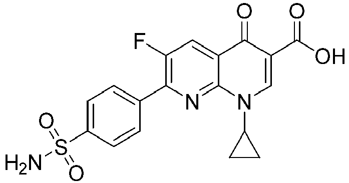 |
| **140** | 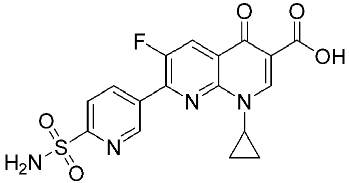 |
| **141** | 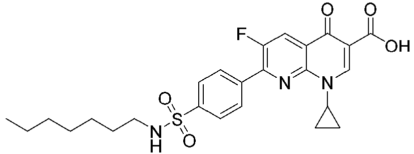 |
| **142** | 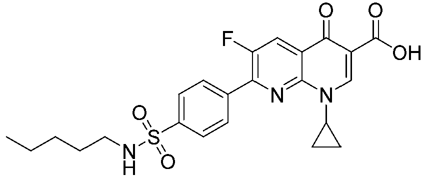 |
| **143** | 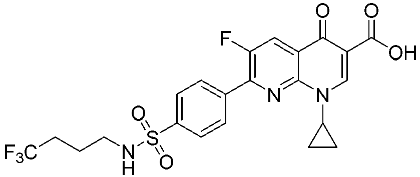 |
| **145** | 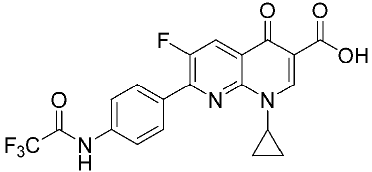 |
| **146** | 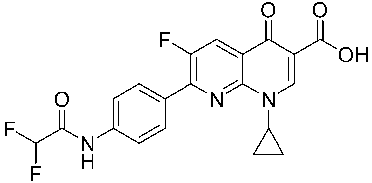 |
| **147** | 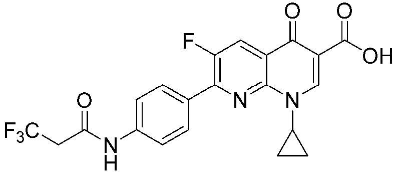 |
| **148** | 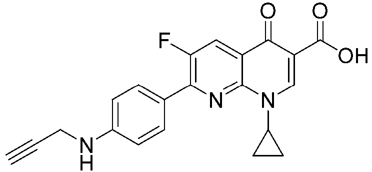 |
| **149** | 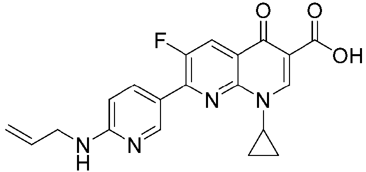 |
| **150** | 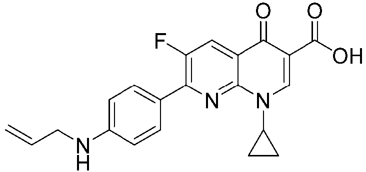 |
| **151** | 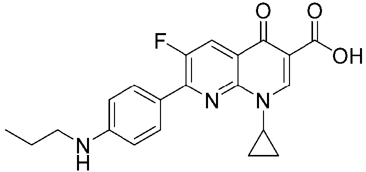 |
| **152** | 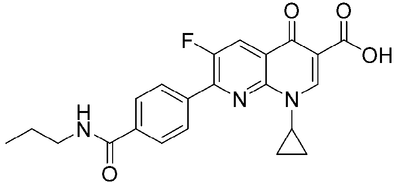 |
| **154** | 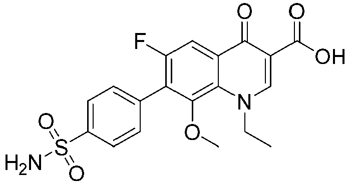 |
| **155** | 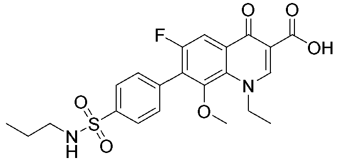 |
| **156** | 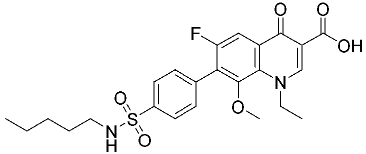 |
| **157** | 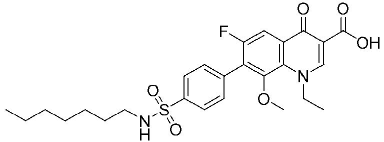 |
| **158** | 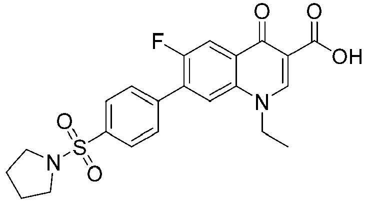 |
| **159** | 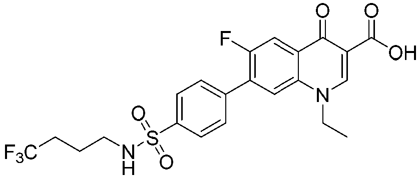 |
| **160** | 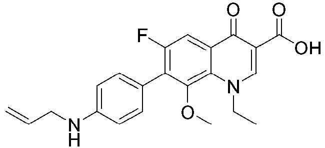 |
| **162** | 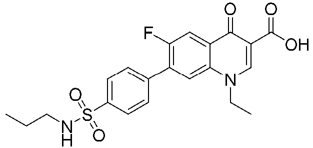 |
| **163** | 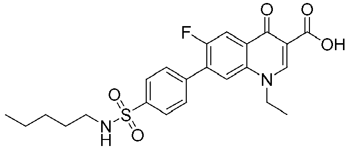 |
| **165** | 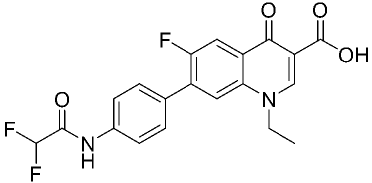 |
| **166** | 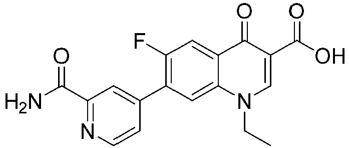 |
| **167** | 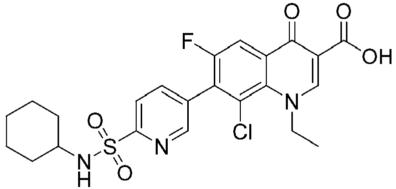 |
| **168** | 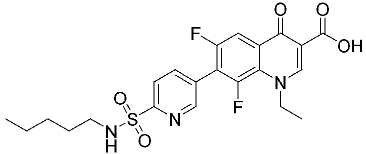 |
| **169** | 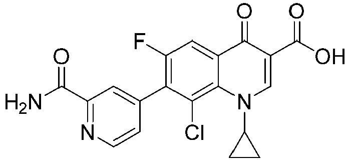 |
| **170** | 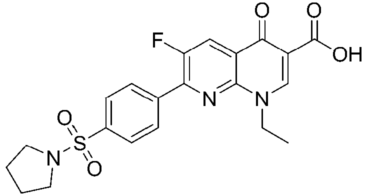 |
| **171** | 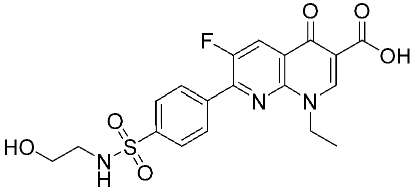 |
| **172** | 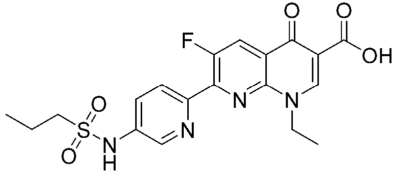 |
| **173** | 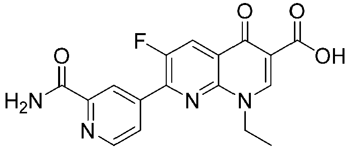 |
or a pharmaceutically acceptable salt, a stereoisomer, a tautomer, a hydrate, or a solvate thereof.

27. A pharmaceutical composition, comprising the compound according to any one of claims 18-26 or a pharmaceutically acceptable salt, a stereoisomer, a tautomer, a hydrate, or a solvate thereof, and a pharmaceutically acceptable excipient.

28. The compound according to any one of claims 18-26 or a pharmaceutically acceptable salt, a stereoisomer, a tautomer, a hydrate, or a solvate thereof, for use as a medicament.

29. The compound according to any one of claims 18-26 or a pharmaceutically acceptable salt, a stereoisomer, a tautomer, a hydrate, or a solvate thereof, for use in the treatment or prevention of a disease or disorder mediated by MRGPRX2, particularly a disease or disorder caused by mast cell degranulation; the disease or disorder is preferably selected from the group consisting of autoimmune diseases, allergic diseases, inflammatory diseases, pruritus, and pain, and more preferably selected from the group consisting of psoriasis, atopic dermatitis, inflammatory bowel disease (IBD), chronic urticaria, allergic rhinitis, asthma, alopecia areata, urticaria, allergic urticaria, rosacea, radiation dermatitis, eczema, pseudoallergy, anaphylactic shock, systemic pruritus, neurogenic pruritus, prurigo nodularis, neurogenic inflammation, ulcerative colitis, Crohn's disease, irritable bowel syndrome, respiratory inflammation, postoperative pain, and vitiligo.

30. A pharmaceutical combination, comprising the compound according to any one of claims 18-26 or a pharmaceutically acceptable salt, a stereoisomer, a tautomer, a hydrate, or a solvate thereof, and one or more other therapeutic agents.

31. A method for antagonizing MRGPRX2 *in vitro,* comprising utilizing an effective amount of the compound according to any one of claims 18-26 or a pharmaceutically acceptable salt, a stereoisomer, a tautomer, a hydrate, or a solvate thereof, in an *in vitro* assay of MRGPRX2.
